# EUROPEAN PATENT APPLICATION

(11) **EP 3 026 051 A1**
(43) Date of publication of application: **01.06.2016**
(21) Application number: 14830099.9
(22) Date of filing: 23.07.2014
(51) Int. Cl.: C07D 471/04, A61K 31/437, A61K 31/4375, A61K 31/519, A61K 31/52, A61K 31/5377, A61K 31/5386, A61P 25/18, A61P 25/28, A61P 43/00, C07D 473/00, C07D 487/04, C07D 519/00

(54) **HETEROCYCLIC COMPOUND**

(30) Priority: 24.07.2013 JP 2013153880; 23.10.2013 JP 2013220193; 20.05.2014 JP 2014104701
(71) Applicant: Takeda Pharmaceutical Company Limited, Osaka-shi, Osaka 541-0045 (JP)
(72) Inventor: KAWASAKI, Masanori, New York, New York 10021 (US); MIKAMI, Satoshi, Fujisawa-shi Kanagawa 251-0012 (JP); NAKAMURA, Shinji, Fujisawa-shi Kanagawa 251-0012 (JP); NEGORO, Nobuyuki, Fujisawa-shi Kanagawa 251-0012 (JP); IKEDA, Shuhei, Fujisawa-shi Kanagawa 251-0012 (JP); NOMURA, Izumi, Fujisawa-shi Kanagawa 251-0012 (JP); ASHIZAWA, Tomoko, Fujisawa-shi Kanagawa 251-0012 (JP); IMAEDA, Toshihiro, New York, New York 10021 (US); SETO, Masaki, Fujisawa-shi Kanagawa 251-0012 (JP); SASAKI, Shigekazu, Fujisawa-shi Kanagawa 251-0012 (JP); MARUI, Shogo, Fujisawa-shi Kanagawa 251-0012 (JP); TANIGUCHI, Takahiko, Fujisawa-shi Kanagawa 251-0012 (JP)
(74) Representative: Held, Stephan
(86) International application number: PCT/JP2014/069494
(87) International publication number: WO 2015/012328

(57) **Abstract**

A compound represented by the formula (I): 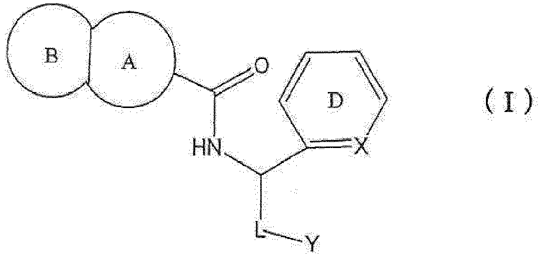 wherein each symbol is as described in the SPECIFICATION, or a salt thereof has a PDE2A inhibitory action, and is useful as a prophylactic or therapeutic drug for schizophrenia, Alzheimer's disease and the like.

## Description

### Technical Field

The present invention relates to a nitrogen-containing heterocyclic compound having a PDE2A selective inhibitory action, and useful as a prophylactic or therapeutic drug for schizophrenia, Alzheimer's disease and the like.

### (Background of the Invention)

Cyclic nucleotide phosphodiesterases (PDEs) are enzymes that regulate the cellular levels of the second messengers, cyclic adenosine monophosphate (cAMP) and cyclic guanosine monophosphate (cGMP), by controlling their rates of degradation. PDEs are a superfamily of enzymes encoded by 21 genes and subdivided into 11 distinct families according to structural and functional properties. The PDE enzymes selectively catalyze the hydrolysis of the 3'-ester bond of cAMP and/or cGMP, forming the inactive 5'-monophosphate. On the basis of substrate specificity, the PDE families can be further classified into three groups: i) the cAMP-PDEs (PDE4, PDE7 and PDE8), ii) the cGMP-PDEs (PDE5, PDE6 and PDE9), and iii) the dual-substrate PDEs (PDE1, PDE2, PDE3, PDE10 and PDE11).

cAMP and cGMP are involved in the regulation of virtually every physiological process such as pro-inflammatory mediator production and action, ion channel function, muscle relaxation, learning and memory formation, differentiation, apoptosis, lipogenesis, glycogenolysis and gluconeogenesis. Especially, in neurons, these second messengers have an important role in the regulation of synaptic transmission as well as in neuronal differentiation and survival (Non-Patent Document 1). Regulation of these processes by cAMP and cGMP are accompanied by activation of protein kinase A (PKA) and protein kinase G (PKG), which in turn phosphorylate a variety of substrates, including transcription factors, ion channels and receptors that regulate a variety of physiological processes. Intracellular cAMP and cGMP concentrations seem to be temporally, spatially, and functionally compartmentalized by regulation of adenylate and guanylate cyclases in response to extracellular signaling and their degradation by PDEs (Non-Patent Document 2). PDEs provide the only means of degrading the cyclic nucleotides cAMP and cGMP in cells, thus PDEs play an essential role in cyclic nucleotide signaling. Thereby, PDEs could be promising targets for various therapeutic drugs.

Phosphodiesterase 2A (PDE2A) is a dual substrate enzyme that hydrolyzes both cAMP and cGMP. It is organized into four domains, N-terminus, GAF-A, GAF-B, and catalytic domains, and functions as a homodimer. PDE2A catalytic activity is allosterically stimulated by cGMP binding. GAF-B domain binds with a high affinity and a high selectivity to cGMP. A conformational change is caused by the cGMP binding in the PDE2A homodimer which causes an increase in the catalytic activity of the enzyme to several-folds or more (Non-Patent Document 3-6). In contrast, there are as yet no known in vivo examples that cAMP stimulates PDE2A catalytic activity. Furthermore, binding affinity of cAMP to the GAF-B is 30-100-fold lower than cGMP (Non-Patent Document 6 and 7). PDE2A activity may become functionally significant under conditions in which cellular cGMP concentrations are elevated, which shows a physiological role for GAF domain-regulation of the enzyme.

PDE2A is expressed in a wide variety of tissues and highly in the brain. The protein was originally purified from heart, liver, adrenal gland, platelets, endothelial cells, and macrophages (Non-Patent Document 8-13). In the brain, the PDE2A mRNA levels are the highest in the caudate lobe, nucleus accumbens, cortex (frontal, parietal and temporal) and the hippocampus, and are at least 10-fold lower expression in other brain regions (Non-Patent Document 14). This suggests that PDE2A may control intraneuronal cAMP and cGMP levels in areas that are important for learning and memory formation.

Inhibition of PDE2A results in increased cAMP and cGMP levels that could improve cognitive function. In both cortical neurons and hippocampal slices, a PDE2A inhibitor potently increased cGMP concentrations in the presence of guanylate cyclase activators and also increased cAMP concentrations in the presence of forskolin (Non-Patent Document 15). The PDE2A inhibitor was also found to potently increase the induction of long-term potentiation (LTP) in hippocampal slices in response to a weak tetanizing stimulus. This effect on LTP in slices suggests that PDE2A inhibition has positive effects on learning and memory in vivo (Non-Patent Document 15). In fact, the same PDE2A inhibitor increased retention on both novel object and social recognition tasks in rats, and improved object memory and object recognition task in 3-, 12-, and 24-month old rats. It also attenuated the extradimensional (ED) shift deficit on extradimensional-intradimensional (ED/ID) cognitive task in subchronic PCP-treated rats (Non-Patent Document 15-17). These results suggest that PDE2A inhibition could facilitate learning and memory processes through cAMP and cGMP-regulated signaling cascades.

Increased cGMP levels by PDE2A inhibition could also influence anxiety and stress-related events. PDE2A inhibitors decreased oxidative stress and induced the expression of NADPH oxidase subunits in oxidative stress inducer-treated mice. It improved anxiety-like behavior in elevated plus maze, open-field, and hole-board tests through the NADPH oxidase pathway (Non-Patent Document 18). In addition, PDE2A inhibitors also produced anxiolytic effects on behavior in non-stressed mice in the elevated plus-maze and hole-board tests (Non-Patent Document 19). PDE2A may be a novel pharmacological target for treatment of not only cognitive deficit, but also anxiety in neuropsychiatric and neurodegenerative disorders.

These unique distribution and functions in the brain indicate that PDE2A represents an important novel target for the treatment of neuropsychiatric and neurodegenerative disorders, in particular schizophrenia and Alzheimer's disease.

Patent document 1 describes that, as a heterocyclic compound, a compound represented by the formula I: 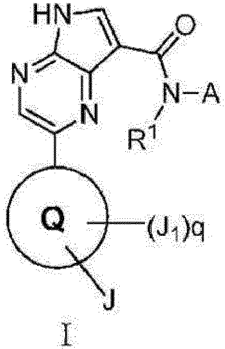 wherein each symbol is as defined in patent document 1, is an ATK kinase inhibitor, and is useful for the treatment of cancer.

Patent document 2 describes that, as a heterocyclic compound, a compound represented by the formula (I): 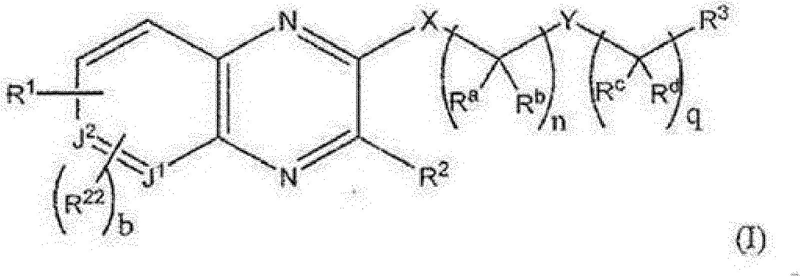 1 wherein each symbol is as defined in patent document 2, is a CRTH2 receptor modulator, and is useful for the treatment of asthma, congestion, allergic rhinitis and COPD.

Patent document 3 describes that, as a heterocyclic compound, a compound represented by the formula I: 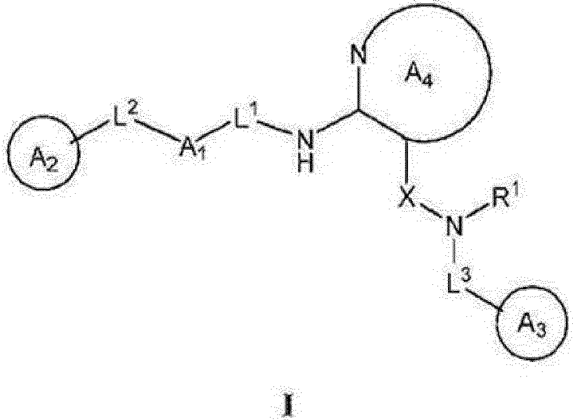 wherein each symbol is as defined in patent document 3, is a protein kinase-1 inhibitor, and is useful for the treatment of cancer.

Patent document 4 describes that, as a heterocyclic compound, a compound represented by the formula: 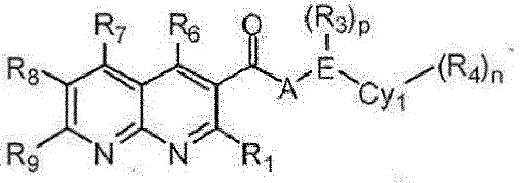 wherein each symbol is as defined in patent document 4, is a PDE3A inhibitor, and is useful for the treatment of respiratory diseases (COPD, asthma etc.) and hypertension.

Patent document 5 describes that, as a heterocyclic compound, a compound represented by the formula (1):

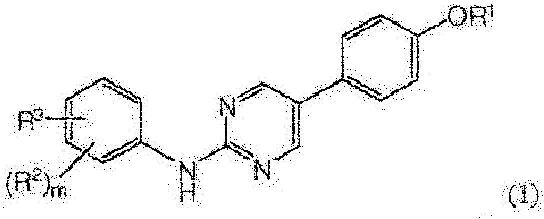

wherein each symbol is as defined in patent document 5, is a protein kinase inhibitor, and is useful for the treatment of cancer and the like.

Patent document 6 describes that, as a heterocyclic compound, a compound represented by the formula (I): 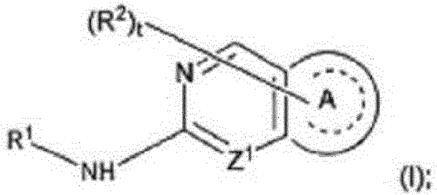 wherein each symbol is as defined in patent document 6, is a JNK modulator, and is useful for the treatment of diabetes and associated diseases.

Patent document 7 describes that, as a heterocyclic compound, a compound represented by the formula I:

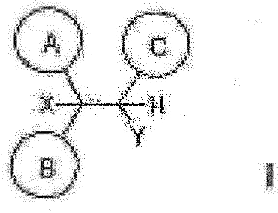

wherein each symbol is as defined in patent document 7, is a potassium channel antagonist.

Patent document 8 describes that, as a heterocyclic compound, a compound represented by the formula XXVIII: 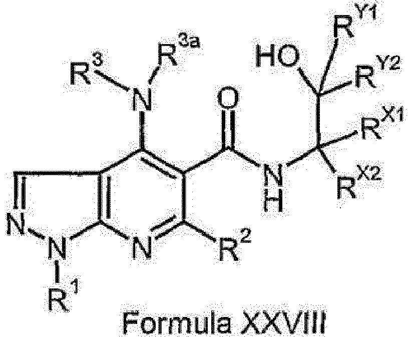 wherein each symbol is as defined in patent document 8, is a synthetic intermediate.

Patent document 9 describes that, as a heterocyclic compound, formula: 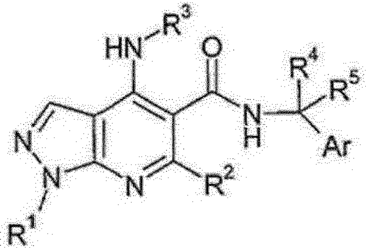 wherein each symbol is as defined in patent document 9, is a PDE4 inhibitor, and is useful for the treatment of inflammation and allergic diseases.

Patent document 10 describes that, as a heterocyclic compound, a compound represented by the formula (1):

**Ar¹(Alk^{a})ᵣL¹Ar²CH(R¹)C(R^{a})(R^{a}')R** **(1)**

wherein each symbol is as defined in patent document 10, is an α4 integrin inhibitor, and is useful for the prophylaxis of immune inflammation and inflammatory diseases.

Patent document 11 describes that, as a heterocyclic compound, a compound represented by the formula (II): 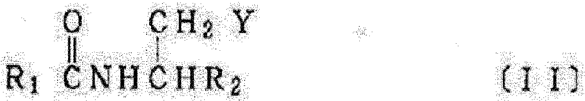 wherein each symbol is as defined in patent document 11, is a synthetic starting compound.

Also, the following compound (CAS registry number: 1422628-80-5) is known.

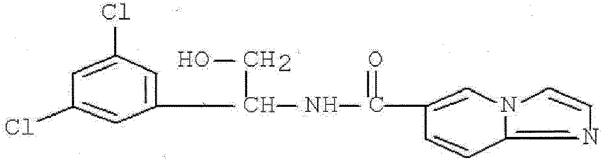

### [document list]

### [patent documents]

patent document 1: WO 2012/178124
patent document 2: WO 2012/087861
patent document 3: WO 2011/044157
patent document 4: WO 2010/097410
patent document 5: WO 2009/026276
patent document 6: WO 2007/125405
patent document 7: WO 2006/015159
patent document 8: WO 2004/056823
patent document 9: WO 2005/058892
patent document 10: WO 00/32575
patent document 11: JP-A-6-145169 [non-patent documents]

non-patent document 1: Nat. Rev. Drug Discov. 2006, vol. 5: 660-670
non-patent document 2: Circ. Res. 2007, vol. 100: 950-966
non-patent document 3: J. Biol. Chem. 1971, vol. 246: 3841-3846
non-patent document 4: J. Biol. Chem. 1973, vol. 248: 1334-1340
non-patent document 5: PNAS 2005, vol. 99: 13260-13265
non-patent document 6: British J. Pharmacol. 2010, vol. 161: 1645-1660
non-patent document 7: J. Biol. Chem. 2004, vol. 279: 37928-37938
non-patent document 8: J. Biol. Chem. 1982, vol. 257: 1973-1979
non-patent document 9: J. Biol. Chem. 1983, vol. 258: 12526-12533
non-patent document 10: Phosphodiesterase Inhibitors, Academic Press: 21-40
non-patent document 11: Rev. Physiol. Biochem. Pharmacol. 1999, vol. 135: 67-104
non-patent document 12: Cell Signal 2004, vol. 16: 365-374
non-patent document 13: J. Histochem. Cytochem. 2009, vol. 57: 933-949
non-patent document 14: Neuropharmacology 2010, vol. 59: 367-374
non-patent document 15: Neuropharmacology 2004, vol. 47: 1081-1092
non-patent document 16: Mol. Neurobiol. 2010, vol. 41: 129-137
non-patent document 17: Neuropharmacology 2012, vol. 62: 1182-1190
non-patent document 18: J. Pharmacol. Exp. Ther. 2008, vol. 326: 369-379
non-patent document 19: J. Pharmacol. Exp. Ther. 2009, vol. 331: 690-699

### SUMMARY OF THE INVENTION

### Problems to be Solved by the Invention

The present invention aims to provide a compound having a PDE2A selective inhibitory action, and useful as a prophylactic or therapeutic drug for schizophrenia, Alzheimer's disease and the like.

### Means of Solving the Problems

The present inventors have conducted intensive studies and found that a compound represented by the formula (I) to be described later unexpectedly has a superior PDE2A selective inhibitory action, and therefore, is useful as a prophylactic or therapeutic drug for schizophrenia, Alzheimer's disease and the like, and completed the present invention based on these findings.

Accordingly, the present invention is as follows.
[1] A compound represented by the formula (I): 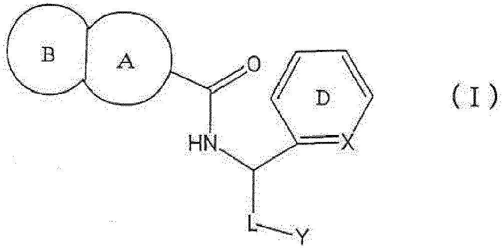 wherein
   ring A is an optionally further substituted 5- or 6-membered nitrogen-containing heterocycle, ring B is an optionally substituted 5- or 6-membered nitrogen-containing heterocycle, and fused ring AB is an optionally further substituted heterocycle having two or more nitrogen atoms as ring-constituting atoms besides carbon atom, and optionally having 1 or 2 hetero atoms selected from an oxygen atom and a sulfur atom, said fused ring AB is aromatic,
   ring D is a benzene ring or a pyridine ring, each of which further has a substituent,
   X is a carbon atom or a nitrogen atom,
   L is a bond or an optionally substituted C₁₋₂ alkylene group, and
   Y is
      (1) a group represented by the formula: 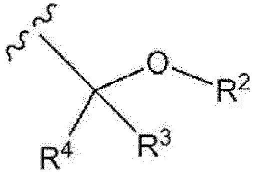 wherein R² and R³ are each independently a hydrogen atom or a substituent, R⁴ is a substituent, or R³ and R⁴ optionally form, together with the adjacent carbon atom, an optionally further substituted ring, or
      (2) a group represented by the formula -CH₂-O-R¹ wherein R¹ is a substituent, (excluding N-[1-(4-fluorophenyl)-2-hydroxy-2,2-di(pyridin-3-yl)ethyl]-pyrazolo[1,5-a]pyridine-2-carboxamide) or a salt thereof (hereinafter to be also referred to as compound (I) or compound (1)).
[2] The compound of the above-mentioned [1], wherein Y is
   (1) a group represented by the formula: 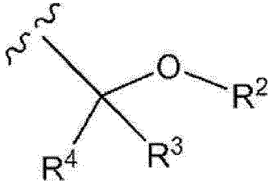
      wherein R² is a hydrogen atom or a C₁₋₆ alkyl-carbonyl group; R³ is a C₁₋₆ alkyl group; and
      R⁴ is a C₁₋₆ alkyl group, or
   (2) a group represented by the formula -CH₂-O-R¹ wherein R¹ is a C₁₋₆ alkyl group,
      or a salt thereof.
[3] The compound of the above-mentioned [1] or [2], wherein ring A is a 5- or 6-membered nitrogen-containing heterocycle optionally substituted by 1 to 3 substituents selected from
   (1) a hydroxy group,
   (2) a C₁₋₆ alkyl group,
   (3) a halogen atom, and
   (4) an amino group
   (excluding a pyrrole ring), or a salt thereof.
[4] The compound of any one of the above-mentioned [1] to [3], wherein ring B is a 5- or 6-membered nitrogen-containing heterocycle optionally substituted by 1 to 3 substituents selected from
   (1) a C₁₋₆ alkyl group optionally substituted by 1 to 3 C₁₋₆ alkoxy groups,
   (2) a halogen atom,
   (3) a hydroxy group,
   (4) a C₂₋₆ alkenyl group,
   (5) a C₃₋₈ cycloalkyl group,
   (6) a di-C₁₋₆ alkyl-amino group,
   (7) a heterocyclic group optionally substituted by 1 to 3 substituents selected from
      (i) a C₁₋₆ alkoxy group,
      (ii) a halogen atom,
      (iii) a C₁₋₆ alkyl group optionally substituted by 1 to 3 halogen atoms,
      (iv) a C₁₋₆ alkoxy group,
      (v) an oxo group, and
      (vi) a cyano group,
   (8) an oxo group,
   (9) a phenyl group optionally substituted by a cyano group, and
   (10) a C₁₋₆ alkoxy group
   (excluding a pyrazine ring),
   or a salt thereof.
[5] The compound of any one of the above-mentioned [1] to [4], wherein fused ring AB is
   (I)
      a 1,5-naphthyridine ring,
      a 1,6-naphthyridine ring,
      a pyrazolo[1,5-a]pyrimidine ring,
      a triazolo[1,5-a]pyrimidine ring,
      an imidazo[4,5-b]pyridine ring,
      a purine ring, or
      a pyrazolo[4,3-c]pyridine ring, each of which is optionally substituted by 1 to 3 substituents selected from
      (1) a hydroxy group,
      (2) a halogen atom,
      (3) a C₁₋₆ alkyl group,
      (4) a C₂₋₆ alkenyl group,
      (5) a C₃₋₈ cycloalkyl group,
      (6) an amino group,
      (7) a di-C₁₋₆ alkyl-amino group,
      (8) a heterocyclic group optionally substituted by 1 to 3 substituents selected from
         (i) a C₁₋₆ alkoxy group,
         (ii) a halogen atom,
         (iii) a C₁₋₆ alkyl group optionally substituted by 1 to 3 halogen atoms,
         (iv) a C₁₋₆ alkoxy group,
         (v) an oxo group, and
         (iv) a cyano group,
      (9) a C₆₋₁₄ aryl group optionally substituted by a cyano group, and
      (10) a C₁₋₆ alkoxy group,
         or
   (II) a 7-oxo-1,7-dihydroa pyrazolo[1,5-a]pyrimidine ring optionally substituted by 1 to 3 substituents selected from
      (1) a C₁₋₆ alkyl group optionally substituted by one C₁₋₆ alkoxy group,
      (2) a C₃₋₈ cycloalkyl group,
      (3) a heterocyclic group, and
      (4) a halogen atom,
   or a salt thereof.
[6] The compound of any one of the above-mentioned [1] to [5], wherein ring D is a benzene ring substituted by 1 to 3 substituents selected from
   (1) a C₁₋₆ alkoxy group optionally substituted by 1 to 5 halogen atoms,
   (2) a halogen atom,
   (3) a heterocyclic group, and
   (4) a C₁₋₆ alkyl group optionally substituted by 1 to 3 halogen atoms,
   or a salt thereof.
[7] The compound of any one of the above-mentioned [1] to [6], wherein ring A is
   a 5- or 6-membered nitrogen-containing heterocycle optionally substituted by 1 to 3 substituents selected from
   (1) a hydroxy group,
   (2) a C₁₋₆ alkyl group,
   (3) a halogen atom, and
   (4) an amino group
      (excluding a pyrrole ring);
      ring B is a 5- or 6-membered nitrogen-containing heterocycle optionally substituted by 1 to 3 substituents selected from
      (1) a C₁₋₆ alkyl group optionally substituted by 1 to 3 C₁₋₆ alkoxy groups,
      (2) a halogen atom,
      (3) a hydroxy group,
      (4) a C₂₋₆ alkenyl group,
      (5) a C₃₋₈ cycloalkyl group,
      (6) a di-C₁₋₆ alkyl-amino group,
      (7) a heterocyclic group optionally substituted by 1 to 3 substituents selected from
         (i) a C₁₋₆ alkoxy group,
         (ii) a halogen atom,
         (iii) a C₁₋₆ alkyl group optionally substituted by 1 to 3 halogen atoms,
         (iv) a C₁₋₆ alkoxy group,
         (v) an oxo group, and
         (iv) a cyano group,
      (8) an oxo group,
      (9) a phenyl group optionally substituted by a cyano group, and
      (10) a C₁₋₆ alkoxy group
         (excluding a pyrazine ring);
         fused ring AB is
         (I)
            a 1,5-naphthyridine ring,
            a 1,6-naphthyridine ring,
            a pyrazolo[1,5-a]pyrimidine ring,
            a triazolo[1,5-a]pyrimidine ring,
            an imidazo[4,5-b]pyridine ring,
            a purine ring, or
            a pyrazolo[4,3-c]pyridine ring, each of which is optionally substituted by 1 to 3 substituents selected from
               (1) a hydroxy group,
               (2) a halogen atom,
               (3) a C₁₋₆ alkyl group,
               (4) a C₂₋₆ alkenyl group,
               (5) a C₃₋₈ cycloalkyl group,
               (6) an amino group,
               (7) a di-C₁₋₆ alkyl-amino group,
               (8) a heterocyclic group optionally substituted by 1 to 3 substituents selected from
                  (i) a C₁₋₆ alkoxy group,
                  (ii) a halogen atom,
                  (iii) a C₁₋₆ alkyl group optionally substituted by 1 to 3 halogen atoms,
                  (iv) a C₁₋₆ alkoxy group,
                  (v) an oxo group, and
                  (iv) a cyano group,
               (9) a C₆₋₁₄ aryl group optionally substituted by a cyano group, and
               (10) a C₁₋₆ alkoxy group,
                  or
         (II) a 7-oxo-1,7-dihydroa pyrazolo[1,5-a]pyrimidine ring optionally substituted by 1 to 3 substituents selected from
            (1) a C₁₋₆ alkyl group optionally substituted by one C₁₋₆ alkoxy group,
            (2) a C₃₋₈ cycloalkyl group,
            (3) a heterocyclic group, and
            (4) a halogen atom;
               ring D is a benzene ring substituted by 1 to 3 substituents selected from
               (1) a C₁₋₆ alkoxy group optionally substituted by 1 to 5 halogen atoms,
               (2) a halogen atom,
               (3) a heterocyclic group, and
               (4) a C₁₋₆ alkyl group optionally substituted by 1 to 3 halogen atoms;
               X is a carbon atom;
               L is a bond or methylene; and
               Y is
               (1) a group represented by the formula: 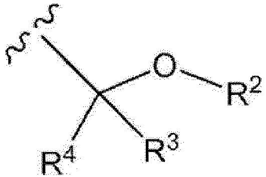
                  wherein is a hydrogen atom or a C₁₋₆ alkyl-carbonyl group; R³ is a C₁₋₆ alkyl group; and
                  R⁴ is a C₁₋₆ alkyl group, or
               (2) a group represented by the formula -CH₂-O-R¹ wherein R¹ is a C₁₋₆ alkyl group,
               or a salt thereof.
[8] The compound of any one of the above-mentioned [1] to [7], wherein ring A is a pyrazole ring, a pyridine ring, a pyrimidine ring, a dihydropyrazole ring, or a dihydropyridine ring, each of which is optionally substituted by 1 to 3 substituents selected from
   (1) a hydroxy group,
   (2) a C₁₋₆ alkyl group,
   (3) a halogen atom, and
   (4) an amino group;
      ring B is
      a pyridine ring,
      a pyrimidine ring,
      a pyrazole ring,
      a triazole ring, dihydropyrimidine ring, or
      an imidazole ring, each of which is optionally substituted by 1 to 3 substituents selected from
      (1) a C₁₋₆ alkyl group optionally substituted by 1 to 3 C₁₋₆ alkoxy groups,
      (2) a halogen atom,
      (3) a hydroxy group,
      (4) a C₂₋₆ alkenyl group,
      (5) a C₃₋₈ cycloalkyl group,
      (6) a di-C₁₋₆ alkyl-amino group,
      (7) an azetidinyl group,
         a pyrrolidinyl group,
         a piperidyl group,
         a morpholinyl group,
         a 3-oxa-8-azabicyclo[3.2.1]octyl group,
         a 3-oxa-6-azabicyclo[3.1.1]heptyl group,
         a 6-oxa-3-azabicyclo[3.1.1]heptyl group,
         a pyrazolyl group,
         a triazolyl group,
         a pyridyl group,
         a pyridazinyl group,
         a thiazolyl group, an imidazolyl group,
         a tetrahydropyranyl group,
         a tetrazolyl group,
         a pyrazinyl group,
         an isoxazolyl group,
         a dihydropyridyl group, or
         a pyrimidinyl group, each of which is optionally substituted by 1 to 3 substituents selected from
         (i) a C₁₋₆ alkoxy group,
         (ii) a halogen atom,
         (iii) a C₁₋₆ alkyl group optionally substituted by 1 to 3 halogen atoms,
         (iv) a C₁₋₆ alkoxy group,
         (v) an oxo group, and
         (vi) a cyano group,
      (8) an oxo group,
      (9) a phenyl group optionally substituted by a cyano group, and
      (10) a C₁₋₆ alkoxy group;
         fused ring AB is
         (I)
            a 1,5-naphthyridine ring,
            a 1,6-naphthyridine ring,
            a pyrazolo[1,5-a]pyrimidine ring,
            a triazolo[1,5-a]pyrimidine ring,
            an imidazo[4,5-b]pyridine ring,
            a purine ring, or
            a pyrazolo[4,3-c]pyridine ring, each of which is optionally substituted by 1 to 3 substituents selected from
            (1) a hydroxy group,
            (2) a halogen atom,
            (3) a C₁₋₆ alkyl group,
            (4) a C₂₋₆ alkenyl group,
            (5) a C₃₋₈ cycloalkyl group,
            (6) an amino group,
            (7) a di-C₁₋₆ alkyl-amino group,
            (8) an azetidinyl group,
               a pyrrolidinyl group,
               a piperidyl group,
               a morpholinyl group,
               a 3-oxa-8-azabicyclo[3.2.1]octyl group,
               a 3-oxa-6-azabicyclo[3.1.1]heptyl group,
               a 6-oxa-3-azabicyclo[3.1.1]heptyl group,
               a pyrazolyl group,
               an imidazolyl group,
               a triazolyl group,
               a pyridyl group,
               a dihydropyridyl group,
               a pyridazinyl group,
               a pyrimidinyl group,
               a thiazolyl group,
               a tetrahydropyranyl group,
               a tetrazolyl group,
               a pyrazinyl group, or
               an isoxazolyl group, each of which is optionally substituted by 1 to 3 substituents selected from
               (i) a C₁₋₆ alkoxy group,
               (ii) a halogen atom,
               (iii) a C₁₋₆ alkyl group optionally substituted by 1 to 3 halogen atoms,
               (iv) a C₁₋₆ alkoxy group, and
               (v) an oxo group,
            (9) a phenyl group optionally substituted by a cyano group, and
            (10) a C₁₋₆ alkoxy group,
               or
         (II) a 7-oxo-1,7-dihydroa pyrazolo[1,5-a]pyrimidine ring optionally substituted by 1 to 3 substituents selected from
            (1) a C₁₋₆ alkyl group optionally substituted by one C₁₋₆ alkoxy group,
            (2) a C₃₋₈ cycloalkyl group,
            (3) a pyridyl group, and
            (4) a halogen atom;
               ring D is a benzene ring substituted by 1 to 3 substituents selected from
               (1) a C₁₋₆ alkoxy group optionally substituted by 1 to 3 halogen atoms,
               (2) a halogen atom,
               (3) a pyrazolyl group, and
               (4) a C₁₋₆ alkyl group optionally substituted by 1 to 3 halogen atoms;
                  X is a carbon atom;
                  L is a bond or methylene; and
                  Y is
                  (1) a group represented by the formula: 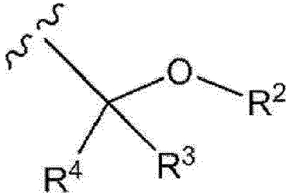
                     wherein R² is a hydrogen atom or a C₁₋₆ alkyl-carbonyl group; R³ is a C₁₋₆ alkyl group; and
                     R⁴ is a C₁₋₆ alkyl group, or
                  (2) a group represented by the formula -CH₂-O-R¹ wherein R¹ is a C₁₋₆ alkyl group,
                     or a salt thereof.
[9] The compound of any one of the above-mentioned [1] to [8], wherein ring A is a pyrazole ring, a pyridine ring, or a dihydropyridine ring each of which is optionally substituted by 1 to 3 substituents selected from
   (1) a hydroxy group, and
   (2) a C₁₋₆ alkyl group;
      ring B is
      a pyridine ring, or
      a pyrimidine ring, each of which is optionally substituted by 1 to 3 substituents selected from
      (1) a C₁₋₆ alkyl group,
      (2) a C₃₋₈ cycloalkyl group,
      (3) a di-C₁₋₆ alkyl-amino group,
      (4) an azetidinyl group,
         a pyrrolidinyl group,
         a morpholinyl group,
         a pyrazolyl group,
         a triazolyl group,
         a pyridyl group, or
         an imidazolyl group, each of which is optionally substituted by 1 to 3 substituents selected from
         (i) a C₁₋₆ alkyl group optionally substituted by 1 to 3 halogen atoms, and
         (ii) an oxo group,
      (5) an oxo group,
      (6) a phenyl group optionally substituted by a cyano group, and
      (7) a C₁₋₆ alkoxy group;
         fused ring AB is
         (I)
            a 1,5-naphthyridine ring,
            a 1,6-naphthyridine ring, or
            a pyrazolo[1,5-a]pyrimidine ring, each of which is optionally substituted by 1 to 3 substituents selected from
            (1) a hydroxy group,
            (2) a C₁₋₆ alkyl group,
            (3) a C₃₋₈ cycloalkyl group,
            (4) a di-C₁₋₆ alkyl-amino group,
            (5)
               an azetidinyl group,
               a pyrrolidinyl group,
               a morpholinyl group,
               a pyrazolyl group,
               an imidazolyl group,
               a triazolyl group, or
               a pyridyl group, each of which is optionally substituted by 1 to 3 substituents selected from
               (i) a C₁₋₆ alkyl group optionally substituted by 1 to 3 halogen atoms, and
               (ii) an oxo group,
            (9) a phenyl group optionally substituted by a cyano group, and
            (10) a C₁₋₆ alkoxy group, or
         (II) a 7-oxo-1,7-dihydroa pyrazolo[1,5-a]pyrimidine ring optionally substituted by 1 to 3 substituents selected from
            (1) a C₁₋₆ alkyl group,
            (2) a C₃₋₈ cycloalkyl group, and
            (3) a pyridyl group;
               ring D is a benzene ring substituted by 1 to 3 substituents selected from
               (1) a C₁₋₆ alkoxy group optionally substituted by 1 to 3 halogen atoms, and
               (2) a halogen atom;
                  X is a carbon atom;
                  L is a bond or methylene; and
                  Y is
                  (1) a group represented by the formula: 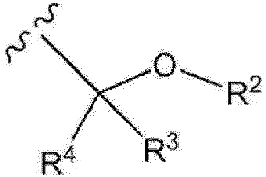
                     wherein R² is a hydrogen atom;
                     R³ is a C₁₋₆ alkyl group; and
                     R⁴ is a C₁₋₆ alkyl group, or
                  (2) a group represented by the formula -CH₂-O-R¹ wherein R¹ is a C₁₋₆ alkyl group,
                     or a salt thereof.
[10] The compound of any one of the above-mentioned [1] to [9], wherein ring A is a pyrazole ring;
   ring B is a pyrimidine ring substituted by 1 to 3 substituents selected from
   (1) a C₁₋₆ alkyl group,
   (2) a triazolyl group optionally substituted by 1 to 3 C₁₋₆ alkyl groups, and
   (3) a pyridyl group optionally substituted by 1 to 3 C₁₋₆ alkyl groups;
      fused ring AB is a pyrazolo[1,5-a]pyrimidine ring optionally substituted by 1 to 3 substituents selected from
      (1) a C₁₋₆ alkyl group,
      (2) a triazolyl group optionally substituted by 1 to 3 C₁₋₆ alkyl groups, and
      (3) a pyridyl group optionally substituted by 1 to 3 C₁₋₆ alkyl groups;
         ring D is a benzene ring substituted by a C₁₋₆ alkoxy group optionally substituted by 1 to 3 halogen atoms;
         X is a carbon atom;
         L is a bond; and
         Y is a group represented by the formula: 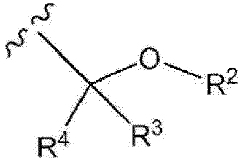
      wherein is a hydrogen atom;
         R³ is a C₁₋₆ alkyl group; and
         R⁴ is a C₁₋₆ alkyl group, or a salt thereof,
         or a salt thereof.
[11] N-((1S)-2-Hydroxy-2-methyl-1-(4-(trifluoromethoxy)phenyl)propyl)-5-(6-methylpyridin-3-yl)pyrazolo[1,5-a]pyrimidine-3-carboxamide, or a salt thereof.
[12] N-((1S)-2-Hydroxy-2-methyl-1-(4-(trifluoromethoxy)phenyl)propyl)-6-methyl-5-(4-methyl-1H-1,2,3-triazol-1-yl)pyrazolo[1,5-a]pyrimidine-3-carboxamide, or a salt thereof.
[13] N-((1S)-2-Hydroxy-2-methyl-1-(4-(trifluoromethoxy)phenyl)propyl)-6-methyl-5-(3-methyl-1H-1,2,4-triazol-1-yl)pyrazolo[1,5-a]pyrimidine-3-carboxamide, or a salt thereof.
[14] A medicament comprising the compound of any one of the above-mentioned [1] to [13], or a salt thereof.
[15] The medicament of the above-mentioned [14], which is a phosphodiesterase 2A inhibitor.
[16] The medicament of the above-mentioned [14], which is a prophylactic or therapeutic drug for schizophrenia.
[17] The compound of any one of the above-mentioned [1] to [13] for use in the prophylaxis or treatment of schizophrenia, or a salt thereof.
[18] A method of inhibiting of phosphodiesterase 2A in a mammal, comprising administering an effective amount of the compound of any one of the above-mentioned [1] to [13] or a salt thereof to the mammal.
[19] A method for the prophylaxis or treatment of schizophrenia in a mammal, comprising administering an effective amount of the compound of any one of the above-mentioned [1] to [13] or a salt thereof to the mammal.
[20] Use of the compound of any one of the above-mentioned [1] to [13] or a salt thereof in the production of a prophylactic or therapeutic drug for schizophrenia.

### Effect of the Invention

According to the present invention, a compound having a PDE2A selective inhibitory action, and useful as a prophylactic or therapeutic drug for schizophrenia, Alzheimer's disease and the like can be provided.

### BRIEF DESCRIPTION OF THE DRAWINGS

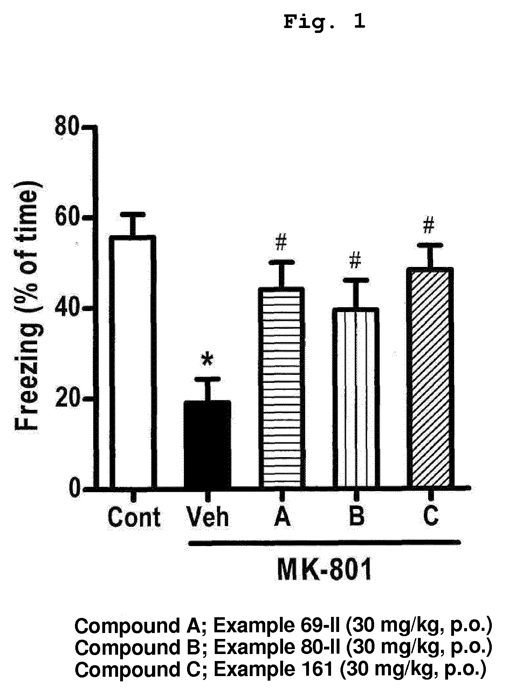

Fig. 1 is a graph showing an improving effect of a test compound on an MK-801 induced disorder in a contextual fear condition test. compound A is a compound obtained in Example 69-II (30 mg/kg, p.o.), compound B is a compound obtained in Example 80-II (30 mg/kg, p.o.), and compound C is a compound obtained in Example 161 (30 mg/kg, p.o.).

### (DETAILED DESCRIPTION OF THE INVENTION)

The present invention is explained in detail in the following.

The definition of each substituent used in the present specification is described in detail in the following. Unless otherwise specified, each substituent has the following definition.

In the present specification, examples of the "halogen atom" include fluorine, chlorine, bromine and iodine.

In the present specification, examples of the "C₁₋₆ alkyl group" include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl and 2-ethylbutyl.

In the present specification, examples of the "optionally halogenated C₁₋₆ alkyl group" include a C₁₋₆ alkyl group optionally having 1 to 7, preferably 1 to 5, halogen atoms. Specific examples include methyl, chloromethyl, difluoromethyl, trichloromethyl, trifluoromethyl, ethyl, 2-bromoethyl, 2,2,2-trifluoroethyl, tetrafluoroethyl, pentafluoroethyl, propyl, 2,2-difluoropropyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl and 6,6,6-trifluorohexyl.

In the present specification, examples of the "C₂₋₆ alkenyl group" include ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl and 5-hexenyl.

In the present specification, examples of the "C₂₋₆ alkynyl group" include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl and 4-methyl-2-pentynyl.

In the present specification, examples of the "C₃₋₁₀ cycloalkyl group" include cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl and adamantyl.

In the present specification, examples of the "optionally halogenated C₃₋₁₀ cycloalkyl group" include a C₃₋₁₀ cycloalkyl group optionally having 1 to 7, preferably 1 to 5, halogen atoms. Specific examples include cyclopropyl, 2,2-difluorocyclopropyl, 2,3-difluorocyclopropyl, cyclobutyl, difluorocyclobutyl, cyclopentyl, cyclohexyl, cycloheptyl and cyclooctyl.

In the present specification, examples of the "C₃₋₁₀ cycloalkenyl group" include cyclopropenyl, cyclobutenyl, cyclopentenyl, cyclohexenyl, cycloheptenyl and cyclooctenyl.

In the present specification, examples of the "C₆₋₁₄ aryl group" include phenyl, 1-naphthyl, 2-naphthyl, 1-anthryl, 2-anthryl and 9-anthryl.

In the present specification, examples of the "C₇₋₁₆ aralkyl group" include benzyl, phenethyl, naphthylmethyl and phenylpropyl.

In the present specification, examples of the "C₁₋₆ alkoxy group" include methoxy, ethoxy, propoxy, isopropoxy, butoxy, isobutoxy, sec-butoxy, tert-butoxy, pentyloxy and hexyloxy.

In the present specification, examples of the "optionally halogenated C₁₋₆ alkoxy group" include a C₁₋₆ alkoxy group optionally having 1 to 7, preferably 1 to 5, halogen atoms. Specific examples thereof include methoxy, difluoromethoxy, trifluoromethoxy, ethoxy, 2,2,2-trifluoroethoxy, propoxy, isopropoxy, butoxy, 4,4,4-trifluorobutoxy, isobutoxy, sec-butoxy, pentyloxy and hexyloxy.

In the present specification, examples of the "C₃₋₁₀ cycloalkyloxy group" include cyclopropyloxy, cyclobutyloxy, cyclopentyloxy, cyclohexyloxy, cycloheptyloxy and cyclooctyloxy.

In the present specification, examples of the "C₁₋₆ alkylthio group" include methylthio, ethylthio, propylthio, isopropylthio, butylthio, sec-butylthio, tert-butylthio, pentylthio and hexylthio.

In the present specification, examples of the "optionally halogenated C₁₋₆ alkylthio group" include a C₁₋₆ alkylthio group optionally having 1 to 7, preferably 1 to 5, halogen atoms. Specific examples include methylthio, difluoromethylthio, trifluoromethylthio, ethylthio, propylthio, isopropylthio, butylthio, 4,4,4-trifluorobutylthio, pentylthio and hexylthio.

In the present specification, examples of the "C₁₋₆ alkyl-carbonyl group" include acetyl, propanoyl, butanoyl, 2-methylpropanoyl, pentanoyl, 3-methylbutanoyl, 2-methylbutanoyl, 2,2-dimethylpropanoyl, hexanoyl and heptanoyl.

In the present specification, examples of the "optionally halogenated C₁₋₆ alkyl-carbonyl group" include a C₁₋₆ alkyl-carbonyl group optionally having 1 to 7, preferably 1 to 5, halogen atoms. Specific examples include acetyl, chloroacetyl, trifluoroacetyl, trichloroacetyl, propanoyl, butanoyl, pentanoyl and hexanoyl.

In the present specification, examples of the "C₁₋₆ alkoxy-carbonyl group" include methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl and hexyloxycarbonyl.

In the present specification, examples of the "C₆₋₁₄ aryl-carbonyl group" include benzoyl, 1-naphthoyl and 2-naphthoyl.

In the present specification, examples of the "C₇₋₁₆ aralkyl-carbonyl group" include phenylacetyl and phenylpropionyl.

In the present specification, examples of the "5- to 14-membered aromatic heterocyclyl-carbonyl group" include nicotinoyl, isonicotinoyl, thenoyl and furoyl.

In the present specification, examples of the "3- to 14-membered non-aromatic heterocyclyl-carbonyl group" include morpholinylcarbonyl, piperidinylcarbonyl and pyrrolidinylcarbonyl.

In the present specification, examples of the "mono- or di-C₁₋₆ alkyl-carbamoyl group" include methylcarbamoyl, ethylcarbamoyl, dimethylcarbamoyl, diethylcarbamoyl and N-ethyl-N-methylcarbamoyl.

In the present specification, examples of the "mono- or di-C₇₋₁₆ aralkyl-carbamoyl group" include benzylcarbamoyl and phenethylcarbamoyl.

In the present specification, examples of the "C₁₋₆ alkylsulfonyl group" include methylsulfonyl, ethylsulfonyl, propylsulfonyl, isopropylsulfonyl, butylsulfonyl, sec-butylsulfonyl and tert-butylsulfonyl.

In the present specification, examples of the "optionally halogenated C₁₋₆ alkylsulfonyl group" include a C₁₋₆ alkylsulfonyl group optionally having 1 to 7, preferably 1 to 5, halogen atoms. Specific examples include methylsulfonyl, difluoromethylsulfonyl, trifluoromethylsulfonyl, ethylsulfonyl, propylsulfonyl, isopropylsulfonyl, butylsulfonyl, 4,4,4-trifluorobutylsulfonyl, pentylsulfonyl and hexylsulfonyl.

In the present specification, examples of the "C₆₋₁₄ arylsulfonyl group" include phenylsulfonyl, 1-naphthylsulfonyl and 2-naphthylsulfonyl.

In the present specification, examples of the "substituent" include a halogen atom, a cyano group, a nitro group, an optionally substituted hydrocarbon group, an optionally substituted heterocyclic group, an acyl group, an optionally substituted amino group, an optionally substituted carbamoyl group, an optionally substituted thiocarbamoyl group, an optionally substituted sulfamoyl group, an optionally substituted hydroxy group, an optionally substituted sulfanyl (SH) group and an optionally substituted silyl group.

In the present specification, examples of the "hydrocarbon group" (containing the "hydrocarbon group" in the "optionally substituted hydrocarbon group") include a C₁₋₆ alkyl group, a C₂₋₆ alkenyl group, a C₂₋₆ alkynyl group, a C₃₋₁₀ cycloalkyl group, a C₃-₁₀ cycloalkenyl group, a C₆₋₁₄ aryl group and a C₇₋₁₆ aralkyl group.

In the present specification, examples of the "optionally substituted hydrocarbon group" include a hydrocarbon group optionally having substituent(s) selected from the following substituent group A.

### [Substituent group A]

(1) a halogen atom,
(2) a nitro group,
(3) a cyano group,
(4) an oxo group,
(5) a hydroxy group,
(6) an optionally halogenated C₁₋₆ alkoxy group,
(7) a C₆₋₁₄ aryloxy group (e.g., phenoxy, naphthoxy),
(8) a C₇₋₁₆ aralkyloxy group (e.g., benzyloxy),
(9) a 5- to 14-membered aromatic heterocycleoxy group (e.g., pyridyloxy),
(10) a 3- to 14-membered non-aromatic heterocycleoxy group (e.g., morpholinyloxy, piperidinyloxy),
(11) a C₁₋₆ alkyl-carbonyloxy group (e.g., acetoxy, propanoyloxy),
(12) a C₆₋₁₄ aryl-carbonyloxy group (e.g., benzoyloxy, 1-naphthoyloxy, 2-naphthoyloxy),
(13) a C₁₋₆ alkoxy-carbonyloxy group (e.g., methoxycarbonyloxy, ethoxycarbonyloxy, propoxycarbonyloxy, butoxycarbonyloxy),
(14) a mono- or di- C₁₋₆ alkyl-carbamoyloxy group (e.g., methylcarbamoyloxy, ethylcarbamoyloxy, dimethylcarbamoyloxy, diethylcarbamoyloxy),
(15) a C₆₋₁₄ aryl-carbamoyloxy group (e.g., phenylcarbamoyloxy, naphthylcarbamoyloxy),
(16) a 5- to 14-membered aromatic heterocyclecarbonyloxy group (e.g., nicotinoyloxy),
(17) a 3- to 14-membered non-aromatic heterocyclecarbonyloxy group (e.g., morpholinylcarbonyloxy, piperidinylcarbonyloxy),
(18) an optionally halogenated C₁₋₆ alkylsulfonyloxy group (e.g., methylsulfonyloxy, trifluoromethylsulfonyloxy),
(19) a C₆₋₁₄ arylsulfonyloxy group optionally substituted by a C₁₋₆ alkyl group (e.g., phenylsulfonyloxy, toluenesulfonyloxy),
(20) an optionally halogenated C₁₋₆ alkylthio group,
(21) a 5- to 14-membered aromatic heterocyclic group,
(22) a 3- to 14-membered nonaromatic heterocyclic group,
(23) a formyl group,
(24) a carboxy group,
(25) an optionally halogenated C₁₋₆ alkyl-carbonyl group,
(26) a C₆₋₁₄ aryl-carbonyl group,
(27) a 5- to 14-membered aromatic heterocyclyl-carbonyl group,
(28) a 3- to 14-membered non-aromatic heterocyclyl-carbonyl group,
(29) a C₁₋₆ alkoxy-carbonyl group,
(30) a C₆₋₁₄ aryloxy-carbonyl group (e.g., phenyloxycarbonyl, 1-naphthyloxycarbonyl, 2-naphthyloxycarbonyl),
(31) a C₇₋₁₆ aralkyloxy-carbonyl group (e.g., benzyloxycarbonyl, phenethyloxycarbonyl),
(32) a carbamoyl group,
(33) a thiocarbamoyl group,
(34) a mono- or di-C₁₋₆ alkyl-carbamoyl group,
(35) a C₆₋₁₄ aryl-carbamoyl group (e.g., phenylcarbamoyl),
(36) a 5- to 14-membered aromatic heterocyclyl-carbamoyl group (e.g., pyridylcarbamoyl, thienylcarbamoyl),
(37) a 3- to 14-membered non-aromatic heterocyclyl-carbamoyl group (e.g., morpholinylcarbamoyl, piperidinylcarbamoyl),
(38) an optionally halogenated C₁₋₆ alkylsulfonyl group,
(39) a C₆₋₁₄ arylsulfonyl group,
(40) a 5- to 14-membered aromatic heterocyclyl-sulfonyl group (e.g., pyridylsulfonyl, thienylsulfonyl),
(41) an optionally halogenated C₁₋₆ alkylsulfinyl group,
(42) a C₆₋₁₄ arylsulfinyl group (e.g., phenylsulfinyl, 1-naphthylsulfinyl, 2-naphthylsulfinyl),
(43) a 5- to 14-membered aromatic heterocyclyl-sulfinyl group (e.g., pyridylsulfinyl, thienylsulfinyl),
(44) an amino group,
(45) a mono- or di- C₁₋₆ alkylamino group (e.g., methylamino, ethylamino, propylamino, isopropylamino, butylamino, dimethylamino, diethylamino, dipropylamino, dibutylamino, N-ethyl-N-methylamino),
(46) a mono- or di- C₆₋₁₄ arylamino group (e.g., phenylamino),
(47) a 5- to 14-membered aromatic heterocyclyl-amino group (e.g., pyridylamino),
(48) a C₇₋₁₆ aralkylamino group (e.g., benzylamino),
(49) a formylamino group,
(50) a C₁₋₆ alkyl-carbonylamino group (e.g., acetylamino, propanoylamino, butanoylamino),
(51) a (C₁₋₆ alkyl) (C₁₋₆ alkyl-carbonyl) amino group (e.g., N-acetyl-N-methylamino),
(52) a C₆₋₁₄ aryl-carbonylamino group (e.g., phenylcarbonylamino, naphthylcarbonylamino),
(53) a C₁₋₆ alkoxy-carbonylamino group (e.g., methoxycarbonylamino, ethoxycarbonylamino, propoxycarbonylamino, butoxycarbonylamino, tert-butoxycarbonylamino),
(54) a C₇₋₁₆ aralkyloxy-carbonylamino group (e.g., benzyloxycarbonylamino),
(55) a C₁₋₆ alkylsulfonylamino group (e.g., methylsulfonylamino, ethylsulfonylamino),
(56) a C₆₋₁₄ arylsulfonylamino group optionally substituted by a C₁₋₆ alkyl group (e.g., phenylsulfonylamino, toluenesulfonylamino),
(57) an optionally halogenated C₁₋₆ alkyl group,
(58) a C₂₋₆ alkenyl group,
(59) a C₂₋₆ alkynyl group,
(60) a C₃₋₁₀ cycloalkyl group,
(61) a C₃₋₁₀ cycloalkenyl group, and
(62) a C₆₋₁₄ aryl group.

The number of the above-mentioned substituents of the "optionally substituted hydrocarbon group" is, for example, 1 to 5, preferably 1 to 3. When the number of the substituents is two or more, the respective substituents may be the same or different.

In the present specification, examples of the "heterocyclic group" (containing the "heterocyclic group" of the "optionally substituted heterocyclic group") include (i) an aromatic heterocyclic group, (ii) a nonaromatic heterocyclic group and (iii) a 7- to 10-membered crosslinked heterocyclic group having, as ring-constituting atom besides carbon atom, 1 to 4 hetero atoms selected from a nitrogen atom, a sulfur atom and an oxygen atom.

In the present specification, examples of the "aromatic heterocyclic group" (containing the "5- to 14-membered aromatic heterocyclic group") include a 5- to 14-membered (preferably 5-to 10-membered) aromatic heterocyclic group having, as ring-constituting atom besides carbon atom, 1 to 4 hetero atoms selected from a nitrogen atom, a sulfur atom and an oxygen atom.

Preferable examples of the "aromatic heterocyclic group" include 5- or 6-membered monocyclic aromatic heterocyclic groups such as thienyl, furyl, pyrrolyl, imidazolyl, pyrazolyl, thiazolyl, isothiazolyl, oxazolyl, isoxazolyl, pyridyl, pyrazinyl, pyrimidinyl, pyridazinyl, 1,2,4-oxadiazolyl, 1,3,4-oxadiazolyl, 1,2,4-thiadiazolyl, 1,3,4-thiadiazolyl, triazolyl, tetrazolyl, triazinyl and the like; 8- to 14-membered fused polycyclic (preferably bicyclic or tricyclic) aromatic heterocyclic groups such as benzothiophenyl, benzofuranyl, benzimidazolyl, benzoxazolyl, benzoisooxazolyl, benzothiazolyl, benzoisothiazolyl, benzotriazolyl, imidazopyridinyl, thienopyridinyl, furopyridinyl, pyrrolopyridinyl, pyrazolopyridinyl, oxazolopyridinyl, thiazolopyridinyl, imidazopyrazinyl, imidazopyrimidinyl, thienopyrimidinyl, furopyrimidinyl, pyrrolopyrimidinyl, pyrazolopyrimidinyl, oxazolopyrimidinyl, thiazolopyrimidinyl, pyrazolotriazinyl, naphtho[2,3-b]thienyl, phenoxathiinyl, indolyl, isoindolyl, 1H-indazolyl, purinyl, isoquinolyl, quinolyl, phthalazinyl, naphthyridinyl, quinoxalinyl, quinazolinyl, cinnolinyl, carbazolyl, β-carbolinyl, phenanthridinyl, acrydinyl, phenazinyl, phenothiazinyl, phenoxazinyl and the like.

In the present specification, examples of the "nonaromatic heterocyclic group" (containing "3- to 14-membered nonaromatic heterocyclic group") include a 3- to 14-membered (preferably 4- to 10-membered) nonaromatic heterocyclic group having, as ring-constituting atom besides carbon atom, 1 to 4 hetero atoms selected from a nitrogen atom, a sulfur atom and an oxygen atom.

Preferable examples of the "nonaromatic heterocyclic group" include 3- to 8-membered monocyclic nonaromatic heterocyclic groups such as aziridinyl, oxiranyl, thiiranyl, azetidinyl, oxetanyl, thietanyl, tetrahydrothienyl, tetrahydrofuranyl, pyrrolinyl, pyrrolidinyl, imidazolinyl, imidazolidinyl, oxazolinyl, oxazolidinyl, pyrazolinyl, pyrazolidinyl, thiazolinyl, thiazolidinyl, tetrahydroisothiazolyl, tetrahydrooxazolyl, tetrahydroisooxazolyl, piperidinyl, piperazinyl, tetrahydropyridinyl, dihydropyridinyl, dihydrothiopyranyl, tetrahydropyrimidinyl, tetrahydropyridazinyl, dihydropyranyl, tetrahydropyranyl, tetrahydrothiopyranyl, morpholinyl, thiomorpholinyl, azepanyl, diazepanyl, azepinyl, oxepanyl, azocanyl, diazocanyl and the like; 9- to 14-membered fused polycyclic (preferably bicyclic or tricyclic) nonaromatic heterocyclic groups such as dihydrobenzofuranyl, dihydrobenzoimidazolyl, dihydrobenzooxazolyl, dihydrobenzothiazolyl, dihydrobenzoisothiazolyl, dihydronaphto[2,3-b]thienyl, tetrahydroisoquinolyl, tetrahydroquinolyl, 4H-quinolizinyl, indolinyl, isoindolinyl, tetrahydrothieno[2,3-c]pyridinyl, tetrahydrobenzoazepinyl, tetrahydroquinoxalinyl, tetrahydrophenanthridinyl, hexahydrophenothiazinyl, hexahydrophenoxazinyl, tetrahydrophthalazinyl, tetrahydronaphthyridinyl, tetrahydroquinazolinyl, tetrahydrocinnolinyl, tetrahydrocarbazolyl, tetrahydro-β-carbolinyl, tetrahydroacrydinyl, tetrahydrophenazinyl, tetrahydrothioxanthenyl, octahydroisoquinolyl and the like.

In the present specification, preferable examples of the "7- to 10-membered crosslinked heterocyclic group" include quinuclidinyl, and 7-azabicyclo[2.2.1]heptanyl.

In the present specification, examples of the "nitrogen-containing heterocyclic group" include, of the "heterocyclic group", those containing one or more nitrogen atoms as a ring constituting atom.

In the present specification, examples of the "optionally substituted heterocyclic group" include a heterocyclic group optionally having substituent(s) selected from the aforementioned substituent group A.

The number of the substituents of the "optionally substituted heterocyclic group" is, for example, 1 to 3. When the number of the substituents is two or more, the respective substituents may be the same or different.

In the present specification, examples of the "acyl group" include a formyl group, a carboxy group, a carbamoyl group, a thiocarbamoyl group, a sulfino group, a sulfo group, a sulfamoyl group and a phosphono group, each of which optionally has "1 or 2 substituents selected from a C₁₋₆ alkyl group, a C₂₋₆ alkenyl group, a C₃₋₁₀ cycloalkyl group, a C₃₋₁₀ cycloalkenyl group, a C₆₋₁₄ aryl group, a C₇₋₁₆ aralkyl group, a 5- to 14-membered aromatic heterocyclic group and a 3- to 14-membered nonaromatic heterocyclic group, each of which optionally has 1 to 3 substituents selected from a halogen atom, an optionally halogenated C₁₋₆ alkoxy group, a hydroxy group, a nitro group, a cyano group, an amino group and a carbamoyl group".

In addition, examples of the "acyl group" include a hydrocarbon-sulfonyl group, a heterocyclyl-sulfonyl group, a hydrocarbon-sulfinyl group, and a heterocyclyl-sulfinyl group.

Here, the hydrocarbon-sulfonyl group means a hydrocarbon group-bonded sulfonyl group, the heterocyclyl-sulfonyl group means a heterocyclic group-bonded sulfonyl group, the hydrocarbon-sulfinyl group means a hydrocarbon group-bonded sulfinyl group, and the heterocyclyl-sulfinyl group means a heterocyclic group-bonded sulfinyl group.

Preferable examples of the "acyl group" include a formyl group, a carboxy group, a C₁₋₆ alkyl-carbonyl group, a C₂₋₆ alkenyl-carbonyl group (e.g., crotonoyl), a C₃₋₁₀ cycloalkylcarbonyl group (e.g., cyclobutanecarbonyl, cyclopentanecarbonyl, cyclohexanecarbonyl, cycloheptanecarbonyl), a C₃₋₁₀ cycloalkenyl-carbonyl group (e.g., 2-cyclohexenecarbonyl), a C₆₋₁₄ aryl-carbonyl group, a C₇₋₁₆ aralkyl-carbonyl group, a 5-to 14-membered aromatic heterocyclyl-carbonyl group, a 3- to 14-membered non-aromatic heterocyclyl-carbonyl group, a C₁₋₆ alkoxy-carbonyl group, a C₆₋₁₄ aryloxy-carbonyl group (e.g., phenyloxycarbonyl, naphthyloxycarbonyl), a C₇₋₁₆ aralkyloxy-carbonyl group (e.g., benzyloxycarbonyl, phenethyloxycarbonyl), a carbamoyl group, a mono- or di- C₁₋₆ alkyl-carbamoyl group, a mono- or di-C₂₋₆ alkenyl-carbamoyl group (e.g., diallylcarbamoyl), a mono- or di-C₃₋₁₀ cycloalkyl-carbamoyl group (e.g., cyclopropylcarbamoyl), a mono- or di- C₆₋₁₄ aryl-carbamoyl group (e.g., phenylcarbamoyl), a mono- or di-C₇₋₁₆ aralkyl-carbamoyl group, a 5- to 14-membered aromatic heterocyclyl carbamoyl group (e.g., pyridylcarbamoyl), a thiocarbamoyl group, a mono- or di- C₁₋₆ alkyl-thiocarbamoyl group (e.g., methylthiocarbamoyl, N-ethyl-N-methylthiocarbamoyl), a mono- or di-C₂₋₆ alkenyl-thiocarbamoyl group (e.g., diallylthiocarbamoyl), a mono- or di-C₃₋₁₀ cycloalkyl-thiocarbamoyl group (e.g., cyclopropylthiocarbamoyl, cyclohexylthiocarbamoyl), a mono- or di- C₆₋₁₄ aryl-thiocarbamoyl group (e.g., phenylthiocarbamoyl), a mono- or di-C₇₋₁₆ aralkyl-thiocarbamoyl group (e.g., benzylthiocarbamoyl, phenethylthiocarbamoyl), a 5- to 14-membered aromatic heterocyclethiocarbamoyl group (e.g., pyridylthiocarbamoyl), sulfino group, a C₁₋₆ alkylsulfinyl group (e.g., methylsulfinyl, ethylsulfinyl), a sulfo group, a C₁₋₆ alkylsulfonyl group, a C₆₋₁₄ arylsulfonyl group, a phosphono group, and a mono- or di- C₁₋₆ alkylphosphono group (e.g., dimethylphosphono, diethylphosphono, diisopropylphosphono, dibutylphosphono).

In the present specification, examples of the "optionally substituted amino group" include an amino group optionally having "1 or 2 substituents selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group, a C₇₋₁₆ aralkyl-carbonyl group, a 5- to 14-membered aromatic heterocyclyl-carbonyl group, a 3- to 14-membered non-aromatic heterocyclyl-carbonyl group, a C₁₋₆ alkoxy-carbonyl group, a 5- to 14-membered aromatic heterocyclic group, a carbamoyl group, a mono- or di-C₁₋₆ alkyl-carbamoyl group, a mono- or di-C₇₋₁₆ aralkyl-carbamoyl group, a C₁₋₆ alkylsulfonyl group and a C₆₋₁₄ arylsulfonyl group, each of which optionally has 1 to 3 substituents selected from substituent group A".

Preferable examples of the optionally substituted amino group include an amino group, a mono- or di-(optionally halogenated C₁₋₆ alkyl)amino group (e.g., methylamino, trifluoromethylamino, dimethylamino, ethylamino, diethylamino, propylamino, dibutylamino), a mono- or di-C₂₋₆ alkenylamino group (e.g., diallylamino), a mono- or di-C₃₋₁₀ cycloalkylamino group (e.g., cyclopropylamino, cyclohexylamino), a mono- or di-C₆₋₁₄ arylamino group (e.g., phenylamino), a mono- or di-C₇₋₁₆ aralkylamino group (e.g., benzylamino, dibenzylamino), a mono- or di-(optionally halogenated C₁₋₆ alkyl)-carbonylamino group (e.g., acetylamino, propionylamino), a mono- or di-C₆₋₁₄ aryl-carbonylamino group (e.g., benzoylamino), a mono- or di-C₇₋₁₆ aralkyl-carbonylamino group (e.g., benzylcarbonylamino), a mono- or di-5- to 14-membered aromatic heterocyclyl-carbonylamino group (e.g., nicotinoylamino, isonicotinoylamino), a mono- or di-3- to 14-membered non-aromatic heterocyclyl-carbonylamino group (e.g., piperidinylcarbonylamino), a mono- or di-C₁₋₆ alkoxy-carbonylamino group (e.g., tert-butoxycarbonylamino), a 5- to 14-membered aromatic heterocyclyl-amino group (e.g., pyridylamino), a carbamoylamino group, (mono- or di-C₁₋₆ alkyl-carbamoyl) an amino group (e.g., methylcarbamoylamino), a (mono- or di-C₇₋₁₆ aralkyl-carbamoyl) amino group (e.g., benzylcarbamoylamino), a C₁₋₆ alkylsulfonylamino group (e.g., methylsulfonylamino, ethylsulfonylamino), a C₆₋₁₄ arylsulfonylamino group (e.g., phenylsulfonylamino), a (C₁₋₆ alkyl) (C₁₋₆ alkyl-carbonyl) amino group (e.g., N-acetyl-N-methylamino), and a (C₁₋₆ alkyl) (C₆₋₁₄ aryl-carbonyl)amino group (e.g., N-benzoyl-N-methylamino).

In the present specification, examples of the "optionally substituted carbamoyl group" include a carbamoyl group optionally having "1 or 2 substituents selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group, a C₇₋₁₆ aralkyl-carbonyl group, a 5-to 14-membered aromatic heterocyclyl-carbonyl group, a 3- to 14-membered non-aromatic heterocyclyl-carbonyl group, a C₁₋₆ alkoxy-carbonyl group, a 5- to 14-membered aromatic heterocyclic group, a carbamoyl group, a mono- or di-C₁₋₆ alkyl-carbamoyl group and a mono- or di-C₇₋₁₆ aralkyl-carbamoyl group, each of which optionally has 1 to 3 substituents selected from substituent group A".

Preferable examples of the optionally substituted carbamoyl group include a carbamoyl group, a mono- or di-C₁₋₆ alkyl-carbamoyl group, a mono- or di- C₂₋₆ alkenyl-carbamoyl group (e.g., diallylcarbamoyl), a mono- or di-C₃₋₁₀ cycloalkyl-carbamoyl group (e.g., cyclopropylcarbamoyl, cyclohexylcarbamoyl), a mono- or di-C₆₋₁₄ aryl-carbamoyl group (e.g., phenylcarbamoyl), a mono- or di-C₇₋₁₆ aralkyl-carbamoyl group, a mono- or di- C₁₋₆ alkyl-carbonyl-carbamoyl group (e.g., acetylcarbamoyl, propionylcarbamoyl), a mono- or di- C₆₋₁₄ aryl-carbonyl-carbamoyl group (e.g., benzoylcarbamoyl), and a 5- to 14-membered aromatic heterocyclyl carbamoyl group (e.g., pyridylcarbamoyl).

In the present specification, examples of the "optionally substituted thiocarbamoyl group" include a thiocarbamoyl group optionally having "1 or 2 substituents selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group, a C₇₋₁₆ aralkyl-carbonyl group, a 5-to 14-membered aromatic heterocyclyl-carbonyl group, a 3- to 14-membered non-aromatic heterocyclyl-carbonyl group, a C₁₋₆ alkoxy-carbonyl group, a 5- to 14-membered aromatic heterocyclic group, a carbamoyl group, a mono- or di-C₁₋₆ alkyl-carbamoyl group and a mono- or di-C₇₋₁₆ aralkyl-carbamoyl group, each of which optionally has 1 to 3 substituents selected from substituent group A".

Preferable examples of the optionally substituted thiocarbamoyl group include a thiocarbamoyl group, a mono- or di-C₁₋₆ alkyl-thiocarbamoyl group (e.g., methylthiocarbamoyl, ethylthiocarbamoyl, dimethylthiocarbamoyl, diethylthiocarbamoyl, N-ethyl-N-methylthiocarbamoyl), a mono- or di-C₂₋₆ alkenyl-thiocarbamoyl group (e.g., diallylthiocarbamoyl), a mono- or di-C₃₋₁₀ cycloalkyl-thiocarbamoyl group (e.g., cyclopropylthiocarbamoyl, cyclohexylthiocarbamoyl), a mono- or di-C₆₋₁₄ aryl-thiocarbamoyl group (e.g., phenylthiocarbamoyl), a mono- or di-C₇₋₁₆ aralkyl-thiocarbamoyl group (e.g., benzylthiocarbamoyl, phenethylthiocarbamoyl), a mono- or di-C₁₋₆ alkyl-carbonyl-thiocarbamoyl group (e.g., acetylthiocarbamoyl, propionylthiocarbamoyl), a mono- or di-C₆₋₁₄ aryl-carbonyl-thiocarbamoyl group (e.g., benzoylthiocarbamoyl), and a 5- to 14-membered aromatic heterocyclethiocarbamoyl group (e.g., pyridylthiocarbamoyl).

In the present specification, examples of the "optionally substituted sulfamoyl group" include a sulfamoyl group optionally having "1 or 2 substituents selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group, a C₇₋₁₆ aralkyl-carbonyl group, a 5-to 14-membered aromatic heterocyclyl-carbonyl group, a 3- to 14-membered non-aromatic heterocyclyl-carbonyl group, a C₁₋₆ alkoxy-carbonyl group, a 5- to 14-membered aromatic heterocyclic group, a carbamoyl group, a mono- or di- C₁₋₆ alkyl-carbamoyl group and a mono- or di-C₇₋₁₆ aralkyl-carbamoyl group, each of which optionally has 1 to 3 substituents selected from substituent group A".

Preferable examples of the optionally substituted sulfamoyl group include a sulfamoyl group, a mono- or di-C₁₋₆ alkyl-sulfamoyl group (e.g., methylsulfamoyl, ethylsulfamoyl, dimethylsulfamoyl, diethylsulfamoyl, N-ethyl-N-methylsulfamoyl), a mono- or di-C₂₋₆ alkenyl-sulfamoyl group (e.g., diallylsulfamoyl), a mono- or di-C₃₋₁₀ cycloalkyl-sulfamoyl group (e.g., cyclopropylsulfamoyl, cyclohexylsulfamoyl), a mono- or di-C₆₋₁₄ aryl-sulfamoyl group (e.g., phenylsulfamoyl), a mono- or di-C₇₋₁₆ aralkyl-sulfamoyl group (e.g., benzylsulfamoyl, phenethylsulfamoyl), a mono- or di-C₁₋₆ alkyl-carbonyl-sulfamoyl group (e.g., acetylsulfamoyl, propionylsulfamoyl), a mono- or di-C₆₋₁₄ aryl-carbonyl-sulfamoyl group (e.g., benzoylsulfamoyl), and a 5- to 14-membered aromatic heterocyclyl-sulfamoyl group (e.g., pyridylsulfamoyl).

In the present specification, examples of the "optionally substituted hydroxy group" include a hydroxyl group optionally having "substituent selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group, a C₇₋₁₆ aralkyl-carbonyl group, a 5- to 14-membered aromatic heterocyclyl-carbonyl group, a 3- to 14-membered non-aromatic heterocyclyl-carbonyl group, a C₁₋₆ alkoxy-carbonyl group, a 5- to 14-membered aromatic heterocyclic group, a carbamoyl group, a mono- or di-C₁₋₆ alkyl-carbamoyl group, a mono- or di-C₇₋₁₆ aralkyl-carbamoyl group, a C₁₋₆ alkylsulfonyl group and a C₆₋₁₄ arylsulfonyl group, each of which optionally has 1 to 3 substituents selected from substituent group A".

Preferable examples of the optionally substituted hydroxy group include a hydroxy group, a C₁₋₆ alkoxy group, a C₂₋₆ alkenyloxy group (e.g., allyloxy, 2-butenyloxy, 2-pentenyloxy, 3-hexenyloxy), a C₃₋₁₀ cycloalkyloxy group (e.g., cyclohexyloxy), a C₆₋₁₄ aryloxy group (e.g., phenoxy, naphthyloxy), a C₇₋₁₆ aralkyloxy group (e.g., benzyloxy, phenethyloxy), a C₁₋₆ alkyl-carbonyloxy group (e.g., acetyloxy, propionyloxy, butyryloxy, isobutyryloxy, pivaloyloxy), a C₆₋₁₄ aryl-carbonyloxy group (e.g., benzoyloxy), a C₇₋₁₆ aralkyl-carbonyloxy group (e.g., benzylcarbonyloxy), a 5- to 14-membered aromatic heterocyclecarbonyloxy group (e.g., nicotinoyloxy), a 3- to 14-membered non-aromatic heterocyclecarbonyloxy group (e.g., piperidinylcarbonyloxy), a C₁₋₆ alkoxy-carbonyloxy group (e.g., tert-butoxycarbonyloxy), a 5- to 14-membered aromatic heterocycleoxy group (e.g., pyridyloxy), a carbamoyloxy group, a C₁₋₆ alkyl-carbamoyloxy group (e.g., methylcarbamoyloxy), a C₇₋₁₆ aralkyl-carbamoyloxy group (e.g., benzylcarbamoyloxy), a C₁₋₆ alkylsulfonyloxy group (e.g., methylsulfonyloxy, ethylsulfonyloxy), and a C₆₋₁₄ arylsulfonyloxy group (e.g., phenylsulfonyloxy).

In the present specification, examples of the "optionally substituted sulfanyl group" include a sulfanyl group optionally having "substituent selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group and a 5- to 14-membered aromatic heterocyclic group, each of which optionally has 1 to 3 substituents selected from substituent group A", and a halogenated sulfanyl group.

Preferable examples of the optionally substituted sulfanyl group include a sulfanyl(-SH) group, a C₁₋₆ alkylthio group, a C₂₋₆ alkenylthio group (e.g., allylthio, 2-butenylthio, 2-pentenylthio, 3-hexenylthio), a C₃₋₁₀ cycloalkylthio group (e.g., cyclohexylthio), a C₆₋₁₄ arylthio group (e.g., phenylthio, naphthylthio), a C₇₋₁₆ aralkylthio group (e.g., benzylthio, phenethylthio), a C₁₋₆ alkyl-carbonylthio group (e.g., acetylthio, propionylthio, butyrylthio, isobutyrylthio, pivaloylthio), a C₆₋₁₄ aryl-carbonylthio group (e.g., benzoylthio), a 5- to 14-membered aromatic heterocyclylthio group (e.g., pyridylthio), and a halogenated thio group (e.g., pentafluorothio).

In the present specification, examples of the "optionally substituted silyl group" include a silyl group optionally having "1 to 3 substituents selected from a C₁₋₆ alkyl group, a C₂₋₆ alkenyl group, a C₃₋₁₀ cycloalkyl group, a C₆₋₁₄ aryl group and a C₇₋₁₆ aralkyl group, each of which optionally has 1 to 3 substituents selected from substituent group A".

Preferable examples of the optionally substituted silyl group include a tri-C₁₋₆ alkylsilyl group (e.g., trimethylsilyl, tert-butyl(dimethyl)silyl).

In the present specification, examples of the "C₁₋₆ alkylene group" include -CH₂-, -(CH₂)₂-, -(CH₂)₃-, -(CH₂)₄-,-(CH₂)₅-, -(CH₂)₆-, -CH(CH₃)-, -C(CH₃)₂-, -CH(C₂H₅)-, -CH(C₃H₇)-,-CH(CH(CH₃)₂)-, -(CH(CH₃))₂-, -CH₂-CH(CH₃)-, -CH(CH₃)-CH₂-, -CH₂-CH₂-C(CH₃)₂-, -C (CH₃)₂-CH₂-CH₂-, -CH₂-CH₂-CH₂-C(CH₃)₂-, and-C(CH₃)₂-CH₂-CH₂-CH₂-.

In the present specification, examples of the "C₂₋₆ alkenylene group" include -CH=CH-, -CH₂-CH=CH-, -CH=CH-CH₂-,-C (CH₃)₂-CH=CH-, -CH=CH-C (CH₃)₂-, -CH₂-CH=CH-CH₂, -CH₂-CH₂-CH=CH-, -CH=CH-CH₂-CH₂-, -CH=CH-CH=CH-, -CH=CH-CH₂-CH₂-CH₂-, and -CH₂-CH₂-CH₂-CH=CH-.

In the present specification, examples of the "C₂₋₆ alkynylene group" include -C=C-, -CH₂-C≡C-, -C≡C-CH₂-, -C(CH₃)₂-C≡C-, -C≡C-C(CH₃)₂-, -CH₂-C≡C-CH₂-, -CH₂-CH₂-C≡C-, -C≡C-CH₂-CH₂-, -C≡C-C≡C-, -C≡C-CH₂-CH₂-CH₂-, and -CH₂-CH₂-CH₂-C≡C-.

The definition of each symbol in the formula (I) is described in detail in the following.

Ring A is an optionally further substituted 5- or 6-membered nitrogen-containing heterocycle.

Examples of the "5- or 6-membered nitrogen-containing heterocycle" of the "optionally further substituted 5- or 6-membered nitrogen-containing heterocycle" for ring A include pyrazole, triazole, oxazole, thiazole, oxadiazole, thiadiazole, pyridine, pyridazine, pyrazine, pyrimidine, triazine, dihydropyrazole and the like.

The "5- or 6-membered nitrogen-containing heterocycle" for ring A may be further substituted by, for example, substituents selected from the aforementioned substituent group A, and the number of the substituents is, for example, 1 to 3. When the number of the substituents is two or more, the respective substituents may be the same or different.

Ring A is preferably a 5- or 6-membered nitrogen-containing heterocycle (e.g., pyrazole, pyridine, pyrimidine, dihydropyrazole) optionally substituted by 1 to 3 substituents selected from
(1) a hydroxy group, and
(2) a C₁₋₆ alkyl group (e.g., methyl).

Note that, preferably, ring A is not optionally further substituted pyrrole. Preferably, the substituent that the "5-or 6-membered nitrogen-containing heterocycle" for ring A optionally has is not a secondary amino group having a substituent (e.g., mono-C₁₋₆ alkylamino group, mono-C₆₋₁₄ arylamino group, 5- to 14-membered aromatic heterocyclyl-amino group, C₇₋₁₆ aralkylamino group, formylamino group, C₁₋₆ alkyl-carbonylamino group, C₆₋₁₄ aryl-carbonylamino group, C₁₋₆ alkoxy-carbonylamino group, C₇₋₁₆ aralkyloxy-carbonylamino group, C₁₋₆ alkylsulfonylamino group, C₆₋₁₄ arylsulfonylamino group optionally substituted by a C₁₋₆ alkyl group).

Ring A is more preferably a 5- or 6-membered nitrogen-containing aromatic heterocycle (e.g., pyrazole, pyridine) optionally substituted by 1 to 3 (preferably one) hydroxy groups.

In another embodiment, ring A is preferably a 5- or 6-membered nitrogen-containing heterocycle (e.g., pyrazole, pyridine, pyrimidine, dihydropyrazole) optionally substituted by 1 to 3 substituents selected from
(1) a hydroxy group,
(2) a C₁₋₆ alkyl group (e.g., methyl),
(3) an amino group, and
(4) a halogen atom (e.g., chlorine atom).

Preferably, ring A is not optionally further substituted pyrrole. In addition, preferably, the substituent that the "5-or 6-membered nitrogen-containing heterocycle" for ring A optionally has is not a secondary amino group having a substituent (e.g., mono-C₁₋₆ alkylamino group, mono-C₆₋₁₄ arylamino group, 5- to 14-membered aromatic heterocyclyl-amino group, C₇₋₁₆ aralkylamino group, formylamino group, C₁₋₆ alkyl-carbonylamino group, C₆₋₁₄ aryl-carbonylamino group, C₁₋₆ alkoxy-carbonylamino group, C₇₋₁₆ aralkyloxy-carbonylamino group, C₁₋₆ alkylsulfonylamino group, C₆₋₁₄ arylsulfonylamino group optionally substituted by a C₁₋₆ alkyl group).

Ring A is more preferably a 5- or 6-membered nitrogen-containing aromatic heterocycle (e.g., pyrazole).

Ring B is an optionally substituted 5- or 6-membered nitrogen-containing heterocycle.

Examples of the "5- or 6-membered nitrogen-containing heterocycle" of the "optionally substituted 5- or 6-membered nitrogen-containing heterocycle" for ring B include pyrrole, pyrazole, triazole, oxazole, thiazole, oxadiazole, thiadiazole, pyridine, pyridazine, pyrimidine, triazine, dihydropyrimidine and the like.

The "5- or 6-membered nitrogen-containing heterocycle" for ring B may be further substituted by, for example, the aforementioned "substituent", and the number of the substituents is, for example, 1 to 3. When the number of the substituents is two or more, the respective substituents may be the same or different.

Ring B is preferably a 5- or 6-membered nitrogen-containing heterocycle (e.g., pyridine, pyrimidine, pyrazole, triazole (e.g., 1,2,4-triazole), dihydropyrimidine, imidazole) optionally substituted by 1 to 3 substituents selected from
(1) a C₁₋₆ alkyl group (e.g., methyl, ethyl, isopropyl) optionally substituted by 1 to 3 C₁₋₆ alkoxy groups (e.g., methoxy),
(2) a halogen atom (e.g., chlorine atom, bromine atom),
(3) a hydroxy group,
(4) a C₂₋₆ alkenyl group (e.g., vinyl, propa-1-en-2-yl),
(5) a C₃₋₈ cycloalkyl group (e.g., cyclopropyl),
(6) a mono- or a di- C₁₋₆ alkyl-amino group (e.g., dimethylamino, ethyl(methyl)amino),
(7) a heterocyclic group (e.g., azetidinyl, pyrrolidinyl, piperidyl, morpholinyl, 3-oxa-8-azabicyclo[3.2.1]octyl, 3-oxa-6-azabicyclo[3.1.1]heptyl, 6-oxa-3-azabicyclo[3.1.1]heptyl, pyrazolyl) optionally substituted by 1 to 3 substituents selected from
   (i) a C₁₋₆ alkoxy group (e.g., methoxy),
   (ii) a halogen atom (e.g., fluorine atom), and
   (iii) a C₁₋₆ alkyl group (e.g., methyl), and
(8) an oxo group.

Preferably, ring B is not pyrazine. In addition, preferably, a substituent that the "5- or 6-membered nitrogen-containing heterocycle" for ring B optionally has is not a secondary amino group having a substituent. Here, examples of the substituent that the "secondary amino group" has include substituents selected from the above-mentioned substituent group A.

Ring B is more preferably a 6-membered nitrogen-containing aromatic heterocycle (e.g., pyridine, pyrimidine) optionally substituted by 1 to 3 C₁₋₆ alkyl groups (e.g., methyl).

In another embodiment, ring B is preferably a 5- or 6-membered nitrogen-containing heterocycle (e.g., pyridine, pyrimidine, pyrazole, triazole (e.g., 1,2,4-triazole), dihydropyrimidine, imidazole) optionally substituted by 1 to 3 substituents selected from
(1) a C₁₋₆ alkyl group (e.g., methyl, ethyl, isopropyl) optionally substituted by 1 to 3 C₁₋₆ alkoxy groups (e.g., methoxy),
(2) a halogen atom (e.g., chlorine atom, bromine atom),
(3) a hydroxy group,
(4) a C₂₋₆ alkenyl group (e.g., vinyl, propa-1-en-2-yl),
(5) a C₃₋₈ cycloalkyl group (e.g., cyclopropyl),
(6) a mono- or a di- C₁₋₆ alkyl-amino group (e.g., dimethylamino, ethyl(methyl)amino),
(7) a heterocyclic group (e.g., azetidinyl, pyrrolidinyl, piperidyl, morpholinyl, 3-oxa-8-azabicyclo[3.2.1]octyl, 3-oxa-6-azabicyclo[3.1.1]heptyl, 6-oxa-3-azabicyclo[3.1.1]heptyl, pyrazolyl, imidazolyl, triazolyl (e.g., 1,2,3-triazolyl), pyridyl, dihydropyridyl) optionally substituted by 1 to 3 substituents selected from
   (i) a C₁₋₆ alkoxy group (e.g., methoxy),
   (ii) a halogen atom (e.g., fluorine atom),
   (iii) a C₁₋₆ alkyl group (e.g., methyl) optionally substituted by 1 to 3 halogen atoms (e.g., fluorine atom), and
   (iv) an oxo group, and
(8) an oxo group.

Preferably, ring B is not pyrazine. In addition, preferably, the substituent that the "5- or 6-membered nitrogen-containing heterocycle" for ring B optionally has is not a secondary amino group having a substituent. Here, examples of the substituent that the "secondary amino group" has include substituents selected from the above-mentioned substituent group A.

Ring B is more preferably a 6-membered nitrogen-containing aromatic heterocycle (e.g., pyridine, pyrimidine) optionally substituted by 1 to 3 substituents selected from (1) a C₁₋₆ alkyl group (e.g., methyl), and (2) a heterocyclic group (e.g., pyrazolyl, imidazolyl, triazolyl (e.g., 1,2,3-triazolyl), pyridyl) optionally substituted by 1 to 3 substituents selected from
(i) a halogen atom (e.g., fluorine atom), and
(ii) a C₁₋₆ alkyl group (e.g., methyl) optionally substituted by 1 to 3 halogen atoms (e.g., fluorine atom).

In a still another embodiment, ring B is preferably a 5-or 6-membered nitrogen-containing heterocycle (e.g., pyridine, pyrimidine, pyrazole, triazole (e.g., 1,2,4-triazole), dihydropyrimidine, imidazole) optionally substituted by 1 to 3 substituents selected from
(1) a C₁₋₆ alkyl group (e.g., methyl, ethyl, isopropyl) optionally substituted by 1 to 3 C₁₋₆ alkoxy groups (e.g., methoxy),
(2) a halogen atom (e.g., fluorine atom, chlorine atom, bromine atom),
(3) a hydroxy group,
(4) a C₂₋₆ alkenyl group (e.g., vinyl, propa-1-en-2-yl),
(5) a C₃₋₈ cycloalkyl group (e.g., cyclopropyl),
(6) a mono- or a di- C₁₋₆ alkyl-amino group (e.g., dimethylamino, ethyl(methyl)amino),
(7) a heterocyclic group (e.g., azetidinyl, pyrrolidinyl, piperidyl, morpholinyl, 3-oxa-8-azabicyclo[3.2.1]octyl, 3-oxa-6-azabicyclo[3.1.1]heptyl, 6-oxa-3-azabicyclo[3.1.1]heptyl, pyrazolyl, imidazolyl, triazolyl (e.g., 1,2,3-triazolyl, 1,2,4-triazolyl), pyridyl, dihydropyridyl, pyridazinyl, pyrimidinyl, thiazolyl, tetrahydropyranyl, tetrazolyl, pyrazinyl, isoxazolyl) optionally substituted by 1 to 3 substituents selected from
   (i) a C₁₋₆ alkoxy group (e.g., methoxy),
   (ii) a halogen atom (e.g., fluorine atom, chlorine atom),
   (iii) a C₁₋₆ alkyl group (e.g., methyl) optionally substituted by 1 to 3 halogen atoms (e.g., fluorine atom), and
   (iv) an oxo group,
(8) a C₆₋₁₄ aryl group optionally substituted by a cyano group (e.g., phenyl),
(9) a C₁₋₆ alkoxy group (e.g., methoxy), and
(10) an oxo group.

Preferably, ring B is not pyrazine. In addition, preferably, the substituent that "5- or 6-membered nitrogen-containing heterocycle" for ring B optionally has is not preferably a secondary amino group having a substituent. Here, examples of the substituent that the "secondary amino group" has include substituents selected from the above-mentioned substituent group A.

Ring B is more preferably a 6-membered nitrogen-containing aromatic heterocycle (e.g., pyrimidine) optionally substituted by 1 to 3 substituents selected from (1) a C₁₋₆ alkyl group (e.g., methyl), and (2) a heterocyclic group (e.g., triazolyl (e.g., 1,2,3-triazolyl, 1,2,4-triazolyl), pyridyl) optionally substituted by 1 to 3 substituents selected from a C₁₋₆ alkyl group (e.g., methyl).

When the "5- or 6-membered nitrogen-containing heterocycle" of the "optionally further substituted 5- or 6-membered nitrogen-containing heterocycle" for ring A and the "optionally substituted 5- or 6-membered nitrogen-containing heterocycle" for ring B contain -N=C(OH)- as shown in the following formula (A), a tautomer of the following formula (B) is present. The tautomer is also contained in ring A and ring B.

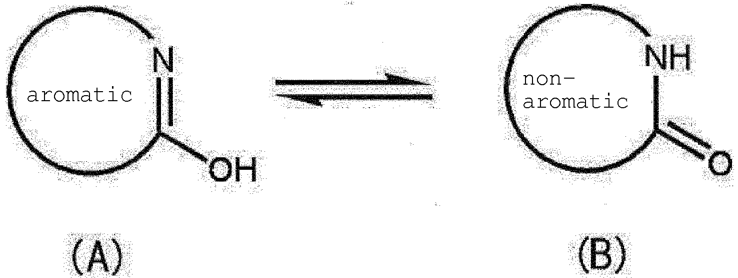

Fused ring AB is an optionally further substituted heterocycle having two or more nitrogen atoms as ring-constituting atoms besides carbon atom, and optionally having 1 or 2 hetero atoms selected from an oxygen atom and a sulfur atom, and the nitrogen atom constituting the fused ring AB may be present at any position on the fused ring. The fused ring AB is aromatic, may show aromaticity only by the fused ring AB, or may show aromaticity due to the substituent further contained.

The "aromaticity" in the present specification means that it has a flat plane structure, and has cyclic **π** electrons in the number of (4n+2) (n is a positive integer including 0) according to the Hückel's rule. When a tautomer is present, "aromaticity" is shown when at least one satisfies the above-mentioned definition.

When ring A or ring B constituting the fused ring AB contains -N=C(OH)-, a tautomer is contained in the fused ring AB.

For example, in a compound wherein ring A is 2-hydroxypyridine, ring B is pyridine, and fused ring AB is 2-hydroxy-1,5-naphthyridine, a compound which is 2-oxo-1,2-dihydro-1,5-naphthyridine is present as a tautomer, and the both are encompassed in compound (I).

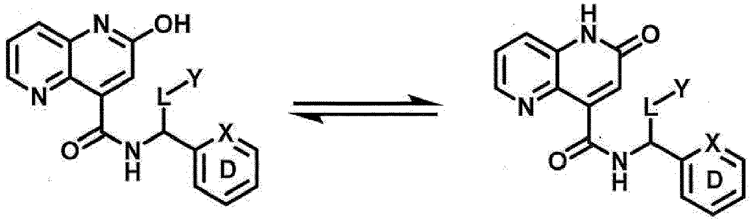

In addition, in a compound wherein ring A is pyrazole substituted by methyl, ring B is dihydropyrimidine substituted by oxo, and fused ring AB is 1-methyl-7-oxo-1,7-dihydropyrazolo[1,5-a]pyrimidine, ionic compounds represented by the following formulas (C) and (D), wherein fused ring AB is aromatic, are present as tautomers.

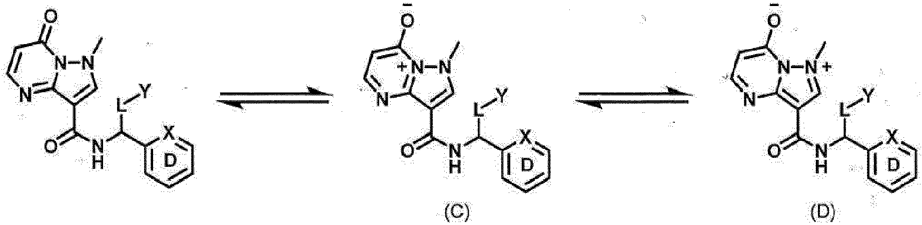

The fused ring AB is preferably
(I) an aromatic heterocycle (e.g., naphthyridine (e.g., 1,5-naphthyridine, 1,6-naphthyridine), pyrazolo[1,5-a]pyrimidine, triazolo[1,5-a]pyrimidine (e.g., 1,2,4-triazolo[1,5-a]pyrimidine), imidazo[4,5-b]pyridine, purine, pyrazolo[4,3-c]pyridine) having, as ring-constituting atom besides carbon atom, 2 to 4 nitrogen atoms optionally substituted by 1 to 3 substituents selected from
   (1) a hydroxy group,
   (2) a halogen atom (e.g., chlorine atom, bromine atom),
   (3) a C₁₋₆ alkyl group (e.g., methyl, ethyl, isopropyl),
   (4) a C₂₋₆ alkenyl group (e.g., vinyl, propa-1-en-2-yl),
   (5) a C₃₋₈ cycloalkyl group (e.g., cyclopropyl),
   (6) a mono- or a di- C₁₋₆ alkyl-amino group (e.g., dimethylamino, ethyl(methyl)amino), and
   (7) a heterocyclic group (e.g., azetidinyl, pyrrolidinyl, piperidyl, morpholinyl, 3-oxa-8-azabicyclo[3.2.1]octyl, 3-oxa-6-azabicyclo[3.1.1]heptyl, 6-oxa-3-azabicyclo[3.1.1]heptyl, pyrazolyl) optionally substituted by 1 to 3 substituents selected from
      (i) a C₁₋₆ alkoxy group (e.g., methoxy),
      (ii) a halogen atom (e.g., fluorine atom), and
      (iii) a C₁₋₆ alkyl group (e.g., methyl), or
(II) a heterocycle (e.g., 1,7-dihydropyrazolo[1,5-a]pyrimidine) having, as ring-constituting atom besides carbon atom, 2 to 4 nitrogen atoms optionally substituted by 1 to 3 (preferably 3) substituents selected from
   (1) a C₁₋₆ alkyl group (e.g., methyl) optionally substituted by one C₁₋₆ alkoxy group (e.g., methoxy), and
   (2) a C₃₋₈ cycloalkyl group (e.g., cyclopropyl), and further having an oxo group to show aromaticity (e.g., 7-oxo-1,7-dihydropyrazolo[1,5-a]pyrimidine).

Preferably, fused ring AB is not optionally substituted 1,8-naphthyridine. In addition, preferably, fused ring AB is not optionally substituted pyrazolo[3,4-b]pyridine.

The fused ring AB is more preferably the aromatic heterocycle of the above-mentioned (I). Of those, it is preferably an aromatic heterocycle (e.g., naphthyridine (e.g., 1,5-naphthyridine), pyrazolo[1,5-a]pyrimidine) having, as ring-constituting atom besides carbon atom, 2 or 3 nitrogen atoms optionally substituted by 1 to 3 substituents selected from (1) a hydroxy group, and (2) a C₁₋₆ alkyl group (e.g., methyl).

In another embodiment, fused ring AB is preferably
(I) an aromatic heterocycle (e.g., naphthyridine (e.g., 1,5-naphthyridine, 1,6-naphthyridine), pyrazolo[1,5-a]pyrimidine, triazolo[1,5-a]pyrimidine (e.g., 1,2,4-triazolo[1,5-a]pyrimidine), imidazo[4,5-b]pyridine, purine, pyrazolo[4,3-c]pyridine) having, as ring-constituting atom besides carbon atom, 2 to 4 nitrogen atoms, optionally substituted by 1 to 3 substituents selected from
   (1) a hydroxy group,
   (2) a halogen atom (e.g., chlorine atom, bromine atom),
   (3) a C₁₋₆ alkyl group (e.g., methyl, ethyl, isopropyl),
   (4) a C₂₋₆ alkenyl group (e.g., vinyl, propa-1-en-2-yl),
   (5) a C₃₋₈ cycloalkyl group (e.g., cyclopropyl),
   (6) a mono- or a di- C₁₋₆ alkyl-amino group (e.g., dimethylamino, ethyl(methyl)amino), and
   (7) a heterocyclic group (e.g., azetidinyl, pyrrolidinyl, piperidyl, morpholinyl, 3-oxa-8-azabicyclo[3.2.1]octyl, 3-oxa-6-azabicyclo[3.1.1]heptyl, 6-oxa-3-azabicyclo[3.1.1]heptyl, pyrazolyl, imidazolyl, triazolyl (e.g., 1,2,3-triazolyl), pyridyl, dihydropyridyl) optionally substituted by 1 to 3 substituents selected from
      (i) a C₁₋₆ alkoxy group (e.g., methoxy),
      (ii) a halogen atom (e.g., fluorine atom),
      (iii) a C₁₋₆ alkyl group (e.g., methyl) optionally substituted by 1 to 3 halogen atoms (e.g., fluorine atom), and
      (iv) an oxo group, or
(II) a heterocycle having, as ring-constituting atom besides carbon atom, 2 to 4 nitrogen atoms (e.g., 1,7-dihydropyrazolo[1,5-a]pyrimidine) optionally substituted by 1 to 3 (preferably 3) substituents selected from
   (1) a C₁₋₆ alkyl group (e.g., methyl) optionally substituted by one C₁₋₆ alkoxy group (e.g., methoxy),
   (2) a C₃₋₈ cycloalkyl group (e.g., cyclopropyl), and
   (3) a heterocyclic group (e.g., pyridyl), and further having an oxo group to show aromaticity (e.g., 7-oxo-1,7-dihydropyrazolo[1,5-a]pyrimidine).

Preferably, fused ring AB is not optionally substituted 1,8-naphthyridine. In addition, preferably, fused ring AB is not optionally substituted pyrazolo[3,4-b]pyridine.

The fused ring AB is more preferably the aromatic heterocycle of the above-mentioned (I). Of those, it is preferably an aromatic heterocycle (e.g., naphthyridine (e.g., 1,5-naphthyridine), pyrazolo[1,5-a]pyrimidine) having, as ring-constituting atom besides carbon atom, 2 or 3 nitrogen atoms, optionally substituted by 1 to 3 substituents selected from
(1) a hydroxy group,
(2) a C₁₋₆ alkyl group (e.g., methyl), and
(3) a heterocyclic group (e.g., pyrazolyl, imidazolyl, triazolyl (e.g., 1,2,3-triazolyl), pyridyl) optionally substituted by 1 to 3 substituents selected from
   (i) a halogen atom (e.g., fluorine atom), and
   (ii) a C₁₋₆ alkyl group (e.g., methyl) optionally substituted by 1 to 3 halogen atoms (e.g., fluorine atom).

In a still another embodiment, fused ring AB is preferably
(I) an aromatic heterocycle (e.g., naphthyridine (e.g., 1,5-naphthyridine, 1,6-naphthyridine), pyrazolo[1,5-a]pyrimidine, triazolo[1,5-a]pyrimidine (e.g., 1,2,4-triazolo[1,5-a]pyrimidine), imidazo[4,5-b]pyridine, purine, pyrazolo[4,3-c]pyridine) having 2 to 4 nitrogen atoms as ring-constituting atoms besides carbon atom, which is optionally substituted by 1 to 3 substituents selected from
   (1) a hydroxy group,
   (2) a halogen atom (e.g., fluorine atom, chlorine atom, bromine atom),
   (3) a C₁₋₆ alkyl group (e.g., methyl, ethyl, isopropyl),
   (4) a C₂₋₆ alkenyl group (e.g., vinyl, propa-1-en-2-yl),
   (5) a C₃₋₈ cycloalkyl group (e.g., cyclopropyl),
   (6) an amino group,
   (7) a mono- or a di- C₁₋₆ alkyl-amino group (e.g., dimethylamino, ethyl(methyl)amino),
   (8) a heterocyclic group (e.g., azetidinyl, pyrrolidinyl, piperidyl, morpholinyl, 3-oxa-8-azabicyclo[3.2.1]octyl, 3-oxa-6-azabicyclo[3.1.1]heptyl, 6-oxa-3-azabicyclo[3.1.1]heptyl, pyrazolyl, imidazolyl, triazolyl (e.g., 1,2,3-triazolyl, 1,2,4-triazolyl), pyridyl, dihydropyridyl, pyridazinyl, pyrimidinyl, thiazolyl, tetrahydropyranyl, tetrazolyl, pyrazinyl, isoxazolyl) optionally substituted by 1 to 3 substituents selected from
      (i) a C₁₋₆ alkoxy group (e.g., methoxy),
      (ii) a halogen atom (e.g., fluorine atom, chlorine atom),
      (iii) a C₁₋₆ alkyl group (e.g., methyl) optionally substituted by 1 to 3 halogen atoms (e.g., fluorine atom), and
      (iv) an oxo group,
   (9) a C₆₋₁₄ aryl group optionally substituted by a cyano group (e.g., phenyl), and
   (10) a C₁₋₆ alkoxy group (e.g., methoxy), or
(II) a heterocycle having, as ring-constituting atom besides carbon atom, 2 to 4 nitrogen atoms (e.g., 1,7-dihydropyrazolo[1,5-a]pyrimidine) optionally substituted by 1 to 3 (preferably 3) substituents selected from
   (1) a C₁₋₆ alkyl group (e.g., methyl, ethyl, isopropyl) optionally substituted by one C₁₋₆ alkoxy group (e.g., methoxy),
   (2) a C₃₋₈ cycloalkyl group (e.g., cyclopropyl),
   (3) a heterocyclic group (e.g., pyridyl), and
   (4) a halogen atom (e.g., chlorine atom), and further having an oxo group to show aromaticity (e.g., 7-oxo-1,7-dihydropyrazolo[1,5-a]pyrimidine).

Preferably, fused ring AB is not optionally substituted 1,8-naphthyridine. In addition, preferably, fused ring AB is not optionally substituted pyrazolo[3,4-b]pyridine.

The fused ring AB is more preferably the aromatic heterocycle of the above-mentioned (I). Of those, it is preferably an aromatic heterocycle (e.g., pyrazolo[1,5-a]pyrimidine) having 2 or 3 nitrogen atoms as ring-constituting atoms besides carbon atom, which is optionally substituted by 1 to 3 substituents selected from
(1) a C₁₋₆ alkyl group (e.g., methyl), and
(2) a heterocyclic group (e.g., triazolyl (e.g., 1,2,3-triazolyl, 1,2,4-triazolyl), pyridyl) optionally substituted by 1 to 3 substituents selected from a C₁₋₆ alkyl group (e.g., methyl).

Ring D is a benzene ring or pyridine ring, each of which further has a substituent.

The "benzene ring or pyridine ring" of the "benzene ring or pyridine ring, each of which further has a substituent" for ring D is substituted by, for example, substituent(s) selected from the aforementioned substituent group A. The number of the substituents is, for example, 1 to 3. When the number of the substituents is two or more, the respective substituents may be the same or different.

Ring D is preferably a benzene ring substituted by 1 to 3 substituents selected from
(1) a C₁₋₆ alkoxy group (e.g., methoxy) optionally substituted by 1 to 5 halogen atoms (e.g., fluorine atom),
(2) a halogen atom (e.g., fluorine atom),
(3) a heterocyclic group (e.g., pyrazolyl), and
(4) a C₁₋₆ alkyl group (e.g., methyl) optionally substituted by 1 to 3 halogen atoms (e.g., fluorine atom).

Preferably, the substituent that the "benzene ring or pyridine ring" for ring D has is not a secondary amino group having a substituent. Here, examples of the substituent that the "secondary amino group" has include substituent(s) selected from the above-mentioned substituent group A.

Ring D is more preferably a benzene ring substituted by 1 to 3 substituents selected from
(1) a halogen atom (e.g., fluorine atom),
(2) a C₁₋₆ alkyl group (e.g., methyl) optionally substituted by 1 to 3 halogen atoms (e.g., fluorine atom), and
(3) a C₁₋₆ alkoxy group (e.g., methoxy)optionally substituted by 1 to 3 halogen atoms (e.g., fluorine atom).

Ring D is more preferably a benzene ring substituted by 1 to 3 substituents selected from a C₁₋₆ alkoxy group (e.g., methoxy) optionally substituted by 1 to 3 halogen atoms (e.g., fluorine atom).

X is a carbon atom or a nitrogen atom.
X is preferably a carbon atom.

L is a bond or an optionally substituted C₁₋₂ alkylene group.
L is preferably a bond or methylene.
L is more preferably a bond.

Y is
(1) a group represented by the formula:

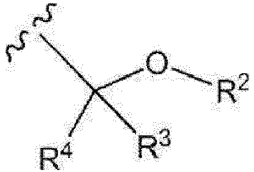

   wherein R² and R³ are each independently a hydrogen atom or a substituent, R⁴ is a substituent, or R³ and R⁴ optionally form, together with the adjacent carbon atom, an optionally further substituted ring; or
(2) a group represented by the formula -CH₂-O-R¹ wherein R¹ is a substituent.

The "ring" of the "optionally further substituted ring" formed by R³ and R⁴ together with the adjacent carbon atom includes a ring corresponding to a C₃₋₁₀ cycloalkyl group, a ring corresponding to a C₃₋₁₀ cycloalkenyl group, and a ring corresponding to a nonaromatic heterocyclic group.

The "ring" of the "optionally further substituted ring" formed by R³ and R⁴ together with the adjacent carbon atom may be further substituted by, for example, a substituent selected from the aforementioned substituent group A, and the number of the substituents is, for example, 1 to 3. When the number of the substituents is two or more, the respective substituents may be the same or different.

Y is preferably
(1) a group represented by the formula:

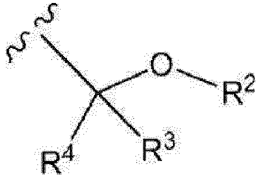

   wherein is a hydrogen atom;
   R³ is a C₁₋₆ alkyl group (e.g., methyl, ethyl); and
   R⁴ is a C₁₋₆ alkyl group (e.g., methyl, ethyl); or
(2) a group represented by the formula -CH₂-O-R¹ wherein R¹ is a C₁₋₆ alkyl group (e.g., methyl).

In another embodiment, preferably, the above-mentioned R³ and R⁴ are not optionally substituted (aromatic) rings (e.g., ring corresponding to C₆₋₁₄ aryl group, ring corresponding to aromatic heterocyclic group).

Furthermore, in another embodiment, preferably, the above-mentioned R³ and R⁴ are not carboxyl groups or derivatives thereof (e.g., acyl group), more preferably, they are not acyl groups.

Y is more preferably
(1) a group represented by the formula:

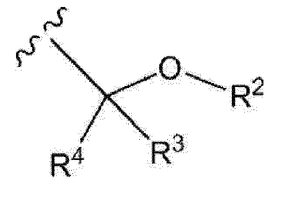

   wherein is a hydrogen atom;
   R³ is a C₁₋₆ alkyl group (e.g., methyl); and
   R⁴ is a C₁₋₆ alkyl group (e.g., methyl); or
(2) a group represented by the formula -CH₂-O-R¹ wherein R¹ is a C₁₋₆ alkyl group (e.g., methyl).

In another embodiment, Y is preferably
(1) a group represented by the formula: 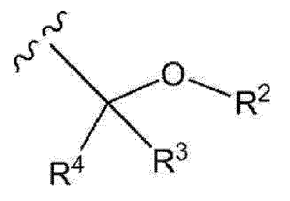
   wherein R² is a hydrogen atom or a C₁₋₆ alkyl-carbonyl group (e.g., acetyl);
   R³ is a C₁₋₆ alkyl group (e.g., methyl, ethyl); and
   R⁴ is a C₁₋₆ alkyl group (e.g., methyl, ethyl); or
(2) a group represented by the formula -CH₂-O-R¹ wherein R¹ is a C₁₋₆ alkyl group (e.g., methyl).

In another embodiment, preferably, the above-mentioned R³ and R⁴ are not optionally substituted (aromatic) rings (e.g., ring corresponding to C₆₋₁₄ aryl group, ring corresponding to aromatic heterocyclic group).

Furthermore, in another embodiment, preferably, the above-mentioned R³ and R⁴ are not carboxyl groups or derivatives thereof (e.g. acyl group), more preferably, they are not acyl groups.

Y is more preferably
a group represented by the formula: 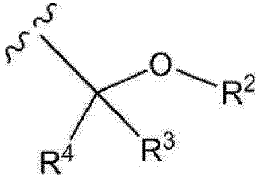 wherein
R² is a hydrogen atom;
R³ is a C₁₋₆ alkyl group (e.g., methyl); and
R⁴ is a C₁₋₆ alkyl group (e.g., methyl).

Preferable examples of compound (I) include the following compounds.

### [Compound A-1]

Compound (I) wherein ring A is a 5- or 6-membered nitrogen-containing heterocycle (e.g., pyrazole, pyridine, pyrimidine, dihydropyrazole) optionally substituted by 1 to 3 substituents selected from
(1) a hydroxy group, and
(2) a C₁₋₆ alkyl group (e.g., methyl);
   ring B is a 5- or 6-membered nitrogen-containing heterocycle (e.g., pyridine, pyrimidine, pyrazole, triazole (e.g., 1,2,4-triazole), dihydropyrimidine, imidazole) optionally substituted by 1 to 3 substituents selected from (1) a C₁₋₆ alkyl group (e.g., methyl, ethyl, isopropyl) optionally substituted by 1 to 3 C₁₋₆ alkoxy groups (e.g., methoxy),
   (2) a halogen atom (e.g., chlorine atom, bromine atom),
(3) a hydroxy group,
(4) a C₂₋₆ alkenyl group (e.g., vinyl, propa-1-en-2-yl),
(5) a C₃₋₈ cycloalkyl group (e.g., cyclopropyl),
(6) a mono- or a di-C₁₋₆ alkyl-amino group (e.g., dimethylamino, ethyl(methyl)amino),
(7) a heterocyclic group (e.g., azetidinyl, pyrrolidinyl, piperidyl, morpholinyl, 3-oxa-8-azabicyclo[3.2.1]octyl, 3-oxa-6-azabicyclo[3.1.1]heptyl, 6-oxa-3-azabicyclo[3.1.1]heptyl, pyrazolyl) optionally substituted by 1 to 3 substituents selected from
   (i) a C₁₋₆ alkoxy group (e.g., methoxy),
   (ii) a halogen atom (e.g., fluorine atom), and
   (iii) a C₁₋₆ alkyl group (e.g., methyl), and
(8) an oxo group;
   fused ring AB is
   (I) an aromatic heterocycle (e.g., naphthyridine (e.g., 1,5-naphthyridine, 1,6-naphthyridine), pyrazolo[1,5-a]pyrimidine, triazolo[1,5-a]pyrimidine (e.g., 1,2,4-triazolo[1,5-a]pyrimidine), imidazo[4,5-b]pyridine, purine, pyrazolo[4,3-c]pyridine) having 2 to 4 nitrogen atoms as ring-constituting atom besides carbon atom, which is optionally substituted by 1 to 3 substituents selected from
      (1) a hydroxy group,
      (2) a halogen atom (e.g., chlorine atom, bromine atom),
      (3) a C₁₋₆ alkyl group (e.g., methyl, ethyl, isopropyl),
      (4) a C₂₋₆ alkenyl group (e.g., vinyl, propa-1-en-2-yl),
      (5) a C₃₋₈ cycloalkyl group (e.g., cyclopropyl),
      (6) a mono- or a di- C₁₋₆ alkyl-amino group (e.g., dimethylamino, ethyl(methyl)amino), and
      (7) a heterocyclic group (e.g., azetidinyl, pyrrolidinyl, piperidyl, morpholinyl, 3-oxa-8-azabicyclo[3.2.1]octyl, 3-oxa-6-azabicyclo[3.1.1]heptyl, 6-oxa-3-azabicyclo[3.1.1]heptyl, pyrazolyl) optionally substituted by 1 to 3 substituents selected from
         (i) a C₁₋₆ alkoxy group (e.g., methoxy),
         (ii) a halogen atom (e.g., fluorine atom), and
         (iii) a C₁₋₆ alkyl group (e.g., methyl), or
   (II) a heterocycle having, as ring-constituting atom besides carbon atom, 2 to 4 nitrogen atoms (e.g., 1,7-dihydropyrazolo[1,5-a]pyrimidine) optionally substituted by 1 to 3 (preferably 3) substituents selected from
      (1) a C₁₋₆ alkyl group (e.g., methyl) optionally substituted by one C₁₋₆ alkoxy group (e.g., methoxy), and
      (2) a C₃₋₈ cycloalkyl group (e.g., cyclopropyl), and further having an oxo group to show aromaticity (e.g., 7-oxo-1,7-dihydropyrazolo[1,5-a]pyrimidine);
         ring D is a benzene ring substituted by 1 to 3 substituents selected from
         (1) a C₁₋₆ alkoxy group (e.g., methoxy) optionally substituted by 1 to 5 halogen atoms (e.g., fluorine atom),
         (2) a halogen atom (e.g., fluorine atom),
         (3) a heterocyclic group (e.g., pyrazolyl), and
         (4) a C₁₋₆ alkyl group (e.g., methyl) optionally substituted by 1 to 3 halogen atoms (e.g., fluorine atom);
            X is a carbon atom;
            L is a bond or methylene; and
            Y is
            (1) a group represented by the formula: 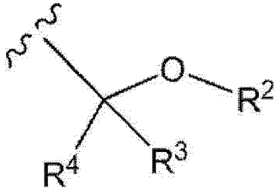
               wherein R² is a hydrogen atom;
               R³ is a C₁₋₆ alkyl group (e.g., methyl, ethyl); and
               R⁴ is a C₁₋₆ alkyl group (e.g., methyl, ethyl); or
            (2) a group represented by the formula -CH₂-O-R¹ wherein R¹ is a
               C₁₋₆ alkyl group (e.g., methyl).

### [Compound A-2]

Compound (I) wherein ring A is a 5- or 6-membered nitrogen-containing heterocycle (e.g., pyrazole, pyridine, pyrimidine, dihydropyrazole) optionally substituted by 1 to 3 substituents selected from
(1) a hydroxy group, and
(2) a C₁₋₆ alkyl group (e.g., methyl);
   ring B is a 5- or 6-membered nitrogen-containing heterocycle (e.g., pyridine, pyrimidine, pyrazole, triazole (e.g., 1,2,4-triazole), dihydropyrimidine, imidazole) optionally substituted by 1 to 3 substituents selected from (1) a C₁₋₆ alkyl group (e.g., methyl, ethyl, isopropyl) optionally substituted by 1 to 3 C₁₋₆ alkoxy groups (e.g., methoxy),
   (2) a halogen atom (e.g., chlorine atom, bromine atom),
(3) a hydroxy group,
(4) a C₂₋₆ alkenyl group (e.g., vinyl, propa-1-en-2-yl),
(5) a C₃₋₈ cycloalkyl group (e.g., cyclopropyl),
(6) a mono- or a di- C₁₋₆ alkyl-amino group (e.g., dimethylamino, ethyl(methyl)amino),
(7) a heterocyclic group (e.g., azetidinyl, pyrrolidinyl, piperidyl, morpholinyl, 3-oxa-8-azabicyclo[3.2.1]octyl, 3-oxa-6-azabicyclo[3.1.1]heptyl, 6-oxa-3-azabicyclo[3.1.1]heptyl, pyrazolyl, imidazolyl, triazolyl (e.g., 1,2,3-triazolyl), pyridyl, dihydropyridyl) optionally substituted by 1 to 3 substituents selected from
   (i) a C₁₋₆ alkoxy group (e.g., methoxy),
   (ii) a halogen atom (e.g., fluorine atom),
   (iii) a C₁₋₆ alkyl group (e.g., methyl) optionally substituted by 1 to 3 halogen atoms (e.g., fluorine atom), and
   (iv) an oxo group, and
(8) an oxo group;
   fused ring AB is
   (I) an aromatic heterocycle (e.g., naphthyridine (e.g., 1,5-naphthyridine, 1,6-naphthyridine), pyrazolo[1,5-a]pyrimidine, triazolo[1,5-a]pyrimidine (e.g., 1,2,4-triazolo[1,5-a]pyrimidine), imidazo[4,5-b]pyridine, purine, pyrazolo[4,3-c]pyridine) having 2 to 4 nitrogen atoms as ring-constituting atom besides carbon atom, which is optionally substituted by 1 to 3 substituents selected from
      (1) a hydroxy group,
      (2) a halogen atom (e.g., chlorine atom, bromine atom),
      (3) a C₁₋₆ alkyl group (e.g., methyl, ethyl, isopropyl),
      (4) a C₂₋₆ alkenyl group (e.g., vinyl, propa-1-en-2-yl),
      (5) a C₃₋₈ cycloalkyl group (e.g., cyclopropyl),
      (6) a mono- or a di- C₁₋₆ alkyl-amino group (e.g., dimethylamino, ethyl(methyl)amino), and
      (7) a heterocyclic group (e.g., azetidinyl, pyrrolidinyl, piperidyl, morpholinyl, 3-oxa-8-azabicyclo[3.2.1]octyl, 3-oxa-6-azabicyclo[3.1.1]heptyl, 6-oxa-3-azabicyclo[3.1.1]heptyl, pyrazolyl, imidazolyl, triazolyl (e.g., 1,2,3-triazolyl), pyridyl, dihydropyridyl) optionally substituted by 1 to 3 substituents selected from
         (i) a C₁₋₆ alkoxy group (e.g., methoxy),
         (ii) a halogen atom (e.g., fluorine atom),
         (iii) a C₁₋₆ alkyl group (e.g., methyl) optionally substituted by 1 to 3 halogen atoms (e.g., fluorine atom), and
         (iv) an oxo group, or
   (II) a heterocycle having, as ring-constituting atom besides carbon atom, 2 to 4 nitrogen atoms (e.g., 1,7-dihydropyrazolo[1,5-a]pyrimidine) optionally substituted by 1 to 3 (preferably 3) substituents selected from
      (1) a C₁₋₆ alkyl group (e.g., methyl) optionally substituted by one C₁₋₆ alkoxy group (e.g., methoxy),
      (2) a C₃₋₈ cycloalkyl group (e.g., cyclopropyl), and
      (3) a heterocyclic group (e.g., pyridyl), and further having an oxo group to show aromaticity (e.g., 7-oxo-1,7-dihydropyrazolo[1,5-a]pyrimidine);
         ring D is a benzene ring substituted by 1 to 3 substituents selected from
         (1) a C₁₋₆ alkoxy group (e.g., methoxy) optionally substituted by 1 to 5 halogen atoms (e.g., fluorine atom),
         (2) a halogen atom (e.g., fluorine atom),
         (3) a heterocyclic group (e.g., pyrazolyl), and
         (4) a C₁₋₆ alkyl group (e.g., methyl) optionally substituted by 1 to 3 halogen atoms (e.g., fluorine atom);
            X is a carbon atom;
            L is a bond or methylene; and
            Y is
            (1) a group represented by the formula: 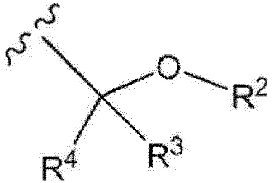
               wherein R² is a hydrogen atom;
               R³ is a C₁₋₆ alkyl group (e.g., methyl, ethyl); and
               R⁴ is a C₁₋₆ alkyl group (e.g., methyl, ethyl); or (2) a group represented by the formula -CH₂-O-R¹ wherein R¹ is a C₁₋₆ alkyl group (e.g., methyl).

### [Compound A-3]

Compound (I) wherein ring A is a 5- or 6-membered nitrogen-containing heterocycle (e.g., pyrazole, pyridine, pyrimidine, dihydropyrazole) optionally substituted by 1 to 3 substituents selected from
(1) a hydroxy group,
(2) a C₁₋₆ alkyl group (e.g., methyl),
(3) an amino group, and
(4) a halogen atom (e.g., chlorine atom); ring B is a 5- or 6-membered nitrogen-containing heterocycle (e.g., pyridine, pyrimidine, pyrazole, triazole (e.g., 1,2,4-triazole), dihydropyrimidine, imidazole) optionally substituted by 1 to 3 substituents selected from
   (1) a C₁₋₆ alkyl group (e.g., methyl, ethyl, isopropyl) optionally substituted by 1 to 3 C₁₋₆ alkoxy groups (e.g., methoxy),
   (2) a halogen atom (e.g., fluorine atom, chlorine atom, bromine atom),
   (3) a hydroxy group,
   (4) a C₂₋₆ alkenyl group (e.g., vinyl, propa-1-en-2-yl),
   (5) a C₃₋₈ cycloalkyl group (e.g., cyclopropyl),
   (6) a mono- or a di- C₁₋₆ alkyl-amino group (e.g., dimethylamino, ethyl(methyl)amino),
   (7) a heterocyclic group (e.g., azetidinyl, pyrrolidinyl, piperidyl, morpholinyl, 3-oxa-8-azabicyclo[3.2.1]octyl, 3-oxa-6-azabicyclo[3.1.1]heptyl, 6-oxa-3-azabicyclo[3.1.1]heptyl, pyrazolyl, imidazolyl, triazolyl (e.g., 1,2,3-triazolyl, 1,2,4-triazolyl), pyridyl, dihydropyridyl, pyridazinyl, pyrimidinyl, thiazolyl, tetrahydropyranyl, tetrazolyl, pyrazinyl, isoxazolyl) optionally substituted by 1 to 3 substituents selected from
      (i) a C₁₋₆ alkoxy group (e.g., methoxy),
      (ii) a halogen atom (e.g., fluorine atom, chlorine atom),
      (iii) a C₁₋₆ alkyl group (e.g., methyl) optionally substituted by 1 to 3 halogen atoms (e.g., fluorine atom), and
      (iv) an oxo group,
   (8) a C₆₋₁₄ aryl group optionally substituted by a cyano group (e.g., phenyl),
   (9) a C₁₋₆ alkoxy group (e.g., methoxy), and
   (10) an oxo group;
      fused ring AB is
      (I) an aromatic heterocycle (e.g., naphthyridine (e.g., 1,5-naphthyridine, 1,6-naphthyridine), pyrazolo[1,5-a]pyrimidine, triazolo[1,5-a]pyrimidine (e.g., 1,2,4-triazolo[1,5-a]pyrimidine), imidazo[4,5-b]pyridine, purine, pyrazolo[4,3-c]pyridine) having 2 to 4 nitrogen atoms as ring-constituting atom besides carbon atom, which is optionally substituted by 1 to 3 substituents selected from
         (1) a hydroxy group,
         (2) a halogen atom (e.g., fluorine atom, chlorine atom, bromine atom),
         (3) a C₁₋₆ alkyl group (e.g., methyl, ethyl, isopropyl),
         (4) a C₂₋₆ alkenyl group (e.g., vinyl, propa-1-en-2-yl),
         (5) a C₃₋₈ cycloalkyl group (e.g., cyclopropyl),
         (6) an amino group,
         (7) a mono- or a di- C₁₋₆ alkyl-amino group (e.g., dimethylamino, ethyl(methyl)amino),
         (8) a heterocyclic group (e.g., azetidinyl, pyrrolidinyl, piperidyl, morpholinyl, 3-oxa-8-azabicyclo[3.2.1]octyl, 3-oxa-6-azabicyclo[3.1.1]heptyl, 6-oxa-3-azabicyclo[3.1.1]heptyl, pyrazolyl, imidazolyl, triazolyl (e.g., 1,2,3-triazolyl, 1,2,4-triazolyl), pyridyl, dihydropyridyl, pyridazinyl, pyrimidinyl, thiazolyl, tetrahydropyranyl, tetrazolyl, pyrazinyl, isoxazolyl) optionally substituted by 1 to 3 substituents selected from
            (i) a C₁₋₆ alkoxy group (e.g., methoxy),
            (ii) a halogen atom (e.g., fluorine atom, chlorine atom),
            (iii) a C₁₋₆ alkyl group (e.g., methyl) optionally substituted by 1 to 3 halogen atoms (e.g., fluorine atom), and
            (iv) an oxo group,
         (9) a C₆₋₁₄ aryl group optionally substituted by a cyano group (e.g., phenyl), and
         (10) a C₁₋₆ alkoxy group (e.g., methoxy), or
      (II) a heterocycle having, as ring-constituting atom besides carbon atom, 2 to 4 nitrogen atoms (e.g., 1,7-dihydropyrazolo[1,5-a]pyrimidine) optionally substituted by 1 to 3 (preferably 3) substituents selected from
         (1) a C₁₋₆ alkyl group (e.g., methyl, ethyl, isopropyl) optionally substituted by one C₁₋₆ alkoxy group (e.g., methoxy),
         (2) a C₃₋₈ cycloalkyl group (e.g., cyclopropyl),
         (3) a heterocyclic group (e.g., pyridyl), and
         (4) a halogen atom (e.g., chlorine atom), and further having an oxo group to show aromaticity (e.g., 7-oxo-1,7-dihydropyrazolo[1,5-a]pyrimidine);
            ring D is a benzene ring substituted by 1 to 3 substituents selected from
            (1) a C₁₋₆ alkoxy group (e.g., methoxy) optionally substituted by 1 to 5 halogen atoms (e.g., fluorine atom),
            (2) a halogen atom (e.g., fluorine atom),
            (3) a heterocyclic group (e.g., pyrazolyl), and
            (4) a C₁₋₆ alkyl group (e.g., methyl) optionally substituted by 1 to 3 halogen atoms (e.g., fluorine atom);
               X is a carbon atom;
               L is a bond or methylene; and
               Y is
               (1) a group represented by the formula: 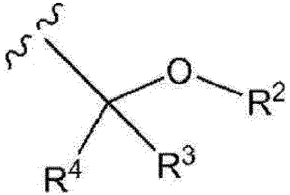
                  wherein R² is a hydrogen atom or a C₁₋₆ alkyl-carbonyl group (e.g., acetyl);
                  R³ is a C₁₋₆ alkyl group (e.g., methyl, ethyl); and
                  R⁴ is a C₁₋₆ alkyl group (e.g., methyl, ethyl); or
               (2) a group represented by the formula -CH₂-O-R¹ wherein R¹ is a C₁₋₆ alkyl group (e.g., methyl).

### [Compound A-4]

Compound (I) wherein ring A is a pyrazole ring, a pyridine ring, a pyrimidine ring, a dihydropyrazole ring, or a dihydropyridine ring, each of which is optionally substituted by 1 to 3 substituents selected from
(1) a hydroxy group,
(2) a C₁₋₆ alkyl group,
(3) a halogen atom, and
(4) an amino group;
   ring B is
   a pyridine ring,
   a pyrimidine ring,
   a pyrazole ring,
   a triazole ring,
   a dihydropyrimidine ring, or
   an imidazole ring, each of which is optionally substituted by 1 to 3 substituents selected from
   (1) a C₁₋₆ alkyl group optionally substituted by 1 to 3 C₁₋₆ alkoxy groups,
   (2) a halogen atom,
   (3) a hydroxy group,
   (4) a C₂₋₆ alkenyl group,
   (5) a C₃₋₈ cycloalkyl group,
   (6) a di- C₁₋₆ alkyl-amino group,
   (7)
      an azetidinyl group,
      a pyrrolidinyl group,
      a piperidyl group,
      a morpholinyl group,
      a 3-oxa-8-azabicyclo[3.2.1]octyl group,
      a 3-oxa-6-azabicyclo[3.1.1]heptyl group,
      a 6-oxa-3-azabicyclo[3.1.1]heptyl group,
      a pyrazolyl group,
      a triazolyl group,
      a pyridyl group,
      a pyridazinyl group,
      a thiazolyl group,
      an imidazolyl group,
      a tetrahydropyranyl group,
      a tetrazolyl group,
      a pyrazinyl group,
      an isoxazolyl group,
      a dihydropyridyl group, or
      a pyrimidinyl group, each of which is optionally substituted by 1 to 3 substituents selected from
      (i) a C₁₋₆ alkoxy group,
      (ii) a halogen atom,
      (iii) a C₁₋₆ alkyl group optionally substituted by 1 to 3 halogen atoms,
      (iv) a C₁₋₆ alkoxy group,
      (v) an oxo group, and
      (vi) a cyano group,
   (8) an oxo group,
   (9) a phenyl group optionally substituted by a cyano group, and
   (10) a C₁₋₆ alkoxy group;
      fused ring AB is
      (I)
         a 1,5-naphthyridine ring,
         a 1,6-naphthyridine ring,
         a pyrazolo[1,5-a]pyrimidine ring,
         a triazolo[1,5-a]pyrimidine ring,
         an imidazo[4,5-b]pyridine ring,
         a purine ring, or
         a pyrazolo[4,3-c]pyridine ring, each of which is optionally substituted by 1 to 3 substituents selected from
         (1) a hydroxy group,
         (2) a halogen atom,
         (3) a C₁₋₆ alkyl group,
         (4) a C₂₋₆ alkenyl group,
         (5) a C₃₋₈ cycloalkyl group,
         (6) an amino group,
         (7) a di- C₁₋₆ alkyl-amino group,
         (8)
            an azetidinyl group,
            a pyrrolidinyl group,
            a piperidyl group,
            a morpholinyl group,
            a 3-oxa-8-azabicyclo[3.2.1]octyl group,
            a 3-oxa-6-azabicyclo[3.1.1]heptyl group,
            a 6-oxa-3-azabicyclo[3.1.1]heptyl group,
            a pyrazolyl group,
            an imidazolyl group,
            a triazolyl group,
            a pyridyl group,
            a dihydropyridyl group,
            a pyridazinyl group,
            a pyrimidinyl group,
            a thiazolyl group,
            a tetrahydropyranyl group,
            a tetrazolyl group,
            a pyrazinyl group, or
            an isoxazolyl group,
            each of which is optionally substituted by 1 to 3 substituents selected from
            (i) a C₁₋₆ alkoxy group,
            (ii) a halogen atom,
            (iii) a C₁₋₆ alkyl group optionally substituted by 1 to 3 halogen atoms,
            (iv) a C₁₋₆ alkoxy group, and
            (v) an oxo group,
         (9) a phenyl group optionally substituted by a cyano group, and
         (10) a C₁₋₆ alkoxy group, or
      (II) a 7-oxo-1,7-dihydroa pyrazolo[1,5-a]pyrimidine ring optionally substituted by 1 to 3 substituents selected from
         (1) a C₁₋₆ alkyl group optionally substituted by one C₁₋₆ alkoxy group,
         (2) a C₃₋₈ cycloalkyl group,
         (3) a pyridyl group, and
         (4) a halogen atom;
            ring D is a benzene ring substituted by 1 to 3 substituents selected from
            (1) a C₁₋₆ alkoxy group optionally substituted by 1 to 3 halogen atoms,
            (2) a halogen atom,
            (3) a pyrazolyl group, and
            (4) a C₁₋₆ alkyl group optionally substituted by 1 to 3 halogen atoms;
               X is a carbon atom;
               L is a bond or methylene; and
               Y is
               (1) a group represented by the formula:

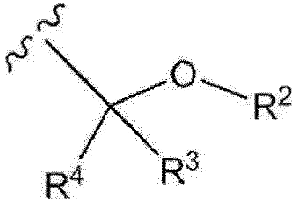

                  wherein R² is a hydrogen atom or a C₁₋₆ alkyl-carbonyl group; R³ is a C₁₋₆ alkyl group; and
                  R⁴ is a C₁₋₆ alkyl group, or
               (2) a group represented by the formula -CH₂-O-R¹ wherein R¹ is a C₁₋₆ alkyl group.

### [Compound A-5]

Compound (I) wherein
ring A is a pyrazole ring, a pyridine ring, or a dihydropyridine ring, each of which is optionally substituted by 1 to 3 substituents selected from
(1) a hydroxy group, and
(2) a C₁₋₆ alkyl group;
ring B is
a pyridine ring, or
a pyrimidine ring, each of which is optionally substituted by 1 to 3 substituents selected from
(1) a C₁₋₆ alkyl group,
(2) a C₃₋₈ cycloalkyl group,
(3) a di- C₁₋₆ alkyl-amino group,
(4)
   an azetidinyl group,
   a pyrrolidinyl group,
   a morpholinyl group,
   a pyrazolyl group,
   a triazolyl group,
   a pyridyl group, or
   an imidazolyl group, each of which is optionally substituted by 1 to 3 substituents selected from
   (i) a C₁₋₆ alkyl group optionally substituted by 1 to 3 halogen atoms, and
   (ii) an oxo group,
(5) an oxo group,
(6) a phenyl group optionally substituted by a cyano group, and
(7) a C₁₋₆ alkoxy group;
   fused ring AB is
   (I) a 1,5-naphthyridine ring,
      a 1,6-naphthyridine ring, or
      a pyrazolo[1,5-a]pyrimidine ring, each of which is optionally substituted by 1 to 3 substituents selected from
      (1) a hydroxy group,
      (2) a C₁₋₆ alkyl group,
      (3) a C₃₋₈ cycloalkyl group,
      (4) a di- C₁₋₆ alkyl-amino group,
      (5)
         an azetidinyl group,
         a pyrrolidinyl group,
         a morpholinyl group,
         a pyrazolyl group,
         an imidazolyl group,
         a triazolyl group, or
         a pyridyl group, each of which is optionally substituted by 1 to 3 substituents selected from
         (i) a C₁₋₆ alkyl group optionally substituted by 1 to 3 halogen atoms, and
         (ii) an oxo group,
(9) a phenyl group optionally substituted by a cyano group, and
(10) a C₁₋₆ alkoxy group, or
   (II) a 7-oxo-1,7-dihydroa pyrazolo[1,5-a]pyrimidine ring optionally substituted by 1 to 3 substituents selected from
      (1) a C₁₋₆ alkyl group,
      (2) a C₃₋₈ cycloalkyl group, and
      (3) a pyridyl group;
         ring D is a benzene ring substituted by 1 to 3 substituents selected from
         (1) a C₁₋₆ alkoxy group optionally substituted by 1 to 3 halogen atoms, and
         (2) a halogen atom;
            X is a carbon atom;
            L is a bond or methylene; and
            Y is
            (1) a group represented by the formula:

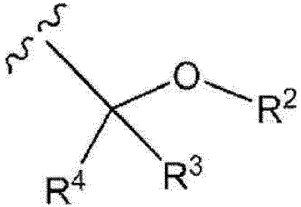

               wherein R² is a hydrogen atom;
               R³ is a C₁₋₆ alkyl group; and
               R⁴ is a C₁₋₆ alkyl group, or
            (2) a group represented by the formula -CH₂-O-R¹ wherein R¹ is a C₁₋₆ alkyl group.

### [Compound A-6]

Compound (I) wherein
ring A is a pyrazole ring;
ring B is a pyrimidine ring substituted by 1 to 3 substituents selected from
(1) a C₁₋₆ alkyl group,
(2) a triazolyl group optionally substituted by 1 to 3 C₁₋₆ alkyl groups, and
(3) a pyridyl group optionally substituted by 1 to 3 C₁₋₆ alkyl groups;
   fused ring AB is a pyrazolo[1,5-a]pyrimidine ring optionally substituted by 1 to 3 substituents selected from
   (1) a C₁₋₆ alkyl group,
   (2) a triazolyl group optionally substituted by 1 to 3 C₁₋₆ alkyl groups, and
   (3) a pyridyl group optionally substituted by 1 to 3 C₁₋₆ alkyl groups;
      ring D is a benzene ring substituted by a C₁₋₆ alkoxy group optionally substituted by 1 to 3 halogen atoms; X is a carbon atom;
      L is a bond; and
      Y is a group represented by the formula:

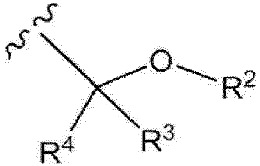

      wherein
      is a hydrogen atom;
      R³ is a C₁₋₆ alkyl group;
      R⁴ is a C₁₋₆ alkyl group.

### [Compound B-1]

Compound (I) wherein ring A is a 5- or 6-membered nitrogen-containing aromatic heterocycle (e.g., pyrazole, pyridine) optionally substituted by 1 to 3 hydroxy groups;
ring B is a 6-membered nitrogen-containing aromatic heterocycle (e.g., pyridine, pyrimidine) optionally substituted by 1 to 3 C₁₋₆ alkyl groups (e.g., methyl);
fused ring AB is an aromatic heterocycle (e.g., naphthyridine (e.g., 1,5-naphthyridine), pyrazolo[1,5-a]pyrimidine) having 2 or 3 nitrogen atoms as ring-constituting atom besides carbon atom, which is optionally substituted by 1 to 3 substituents selected from
(1) a hydroxy group, and
(2) a C₁₋₆ alkyl group (e.g., methyl);
   ring D is a benzene ring substituted by 1 to 3 substituents selected from
   (1) a halogen atom (e.g., fluorine atom),
   (2) a C₁₋₆ alkyl group (e.g., methyl) optionally substituted by 1 to 3 halogen atoms (e.g., fluorine atom), and
   (3) a C₁₋₆ alkoxy group (e.g., methoxy) optionally substituted by 1 to 3 halogen atoms (e.g., fluorine atom);
      X is a carbon atom;
      L is a bond; and
      Y is
      (1) a group represented by the formula:

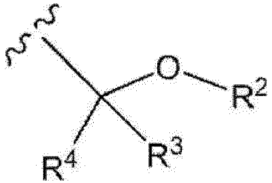

         wherein R² is a hydrogen atom;
         R³ is a C₁₋₆ alkyl group (e.g., methyl); and
         R⁴ is a C₁₋₆ alkyl group (e.g., methyl); or
      (2) a group represented by the formula -CH₂-O-R¹ wherein R¹ is a C₁₋₆ alkyl group (e.g., methyl).

### [Compound B-2]

Compound (I) wherein
ring A is a 5- or 6-membered nitrogen-containing aromatic heterocycle (e.g., pyrazole, pyridine) optionally substituted by 1 to 3 hydroxy groups;
ring B is a 6-membered nitrogen-containing aromatic heterocycle (e.g., pyridine, pyrimidine) optionally substituted by 1 to 3 substituents selected from
(1) a C₁₋₆ alkyl group (e.g., methyl), and
(2) a heterocyclic group (e.g., pyrazolyl, imidazolyl, triazolyl (e.g., 1,2,3-triazolyl), pyridyl) optionally substituted by 1 to 3 substituents selected from
   (i) a halogen atom (e.g., fluorine atom), and
   (ii) a C₁₋₆ alkyl group (e.g., methyl) optionally substituted by 1 to 3 halogen atoms (e.g., fluorine atom);
      fused ring AB is an aromatic heterocycle (e.g., naphthyridine (e.g., 1,5-naphthyridine), pyrazolo[1,5-a]pyrimidine) having 2 or 3 nitrogen atoms as ring-constituting atom besides carbon atom, which is optionally substituted by 1 to 3 substituents selected from
      (1) a hydroxy group,
      (2) a C₁₋₆ alkyl group (e.g., methyl), and
      (3) a heterocyclic group (e.g., pyrazolyl, imidazolyl, triazolyl (e.g., 1,2,3-triazolyl), pyridyl) optionally substituted by 1 to 3 substituents selected from
         (i) a halogen atom (e.g., fluorine atom), and
         (ii) a C₁₋₆ alkyl group (e.g., methyl) optionally substituted by 1 to 3 halogen atoms (e.g., fluorine atom);
            ring D is a benzene ring substituted by 1 to 3 substituents selected from
            (1) a halogen atom (e.g., fluorine atom),
            (2) a C₁₋₆ alkyl group (e.g., methyl) optionally substituted by 1 to 3 halogen atoms (e.g., fluorine atom), and
            (3) a C₁₋₆ alkoxy group (e.g., methoxy) optionally substituted by 1 to 3 halogen atoms (e.g., fluorine atom);
               X is a carbon atom;
               L is a bond; and
               Y is
               (1) a group represented by the formula:

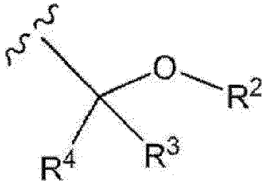

                  wherein R² is a hydrogen atom;
                  R³ is a C₁₋₆ alkyl group (e.g., methyl); and
                  R⁴ is a C₁₋₆ alkyl group (e.g., methyl); or
               (2) a group represented by the formula -CH₂-O-R¹ wherein R¹ is a C₁₋₆ alkyl group (e.g., methyl).

### [Compound B-3]

Compound (I) where in ring A is a 5- or 6-membered nitrogen-containing aromatic heterocycle (e.g., pyrazole);
ring B is a 6-membered nitrogen-containing aromatic heterocycle (e.g., pyrimidine) optionally substituted by 1 to 3 substituents selected from
(1) a C₁₋₆ alkyl group (e.g., methyl), and
(2) a heterocyclic group (e.g., triazolyl (e.g., 1,2,3-triazolyl, 1,2,4-triazolyl), pyridyl) optionally substituted by 1 to 3 substituents selected from a C₁₋₆ alkyl group (e.g., methyl);
   fused ring AB is an aromatic heterocycle (e.g., pyrazolo[1,5-a]pyrimidine) having 2 or 3 nitrogen atoms as ring-constituting atom besides carbon atom, which is optionally substituted by 1 to 3 substituents selected from
   (1) a C₁₋₆ alkyl group (e.g., methyl), and
   (2) a heterocyclic group (e.g., triazolyl (e.g., 1,2,3-triazolyl, 1,2,4-triazolyl), pyridyl) optionally substituted by 1 to 3 substituents selected from a C₁₋₆ alkyl group (e.g., methyl);
      ring D is a benzene ring substituted by 1 to 3 substituents selected from a C₁₋₆ alkoxy group (e.g., methoxy) optionally substituted by 1 to 3 halogen atoms (e.g., fluorine atom);
      X is a carbon atom;
      L is a bond; and
      Y is
      a group represented by the formula:

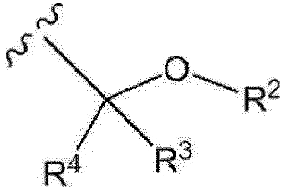

      wherein R² is a hydrogen atom;
      R³ is a C₁₋₆ alkyl group (e.g., methyl); and
      R⁴ is a C₁₋₆ alkyl group (e.g., methyl).

As specific examples of compound (I), for example, compounds of Examples 1 - 167 can be mentioned. Among those, N-((1S)-2-hydroxy-2-methyl-1-(4-(trifluoromethoxy)phenyl)propyl)-5-(6-methylpyridin-3-yl)pyrazolo[1,5-a]pyrimidine-3-carboxamide, N-((1S)-2-hydroxy-2-methyl-1-(4-(trifluoromethoxy)phenyl)propyl)-6-methyl-5-(4-methyl-1H-1,2,3-triazol-1-yl)pyrazolo[1,5-a]pyrimidine-3-carboxamide, and N-((1S)-2-hydroxy-2-methyl-1-(4-(trifluoromethoxy)phenyl)propyl)-6-methyl-5-(3-methyl-1H-1,2,4-triazol-1-yl)pyrazolo[1,5-a]pyrimidine-3-carboxamide, and salts thereof are preferable.

Examples of the salt of the compound represented by the formula (I) include metal salt, ammonium salt, salt with organic base, salt with inorganic acid, salt with organic acid, salt with basic or acidic amino acid, and the like.

Preferable examples of the metal salt include alkali metal salts such as sodium salt, potassium salt and the like; alkaline earth metal salts such as calcium salt, magnesium salt, barium salt and the like; an aluminum salt, and the like.

Preferable examples of the salt with organic base include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine and the like.

Preferable examples of the salt with inorganic acid include salts with hydrochloric acid, hydrobromic acid, nitric acid, sulfuric acid, phosphoric acid and the like.

Preferable examples of the salt with organic acid include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like.

Preferable examples of the salt with basic amino acid include salts with arginine, lysine, ornithine and the like, and preferable examples of the salt with acidic amino acid include salts with aspartic acid, glutamic acid and the like.

Of the above-mentioned salts, a pharmaceutically acceptable salt is preferable.

A prodrug of compound (I) means a compound which is converted to the compound (I) with a reaction due to an enzyme, an gastric acid, etc. under the physiological condition in the living body, that is, a compound which is converted to the compound (I) with oxidation, reduction, hydrolysis, etc. according to an enzyme; a compound which is converted to the compound (I) by hydrolysis etc. due to gastric acid, etc.

A prodrug for compound (I) may be a compound obtained by subjecting an amino group in compound (I) to an acylation, alkylation or phosphorylation (e.g., a compound obtained by subjecting an amino group in compound (I) to an eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation and tert-butylation, etc.); a compound obtained by subjecting a hydroxy group in compound (I) to an acylation, alkylation, phosphorylation or boration (e.g., a compound obtained by subjecting an hydroxy group in compound (I) to an acetylation, palmitoylation, propanoylation, pivaloylation, succinylation, fumarylation, alanylation, dimethylaminomethylcarbonylation, etc.); a compound obtained by subjecting a carboxyl group in compound (I) to an esterification or amidation (e.g., a compound obtained by subjecting a carboxyl group in compound (I) to C₁₋₆ alkyl esterification, phenyl esterification, carboxymethyl esterification, dimethylaminomethyl esterification, pivaloyloxymethyl esterification, ethoxycarbonyloxyethyl esterification, phthalidyl esterification, (5-methyl-2-oxo-1,3-dioxolen-4-yl)methyl esterification, cyclohexyloxycarbonylethyl esterification and methylamidation, etc.) and the like. Of these, compound (I) wherein a carboxyl group is esterified with C₁₋₆ alkyl such as methyl, ethyl, tert-butyl and the like is preferably used. These compounds can be produced from compound (I) by a method known *per se.*

The prodrug of compound (I) may be a compound that converts to compound (I) under physiological conditions as described in Development of Pharmaceutical Products, vol. 7, Molecule Design, 163-198, Hirokawa Shoten (1990).

Unless otherwise specified, each symbol in the compounds in the following reaction schemes is as defined above. Each compound described in the reaction schemes may form a salt as long as it does not inhibit the reaction. Examples of such salt include those similar to the salts of compound (I).

While the compound obtained in each step can be directly used as a crude product in the form of a reaction mixture for the next reaction, it can also be isolated from the reaction mixture according to a conventional method, and further purified with ease by a separation means such as recrystallization, distillation, chromatography and the like.

The production methods of the compound of the present invention are described in the following.

Compound (I) can be produced by a method known per se, for example, the production methods shown in reaction scheme 1 to reaction scheme 11 or a method analogous thereto. In each production method below, each starting compound used for the production of compound (I) may form a salt. Examples of such salt include those similar to the salts of compound (I).

Each starting compound to be used for the production of compound (I) can be directly used as a crude product in the form of a reaction mixture for the next reaction. The compound can also be isolated from the reaction mixture according to a conventional method, and can be purified by a means known per se such as extraction, concentration, neutralization, filtration, distillation, recrystallization, chromatography and the like. Examples of the solvent to be used for the above-mentioned recrystallization include water, alcohols, ethers, hydrocarbons, amides, hydrocarbon halides, nitriles, ketones, esters, sulfoxides, organic acids and the like. These solvents may be used alone or two or more kinds of solvents may be mixed at a suitable ratio, for example, at a ratio of 1:1 - 1:10. In addition, as a compound in the formula, a commercially available product can be used as it is, or a compound produced by a method known per se or a method analogous thereto can also be used.

When compound (I) and a production intermediate of compound (I) have a convertible functional group (e.g., carboxyl group, amino group, hydroxy group, carbonyl group, mercapto group, C₁₋₆ alkoxy-carbonyl group, C₆₋₁₄ aryloxy-carbonyl group, C₇₋₁₆ aralkyloxy-carbonyl group, sulfo group, a halogen atom, optionally halogenated C₁₋₆ alkylsulfonyloxy group, cyano group, aminocarbonyl group, boryl group etc.), various compounds can be produced by converting these functional groups by a method known per se or a method analogous thereto.

The carboxyl group can be converted by reactions such as esterification, reduction, amidation, conversion reaction to an optionally protected amino group and the like.

The amino group can be converted by reactions such as amidation, sulfonylation, nitrosation, alkylation, arylation, imidation and the like.

The hydroxy group can be converted by reactions such as esterification, carbamoylation, sulfonylation, alkylation, fluorination, arylation, oxidation, halogenation and the like.

The carbonyl group can be converted by reactions such as reduction, oxidation, fluorination, imination (including oximation, hydrazonation), (thio)ketalization, alkylidenation, thiocarbonylation and the like.

The mercapto group can be converted by reactions such as alkylation, oxidation and the like.

The C₁₋₆ alkoxy-carbonyl group, C₆₋₁₄ aryloxy-carbonyl group and C₇₋₁₆ aralkyloxy-carbonyl group can be converted by reactions such as reduction, hydrolysis and the like.

The sulfo group can be converted by reactions such as sulfonamidation, reduction and the like.

The halogen atom can be converted by reactions such as various nucleophilic substitution reactions, various coupling reactions and the like.

The optionally halogenated C₁₋₆ alkylsulfonyloxy group can be converted by reactions such as various nucleophilic substitution reactions, various coupling reactions and the like.

The cyano group can be converted by reactions such as reduction, hydrolysis and the like.

The aminocarbonyl group can be converted by reactions such as dehydrating, reduction and the like.

The boryl group can be converted by reactions such as oxidation, various coupling reactions and the like.

In the aforementioned respective reactions, when a compound is obtained in a free form, it may be converted to a salt according to a conventional method. When a compound is obtained as a salt, it can be converted to a free form or other salt according to a conventional method.

These functional groups can be converted according to a method known per se, for example, the method described in Comprehensive Organic Transformations, Second Edition, Wiley-VCH, Richard C. Larock, and the like.

In each reaction of the production method of compound (I) and each reaction of synthesis of the starting compound, when starting compound has an amino group, a carboxyl group, a hydroxy group, carbonyl group, a carbonyl group or a mercapto group as a substituent, a protecting group generally used in peptide chemistry and the like may be introduced into these groups. The objective compound can be obtained by removing the protecting group as necessary after the reaction.

As the amino-protecting group, for example, a formyl group, and a C₁₋₆ alkyl-carbonyl group (e.g., acetyl, ethylcarbonyl etc.), a phenylcarbonyl group, a C₁₋₆ alkyloxycarbonyl group (e.g., methoxycarbonyl, ethoxycarbonyl, tert-butoxycarbonyl (Boc) etc.), an allyloxycarbonyl (Alloc) group, a phenyloxycarbonyl group, a fluorenylmethoxycarbonyl (Fmoc) group, a C₇₋₁₀ aralkyl-carbonyl group (e.g., benzylcarbonyl etc.), a C₇₋₁₀ aralkyl-oxycarbonyl group (e.g., benzyloxycarbonyl (Z) etc.), a C₇₋₁₀ aralkyl group (e.g., benzyl etc.), a 2-(trimethylsilyl)ethoxymethyl (SEM) group, a trityl group, a phthaloyl group, a N,N-dimethylaminomethylene group, an allyl group, a tert-butyl group and the like, each of which optionally has substituent(s), are used. As these substituents, a phenyl group, a halogen atom (e.g., fluorine, chlorine, bromine, iodine etc.), a C₁₋₆ alkyl-carbonyl group (e.g., methylcarbonyl, ethylcarbonyl, butylcarbonyl etc.), a nitro group and the like are used. The number of the substituents is about 1 to 3.

As the carboxyl-protecting group, for example, a C₁₋₆ alkyl group (e.g., methyl, ethyl, n-propyl, isopropyl, n-butyl, tert-butyl etc.), an allyl group, a benzyl group, a phenyl group, a trityl group, a trialkylsilyl group, and the like, each of which optionally have substituent(s), are used. As these substituents, a halogen atom (e.g., fluorine, chlorine, bromine, iodine etc.), a formyl group, a C₁₋₆ alkyl-carbonyl group (e.g., acetyl, ethylcarbonyl, butylcarbonyl etc.), a nitro group and the like are used. The number of the substituents is about 1 to 3.

As the protecting group of the hydroxy group, for example, a C₁₋₆ alkyl group (e.g., methyl, ethyl, n-propyl, isopropyl, n-butyl, tert-butyl etc.), a C₇₋₁₀ aralkyl group (e.g., benzyl etc.), a formyl group, a C₁₋₆ alkyl-carbonyl group (e.g., acetyl, ethylcarbonyl etc.), a benzoyl group, a C₇₋₁₀ aralkyl-carbonyl group (e.g., benzylcarbonyl etc.), a tetrahydropyranyl group, a furanyl group, a silyl group and the like, each of which optionally has substituent(s), are used. As these substituents, a halogen atom (e.g., fluorine, chlorine, bromine, iodine etc.), a C₁₋₆ alkyl group (e.g., methyl, ethyl, n-propyl etc.), a phenyl group, a C₇₋₁₀ aralkyl group (e.g., benzyl etc.), a C₁₋₆ alkoxy group (e.g., methoxy, ethoxy, n-propoxy etc.), a nitro group and the like are used. The number of the substituents is about 1 to 4.

Examples of the protected carbonyl group include cyclic acetal (e.g., 1,3-dioxane), non-cyclic acetal (e.g., di-C₁₋₆ alkylacetal) and the like.

Examples of the mercapto-protecting group include a C₁₋₆ alkyl group, a phenyl group, a trityl group, a C₇₋₁₀ aralkyl group (e.g., benzyl), a C₁₋₆ alkyl-carbonyl group, a benzoyl group, a C₇₋₁₀ aralkyl-carbonyl group (e.g., benzylcarbonyl), a C₁₋₆ alkoxy-carbonyl group, a C₆₋₁₄ aryloxy-carbonyl group (e.g., phenyloxycarbonyl), a C₇₋₁₄ aralkyloxy-carbonyl group (e.g., benzyloxycarbonyl, 9-fluorenylmethoxycarbonyl), a 2-tetrahydropyranyl group, a C₁₋₆ alkylamino-carbonyl group (e.g., methylaminocarbonyl, ethylaminocarbonyl) and the like. These protecting groups are optionally substituted by 1 to 3 substituents selected from a halogen atom, a C₁₋₆ alkyl group, a C₁₋₆ alkoxy group or a nitro group.

These protecting groups can be introduced or removed by a method known per se, for example, the method described in Greene's Protective Groups in Organic Synthesis, 4th Edition, Wiley-Interscience, Theodora W. Greene, Peter G. M. Wuts, and the like. To be specific, a method using acid, base, ultraviolet rays, hydrazine, phenylhydrazine, sodium N-methyldithiocarbamate, tetrabutylammonium fluoride, palladium acetate, trialkylsilyl halide (e.g., trimethylsilyl iodide, trimethylsilyl bromide) and the like, a reduction method, and the like can be mentioned.

When compound (I) is present as a configurational isomer, a diastereomer, a conformer and the like, each of them can be isolated by a known means. When compound (I) contains an optical isomer, an optically active form ((+) form, (-) form) can be obtained by resolving the racemate by a general means for optical re solution.

When compound (I) contains an optical isomer, a stereoisomer, a regioisomer, a rotamer or a tautomer, these are also encompassed in compound (I), and can be obtained as a single product according to a synthesis method and separation method known per se.

For example, the method of optical re solution may be a method known per se, such as a fractional recrystallization method, a chiral column method, a diastereomer method etc.

### 1) Fractional recrystallization method

A method wherein a salt of a racemate with an optically active compound (e.g., (+)-mandelic acid, (-)-mandelic acid, (+)-tartaric acid, (-)-tartaric acid, (+)-1-phenethylamine, (-)-1-phenethylamine, cinchonine, (-)-cinchonidine, brucine etc.) is formed, which is separated by a fractional recrystallization method, and if desired, a neutralization step to give a free optical isomer.

### 2) Chiral column method

A method wherein a racemate or a salt thereof is applied to a column (a chiral column) for separation of an optical isomer to allow separation. In the case of a liquid chromatography, for example, a mixture of the optical isomers is applied to a chiral column such as ENALTIO-OVM (manufactured by Tosoh Corporation), CHIRAL series (manufactured by Daicel Chemical Industries, Ltd.) and the like, and developed with water, various buffers (e.g., phosphate buffer, etc.) and organic solvents (e.g., ethanol, methanol, isopropanol, acetonitrile, trifluoroacetic acid, diethylamine, etc.) as an eluent, solely or in admixture to separate the optical isomer. In the case of a gas chromatography, for example, a chiral column such as CP-Chirasil-DeX CB (manufactured by GL Sciences Inc.) and the like is used to allow separation.

### 3) Diastereomer method

A method wherein a racemic mixture is prepared into a diastereomeric mixture by chemical reaction with an optically active reagent, which is made into a single substance by a typical separation means (e.g., a fractional recrystallization, a chromatography method etc.) and the like, and is subjected to a chemical treatment such as hydrolysis reaction and the like to separate an optically active reagent moiety, whereby an optical isomer is obtained. For example, when compound (I) contains hydroxy group or primary or secondary amino group in a molecule, the compound and an optically active organic acid (e.g., MTPA [α-methoxy-α-(trifluoromethyl)phenylacetic acid], (-)-menthoxyacetic acid etc.) and the like are subjected to condensation reaction to give diastereomers of the ester compound or the amide compound, respectively. When compound (I) has a carboxyl group, the compound and an optically active amine or an optically active alcohol reagent are subjected to condensation reaction to give diastereomers of the amide compound or the ester compound, respectively. The separated diastereomer is converted to an optical isomer of the original compound by acid hydrolysis or base hydrolysis reaction.

The solvent, acid and base mentioned in the production methods of the compound of the present invention are explained below.

As the "solvent", for example, "alcohols", "ethers", "hydrocarbons", "amides", "hydrocarbon halide", "nitriles", "ketones", "esters", "sulfoxides", "water" and the like can be used.

Examples of the "alcohols" include methanol, ethanol, 1-propanol, 2-propanol, tert-butyl alcohol and the like.

Examples of the "ethers" include diethyl ether, diisopropyl ether, diphenyl ether, tetrahydrofuran, 1,4-dioxane, 1,2-dimethoxyethane, tert-butyl methyl ether and the like.

Examples of the "hydrocarbons" include "aromatic hydrocarbons" and "saturated hydrocarbons". Examples of the "aromatic hydrocarbons" include benzene, toluene and the like. Examples of the "saturated hydrocarbons" include cyclohexane, hexane, petroleum ether and the like.

Examples of the "amides" include N,N-dimethylformamide, N,N-dimethylacetamide, N-methylpyrrolidone, hexamethylphosphoric triamide and the like.

Examples of the "hydrocarbon halide" include dichloromethane, chloroform, carbon tetrachloride, 1,2-dichloroethane, chlorobenzene, benzotrifluoride and the like.

Examples of the "nitriles" include acetonitrile, propionitrile and the like.

Examples of the "ketones" include acetone, ethyl methyl ketone and the like.

Examples of the "esters" include ethyl acetate, tert-butyl acetate and the like.

Examples of the "sulfoxides" include dimethyl sulfoxide and the like.

Examples of the "acid" include "organic acids", "mineral acids", "Lewis acids" and the like.

Examples of the "organic acids" include formic acid, acetic acid, propionic acid, trifluoroacetic acid, citric acid, methanesulfonic acid, p-toluenesulfonic acid and the like.

Examples of the "mineral acids" include hydrochloric acid, sulfuric acid and the like.

Examples of the "Lewis acids" include boron trichloride, boron tribromide and the like.

Examples of the "base" include "inorganic bases", "basic salts", "aromatic amines", "tertiary amines", "alkali metal hydrides", "alkali metals", "metal amides", "alkyl metals", "aryl metals", "metal alkoxides" and the like.

Examples of the "inorganic bases" include sodium hydroxide, potassium hydroxide, lithium hydroxide, barium hydroxide and the like.

Examples of the "basic salts" include sodium carbonate, potassium carbonate, cesium carbonate, sodium hydrogen carbonate, sodium acetate, tripotassium phosphate, ammonium acetate and the like.

Examples of the "aromatic amines" include pyridine, 2,6-lutidine and the like.

Examples of the "tertiary amines" include triethylamine, tripropylamine, tributylamine, diisopropylethylamine, cyclohexyldimethylamine, 4-dimethylaminopyridine, N,N-dimethylaniline, N-methylpiperidine, N-methylpyrrolidine, N-methylmorpholine, 1,8-diazabicyclo[5,4,0]undec-7-ene and the like.

Examples of the "alkali metal hydrides" include sodium hydride, potassium hydride and the like.

Examples of the "alkali metals" include sodium, lithium, potassium and the like.

Examples of the "metal amides" include sodium amide, lithium diisopropylamide, lithium hexamethyl disilazide and the like.

Examples of the "alkyl metals" include butyllithium, sec-butyllithium, tert-butyllithium and the like.

Examples of the "aryl metals" include phenyllithium and the like.

Examples of the "metal alkoxides" include sodium methoxide, sodium ethoxide, sodium tert-butoxide, potassium tert-butoxide and the like.

### Reaction scheme 1

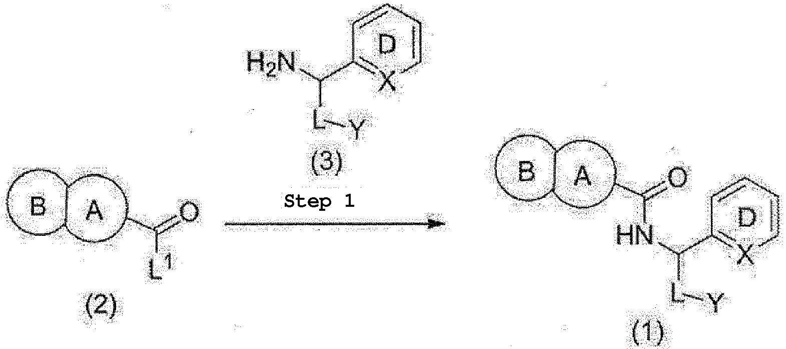

wherein L¹ is a leaving group, and other symbols are as defined above.

Compound (2) can be obtained as a commercially available product, or can be produced by a method known per se or a method analogous thereto.

Examples of the leaving group for L¹ include a hydroxy group, a halogen atom, an optionally halogenated C₁₋₆ alkylsulfonyloxy group, an optionally halogenated C₁₋₆ alkoxy group, an optionally substituted aryloxy group, an optionally substituted aralkyloxy group, a 1-1H-imidazolyl group and the like, with preference given to a hydroxy group, a halogen atom and a C₁₋₂ alkoxy group.

### <Step 1>

Compound (1) can be produced by a condensation reaction of compound (2) and compound (3).

### <Step 1-1>

Condensation of compound (2) wherein L¹ is a hydroxy group and compound (3) is performed in the presence of a condensing agent in a solvent that does not adversely influence the reaction. Examples of the condensing agent include generally known condensing agents such as carbodiimide condensation reagent (e.g., dicyclohexylcarbodiimide (DCC), diisopropylcarbodiimide (DIC), N-ethyl-N'-3-dimethylaminopropylcarbodiimide and hydrochloride thereof (WSC, WSC-HCl, EDCI) and the like), phosphoric acid condensation reagents (e.g., benzotriazol-1-yloxy-tris(dimethylamino)phosphonium hexafluorophosphate (BOP), diphenylphosphoryl azide (DPPA) and the like), N,N'-carbonyldiimidazole, 2-methyl-6-nitrobenzoic anhydride, 2-chloro-1,3-dimethylimidazolium tetrafluoroborate, 2-chloro-1-methyl pyridinium, N,N-dimethylsulfamoyl chloride, 2-chloro-4,6-dimethoxytriazine, 4-(4,6-dimethoxy-1,3,5-triazin-2-yl)-4-methyl-morpholinium chloride and the like.

When the carbodiimide condensation reagent, 2-methyl-6-nitrobenzoic anhydride and the like are used as the condensing agent, the reaction efficiency can be improved by using, where necessary, a suitable condensation promoter (e.g., 1-hydroxy-7-azabenzotriazole, 1-hydroxybenzotriazole, N-hydroxysuccinimide, N-hydroxyphthalimide, 4-dimethylaminopyridine etc.). When the phosphoric acid condensation reagent (e.g., benzotriazol-1-yloxy-tris(dimethylamino)phosphonium hexafluorophosphate (BOP), diphenylphosphoryl azide (DPPA) and the like), 2-methyl-6-nitrobenzoic anhydride and the like are used as the condensing agent, the reaction efficiency can be generally improved by adding an organic amine base such as triethylamine, diisopropylethylamine and the like.

When compound (3) forms a salt, this reaction is generally performed by adding a base. Examples of such base include tertiary amines, basic salts, metal hydride complex compounds and the like. These bases are generally used in an amount of about 1 - 100 mol, preferably about 1 - 10 mol, per 1 mol of compound (3).

Compound (3) is generally used in an amount of about 0.1 - 10 mol, preferably about 0.5 - 2 mol, per 1 mol of compound (2). The amount of the condensing agent to be used is generally about 0.1 - 10 mol, preferably about 1 - 3 mol, per 1 mol of compound (2). The amounts of the above-mentioned condensation promoter and the base to be used are generally about 0.1 - 10 mol, preferably about 0.3 - 3 mol, per 1 mol of compound (2).

This reaction is advantageously performed in a solvent inert to the reaction. While such solvent is not particularly limited as long as the reaction proceeds, for example, solvents such as hydrocarbon halides, amides, sulfoxides, ethers, nitriles, esters, hydrocarbons, water and the like, a mixed solvent thereof and the like are preferable.

While the reaction time varies depending on the reagents and solvents to be used, it is generally about 0.1 - 48 hr, preferably about 0.5 - 24 hr.

The reaction temperature is generally about 0 - 200°C, preferably about 20 - 100°C.

### <Step 1-2>

Condensation of compound (2) wherein L¹ is a leaving group other than a hydroxy group and compound (3) and preferably condensation of compound (2) wherein L¹ is a leaving group other than a C₁₋₂ alkoxy group and compound (3) are performed in a solvent that does not adversely influence the reaction and by adding a base as necessary.

Examples of the base to be used here include tertiary amines, aromatic amines, basic salts and the like. The amount of the bases to be used is generally about 0.1 - 10 mol, preferably about 0.3 - 3 mol, per 1 mol of compound (2).

This reaction is advantageously performed in a solvent inert to the reaction. While such solvent is not particularly limited as long as the reaction proceeds, for example, solvents such as hydrocarbon halide, amides, ethers, nitriles, esters, hydrocarbons, water and the like, a mixed solvent thereof and the like are preferable.

While the reaction time varies depending on the reagents and solvents to be used, it is generally about 0.1 - 48 hr, preferably about 0.5 - 24 hr.

The reaction temperature is generally about -40 to 200°C, preferably about 0 - 100°C.

### <Step 1-3>

Condensation of compound (2) wherein L¹ is a leaving group other than a hydroxy group and compound (3) and preferably condensation of compound (2) wherein L¹ is a C₁₋₂ alkoxy and compound (3) are performed in the presence of an organic aluminum reagent in a solvent that does not adversely influence the reaction. Examples of the organic aluminum reagent include generally known reagents such as trimethylaluminum, triethylaluminum, dimethylaluminum chloride, diisobutylaluminum hydride and the like.

The amount of the organic aluminum reagent to be used is generally about 0.1 - 10 mol, preferably about 1 - 3 mol, per 1 mol of compound (2).

For this reaction, a suitable activator such as hydrochloric acid and the like can be used as necessary.

When compound (3) forms a salt such as hydrochloride and the like, this reaction can be performed by adding a base to suppressive action of an activator such as hydrochloric acid and the like. Examples of such base include tertiary amines, basic salts, metal hydride complex compounds and the like. These activators and bases are generally used in an amount of about 1 - 100 mol, preferably about 1 - 10 mol, per 1 mol of compound (3).

This reaction is advantageously performed in a solvent inert to the reaction. While such solvent is not particularly limited as long as the reaction proceeds, for example, solvents such as hydrocarbon halide, hydrocarbons and the like, a mixed solvent thereof and the like are preferable.

While the reaction time varies depending on the reagents and solvents to be used, it is generally about 0.1 - 48 hr, preferably about 0.5 - 24 hr.

The reaction temperature is generally about 0 - 200°C, preferably about 20 - 100°C.

Compound (17) which is compound (3) in reaction scheme 1 wherein -L-Y is -C(R³)(R⁴)-OH, for example, can be produced by a method shown in reaction scheme 2 or a method analogous thereto.

### Reaction scheme 2

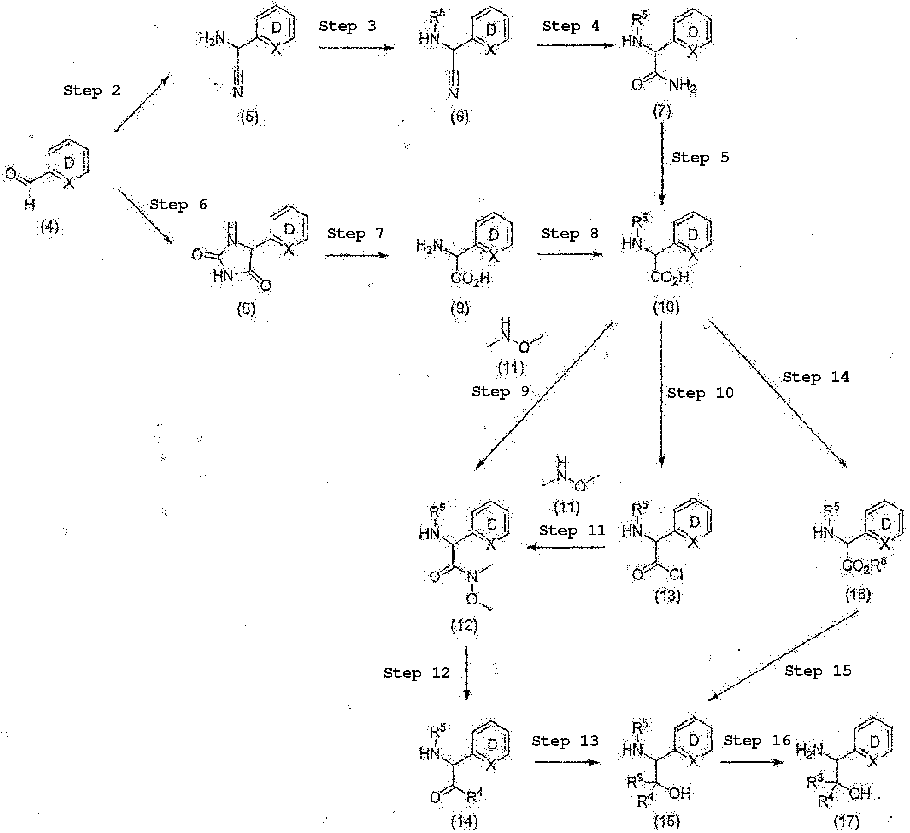

wherein R⁵ is an amino-protecting group, R⁶ is a carboxyl-protecting group, and other symbols are as defined above.

Compound (4) can be obtained as a commercially available product, or can be produced by a method known per se or a method analogous thereto, and compound (11) can be obtained as a commercially available product.

### <Step 2>

Compound (5) can be produced by subjecting compound (4) to the Strecker reaction.

This reaction generally condenses compound (4) and ammonia or an equivalent thereof, and hydrogen cyanide or an equivalent thereof to lead the corresponding α-aminonitrile (5).

Examples of the equivalent of ammonia include ammonium chloride, ammonium carbonate, benzylamine and the like. Ammonia or an equivalent thereof is generally used in an amount of about 1 - 50 mol, preferably about 1 - 10 mol, per 1 mol of compound (4).

Examples of the equivalent of hydrogen cyanide include sodium cyanide, potassium cyanide, trimethylsilyl cyanide and the like. Hydrogen cyanide or an equivalent thereof is generally used in an amount of about 1 - 50 mol, preferably about 1 - 10 mol, per 1 mol of compound (4).

This reaction may be performed by adding a Lewis acid such as titanium (IV) tetraisopropoxide and the like as necessary. The Lewis acid is generally used in an amount of about 0.05 - 50 mol, preferably about 0.1 - 10 mol, per 1 mol of compound (4).

This reaction is advantageously performed in a solvent inert to the reaction. While such solvent is not particularly limited as long as the reaction proceeds and, for example, solvents such as alcohols, hydrocarbon halide, ethers, hydrocarbons, nitriles, water and the like, a mixed solvent thereof and the like are preferable.

While the reaction time varies depending on the reagents and solvents to be used, it is generally about 0.1 - 100 hr, preferably about 0.5 - 48 hr.

The reaction temperature is generally about -78 to 200°C, preferably about -78 to 100°C.

### <Step 3>

Compound (6) can be produced by protecting amino group of compound (5).

Examples of such protecting group include a tert-butoxycarbonyl (Boc) group, a benzyloxycarbonyl (Z) group and the like.

Introduction of a protecting group in this step can be performed by a method known per se, for example, the method described in Greene's Protective Groups in Organic Synthesis, 4th Edition, Wiley-Interscience, Theodora W. Greene, Peter G. M. Wuts and the like.

### <Step 4>

Compound (7) can be produced from compound (6).

This reaction can be performed by a method known per se, for example, the method described in Synthesis, vol. 12, pp. 949-950, 1989, or a method analogous thereto. For example, a reaction using potassium carbonate and aqueous hydrogen peroxide and the like can be mentioned.

This reaction is advantageously performed in a solvent inert to the reaction. While such solvent is not particularly limited as long as the reaction proceeds, for example, solvents such as sulfoxides, alcohols, water and the like, a mixed solvent thereof and the like are preferable.

While the reaction time varies depending on the reagents and solvents to be used, it is generally about 0.1 - 100 hr, preferably about 0.5 - 36 hr.

The reaction temperature is generally about -30 to 150°C, preferably about 0 - 120°C.

### <Step 5>

Compound (10) can be produced by subjecting compound (7) to a hydrolysis reaction.

This reaction is performed according to a conventional method and using an acid or a base.

Examples of the acid include mineral acids, organic acids and the like. Examples of the base include inorganic bases, basic salts and the like. These acids and bases are generally used in an amount of about 0.5 - 100 mol, preferably about 1 - 20 mol, per 1 mol of compound (7).

This reaction is advantageously performed in a solvent inert to the reaction. While such solvent is not particularly limited as long as the reaction proceeds, for example, solvents such as alcohols, ethers, water and the like, a mixed solvent thereof and the like are preferable.

While the reaction time varies depending on the reagents and solvents to be used, it is generally about 0.1 - 100 hr, preferably about 0.1 - 48 hr.

The reaction temperature is generally about -10 to 200°C, preferably about 0 - 150°C.

### <Step 6>

Compound (8) can be produced from compound (4).

This reaction can be performed by a method known per se, for example, the method described in ORGANIC PREPARATIONS AND PROCEDURES INT., vol. 36, pp. 391-443, 2004, or a method analogous thereto.

For example, a method using ammonium carbonate and potassium cyanide and the like can be mentioned.

The ammonium carbonate to be used in this reaction is generally used in an amount of about 0.5 - 50 mol, preferably about 1 - 10 mol, per 1 mol of compound (4). Potassium cyanide to be used in this reaction is generally used in an amount of about 1 - 50 mol, preferably about 1 - 5 mol, per 1 mol of compound (4).

This reaction is advantageously performed in a solvent inert to the reaction. While such solvent is not particularly limited as long as the reaction proceeds, for example, solvents such as alcohols, water and the like, a mixed solvent thereof and the like are preferable.

While the reaction time varies depending on the reagents and solvents to be used, it is generally about 0.5 - 72 hr, preferably about 0.5 - 12 hr.

The reaction temperature is generally about 0 - 150°C, preferably about 20 - 100°C.

### <Step 7>

Compound (9) can be produced by subjecting compound (8) to a hydrolysis reaction.

This reaction is performed in the same manner as in step 5.

### <Step 8>

Compound (10) can be produced by protecting the amino group of compound (9).

This reaction is performed in the same manner as in step 3.

### <Step 9>

Compound (12) can be produced by reacting compound (10) with compound (11).
(i) This reaction is performed in the presence of a condensing agent in a solvent inert to the reaction. Examples of the condensing agent include generally known condensing agents such as carbodiimide condensation reagent (e.g., dicyclohexylcarbodiimide (DCC), diisopropylcarbodiimide (DIC), N-ethyl-N'-3-dimethylaminopropylcarbodiimide and hydrochloride thereof (WSC, WSC-HCl, EDCI) and the like), phosphoric acid condensation reagents (e.g., benzotriazol-1-yloxy-tris(dimethylamino)phosphonium hexafluorophosphate (BOP), diphenylphosphoryl azide (DPPA) and the like), N,N'-carbonyldiimidazole, 2-methyl-6-nitrobenzoic anhydride, 2-chloro-1,3-dimethylimidazolium tetrafluoroborate, 2-chloro-1-methyl pyridinium, N,N-dimethylsulfamoyl chloride, 2-chloro-4,6-dimethoxytriazine, 4-(4,6-dimethoxy-1,3,5-triazin-2-yl)-4-methyl-morpholinium chloride and the like.

When the carbodiimide condensation reagent (e.g., dicyclohexylcarbodiimide (DCC), diisopropylcarbodiimide (DIC), N-ethyl-N'-3-dimethylaminopropylcarbodiimide and hydrochloride thereof (WSC, WSC-HCl, EDCI) and the like), 2-methyl-6-nitrobenzoic anhydride and the like are used as the condensing agent, the reaction efficiency can be improved by using, where necessary, a suitable condensation promoter (e.g., 1-hydroxy-7-azabenzotriazole, 1-hydroxybenzotriazole, N-hydroxysuccinimide, N-hydroxyphthalimide, 4-dimethylaminopyridine etc.). When the phosphoric acid condensation reagent (e.g., benzotriazol-1-yloxy-tris(dimethylamino)phosphonium hexafluorophosphate (BOP), diphenylphosphoryl azide (DPPA) and the like), 2-methyl-6-nitrobenzoic anhydride and the like are used as the condensing agent, the reaction efficiency can be generally improved by adding an organic amine base such as triethylamine, diisopropylethylamine and the like. When compound (11) forms a salt, this reaction is generally performed by adding a base. Examples of such base include tertiary amines, basic salts, metal hydride complex compounds and the like. These base are generally used in an amount of about 1 - 100 mol, preferably about 1 - 10 mol, per 1 mol of compound (11).

Compound (11) is generally used in an amount of about 1 - 10 mol, preferably about 1 - 2 mol, per 1 mol of compound (10). The amount of the condensing agent to be used is generally about 0.1 - 10 mol, preferably about 1 - 3 mol, per 1 mol of compound (10). The amounts of the above-mentioned condensation promoter and the base to be used is generally about 0.1 - 10 mol, preferably about 0.3 - 3 mol, per 1 mol of compound (10).

This reaction is advantageously performed in a solvent inert to the reaction. While such solvent is not particularly limited as long as the reaction proceeds, for example, solvents such as hydrocarbon halide, amides, sulfoxides, ethers, nitriles, esters, hydrocarbons, water and the like, a mixed solvent thereof and the like are preferable.

While the reaction time varies depending on the reagents and solvents to be used, it is generally about 0.1 - 48 hr, preferably about 0.5 - 24 hr.

The reaction temperature is generally about 0 - 200°C, preferably about 20 - 100°C.

### <Step 10>

Compound (13) can be produced from compound (10).

This reaction is performed, for example, according to a conventional method and using thionyl chloride, oxalyl chloride and the like. These reagent are generally used in an amount of about 1 - 100 mol, preferably about 1 - 30 mol, per 1 mol of compound (10).

This reaction is advantageously performed without solvent or in a solvent inert to the reaction. While such solvent is not particularly limited as long as the reaction proceeds, for example, solvents such as hydrocarbon halide, hydrocarbons and the like, or a mixed solvent thereof are preferable.

While the reaction time varies depending on the reagents and solvents to be used, it is generally about 0.1 - 100 hr, preferably about 0.1 - 24 hr.

The reaction temperature is generally about -20 to 150°C, preferably about 0 - 100°C.

### <Step 11>

Compound (12) can be produced by reacting compound (13) with compound (11).

Compound (11) is generally used in an amount of about 1 - 50 mol, preferably about 1 - 5 mol, per 1 mol of compound (13).

When compound (11) forms a salt, this reaction is generally performed by adding a base. Examples of such base include tertiary amines, basic salts, metal hydride complex compounds and the like. These bases are generally used in an amount of about 1 - 100 mol, preferably about 1 - 10 mol, per 1 mol of compound (11).

This reaction is advantageously performed in a solvent inert to the reaction. While such solvent is not particularly limited as long as the reaction proceeds, for example, solvents such as hydrocarbon halide, amides, sulfoxides, ethers, nitriles, esters, hydrocarbons and the like, a mixed solvent thereof and the like are preferable.

While the reaction time varies depending on the reagents and solvents to be used, it is generally about 0.1 - 48 hr, preferably about 0.5 - 24 hr.

The reaction temperature is generally about -30 to 150°C, preferably about 0 - 100°C.

### <Step 12>

Compound (14) can be produced by reacting compound (12) with an organic metal reagent for introducing a substituent for R⁴.

Examples of such organic metal reagent include organic magnesiums, organic lithiums and the like. These reagent are generally used in an amount of about 1 - 50 mol, preferably about 1 - 5 mol, per 1 mol of compound (14).

This reaction is advantageously performed in a solvent inert to the reaction. While such solvent is not particularly limited as long as the reaction proceeds, for example, solvents such as hydrocarbon halide, ethers, hydrocarbons and the like, a mixed solvent thereof and the like are preferable.

While the reaction time varies depending on the reagents and solvents to be used, it is generally about 0.1 - 48 hr, preferably about 0.5 - 24 hr.

The reaction temperature is generally about -100 to 100°C, preferably about -40 to 50°C.

### <Step 13>

Compound (15) can be produced from compound (14).
(i) When compound (15) wherein R³ is a substituent is produced, this reaction is performed in the same manner as in step 12.
(ii) When compound (15) wherein R³ is a hydrogen atom is produced, this reaction is performed in the presence of a reducing agent in a solvent inert to the reaction. Examples of the reducing agent include aluminum hydride, diisobutylaluminum hydride, lithium aluminum hydride and the like.

These reducing agents are generally used in an amount of about 0.25 - 10 mol, preferably about 0.5 - 5 mol, relative to compound (14).

This reaction is advantageously performed in a solvent inert to the reaction. While such solvent is not particularly limited as long as the reaction proceeds, for example, ethers, hydrocarbon halide, hydrocarbons and the like, a mixed solvent thereof and the like are preferable.

While the reaction time varies depending on the reagents and solvents to be used, it is generally about 0.1 - 72 hr, preferably, about 0.5 - 24 hr.

The reaction temperature is generally about -40 to 150°C, preferably about -20 to 100°C.

### <Step 14>

Compound (16) can be produced by protecting the carboxyl group of compound (10).

Examples of such protecting group include methyl, ethyl, n-propyl, isopropyl, n-butyl, tert-butyl group, benzyl group and the like.

Introduction of the protecting group in this step can be performed by a method known per se, for example, the method described in Greene's Protective Groups in Organic Synthesis, 4th Edition, Wiley-Interscience, Theodora W. Greene, Peter G. M. Wuts et al.

### <Step 15>

Compound (15) wherein both R³ and R⁴ are substituents can be produced by reacting compound (16) with an organic metal reagent for introducing a substituent for R³ and R⁴.

This reaction is performed in the same manner as in step 12.

### <Step 16>

Compound (17) can be produced by removing the amino-protecting group of compound (15).

Examples of such protecting group include a tert-butoxycarbonyl (Boc) group, a benzyloxycarbonyl (Z) group and the like.

Removal of the protecting group in this step can be performed by a method known per se, for example, the method described in Greene's Protective Groups in Organic Synthesis, 4th Edition, Wiley-Interscience, Theodora W. Greene, Peter G. M. Wuts et al.

Compound (17) which is compound (3) in reaction scheme 1 wherein -L-Y is -C(R³)(R⁴)-OH can be produced by, for example, the method shown in reaction scheme 3 or a method analogous thereto.

### Reaction scheme 3

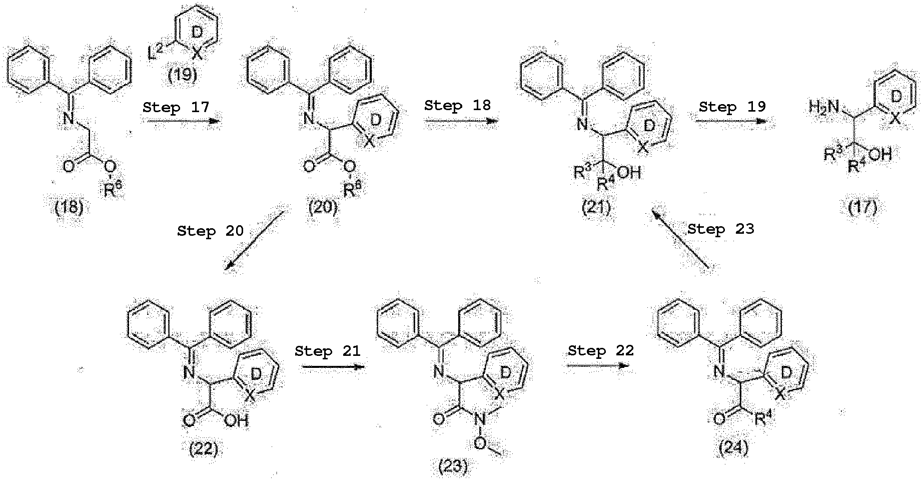

wherein L² is a leaving group, and other symbols are as defined above.

Examples of the leaving group for L² include a halogen atom, an optionally halogenated C₁₋₆ alkylsulfonyloxy group, an optionally substituted arylsulfonyloxy group and the like.

Compound (18) and compound (19) can be obtained as commercially available products, or can be produced by a method known per se or a method analogous thereto.

### <Step 17>

Compound (20) can be produced by subjecting compound (18) and compound (19) to a cross coupling reaction.

This reaction is performed according to a conventional method and using a transition metal catalyst and a base.

Examples of the transition metal catalyst include palladium catalysts and the like. Examples of the palladium catalyst include bis(tri-tert-butylphosphine)palladium(0), tris(dibenzylideneacetone)dipalladium(0) and the like. The transition metal catalyst is used in an amount of about 0.01 - 5 mol, preferably about 0.03 - 0.5 mol, per 1 mol of compound (18). Examples of the base include organic bases, inorganic bases, basic salts and the like. These bases are generally used in an amount of about 0.5 - 100 mol, preferably about 1 - 20 mol, per 1 mol of compound (18). compound (19) is generally used in an amount of about 0.5 - 50 mol, preferably about 0.9 - 10 mol, per 1 mol of compound (18).

This reaction is also performed in an inactive gas (e.g., argon gas or nitrogen gas) atmosphere or stream in the co-presence of a phosphine ligand. Examples of such phosphine ligand include tri-tert-butylphosphine and the like. These phosphine ligands are generally used in an amount of about 0.1 - 10 mol, preferably about 0.5 - 5 mol, per 1 mol of the transition metal catalyst.

This reaction is advantageously performed in a solvent inert to the reaction. While such solvent is not particularly limited as long as the reaction proceeds, for example, solvents such as hydrocarbons, ethers, nitriles, water and the like, a mixed solvent thereof and the like are preferable.

While the reaction time varies depending on the reagents and solvents to be used, it is generally about 0.1 - 100 hr, preferably about 0.1 - 72 hr.

The reaction temperature is generally about -10 to 200°C, preferably about 0 - 150°C.

### <Step 18>

Compound (21) can be produced from compound (20).

This reaction is performed in the same manner as in step 12.

### <Step 19>

Compound (17) can be produced by deprotecting the diphenylmethylene group of compound (21).

Removal of the protecting group in this step can be performed by a method known per se, for example, the method described in Greene's Protective Groups in Organic Synthesis, 4th Edition, Wiley-Interscience, Theodora W. Greene, Peter G. M. Wuts et al.

### <Step 20>

Compound (22) can be produced by subjecting compound (20) to a hydrolysis reaction.

This reaction is performed in the same manner as in step 5.

### <Step 21>

Compound (23) can be produced from compound (22).

This reaction is performed in the same manner as in step 9.

### <Step 22>

Compound (24) can be produced by reacting compound (23) with an organic metal reagent for introducing a substituent for R⁴.

This reaction is performed in the same manner as in step 12.

### <Step 23>

Compound (21) can be produced from compound (24).

This reaction is performed in the same manner as in step 13.

Compound (29) which is compound (3) in reaction scheme 1 wherein -L-Y is -CH₂-OR¹ can be produced by the method shown in reaction scheme 4 or a method analogous thereto.

### Reaction scheme 4

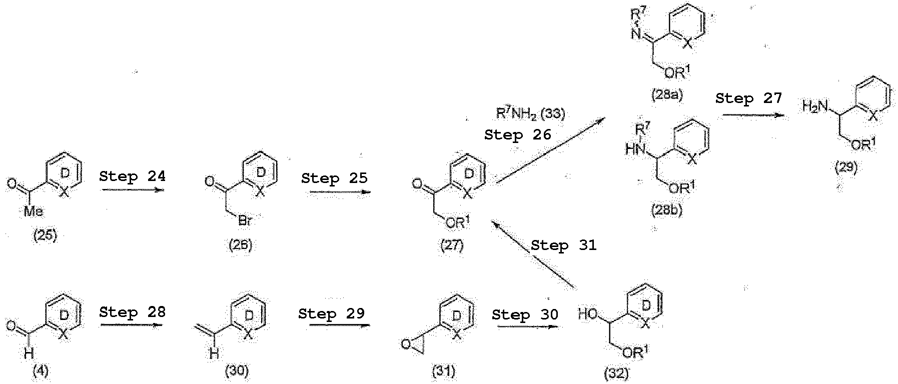

wherein R⁷ is a hydroxy group, an optionally substituted C₁₋₃ alkoxy group or an optionally substituted benzyl group, and other symbols are as defined above.

Compound (25), compound (33) and compound (4) can be obtained as commercially available products, or can be produced by a method known per se or a method analogous thereto.

### <Step 24>

Compound (26) can be produced by bromination of compound (25).

Examples of such brominating agent include bromine, phenyltrimethylammonium tribromide, N-bromosuccinimide and the like. These brominating agents are generally used in an amount of about 0.5 - 2 mol, preferably about 0.8 - 1.5 mol, per 1 mol of compound (25).

This reaction is advantageously performed in a solvent inert to the reaction. While such solvent is not particularly limited as long as the reaction proceeds, for example, solvents such as organic acids, alcohols, ethers, amides, hydrocarbon halide, hydrocarbons, water and the like, a mixed solvent thereof and the like are preferable.

While the reaction time varies depending on the reagents and solvents to be used, it is generally about 0.1 - 100 hr, preferably about 0.1 - 48 hr.

The reaction temperature is generally about -30 to 200°C, preferably about 0 - 100°C.

### <Step 25>

Compound (27) can be produced from compound (26).
(i) This reaction is performed by, for example, reacting compound (26) with desired alcohol (R¹OH) in the presence of silver(I) carbonate or iron(I) oxide and boron trifluoride diethyl ether complex.

Silver(I) carbonate and iron(I) oxide are generally used in an amount of about 1 - 10 mol, preferably about 1 - 5 mol, per 1 mol of compound (26). The boron trifluoride diethyl ether complex is generally used in an amount of about 1 - 10 mol, preferably about 1 - 5 mol, per 1 mol of compound (26).

This reaction is advantageously performed in a solvent inert to the reaction. While such solvent is not particularly limited as long as the reaction proceeds, for example, solvents such as alcohols corresponding to R¹OH, ethers, hydrocarbon halide, hydrocarbons and the like, a mixed solvent thereof and the like are preferable.

While the reaction time varies depending on the reagents and solvents to be used, it is generally about 0.1 - 100 hr, preferably about 0.1 - 48 hr.

The reaction temperature is generally about -30 - 150°C, preferably about 0 - 80°C.
(ii) This reaction can also be performed by reacting compound (26) with desired alcohol (R¹OH) in the presence of a base.

Examples of such base include tertiary amines, basic salts, metal hydride complex compounds, metal alkoxides, metal amides, alkyl metals, aryl metals and the like. These bases are generally used in an amount of about 1 - 10 mol, preferably about 1 - 5 mol, per 1 mol of compound (26).

This reaction is advantageously performed in a solvent inert to the reaction. While such solvent is not particularly limited as long as the reaction proceeds, for example, solvents such as alcohols corresponding to R¹OH, ethers, amides, sulfoxides, hydrocarbon halide, hydrocarbons and the like, a mixed solvent thereof and the like are preferable.

While the reaction time varies depending on the reagents and solvents to be used, it is generally about 0.1 - 48 hr, preferably about 0.5 - 24 hr.

The reaction temperature is generally about 0 - 150°C, preferably about 20 - 100°C.

### <Step 26>

Compound (28a) or compound (28b) can be produced by reacting compound (27) with compound (33). (i) compound (28a) can be produced by a dehydration condensation reaction of compound (27) and compound (33).

Compound (33) is generally used in an amount of about 1 - 10 mol, preferably about 1 - 5 mol, per 1 mol of compound (27).

When compound (33) forms a salt, this reaction is generally performed by adding a base. Examples of such base include tertiary amines, aromatic amines, basic salts, inorganic bases, alkali metal hydrides, metal alkoxides and the like. These bases are generally used in an amount of about 1 - 10 mol, preferably about 1 - 5 mol, per 1 mol of compound (33).

This reaction can also be promoted by adding a dehydrating agent such as molecular sieve and the like, or p-toluenesulfonic acid, zinc chloride, phosphoryl chloride, boron trifluoride, titanium tetrachloride, acetic acid, trifluoroacetic acid and the like to the system, or removing water generated in the system by using Dean-Stark and the like, or combining these.

This reaction is advantageously performed in a solvent inert to the reaction. While such solvent is not particularly limited as long as the reaction proceeds, for example, solvents such as alcohols, ethers, hydrocarbons, esters and the like, a mixed solvent thereof and the like are preferable.

While the reaction time varies depending on the reagents and solvents to be used, it is generally about 0.1 - 48 hr, preferably about 0.5 - 24 hr.

The reaction temperature is generally about 0 - 150°C, preferably about 20 - 100°C.
(ii) compound (28b) can be produced by a reductive amination reaction (e.g., described in Jikken Kagaku Kouza, 4th Edition, vol. 20, pp. 282-284, 366-368 (The Chemical Society of Japan ed.); J. Am. Chem. Soc., vol. 93, pp. 2897-2904, 1971; Synthesis, p. 135, 1975 and the like) of compound (27) and compound (33).

In this reaction, compound (28b) is obtained by subjecting an imine form formed by a dehydration reaction of compound (27) and compound (33) to a reduction reaction.

Compound (33) is generally used in an amount of about 1 - 10 mol, preferably about 1 - 3 mol, per 1 mol of compound (27).

The dehydration reaction can be promoted by adding a dehydrating agent such as molecular sieve and the like, or p-toluenesulfonic acid, zinc chloride, phosphoryl chloride, boron trifluoride, titanium tetrachloride, acetic acid, trifluoroacetic acid and the like to the system, or removing water generated in the system by using Dean-Stark and the like, or combining these.

The reduction reaction is performed according to a conventional method and generally using a reducing agent. Examples of the reducing agent include metal hydrides such as aluminum hydride, diisobutylaluminum hydride, tributyltin hydride and the like, metal hydride complex compounds such as sodium cyanoborohydride, sodium triacetoxyborohydride, sodium borohydride, lithium aluminum hydride and the like, borane complexes such as borane tetrahydrofuran complex, borane dimethylsulfide complex, picoline-borane complex and the like, alkyl boranes such as thexylborane, disiamylborane and the like, and the like.

These reducing agents are generally used in an amount of about 0.25 - 10 mol, preferably about 0.5 - 5 mol, per 1 mol of compound (27).

This reaction is advantageously performed in a solvent inert to the reaction. While such solvent is not particularly limited as long as the reaction proceeds, for example, solvents such as hydrocarbon halide, alcohols, ethers, nitriles, esters, hydrocarbons, amides, organic acids and the like, a mixed solvent thereof and the like are preferable.

While the reaction time varies depending on the reagents and solvents to be used, it is generally about 0.1 - 100 hr, preferably about 0.5 - 24 hr.

The reaction temperature is generally about -20 to 200°C, preferably about 0 - 100°C.

### <Step 27>

Compound (29) can be produced from compound (28a) or compound (28b). (i) This reaction is performed by, for example, using a catalyst such as palladium-carbon, palladium hydroxide, palladium black, platinum dioxide, Raney-nickel, Raney cobalt and the like. These catalysts are generally used in an amount of about 1 - 1000 wt%, preferably about 5 - 300 wt%, relative to compound (28a) or compound (28b).

This reaction is also performed using various hydrogen sources instead of gaseous hydrogen. Examples of such hydrogen source include formic acid, ammonium formate, triethylammonium formate, sodium phosphinate, hydrazine and the like. These hydrogen sources are generally used in an amount of about 1 - 10 mol, preferably about 1 - 5 mol, per 1 mol of compound (28a) or compound (28b).

This reaction is advantageously performed in a solvent inert to the reaction. While such solvent is not particularly limited as long as the reaction proceeds, for example, solvents such as hydrocarbon halide, alcohols, ethers, esters, organic acids, amides and the like, a mixed solvent thereof and the like are preferable.

While the reaction time varies depending on the reagents and solvents to be used, it is generally about 0.1 - 100 hr, preferably about 0.5 - 50 hr.

The reaction temperature is generally about -20 - 150°C, preferably about 0 - 100°C.
(ii) compound (29) can be produced by, for example, subjecting compound (28a) or compound (28b) wherein R⁷ is a hydroxy group or an optionally substituted C₁₋₃ alkoxy group to a reduction reaction.

Examples of the reducing agent include borane complex, metal hydride complex compound and the like.

These reducing agents are generally used in an amount of about 0.25 - 100 mol, preferably about 0.5 - 10 mol, per 1 mol of compound (28a) or compound (28b).

This reaction is advantageously performed in a solvent inert to the reaction. While such solvent is not particularly limited as long as the reaction proceeds, for example, solvents such as ethers, alcohols, water and the like, a mixed solvent thereof and the like are preferable.

While the reaction time varies depending on the reagents and solvents to be used, it is generally about 0.1 - 48 hr, preferably about 0.5 - 24 hr.

The reaction temperature is generally about -50 to 150°C, preferably about -20 to 100°C.

### <Step 28>

Compound (30) can be produced by subjecting compound (4) to the Wittig reaction and the like.

This reaction is performed by, for example, reacting compound (4) with phosphorus ylide prepared from methyl(triphenyl)phosphonium salt and a base.

Examples of such base include metal alkoxides, alkyl metals, alkali metal hydrides, metal amides and the like. These bases are generally used in an amount of about 1 - 10 mol, preferably about 1 - 5 mol, per 1 mol of compound (4).

The methyl(triphenyl)phosphonium salt is generally used in an amount of about 1 - 10 mol, preferably about 1 - 5 mol, per 1 mol of compound (4).

This reaction is advantageously performed in a solvent inert to the reaction. While such solvent is not particularly limited as long as the reaction proceeds, for example, solvents such as ethers, hydrocarbon halide, hydrocarbons, sulfoxides and the like, a mixed solvent thereof and the like are preferable.

While the reaction time varies depending on the reagents and solvents to be used, it is generally about 0.1 - 48 hr, preferably about 0.1 - 24 hr.

The reaction temperature is generally about -78 to 100°C, preferably about 0 - 100°C.

### <Step 29>

Compound (31) can be produced by reacting compound (30) with an oxidizing agent.

Examples of such oxidizing agent include 3-chloroperbenzoic acid, peracetic acid and the like.

These oxidizing agents are generally used in an amount of about 1 - 10 mol, preferably about 1 - 5 mol, per 1 mol of compound (30).

This reaction is advantageously performed in a solvent inert to the reaction. While such solvent is not particularly limited as long as the reaction proceeds, for example, solvents such as hydrocarbon halide, organic acids, esters, ethers, hydrocarbons, nitriles, water and the like, a mixed solvent thereof and the like are preferable.

While the reaction time varies depending on the reagents and solvents to be used, it is generally about 0.1 - 48 hr, preferably about 0.1 - 24 hr.

The reaction temperature is generally about -78 to 150°C, preferably about -20 to 80°C.

### <Step 30>

Compound (32) can be produced by reacting compound (31) with desired alcohol (R¹OH) in the presence of a base.

Examples of the base include metal alkoxides prepared from R¹OH, basic salts, metal hydride complex compounds, tertiary amines and the like. These bases are generally used in an amount of about 1 - 100 mol, preferably about 1 - 10 mol, per 1 mol of compound (31).

This reaction is advantageously performed in a solvent inert to the reaction. While such solvent is not particularly limited as long as the reaction proceeds, for example, solvents such as alcohols corresponding to R¹OH, amides, hydrocarbon halide, ethers, hydrocarbons, nitriles, water and the like, a mixed solvent thereof and the like are preferable.

While the reaction time varies depending on the reagents and solvents to be used, it is generally about 0.1 - 48 hr, preferably about 0.1 - 24 hr.

The reaction temperature is generally about -30 to 150°C, preferably about 0 - 100°C.

### <Step 31>

Compound (27) can be produced by subjecting compound (32) to an oxidation reaction.

This reaction is performed according to a conventional method and using an oxidizing agent.

Examples of the oxidizing agent include metal salts and metal oxides such as chrome (VI) oxide, pyridinium chlorochromate, manganese dioxide and the like, organic oxidizing agents such as o-iodoxybenzoic acid (IBX), 1,1,1-triacetoxy-1,1-dihydro-1,2-benziodoxol-3(1H)-one (Dess-Martin periodinane) and the like. These oxidizing agents are generally used in an amount of about 1 - 100 mol, preferably about 1 - 50 mol, per 1 mol of compound (32).

This reaction is advantageously performed in a solvent inert to the reaction. While such solvent is not particularly limited as long as the reaction proceeds, for example, solvents such as hydrocarbon halide, aromatic hydrocarbons, saturated hydrocarbons, nitriles, esters, ethers, sulfoxides, water and the like, a mixed solvent thereof and the like are preferable.

While the reaction time varies depending on the reagents and solvents to be used, it is generally about 0.1 - 48 hr, preferably about 0.1 - 24 hr.

The reaction temperature is generally about -78 to 150°C, preferably about -78 to 100°C.

Compound (37) which is compound (3) in reaction scheme 1 wherein -L-Y is -CH₂CH₂-OR¹ can be produced by, for example, the method shown in reaction scheme 5 or a method analogous thereto. Reaction scheme 5 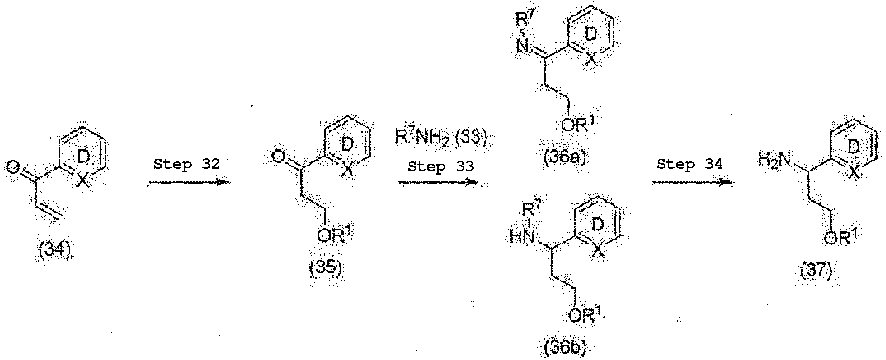 wherein each symbol is as defined above.

Compound (33) and compound (34) can be obtained as a commercially available product, or can be produced by a method known per se or a method analogous thereto.

### <Step 32>

Compound (35) can be produced by reacting compound (34) with desired alcohol (R¹OH) in the presence of a palladium (II) catalyst.

Examples of the palladium (II) catalyst include bis(acetonitrile)palladium chloride, palladium chloride, palladium acetate and the like. These palladium (II) catalysts are generally used in an amount of about 0.005 - 1 mol, preferably about 0.01 - 1 mol, per 1 mol of compound (34).

The alcohol (R¹OH) is generally used in an amount of about 1 - 10 mol, preferably about 1 - 3 mol, per 1 mol of compound (34).

This reaction is advantageously performed in a solvent inert to the reaction. While such solvent is not particularly limited as long as the reaction proceeds, for example, solvents such as hydrocarbon halide, alcohols corresponding to R¹OH, ethers, hydrocarbons and the like, a mixed solvent thereof and the like are preferable.

While the reaction time varies depending on the reagents and solvents to be used, it is generally about 0.5 - 48 hr, preferably about 1 - 24 hr.

The reaction temperature is generally about 0 - 100°C, preferably about 20 - 80°C.

### <Step 33>

Compound (36a) or compound (36b) can be produced by reacting compound (35) with compound (33).

This reaction is performed in the same manner as in step 26.

### <Step 34>

Compound (37) can be produced from compound (36a) or compound (36b).

This reaction is performed in the same manner as in step 27.

Compound (42) which is compound (3) in reaction scheme 1 wherein -L-Y is -CH₂-C(R³)(R⁴)-OH can be produced by, for example, the method shown in reaction scheme 6 or a method analogous thereto.

### Reaction scheme 6

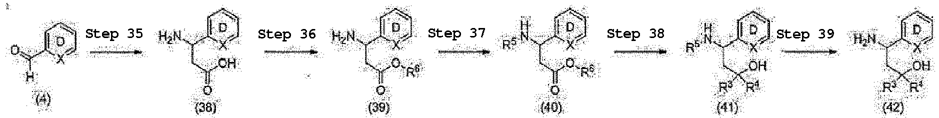

wherein each symbol is as defined above.

Compound (4) can be obtained as a commercially available product, or can be produced by a method known per se or a method analogous thereto.

### <Step 35>

Compound (38) can be produced from compound (4).

This reaction is performed by, for example, reacting ammonia or an equivalent thereof with malonic acid. Examples of such equivalent of ammonia include ammonium acetate, ammonium chloride, hexamethyldisilazane and the like. The ammonia and an equivalent thereof are generally used in an amount of about 1 - 100 mol, preferably about 1 - 10 mol, per 1 mol of compound (4).

The malonic acid is generally used in an amount of about 1 - 100 mol, preferably about 1 - 5 mol, per 1 mol of compound (4).

This reaction is advantageously performed in a solvent inert to the reaction. While such solvent is not particularly limited as long as the reaction proceeds and, for example, solvents such as hydrocarbon halide, alcohols, ethers, hydrocarbons, amides, water and the like, a mixed solvent thereof and the like are preferable.

While the reaction time varies depending on the reagents and solvents to be used, it is generally about 0.5 - 48 hr, preferably about 1 - 24 hr.

The reaction temperature is generally about 0 - 200°C, preferably about 20 - 150°C.

### <Step 36>

Compound (39) can be produced by protecting the carboxyl group of compound (38).

This reaction is performed in the same manner as in step 14.

### <Step 37>

Compound (40) can be produced by protecting the amino group of compound (39).

This reaction is performed in the same manner as in step 3.

### <Step 38>

Compound (41) can be produced from compound (40).

This reaction is performed in the same manner as in step 12.

### <Step 39>

Compound (42) can be produced by removing the amino-protecting group of compound (41).

This reaction is performed in the same manner as in step 16.

Compound (47) which is compound (2) in reaction scheme 1 wherein fused ring AB is pyrazolo[1,5-a]pyrimidine having R⁹ at the 6-position and L¹ is a hydroxy group can be produced by, for example, the method shown in reaction scheme 7 or a method analogous thereto.

### Reaction scheme 7

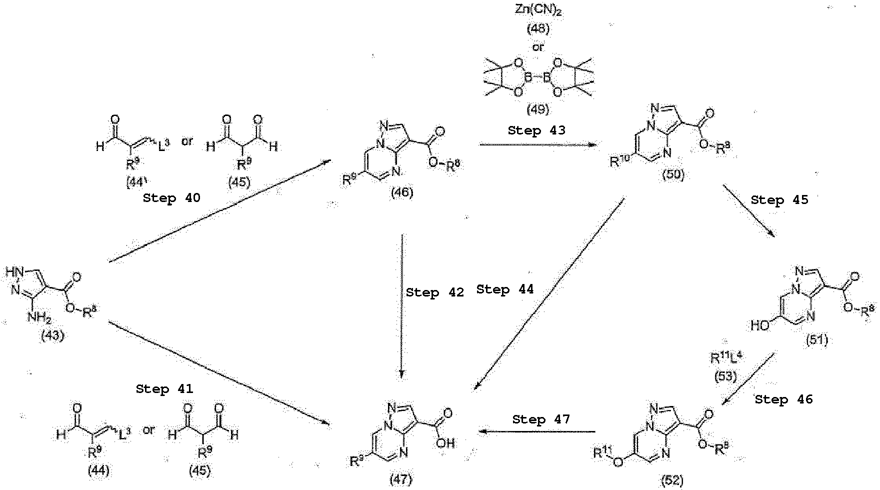

wherein R⁸ is a hydrogen atom or a carboxyl-protecting group, R⁹ is a hydrogen atom, an optionally substituted C₁₋₆ alkyl group, an optionally substituted C₁₋₆ alkoxy group, a mono- or di-C₁₋₆ alkylamino group, a halogen atom or a cyano group, R¹⁰ is a cyano group or an optionally substituted boryl group, R¹¹ is an optionally substituted C₁₋₆ alkyl group, L³ and L⁴ are each a leaving group, and other symbols are as defined above.

Examples of the "optionally substituted boryl group" for R¹⁰ include a boryl group optionally substituted by two hydroxy groups, a tetramethylethylenedioxy group and the like.

Examples of the leaving group for L³ include an optionally substituted C₁₋₃ alkoxy group, an optionally substituted C₁₋₃ dialkylamino group and the like.

Examples of the leaving group for L⁴ include a halogen atom, an optionally halogenated C₁₋₆ alkylsulfonyloxy group, a C₆₋₁₀ arylsulfonyloxy group optionally substituted by C₁₋₆ and the like.

Compound (43), compound (44), compound (45), compound (48) and compound (53) can be obtained as a commercially available product, or can be produced by a method known per se or a method analogous thereto.

### <Step 40>

Compound (46) wherein R⁸ is a carboxyl-protecting group can be produced by reacting compound (43) wherein R⁸ is a carboxyl-protecting group with compound (44) or compound (45).

Compound (44) or compound (45) is generally used in an amount of about 0.5 - 10 mol, preferably about 1 - 5 mol, per 1 mol of compound (43).

This reaction is also performed by adding an acid or base. Examples of such acid include organic acids and the like. Examples of such base include tertiary amines and the like. These acids or bases are generally used in an amount of about 0.5 - 50 mol, preferably about 1 - 10 mol, per 1 mol of compound (43).

This reaction is advantageously performed without solvent or in a solvent inert to the reaction. While such solvent is not particularly limited as long as the reaction proceeds, for example, solvents such as alcohols, ethers, hydrocarbon halide, hydrocarbons, organic acids, mineral acids and the like, a mixed solvent thereof and the like are preferable.

While the reaction time varies depending on the reagents and solvents to be used, it is generally about 0.1 - 48 hr, preferably about 0.5 - 24 hr.

The reaction temperature is generally about 0 - 200°C, preferably about 20 - 150°C.

### <Step 41>

Compound (47) can be produced by reacting compound (43) wherein R⁸ is a hydrogen atom with compound (44) or compound (45).

This reaction is performed in the same manner as in step

### 40. <Step 42>

Compound (47) can be produced by subjecting compound (46) to a hydrolysis reaction.

This reaction is performed in the same manner as in step 5.

### <Step 43>

Compound (50) wherein R⁸ is a carboxyl-protecting group can be produced by subjecting compound (46) wherein R⁹ is a halogen atom and zinc cyanide (48) or bis(pinacolato)diboron (49) to a coupling reaction in the presence of a palladium catalyst.

The zinc cyanide (48) is generally used in an amount of about 0.5 - 10 mol, preferably about 1 - 5 mol, per 1 mol of compound (46). Also, zinc metal may be added as appropriate. The zinc metal is generally used in an amount of about 0.005 - 10 mol, preferably about 0.01 - 5 mol, per 1 mol of compound (46).

The bis(pinacolato)diboron (49) is generally used in an amount of about 1 - 10 mol, preferably about 1 - 5 mol, per 1 mol of compound (46). This reaction is generally performed in the presence of a base. Examples of the base include basic salts and the like.

Examples of the palladium catalyst include palladium(II) acetate, tris(dibenzylideneacetone)dipalladium(0), tetrakis(triphenylphosphine)palladium(0), bis(tri-tert-butylphosphine)palladium(0), bis(triphenylphosphine)palladium(II) chloride, [1,1'-bis(diphenylphosphino)ferrocene]palladium(II) chloride and the like. These palladium catalyst is generally used in an amount of about 0.005 - 1 mol, preferably about 0.01 - 1 mol, per 1 mol of compound (46).

This reaction is generally performed in an inactive gas (e.g., argon gas or nitrogen gas) atmosphere or stream in the co-presence of a phosphine ligand. Examples of the phosphine ligand include tri-tert-butylphosphine, triphenylphosphine, 1,1'-bis(diphenylphosphino)ferrocene and the like. These phosphine ligands are generally used in an amount of about 0.1 - 10 mol, preferably about 0.5 - 5 mol, per 1 mol of the palladium catalyst.

This reaction is advantageously performed in a solvent inert to the reaction. While such solvent is not particularly limited as long as the reaction proceeds, for example, solvents such as amides, ethers, hydrocarbons, sulfoxides and the like, a mixed solvent thereof and the like are preferable.

While the reaction time varies depending on the reagents and solvents to be used, it is generally about 0.5 - 100 hr, preferably about 1 - 48 hr.

The reaction temperature is generally about 0 - 200°C, preferably about 20 - 150°C.

### <Step 44>

Compound (47) wherein R⁹ is a cyano group can be produced by subjecting compound (50) wherein R¹⁰ is a cyano group to a hydrolysis reaction.

This reaction is performed in the same manner as in step 5.

### <Step 45>

Compound (51) can be produced by reacting compound (50) wherein R¹⁰ is an optionally substituted boryl group with an oxidizing agent.

Examples of such oxidizing agent include hydrogen peroxide water, Oxone (registered trade mark), sodium perborate, hydroxylamine, tertiary amine-N-oxide, oxygen and the like. Such oxidizing agent is generally used in an amount of about 1 - 100 mol, preferably about 1 - 30 mol, per 1 mol of compound (50).

In this reaction, a base or a transition metal catalyst can also be added. Examples of such base include inorganic bases, basic salts, tertiary amines and the like. These bases are generally used in an amount of about 1 - 100 mol, preferably about 1 - 50 mol, per 1 mol of compound (50). Examples of such transition metal catalyst include copper (II) sulfate, copper (I) oxide, copper (II) bromide, copper (II) chloride, copper (II) acetate and the like. These transition metal catalysts are generally used in an amount of about 0.01 - 10 mol, preferably about 0.05 - 1 mol, per 1 mol of compound (50).

This reaction is advantageously performed in a solvent inert to the reaction. While such solvent is not particularly limited as long as the reaction proceeds, for example, solvents such as amides, ethers, nitriles, hydrocarbon halide, hydrocarbons, alcohols and the like, a mixed solvent thereof and the like are preferable.

While the reaction time varies depending on the reagents and solvents to be used, it is generally about 0.1 - 100 hr, preferably about 0.5 - 48 hr.

The reaction temperature is generally about -40 - 150°C, preferably about -10 - 100°C.

### <Step 46>

Compound (52) can be produced by reacting compound (51) with compound (53).

This reaction is performed in the presence of a base. Examples of such base include basic salts, aromatic amines, tertiary amines, alkali metal hydrides, metal amides and the like. These bases are generally used in an amount of about 1 - 50 mol, preferably about 1 - 10 mol, per 1 mol of compound (51).

Compound (53) is generally used in an amount of about 1 - 50 mol, preferably about 1 - 10 mol, per 1 mol of compound (51).

This reaction is advantageously performed in a solvent inert to the reaction. While such solvent is not particularly limited as long as the reaction proceeds, for example, solvents such as amides, ethers, nitriles, hydrocarbon halide, hydrocarbons, sulfoxides, ketones and the like, a mixed solvent thereof and the like are preferable.

While the reaction time varies depending on the reagents and solvents to be used, it is generally about 0.1 - 100 hr, preferably about 0.5 - 48 hr.

The reaction temperature is generally about -100 - 150°C, preferably about -40 - 100°C.

### <Step 47>

Compound (47) wherein R⁹ is an optionally substituted C₁₋₆ alkoxy group can be produced by subjecting compound (52) to a hydrolysis reaction.

This reaction is performed in the same manner as in step 5.

Compound (59) which is compound (2) in reaction scheme 1 wherein fused ring AB is pyrazolo[1,5-a]pyrimidine having R¹² at the 5-position and R⁹ at the 6-position, and L¹ is a hydroxy group can be produced by, for example, the method shown in reaction scheme 8 or a method analogous thereto. Reaction scheme 8 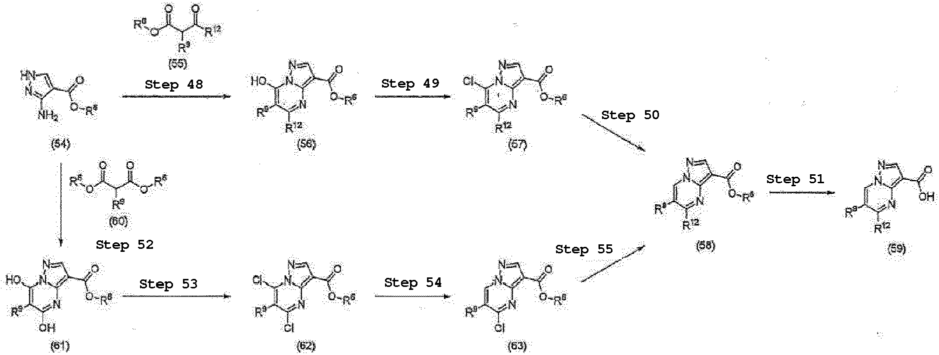 wherein R¹² is an optionally substituted hydrocarbon group, an optionally substituted C₁₋₆ alkoxy group, a halogen atom, a cyano group, an optionally substituted amino group or an optionally substituted heterocyclic group, and other symbols are as defined above.

Compound (54), compound (55) and compound (60) can be obtained as a commercially available product, or can be produced by a method known per se or a method analogous thereto.

### <Step 48>

Compound (56) can be produced by reacting compound (54) with compound (55) in the presence of an acid.

Examples of such acid include organic acids and the like.

These acids are generally used in an amount of about 0.5 - 200 mol, preferably 1 - 100 mol, per 1 mol of compound (54).

Compound (55) is generally used in an amount of about 1 - 10 mol, preferably about 1 - 5 mol, per 1 mol of compound (54).

This reaction is advantageously performed without solvent or in a solvent inert to the reaction.

While such solvent is not particularly limited as long as the reaction proceeds, for example, solvents such as hydrocarbon halide, aromatic hydrocarbons, saturated hydrocarbons, nitriles, ethers and the like, a mixed solvent thereof and the like are preferable.

While the reaction time varies depending on the reagents and solvents to be used, it is generally about 0.1 - 100 hr, preferably about 0.5 - 50 hr.

The reaction temperature is generally about 0 - 200°C, preferably about 20 - 150°C.

### <Step 49>

Compound (57) can be produced by method (A) comprising reacting compound (56) with a halogenating agent, or method (B) comprising halogenating compound (56) by using triphenylphosphine and a halogen source.
(i) When method (A) is used, examples of the halogenating agent include phosphorus oxychloride, thionyl chloride and the like. These halogenating agents are generally used in an amount of about 1 - 200 mol, preferably about 1 - 50 mol, per 1 mol of compound (56).

This reaction can also be promoted by adding a base. Examples of such base include tertiary amines and the like. These bases are generally used in an amount of about 0.5 - 50 mol, preferably about 1 - 5 mol, per 1 mol of compound (56).

This reaction can also be promoted by adding amides. Examples of such amides include N,N-dimethylformamide and the like. These amides are generally used in an amount of about 0.01 - 10 mol, preferably about 0.1 - 5 mol, per 1 mol of compound (56).

This reaction is advantageously performed without solvent or in a solvent inert to the reaction. While such solvent is not particularly limited as long as the reaction proceeds, for example, solvents such as hydrocarbon halide, aromatic hydrocarbons, saturated hydrocarbons, nitriles, ethers and the like, a mixed solvent thereof and the like are preferable.

While the reaction time varies depending on the reagents and solvents to be used, it is generally about 0.1 - 200 hr, preferably about 0.5 - 150 hr.

The reaction temperature is generally about 0 - 200°C, preferably about 20 - 150°C.
(ii) When method (B) is used, carbon tetrachloride or carbon tetrabromide can be used as the halogen source.

The halogen source is generally used in an amount of about 1 - 50 mol, preferably about 1 - 10 mol, per 1 mol of compound (56).

The triphenylphosphine is generally used in an amount of about 1 - 50 mol, preferably about 1 - 10 mol, per 1 mol of compound (56).

This reaction is advantageously performed by using a solvent inert to the reaction.

While such solvent is not particularly limited as long as the reaction proceeds, for example, solvents such as hydrocarbon halide, aromatic hydrocarbons, saturated hydrocarbons, nitriles, ethers and the like, a mixed solvent thereof and the like are preferable.

While the reaction time varies depending on the reagents and solvents to be used, it is generally about 0.1 - 100 hr, preferably about 0.5 - 50 hr.

The reaction temperature is generally about 0 - 150°C, preferably about 20 - 100°C.

### <Step 50>

Compound (58) can be produced by subjecting compound (57) to a reduction reaction.

Examples of such reducing agent include zinc powder, zinc-copper alloy powder and the like. These reducing agents are generally used in an amount of about 1 - 50 mol, preferably about 1 - 10 mol, per 1 mol of compound (57).

This reaction can also be promoted by adding a suitable additive. Examples of such additive include sodium chloride, ammonia and the like. A mixture of these additives may be used. These additives are generally used in an amount of about 1 - 200 mol, preferably about 1 - 100 mol, per 1 mol of compound (57).

This reaction can also be performed by a hydrogenation reaction. In this case, for example, catalysts such as palladium carbon, palladium hydroxide carbon, palladium black, platinum carbon, platinum dioxide, Raney-nickel, Raney cobalt and the like are used. These catalysts are generally used in an amount of about 5 - 1000 wt%, preferably about 10 - 300 wt%, per 1 mol of compound (57).

The hydrogenation reaction can also be performed by using various hydrogen sources instead of gaseous hydrogen. Examples of such hydrogen source include formic acid, ammonium formate, triethylammonium formate, sodium phosphinate, hydrazine and the like. These hydrogen sources are generally used in an amount of about 1 - 10 mol, preferably about 1 - 5 mol, per 1 mol of compound (57).

This reaction is advantageously performed in a solvent inert to the reaction. While such solvent is not particularly limited as long as the reaction proceeds, for example, solvents such as aromatic hydrocarbons, alcohols, esters, saturated hydrocarbons, ethers, organic acids, mineral acids, amides, water and the like, a mixed solvent thereof and the like are preferable.

While the reaction time varies depending on the reagents and solvents to be used, it is generally about 0.1 - 100 hr, preferably about 0.5 - 50 hr.

The reaction temperature is generally about 0 - 150°C, preferably about 20 - 100°C.

### <Step 51>

Compound (59) can be produced by subjecting compound (58) to a hydrolysis reaction.

This reaction is performed in the same manner as in step 5.

### <Step 52>

Compound (61) can be produced by reacting compound (54) with compound (60).

This reaction is performed in the same manner as in step 48.

### <Step 53>

Compound (62) can be produced from compound (61).

This reaction is performed in the same manner as in step 49.

### <Step 54>

Compound (63) can be produced from compound (62).

This reaction is performed in the same manner as in step 50.

### <Step 55>

Compound (58) can be produced from compound (63).
(i) compound (58) wherein R¹² is an optionally substituted hydrocarbon group or an optionally substituted heterocyclic group can be produced by the Suzuki-Miyaura coupling reaction, the Stille coupling reaction and the like. The coupling reaction can be performed by a method known per se, for example, the method described in Topics in Current Chemistry 219. Cross-Coupling Reactions: A Practical Guide, Springer-Verlag, Norio Miyaura et al. and the like, or a method analogous thereto.
(ii) compound (58) wherein R¹² is an optionally substituted amino group, or an optionally substituted heterocyclic group (e.g., pyrrolidin-1-yl, morpholino, 1H-pyrazol-1-yl), an optionally substituted C₁₋₆ alkoxy group can be produced by a nucleophilic substitution reaction known per se or Buchwald-Hartwig coupling reaction. The Buchwald-Hartwig coupling reaction can be performed by a method known per se, for example, the method described in Topics in Current Chemistry 219. Cross-Coupling Reactions: A Practical Guide, Springer-Verlag, Norio Miyaura et al. and the like, or a method analogous thereto.

Compound (69) which is compound (1) in reaction scheme 1 wherein fused ring AB is pyrazolo[4,3-c]pyridin-4(5H)-one can be produced by, for example, the method shown in reaction scheme 9 or a method analogous thereto.

### Reaction scheme 9

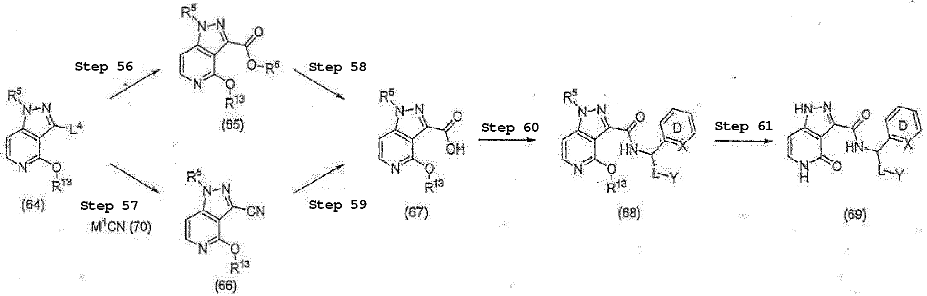

wherein R¹³ is a protecting group of a hydroxy group, M¹CN is an organic metal reagent for introduction of a cyano group, and other symbols are as defined above.

Compound (64) and compound (70) can be obtained as commercially available products, or can be produced by a method known per se or a method analogous thereto.

### <Step 56>

Compound (65) can be produced by reacting compound (64) with carbon monoxide and R⁶OH under a transition metal catalyst.

Examples of the transition metal catalyst include palladium (II) acetate, tris(dibenzylideneacetone)dipalladium (0), bis(tri-tert-butylphosphine)palladium (0), bis(triphenylphosphine)palladium (II) chloride, [1,1'-bis(diphenylphosphino)ferrocene]palladium (II) chloride and the like. These palladium catalysts are generally used in an amount of about 0.005 - 1 mol, preferably about 0.01 - 1 mol, per 1 mol of compound (64).

This reaction is generally performed by using a base. Examples of the base include tertiary amines and the like.

This reaction is performed under a carbon monoxide atmosphere. The carbon monoxide pressure is generally about 1 - 100 atm, preferably about 1 - 20 atm.

This reaction is also performed in the co-presence of a phosphine ligand. Examples of such phosphine ligand include tri-tert-butylphosphine, triphenylphosphine, 1,1'-bis(diphenylphosphino)ferrocene and the like. These phosphine ligands are generally used in an amount of about 1 - 10 mol, preferably about 1 - 5 mol, per 1 mol of the palladium catalyst.

This reaction is advantageously performed in a solvent inert to the reaction. While such solvent is not particularly limited as long as the reaction proceeds, for example, solvents such as amides, hydrocarbons, alcohols corresponding to R⁶OH, ethers and the like, a mixed solvent thereof and the like are preferable.

While the reaction time varies depending on the reagents and solvents to be used, it is generally about 0.5 - 100 hr, preferably about 1 - 48 hr.

The reaction temperature is generally about 0 - 200°C, preferably about 20 - 150°C.

### <Step 57>

Compound (66) can be produced by reacting compound (64) with metal cyanide (70).

The metal cyanide (70) is generally used in an amount of about 0.5 - 10 mol, preferably about 1 - 5 mol, per 1 mol of compound (64).

This reaction can be performed in the co-presence of an adduct such as copper iodide, nickel bromide and the like.

This reaction is advantageously performed in a solvent inert to the reaction. While such solvent is not particularly limited as long as the reaction proceeds, for example, solvents such as hydrocarbon halide, alcohols, ethers, nitriles, esters, hydrocarbons, amides, organic acids and the like, a mixed solvent thereof and the like are preferable.

While the reaction time varies depending on the reagents and solvents to be used, it is generally about 0.1 - 100 hr, preferably about 0.5 - 24 hr.

The reaction temperature is generally about -20 - 200°C, preferably about 0 - 100°C.

This reaction can be performed by reacting compound (64) with metal cyanide (70) in the presence of a metal catalyst such as palladium catalyst and the like.

Examples of the palladium catalyst include palladium (II) acetate, tris(dibenzylideneacetone)dipalladium (0), tetrakis(triphenylphosphine)palladium (0), bis(tri-tert-butylphosphine)palladium (0), bis(triphenylphosphine)palladium (II) chloride, [1,1'-bis(diphenylphosphino)ferrocene]palladium (II) chloride and the like can be mentioned. These palladium catalysts are generally used in an amount of about 0.005 - 1 mol, preferably about 0.01 - 1 mol, per 1 mol of compound (64).

The catalyst reaction is generally performed in an inactive gas (e.g., argon gas or nitrogen gas) atmosphere or stream in the co-presence of a phosphine ligand. Examples of such phosphine ligand include tri-tert-butylphosphine, triphenylphosphine, 1,1'-bis(diphenylphosphino)ferrocene and the like. These phosphine ligands are generally used in an amount of about 1 - 10 mol, preferably about 1 - 5 mol, per 1 mol of the palladium catalyst. Also, a zinc metal may be added as appropriate. The zinc metal is generally used in an amount of about 0.005 - 10 mol, preferably about 0.01 - 5 mol, per 1 mol of compound (64).

This reaction is advantageously performed in a solvent inert to the reaction. While such solvent is not particularly limited as long as the reaction proceeds, for example, solvents such as amides, ethers, hydrocarbons and the like, a mixed solvent thereof and the like are preferable.

While the reaction time varies depending on the reagents and solvents to be used, it is generally about 0.5 - 100 hr, preferably about 1 - 48 hr.

The reaction temperature is generally about 0 - 200°C, preferably about 20 - 150°C.

### <Step 58>

Compound (67) can be produced by subjecting compound (65) to a hydrolysis reaction.

This reaction is performed in the same manner as in step 5.

### <Step 59>

Compound (67) can be produced by subjecting compound (66) to a hydrolysis reaction.

This reaction is performed in the same manner as in step 4 and step 5.

### <Step 60>

Compound (68) can be produced by subjecting compound (67) and compound (3) to a condensation reaction.

This reaction is performed in the same manner as in step 1.

### <Step 61>

Compound (69) can be produced by subjecting compound (68) to a deprotection reaction.

Removal of the protecting group of compound (68) can be performed by a method known per se, for example, the method described in Greene's Protective Groups in Organic Synthesis, 4th Edition, Wiley-Interscience, Theodora W. Greene, Peter G. M. Wuts et al., or a method analogous thereto and, for example, a method using an acid or a base, hydrogenation reaction and the like can be mentioned.

Of compounds (2) in reaction scheme 1, compound (81) can be produced by, for example, the method shown in reaction scheme 10, or a method analogous thereto.

### Reaction scheme 10

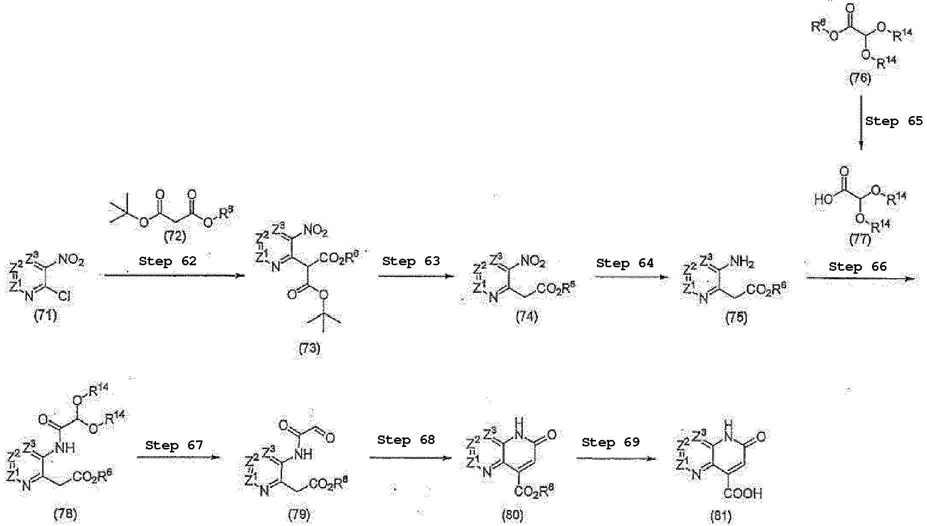

wherein Z¹ is a group represented by CR^{Z1} (R^{Z1} is a hydrogen atom, a halogen atom, an optionally substituted hydrocarbon group, an optionally substituted C₁₋₆ alkoxy group, an optionally substituted amino group, a cyano group or an optionally substituted heterocyclic group), or a nitrogen atom, Z² is a group represented by CR^{Z2} (R^{Z2} is a hydrogen atom, a halogen atom, an optionally substituted hydrocarbon group, an optionally substituted C₁₋₆ alkoxy group, an optionally substituted amino group, a cyano group or an optionally substituted heterocyclic group), or a nitrogen atom, Z³ is a group represented by CR^{Z3} (R^{Z3} is a hydrogen atom, a halogen atom, an optionally substituted hydrocarbon group, an optionally substituted C₁₋₆ alkoxy group, an optionally substituted amino group, a cyano group, an optionally substituted C₁₋₆ alkyl-carbonyl group or an optionally substituted heterocyclic group), or a nitrogen atom, R¹⁴ is a carbonyl-protecting group, and other symbols are as defined above.

Examples of the carbonyl-protecting group for R¹⁴ include cyclic acetal (e.g., 1,3-dioxane), non-cyclic acetal (e.g., di-C₁₋₆ alkylacetal) and the like.

Compound (71), compound (72) and compound (76) can be obtained as commercially available products, or can be produced by a method known per se or a method analogous thereto.

### <Step 62>

Compound (73) can be produced by reacting compound (71) with compound (72).

Compound (72) is generally used in an amount of about 1 - 100 mol, preferably about 1 - 10 mol, per 1 mol of compound (71).

This reaction is performed in the presence of a base. Examples of such base include tertiary amines, aromatic amines, alkali metal hydrides, metal amides, basic salts, metal alkoxides and the like. These bases are generally used in an amount of about 1 - 100 mol, preferably about 1 - 10 mol, per 1 mol of compound (71).

This reaction is advantageously performed in a solvent inert to the reaction. While such solvent is not particularly limited as long as the reaction proceeds, for example, solvents such as hydrocarbon halide, aromatic amines, amides, alcohols, ethers, nitriles, hydrocarbons, sulfoxides and the like, a mixed solvent thereof and the like are preferable.

While the reaction time varies depending on the reagents and solvents to be used, it is generally about 0.1 - 48 hr, preferably about 0.5 - 24 hr.

The reaction temperature is generally about -50 - 150°C, preferably about -20 - 100°C.

### <Step 63>

Compound (74) can be produced by subjecting compound (73) to an acid hydrolysis reaction.

Examples of such acid include organic acids, mineral acids and the like. These acids are generally used in an amount of about 1 - 200 mol, preferably about 10 - 100 mol, per 1 mol of compound (73).

This reaction is advantageously performed in a solvent inert to the reaction. While such solvent is not particularly limited as long as the reaction proceeds, for example, solvents such as hydrocarbon halide, ethers, nitriles, hydrocarbons and the like, a mixed solvent thereof and the like are preferable.

While the reaction time varies depending on the reagents and solvents to be used, it is generally about 0.1 - 48 hr, preferably about 0.5 - 24 hr.

The reaction temperature is generally about -50 - 150°C, preferably about -20 - 100°C.

### <Step 64>

Compound (75) can be produced by reducing the nitro group of compound (74).

Conversion of nitro group to amino group can be performed by a method known per se, for example, the method described in Reductions in Organic Chemistry, Second Edition, The American Chemical Society, 1996, or a method analogous thereto and, for example, hydrogenation reaction, a reaction using a metal or a metal salt and the like can be mentioned.

### <Step 65>

Compound (77) can be produced by subjecting compound (76) to a hydrolysis reaction.

This reaction is performed in the same manner as in step 5.

### <Step 66>

Compound (78) can be produced by subjecting compound (75) and compound (77) to a condensation reaction.

This reaction is performed in the same manner as in step 1.

### <Step 67>

Compound (79) can be produced by removing the carbonyl-protecting group of compound (78).

Removal of the carbonyl-protecting group can be performed by a method known per se, for example, the method described in Greene's Protective Groups in Organic Synthesis, 4th Edition, Wiley-Interscience, Theodora W. Greene, Peter G. M. Wuts et al., or a method analogous thereto.

This reaction is performed by, for example, a method using an acid and the like.

### <Step 68>

Compound (80) can be produced from compound (79).

This reaction is performed using a base. Examples of such base include basic salts, aromatic amines, tertiary amines, metal alkoxides, metal amides, alkali metal hydrides, secondary amines and the like.

Examples of the secondary amines include pyrrolidine, piperidine and the like.

These bases are generally used in an amount of about 1 - 100 mol, preferably about 1 - 50 mol, relative to compound (79).

This reaction is advantageously performed in a solvent inert to the reaction. While such solvent is not particularly limited as long as the reaction proceeds, for example, solvents such as hydrocarbon halide, ethers, nitriles, hydrocarbons, amides, sulfoxides and the like, a mixed solvent thereof and the like are preferable.

While the reaction time varies depending on the reagents and solvents to be used, it is generally about 0.1 - 48 hr, preferably about 0.5 - 24 hr.

The reaction temperature is generally about -50 - 150°C, preferably about -20 - 100°C.

### <Step 69>

Compound (81) can be produced by subjecting compound (80) to a hydrolysis reaction.

This reaction is performed in the same manner as in step 5.

Of compounds (2) in reaction scheme 1, compound (84) can be produced by, for example, the method shown in reaction scheme 11, or a method analogous thereto.

### Reaction scheme 11

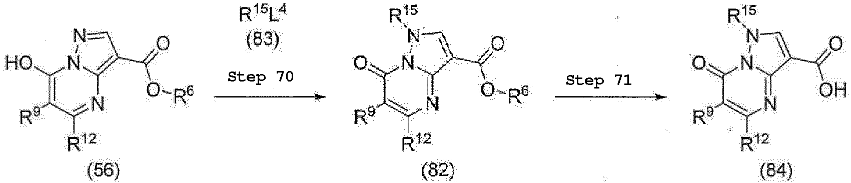

wherein R¹⁵ is an optionally substituted C₁₋₆ alkyl group, and other symbols are as defined above.

Compound (83) can be obtained as a commercially available product, or can be produced by a method known per se or a method analogous thereto.

### <Step 70>

Compound (82) can be produced by reacting compound (56) with compound (83).

Compound (83) is generally used in an amount of about 1 - 50 mol, preferably about 1 - 10 mol, per 1 mol of compound (56).

This reaction is performed in the presence of a base. Examples of such base include basic salts, tertiary amines, alkali metal hydrides, metal amides and the like. These bases are generally used in an amount of about 0.5 - 50 mol, preferably about 1 - 10 mol, per 1 mol of compound (56).

This reaction is advantageously performed in a solvent inert to the reaction. While such solvent is not particularly limited as long as the reaction proceeds, for example, ethers, hydrocarbons, amides, hydrocarbon halide, nitriles, sulfoxides and the like.

While the reaction time varies depending on the reagents and solvents to be used, it is generally about 0.1 - 72 hr, preferably about 0.5 - 36 hr.

The reaction temperature is generally about -80 - 150°C, preferably about -40 - 120°C.

### <Step 71>

Compound (84) can be produced by subjecting compound (82) to a hydrolysis reaction.

This reaction is performed in the same manner as in step 5.

Compounds (63) in reaction scheme 8 can also be produced by, for example, the method shown in reaction scheme 12 or a method analogous thereto.

### Reaction scheme 12

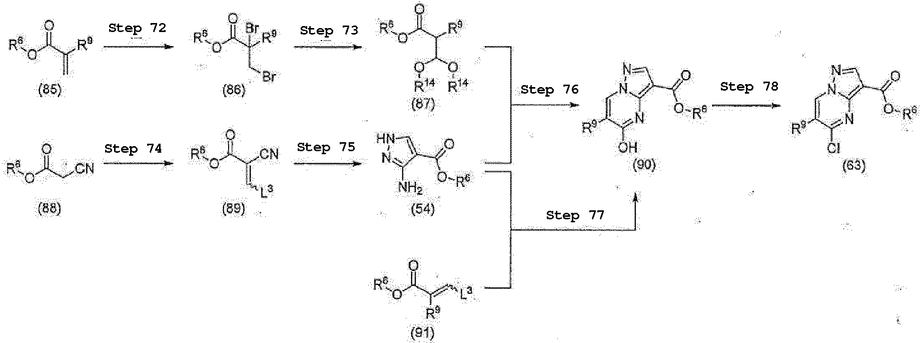

wherein each symbol is as defined above.

Compound (85), compound (88) and compound (91) can be obtained as commercially available products, or can be produced by a method known per se or a method analogous thereto.

### <Step 72>

Compound (86) can be produced by bromination of compound (85).

Examples of such brominating agent include bromine, phenyltrimethylammonium tribromide and the like. These brominating agents are generally used in an amount of about 0.5 - 5 mol, preferably about 1 - 2 mol, per 1 mol of compound (85).

This reaction is advantageously performed in a solvent inert to the reaction. While such solvent is not particularly limited as long as the reaction proceeds, for example, alcohols, ethers, amides, esters, hydrocarbon halide, hydrocarbons and the like, or a mixed solvent thereof are preferable.

While the reaction time varies depending on the reagents and solvents to be used, it is generally about 0.1 - 48 hr, preferably about 0.5 - 24 hr.

The reaction temperature is generally about -20 to 100°C, preferably about 0 - 50°C.

### <Step 73>

Compound (87) can be produced by reacting compound (86) with a base.

Examples of such base include metal alkoxides, basic salts and the like. These bases are generally used in an amount of about 1 - 20 mol, preferably about 1 - 5 mol, per 1 mol of compound (86).

This reaction is advantageously performed in a solvent inert to the reaction. While such solvent is not particularly limited as long as the reaction proceeds, for example, alcohols corresponding to R¹⁴OH, amides, hydrocarbon halide, hydrocarbons and the like, or a mixed solvent thereof are preferable.

While the reaction time varies depending on the reagents and solvents to be used, it is generally about 0.1 - 48 hr, preferably about 1 - 24 hr.

The reaction temperature is generally about -20 to 100°C, preferably about 0 - 80°C.

### <Step 74>

Compound (89) can be produced by reacting compound (88) with a formyl group equivalent.

Examples of such formyl group equivalent include N,N-dimethylformamide dimethylacetal, tert-butoxybis(dimethylamino)methane, trimethyl orthoformate, triethyl orthoformate and the like. These formyl group equivalents are generally used in an amount of about 0.5 - 100 mol, preferably about 1 - 10 mol, per 1 mol of compound (88).

This reaction can be promoted by an additive. Examples of such additive include organic acids, Lewis acids, metal alkoxides, tertiary amines and the like. These additives are generally used in an amount of about 0.01 - 10 mol, preferably about 0.05 - 5 mol, per 1 mol of compound (88).

This reaction is advantageously performed without solvent or in a solvent inert to the reaction. While such solvent is not particularly limited as long as the reaction proceeds, for example, amides, esters, hydrocarbon halide, hydrocarbons, organic acids, anhydrous acetic acid and the like, or a mixed solvent thereof are preferable.

While the reaction time varies depending on the reagents and solvents to be used, it is generally about 0.1 - 48 hr, preferably about 1 - 24 hr.

The reaction temperature is generally about -20 to 250°C, preferably about 0 - 200°C.

### <Step 75>

Compound (54) can be produced from compound (89).

This reaction is performed by, for example, reacting compound (89) with hydrazine, hydrazine monohydrate, hydrazine aqueous solution and the like. These hydrazines are generally used in an amount of about 0.5 - 10 mol, preferably about 1 - 3 mol, per 1 mol of compound (89).

This reaction is advantageously performed in a solvent inert to the reaction. While such solvent is not particularly limited as long as the reaction proceeds, for example, amides, ethers, hydrocarbon halide, hydrocarbons, alcohols and the like, or a mixed solvent thereof are preferable.

While the reaction time varies depending on the reagents and solvents to be used, it is generally about 0.1 - 48 hr, preferably about 1 - 24 hr.

The reaction temperature is generally about 0 - 150°C, preferably about 10 - 100°C.

### <Step 76>

Compound (90) can be produced by reacting compound (87) with compound (54) in the presence of a base.

Examples of such base include basic salts, metal alkoxides and the like. These bases are generally used in an amount of about 0.5 - 10 mol, preferably about 1 - 3 mol, per 1 mol of compound (54).

Compound (87) is generally used in an amount of about 0.5 - 10 mol, preferably about 1 - 3 mol, per 1 mol of compound (54).

This reaction is advantageously performed in a solvent inert to the reaction. While such solvent is not particularly limited as long as the reaction proceeds, for example, amides, ethers, hydrocarbon halide, hydrocarbons, nitriles and the like, or a mixed solvent thereof are preferable.

While the reaction time varies depending on the reagents and solvents to be used, it is generally about 0.1 - 48 hr, preferably about 1 - 24 hr.

The reaction temperature is generally about -20 - 150°C, preferably about 0 - 110°C.

### <Step 77>

Compound (90) can also be produced by reacting compound (54) with compound (91).

This reaction is performed in the same manner as in step 76.

### <Step 78>

Compound (63) can be produced by reacting compound (90) with a halogenating agent, or triphenylphosphine and a halogen source.

This reaction is performed in the same manner as in step 49.

Compound (97) which is compound (59) in reaction scheme 8 wherein R¹² is an optionally substituted 1H-1,2,3-triazol-1-yl group can also be produced by, for example, the method described in reaction scheme 13 or a method analogous thereto. Reaction scheme 13 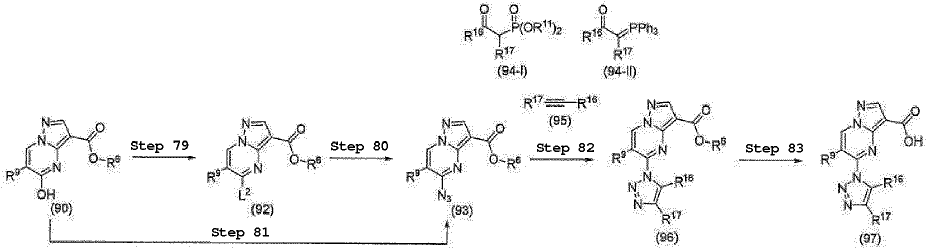 wherein R¹⁶ and R¹⁷ are each independently a hydrogen atom or an optionally substituted hydrocarbon group, and other symbols are as defined above.

Compound (94) and compound (95) can be obtained as commercially available products, or can be produced by a method known per se or a method analogous thereto.

### <Step 79>

Compound (92) can be produced from compound (90).
(i) compound (92) wherein L² is a halogen atom can be produced by reacting compound (90) with a halogenating agent, or triphenylphosphine and a halogen source.

This reaction is performed in the same manner as in step 49.
(ii) compound (92) wherein L² is an optionally halogenated C₁₋₆ alkylsulfonyloxy group, an optionally substituted arylsulfonyloxy group and the like can be produced by reacting the corresponding sulfonyl chloride or sulfonic anhydride with compound (90).

The corresponding sulfonyl chloride or sulfonic anhydride is generally used in an amount of about 1 - 50 mol, preferably about 1 - 10 mol, per 1 mol of compound (90).

This reaction is performed in the presence of a base. Examples of such base include aromatic amines, tertiary amines and the like. These bases are generally used in an amount of about 1 - 50 mol, preferably about 1 - 10 mol, per 1 mol of compound (90).

This reaction is advantageously performed in a solvent inert to the reaction. While such solvent is not particularly limited as long as the reaction proceeds, for example, amides, esters, hydrocarbon halide, hydrocarbons, ethers, nitriles, aromatic amines and the like, or a mixed solvent thereof are preferable.

While the reaction time varies depending on the reagents and solvents to be used, it is generally about 0.1 - 48 hr, preferably about 1 - 24 hr.

The reaction temperature is generally about -20 - 150°C, preferably about 0 - 100°C.

### <Step 80>

Compound (93) can be produced by reacting compound (92) with azide.

Examples of such azide include sodium azide, tetra-n-butylammonium azide and the like. These azides are generally used in an amount of about 1 - 10 mol, preferably about 1 - 3 mol, per 1 mol of compound (92).

This reaction is advantageously performed in a solvent inert to the reaction. While such solvent is not particularly limited as long as the reaction proceeds, for example, amides, nitriles, alcohols, sulfoxides and the like, or a mixed solvent thereof are preferable.

While the reaction time varies depending on the reagents and solvents to be used, it is generally about 0.1 - 48 hr, preferably about 1 - 24 hr.

The reaction temperature is generally about 0 - 150°C, preferably about 0 - 100°C.

### <Step 81>

Compound (93) can be produced by reacting compound (90) with an azidating agent.

Examples of such azidating agent include diphenylphosphoryl azide and the like. These azidating agents are generally used in an amount of about 1 - 30 mol, preferably about 1 - 15 mol, per 1 mol of compound (90).

This reaction is performed in the presence of a base. Examples of such base include aromatic amines, tertiary amines and the like. These bases are generally used in an amount of about 1 - 50 mol, preferably about 1 - 20 mol, per 1 mol of compound (90).

This reaction is advantageously performed in a solvent inert to the reaction. While such solvent is not particularly limited as long as the reaction proceeds, for example, ethers, amides, hydrocarbons, hydrocarbon halide, nitriles and the like, or a mixed solvent thereof are preferable.

While the reaction time varies depending on the reagents and solvents to be used, it is generally about 0.1 - 48 hr, preferably about 1 - 24 hr.

The reaction temperature is generally about 0 - 150°C, preferably about 0 - 100°C.

### <Step 82>

Compound (96) can be produced by reacting compound (93) with compound (94) or compound (95).
(i) When compound (93) and compound (94-I) are reacted, this reaction is performed using a base. Examples of such base include alkali metal hydrides, metal amides, alkyl metals, aryl metals, metal alkoxides and the like. These bases are generally used in an amount of about 1 - 10 mol, preferably about 1 - 5 mol, per 1 mol of compound (93).

Compound (94-I) is generally used in an amount of about 1 - 10 mol, preferably about 1 - 5 mol, per 1 mol of compound (93).

This reaction is advantageously performed in a solvent inert to the reaction. While such solvent is not particularly limited as long as the reaction proceeds, for example, ethers, amides, hydrocarbons, hydrocarbon halide, sulfoxides and the like, or a mixed solvent thereof are preferable.

While the reaction time varies depending on the reagents and solvents to be used, it is generally about 0.1 - 48 hr, preferably about 1 - 24 hr.

The reaction temperature is generally about -78 - 150°C, preferably about -78 - 120°C.
(ii) When compound (93) and compound (94-II) are reacted, compound (94-II) is generally used in an amount of about 1 - 10 mol, preferably about 1 - 3 mol, per 1 mol of compound (93).

This reaction is advantageously performed in a solvent inert to the reaction. While such solvent is not particularly limited as long as the reaction proceeds, for example, ethers, amides, hydrocarbons, hydrocarbon halide, esters, nitriles and the like, or a mixed solvent thereof are preferable.

While the reaction time varies depending on the reagents and solvents to be used, it is generally about 0.1 - 48 hr, preferably about 1 - 24 hr.

The reaction temperature is generally about 0 - 150°C, preferably about 0 - 100°C.
(iii) When compound (93) and compound (95) are reacted, compound (95) is generally used in an amount of about 1 - 20 mol, preferably about 1 - 10 mol, per 1 mol of compound (93).

This reaction is accelerated by a copper catalyst. Examples of such copper catalyst include copper (I) iodide and the like. These copper catalysts are generally used in an amount of about 0.01 - 5 mol, preferably about 0.05 - 0.5 mol, per 1 mol of compound (93).

This reaction is advantageously performed in a solvent inert to the reaction. While such solvent is not particularly limited as long as the reaction proceeds, for example, ethers, amides, hydrocarbons, hydrocarbon halide and the like, or a mixed solvent thereof are preferable.

While the reaction time varies depending on the reagents and solvents to be used, it is generally about 0.1 - 48 hr, preferably about 1 - 24 hr.

The reaction temperature is generally about 0 - 200°C, preferably about 10 - 170°C.

### <Step 83>

Compound (97) can be produced by subjecting compound (96) to a deprotection reaction.

Removal of the protecting group of compound (96) can be performed by a method known per se, for example, the method described in Greene's Protective Groups in Organic Synthesis, 4th Edition, Wiley-Interscience, Theodora W. Greene, Peter G. M. Wuts et al., or a method analogous thereto and, for example, a method using an acid, hydrogenation reaction, a method using a transition metal catalyst and the like can be mentioned.

Of compounds (1) in reaction scheme 1, compound (100) can also be produced by, for example, the method shown in reaction scheme 14 or a method analogous thereto.

### Reaction scheme 14

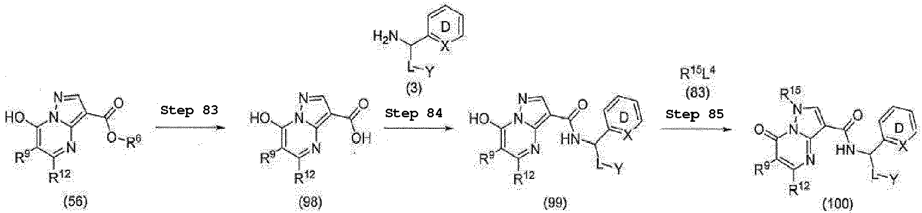

wherein each symbol is as defined above.

Compound (83) can be obtained as a commercially available product, or can be produced by a method known per se or a method analogous thereto.

### <Step 83>

Compound (98) can be produced by subjecting compound (56) to a hydrolysis reaction.

This reaction is performed in the same manner as in step 5.

### <Step 84>

Compound (99) can be produced by subjecting compound (98) and compound (3) to a condensation reaction.

This reaction is performed in the same manner as in step 1.

### <Step 85>

Compound (100) can be produced by reacting compound (99) with compound (83).

This reaction is performed in the same manner as in step 70.

Of compounds (1) in reaction scheme 1, compound (104) can also be produced by, for example, the method shown in reaction scheme 15 or a method analogous thereto.

### Reaction scheme 15

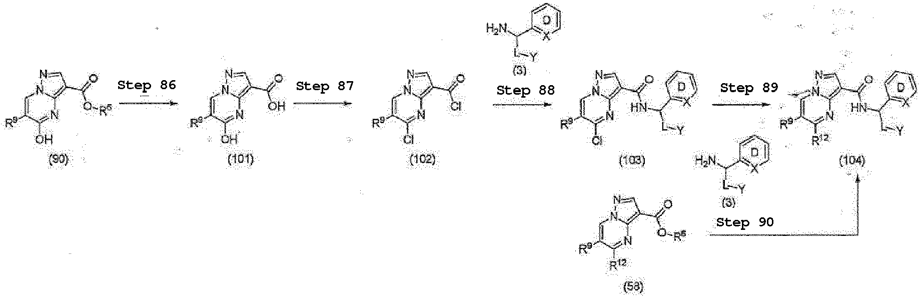

wherein each symbol is as defined above.

### <Step 86>

Compound (101) can be produced by subjecting compound (90) to a hydrolysis reaction.

This reaction is performed in the same manner as in step 5.

### <Step 87>

Compound (102) can be produced by reacting compound (101) with a halogenating agent, or using triphenylphosphine and a halogen source.

This reaction is performed in the same manner as in step 49.

### <Step 88>

Compound (103) can be produced by reacting compound (102) with compound (3).

This reaction is performed using a base. Examples of such base include tertiary amines, aromatic amines, basic salts and the like. These bases are generally used in an amount of about 1 - 50 mol, preferably about 1 - 5 mol, per 1 mol of compound (102).

This reaction is advantageously performed in a solvent inert to the reaction. While such solvent is not particularly limited as long as the reaction proceeds, for example, ethers, amides, hydrocarbons, hydrocarbon halide, nitriles, aromatic amines and the like, or a mixed solvent thereof are preferable.

While the reaction time varies depending on the reagents and solvents to be used, it is generally about 0.1 - 48 hr, preferably about 1 - 24 hr.

The reaction temperature is generally about -40 - 150°C, preferably about 0 - 100°C.

### <Step 89>

Compound (104) can be produced from compound (103).

This reaction is performed in the same manner as in step 55.

### <Step 90>

Compound (104) can be produced by reacting compound (58) with compound (3).

This reaction is performed in the same manner as in step 1-3.

Compounds (I) obtained by each of the above-mentioned production methods can be isolated and purified by a known means such as concentration, and concentrated under reduced pressure, solvent extraction, crystallization, recrystallization, phase transfer, chromatography and the like. Respective starting compounds used in each of the above-mentioned production methods can be isolated and purified by a known means similar to the aforementioned means. Alternatively, these starting compounds in the form of a reaction mixture may be directly used without isolation as a starting material for the next step.

When compound (I) contains an isomer such as an optical isomer, a stereoisomer, a regioisomer, a rotamer and the like, any isomer and a mixture thereof are also encompassed in compound (I). For example, when compound (I) has an optical isomer, an optical isomer resolved from racemate is also encompassed in compound (I). These isomers can be obtained as independent products by a synthesis means or a separation means known per se (e.g., concentration, solvent extraction, column chromatography, recrystallization etc.), optical re solution method (e.g., fractional recrystallization method, chiral column method, diastereomer method etc.) and the like.

Compound (I) may be a crystal. Even if compound (I) is in a single crystal form or mixed crystal form, it can be provided as compound (I) of the present invention. Crystal can be produced by crystallization by applying a crystallization method known per se.

Compound (I) may be a solvate (e.g., hydrate etc.) or a non-solvate (e.g., non-hydrate etc.), both of which are encompassed in compound (I).

Compound (I) also encompasses a compound labeled or substituted with an isotope (e.g., ²H, ³H, ¹³C, ¹⁴C, ¹⁸F, ³⁵S, ¹²⁵I etc.) and the like.

Compound (I) also encompasses a deuterium conversion form wherein ¹H is converted to ²H(D).

Compound (I) labeled or substituted with an isotope can be used, for example, as a tracer (PET tracer) used for positron emission tomography (PET), and is useful in the field such as medical diagnosis and the like.

Since the compound of the present invention has a superior PDE2A inhibitory action, shows low toxicity (e.g., phototoxicity, acute toxicity, chronic toxicity, genetic toxicity, reproductive toxicity, cardiac toxicity, drug interaction, carcinogenicity etc., particularly phototoxicity), is superior in stability (particularly, metabolic stability) and in vivo kinetics (absorbability, distribution, metabolism, excretion etc.), and further shows high solubility, it is useful as a pharmaceutical product. The compound of the present invention has a PDE2A inhibitory action on mammals (e.g., mouse, rat, hamster, rabbit, cat, dog, bovine, horse, sheep, monkey, human etc.), and can be used as a prophylactic or therapeutic drug for the following diseases and symptoms:
(1) psychotic disorder (e.g., brief psychotic disorder, shared psychotic disorder),
(2) psychosis induced by alcohol, amphetamine, cannabis, cocaine, hallucinogens, obesity, inhalants, opioids or phencyclidine,
(3) delusional disorder,
(4) anxiety disorder,
(5) movement disorder,
(6) mood disorder,
(7) major depressive disorder,
(8) a major depressive disorder superimposed on a psychotic disorder (including delusional disorder and schizophrenia),
(9) major depressive episode of the mild, moderate or severe type,
(10) manic or mixed mood episode,
(11) hypomanic mood episode,
(12) depressive episode with atypical features,
(13) depressive episode with melancholic features,
(14) depressive episode with catatonic features,
(15) mood episode with postpartum onset;
(16) post-stroke depression,
(17) dysthymic disorder,
(18) minor depressive disorder,
(19) autism;
(20) drug addiction,
(21) neurodegenerative disorder,
(22) neurodegeneration associated with cerebral trauma,
(23) neurodegeneration associated with stroke,
(24) neurodegeneration associated with cerebral infarct,
(25) neurodegeneration associated with hypoglycemia,
(26) neurodegeneration associated with epileptic seizure,
(27) neurodegeneration associated with neurotoxin poisoning,
(28) multi-system atrophy,
(29) Alzheimer's disease,
(30) dementia,
(31) multi-infarct dementia,
(32) alcoholic dementia or other drug-related dementia,
(33) dementia associated with intracranial tumors or cerebral trauma,
(34) dementia associated with Huntington's disease or Parkinson's disease,
(35) AIDS-related dementia,
(36) frontotemperal dementia,
(37) delirium,
(38) amnestic disorder,
(39) post-traumatic stress disorder,
(40) mental retardation,
(41) learning disorder (e.g., reading disorder, mathematics disorder, or a disorder of written expression),
(42) attention-deficit/hyperactivity disorder;
(43) age-related cognitive decline,
(44) premenstrual dysphoric disorder,
(45) post-psychotic depressive disorder of schizophrenia,
(46) bipolar disorder (including bipolar I disorder and bipolar II disorder),
(47) cyclothymic disorder,
(48) Parkinson's disease,
(49) Huntington's disease,
(50) paranoia,
(51) schizophrenia (positive symptom, a negative symptom, cognitive functional disorder as main symptoms) (e.g., paranoid schizophrenia, disorganized schizophrenia, catatonic schizophrenia, undifferentiated schizophrenia, residual schizophrenia),
(52) schizophreniform disorder,
(53) schizoaffective disorder of the delusional type or the depressive type,
(54) personality disorder of the paranoid type,
(55) personality disorder of the schizoid type,
(56) obesity,
(57) metabolic syndrome,
(58) non-insulin dependent diabetes (NIDDM),
(59) glucose intolerance,
(60) pneumonia,
(61) osteoarthritis,
(62) migraine headache,
(63) fragile X syndrome.

Particularly, the compound of the present invention is useful for the prophylaxis or treatment of schizophrenia and Alzheimer's disease.

Since the compound of the present invention is superior in metabolic stability, it is expected to show a superior treatment effect on the above-mentioned diseases even at a low dose. Moreover, the compound of the present invention is superior in intracerebral transferability.

Since the compound of the present invention shows low toxicity, a pharmaceutical composition containing the compound of the present invention (hereinafter to be referred to as "the medicament of the present invention") can be safely administered solely or by mixing with a pharmacologically acceptable carrier according to a method known per se (e.g., the method described in the Japanese Pharmacopoeia etc.) as the production method of a pharmaceutical preparation, and in the form of, for example, tablet (including sugar-coated tablet, film-coated tablet, sublingual tablet, orally disintegrating tablet, buccal and the like), pill, powder, granule, capsule (including soft capsule, microcapsule), troche, syrup, liquid, emulsion, suspension, release control preparation (e.g., immediate-release preparation, sustained-release preparation, sustained-release microcapsule), aerosol, film (e.g., orally disintegrating film, oral mucosa-adhesive film), injection (e.g., subcutaneous injection, intravenous injection, intramuscular injection, intraperitoneal injection), drip infusion, transdermal absorption type preparation, ointment, lotion, adhesive preparation, suppository (e.g., rectal suppository, vaginal suppository), pellet, nasal preparation, pulmonary preparation (inhalant), eye drop and the like, orally or parenterally (e.g., intravenous, intramuscular, subcutaneous, intraorgan, intranasal, intradermal, instillation, intracerebral, intrarectal, intravaginal, intraperitoneal and intratumor administrations, administration to the vicinity of tumor, and direct administration to the lesion).

The above-mentioned "pharmacologically acceptable carrier" may be exemplified by various organic or inorganic carrier materials that are conventionally used as preparation materials, for example, excipient, lubricant, binding agent and disintegrant for solid preparations; or solvent, solubilizing agent, suspending agent, isotonic agent, buffering agent, soothing agent and the like for liquid preparations. Furthermore, when necessary, ordinary additives such as preservative, antioxidizing agent, colorant, sweetening agent, adsorbing agent, wetting agent and the like can also be used as appropriate in an appropriate amount.

Examples of the excipient include lactose, sucrose, D-mannitol, D-sorbitol, starch, α-starch, cornstarch, dextrin, crystalline cellulose, low-substituted hydroxypropylcellulose, sodium carboxymethylcellulose, gum arabic, pullulan, light anhydrous silicic acid, synthetic aluminum silicate, magnesium aluminometasilicate and the like.

Examples of the lubricant include magnesium stearate, calcium stearate, talc, colloidal silica and the like.

Examples of the binder include α-starch, crystalline cellulose, sucrose, gum arabic, D-mannitol, trehalose, dextrin, pullulan, hydroxypropylcellulose, hydroxypropylmethylcellulose, polyvinylpyrrolidone, starch, sucrose, gelatin, methylcellulose, carboxymethylcellulose, carboxymethylcellulose sodium and the like.

Examples of the disintegrant include lactose, sucrose, starch, carboxymethylcellulose, calcium carboxymethylcellulose, croscarmellose sodium, carboxymethylstarch sodium, light anhydrous silicic acid, low-substituted hydroxypropylcellulose and the like.

Examples of the solvent include water for injection, physiological saline, Ringer's injection, alcohol, propylene glycol, polyethylene glycol, macrogol, sesame oil, corn oil, olive oil, cottonseed oil and the like.

Examples of the solubilizing agent include polyethylene glycol, propylene glycol, D-mannitol, trehalose, benzyl benzoate, ethanol, trisaminomethane, cholesterol, triethanolamine, sodium carbonate, sodium citrate, sodium salicylate, sodium acetate and the like.

Examples of the suspending agent include surfactants such as stearyl triethanolamine, sodium lauryl sulfate, laurylaminopropionic acid, lecithin, benzalkonium chloride, benzetonium chloride, glycerin monostearate and the like; hydrophilic polymers such as polyvinyl alcohol, polyvinylpyrrolidone, carboxymethylcellulose sodium, methylcellulose, hydroxymethylcellulose, hydroxyethylcellulose, hydroxypropylcellulose, polysorbate, polyoxyethylene hydrogenated castor oil and the like; and the like.

Examples of the isotonic agent include glucose, D-sorbitol, sodium chloride, glycerin, D-mannitol and the like.

Examples of the buffering agent include buffer solutions such as phosphates, acetates, carbonates, citrates and the like.

Examples of the soothing agent include benzyl alcohol and the like.

Examples of the preservative include parahydroxybenzoates, chlorobutanol, benzyl alcohol, phenethyl alcohol, dehydroacetic acid, sorbic acid and the like.

Examples of the antioxidizing agent include sulfites, ascorbic acid, α-tocopherol and the like.

Examples of the colorant include water-soluble food tar color (e.g., Food Color Red No. 2 and No. 3, Food Color Yellow No. 4 and No. 5, Food Color Blue No. 1 and No. 2 etc.), water-insoluble lake dye (e.g., aluminum salt of the aforementioned water-soluble food tar color), natural dye (e.g., β-carotene, chlorophyll, ferric oxide red) and the like.

Examples of the sweetening agent include saccharin sodium, dipotassium glycyrrhizinate, aspartame, stevia and the like.

While the content of the compound of the present invention in the medicament of the present invention varies depending on the dosage form, dose of the compound of the present invention and the like, it is, for example, about 0.01 - 100 wt%, preferably about 0.1 - 95 wt%, of the whole medicament.

While the dose of the compound of the present invention varies depending on the subject of administration, administration route, target disease, symptom and the like, it is generally about 0.1 - about 20 mg/kg body weight, preferably about 0.2 - about 10 mg/kg body weight, more preferably about 0.5 - about 10 mg/kg body weight, for oral administration to schizophrenia patients (adult, about 60 kg body weight), and the dose is desirably administered in about one to several (e.g., 1-3) portions per day according to the symptoms.

The compound of the present invention can be administered as a single active substance, or can be administered in combination with other medicaments such as other drugs used in the treatment of psychotic disorder (particularly schizophrenia and bipolar disorder), obsessive-compulsive disorder, major depression, Parkinson's disease, Alzheimer's disease, cognitive disorder, memory loss and the like (hereinafter to be abbreviated as concomitant drug).

Examples of the concomitant drug include nicotinic α7 agonists, nicotinic α7 partial agonists, nicotinic α7 positive allosteric modulators, PDE2 inhibitors, PDE4 inhibitors, PDE5 inhibitors, PDE10 inhibitors, other PDE inhibitors, calcium channel blockers, muscarinic m1 and m2 modulators, adenosine receptor modulators, ampakines, Glycine transporter 1 inhibitors, NMDA-R modulators, mGluR modulators, dopamine modulators, serotonin modulators, selective serotonin reuptake inhibitors, serotonin and norepinephrine reuptake inhibitors, norepinephrine and dopamine reuptake inhibitors, triple reuptake inhibitors, cannabinoid modulators, cholinesterase inhibitors (e.g., donepezil, rivastigmine, galanthamine) and the like.

In addition, examples of the concomitant drug include, but are not limited to, other suitable schizophrenia drugs (e.g., Haloperidol, Clozapine, Olanzapine, Risperidone, Aripiprazole, Ziprasidone, Paliperidone, Quetiapine fumarate etc.), bipolar disorder drug (e.g., Lithium, Olanzapine, Aripiprazole, Valproic acid etc.), Parkinson's disease drugs (e.g., Levodopa, Bromocriptine, Pergolide, Pramipexole, Tolcapone, Procyclidine, Trihexyphenidyl, Benztropine etc.), agents used in the treatment of major depression (e.g., Amitriptyline, Imipramine, Desipramine, Nortriptyline, Paroxetine, Fluoxetine, Sertraline, Bupropion, Escitalopram, Mirtazapine, Venlafaxine, Duloxetine etc.), agents used in the treatment of Alzheimer's disease (e.g., Galanthamine, Tacrine, Donepezil, Rivastigmine, Memantine, Neotropin, Selegiline, Estrogen, Clioquinol etc.), agents used in the treatment of dementia (e.g., Thioridazine, Haloperidol, Risperidone, Tacrine, Donepezil, Rivastigmine etc.), agents used in the treatment of epilepsy (e.g., Phenytoin, Phenobarbital, Carbamazepine, Valproic acid, Ethosuximide, Gabapentin, Solfeton, Felbatol etc.), agents used in the treatment of multiple sclerosis (e.g., Tolterodine, Oxybutynin, Oxycodone, Interferon beta-1b, Interferon beta-la, Azathioprine, Methotrexate, Glatiramer etc.), agents used in the treatment of Huntington's disease (e.g., Amitriptyline, Imipramine, Desipramine, Nortriptyline, Paroxetine, Fluoxetine, Sertraline, Tetrabenazine, Haloperidol, Chlorpromazine, Thioridazine, Sulpiride, Quetiapine, Clozapine, Risperidone etc.), agents useful in the treatment of diabetes [e.g., PPAR ligands (e.g. agonists or antagonists such as Rosiglitazone, Troglitazone, Pioglitazone etc.), insulin secretagogues (e.g., sulfonylurea drugs such as Glyburide, Glimepiride, Chlopropamide, Tolbutamide, Glipizide etc., and non-sulfonyl secretagogues), α-glucosidase inhibitors (e.g., Acarbose, Miglitol, Voglibose etc), insulin sensitizers (e.g., PPAR-γ agonists (e.g., the glitazones); biguanides, PTP-1B inhibitors, DPP-IV inhibitors, 11beta-HSD inhibitors etc.), hepatic glucose output lowering compounds (e.g., glucagon antagonists and metformin (e.g., Glucophage, Glucophage XR etc.)), insulin and insulin derivatives (including both long and short acting forms of insulin and insulin formulations)], antiobesity drugs [e.g., β-3 agonists, CB-1 agonists, neuropeptide Y5 inhibitors, Ciliary Neurotrophic Factor and derivatives (e.g., Axokine), appetite suppressants (e.g., Sibutramine), lipase inhibitors (e.g., Orlistat) etc.].

The administration form of the combination agent of the present invention is not particularly limited, and the compound of the present invention and a concomitant drug only need to be combined on administration. Examples of such administration mode include the following:
(1) administration of a single preparation obtained by simultaneously processing the compound of the present invention and the concomitant drug,
(2) simultaneous administration of two kinds of preparations of the compound of the present invention and the concomitant drug, which have been separately produced, by the same administration route,
(3) administration of two kinds of preparations of the compound of the present invention and the concomitant drug, which have been separately produced, by the same administration route in a staggered manner,
(4) simultaneous administration of two kinds of preparations of the compound of the present invention and the concomitant drug, which have been separately produced, by different administration routes,
(5) administration of two kinds of preparations of the compound of the present invention and the concomitant drug, which have been separately produced, by different administration routes in a staggered manner (e.g., administration in the order of the compound of the present invention and the concomitant drug, or in the reverse order) and the like.

These administration forms are summarized below, and abbreviated as the concomitant drug of the present invention.

When the concomitant drug of the present invention is administered, the concomitant drug and the compound of the present invention can be administered simultaneously. In addition, the compound of the present invention can be administered after administration of the concomitant drug administration, and the concomitant drug can be administered after administration of the compound of the present invention. When administered in a staggered manner, the administration time varies depending on the active ingredient to be administered, dosage form and administration method.

For example, when the concomitant drug or a pharmaceutical composition thereof is to be administered first, the compound of the present invention or a pharmaceutical composition thereof can be administered within 1 min to 3 days, preferably within 10 min to 1 day, more preferably within 15 min to 1 hour after administration of the concomitant drug or a pharmaceutical composition thereof. In addition, when the compound of the present invention or a pharmaceutical composition thereof is to be administered first, the concomitant drug or a pharmaceutical composition thereof can be administered within 1 min to 1 day, preferably within 10 min to 6 hours, more preferably within 15 min to 1 hour after administration of the compound of the present invention or a pharmaceutical composition thereof.

When the concomitant drug does not pose problems of side effects, it can be administered at any dose. While the dose of the concomitant drug varies depending on the dosage form, subject of administration, administration route, target disease, symptom and the like, it is, for example, generally about 0.1 - about 20 mg/kg body weight, preferably about 0.2 - about 10 mg/kg body weight, more preferably about 0.5 - about 10 mg/kg body weight for oral administration to schizophrenia patients (adult, about 60 kg body weight), and the dose is desirably administered in about one to several (e.g., 1-3) portions per day according to the symptoms.

When the compound of the present invention is used in combination with a concomitant drug, the dose thereof can be reduced within the safe range in consideration of the opposite effects of the both drugs.

The concomitant drug of the present invention shows low toxicity and, for example, the compound of the present invention or(and) the above-mentioned concomitant drug may be mixed with a pharmacologically acceptable carrier according to a method known per se to give a pharmaceutical composition, for example, tablet (including sugar-coated tablet, film-coated tablet and the like), powder, granule, capsule (including soft capsule), liquid, emulsion, suspension, injection, suppository, sustained-release preparation (e.g., sublingual tablet, microcapsule etc.), plaster, orally disintegrating tablet, orally disintegrable film and the like, which can be safely administered orally or parenterally (e.g., subcutaneous, topical, rectal, intravenous administration etc.).

The pharmaceutically acceptable carriers that can be used for manufacturing the combination drug of the present invention can be the same as those used in the medicament of the present invention as mentioned above.

A mixing ratio between the compound of the present invention and the concomitant drug in the combination drug of the present invention can be selected appropriately based on the administration subjects, administration routes, target diseases and the like.

The concomitant drug in the combination drug of the present invention can be combined at an appropriate proportion if two or more drugs are combined.

A dosage of the concomitant drug can be selected appropriately based on the dosages used clinically. In addition, a mixing ratio between the compound of the present invention and the concomitant drug can be selected appropriately based on the administration subjects, administration routes, target diseases, symptoms, combinations, etc. For example, if the administration subject is humans, a concomitant drug may be used in an amount ranging from about 0.01 to 100 parts by weight relative to 1 part by weight of the compound of the present invention.

For example, while the content of the compound of the present invention in the combination agent of the present invention varies depending on the preparation form, it is generally about 0.01 - 99.9 wt%, preferably about 0.1 - 50 wt%, more preferably about 0.5 - 20 wt%, of the whole preparation.

The content of the concomitant drug in the combination agent of the present invention varies depending on the preparation form, and is generally about 0.01 to 99.9% by weight, preferably about 0.1 to 50% by weight, further preferably about 0.5 to 20% by weight, of the whole preparation.

While the content of the additive such as a carrier and the like in the combination agent of the present invention varies depending on the form of a preparation, it is generally about 1 to 99.99% by weight, preferably about 10 to 90% by weight, based on the whole preparation.

When the compound of the present invention and the concomitant drug are separately prepared, the same content may be adopted.

Since the dosage may vary under various conditions as mentioned above, a dosage less than the dosages may be sufficient or it may be necessary to administer at a dosage exceeding the above-mentioned ranges.

### Examples

The present invention is explained in detail in the following by referring to Examples, Formulation Examples and Experimental Examples and which are merely exemplified and not to be construed as limitative, and the invention may be changed within the scope of the present invention. In the following Examples, the "room temperature" generally means about 10°C to about 35°C. The ratios indicated for mixed solvents are volume mixing ratios, unless otherwise specified. % means wt%, unless otherwise specified.

In silica gel column chromatography, NH means use of aminopropylsilane-bonded silica gel. In HPLC (high performance liquid chromatography), C18 means use of octadecyl-bonded silica gel. The ratios of elution solvents are volume mixing ratios, unless otherwise specified.

¹H NMR (proton nuclear magnetic resonance spectrum) was measured by Fourier-transform type NMR. Broad and unidentified peaks of protons of a hydroxyl group, an amino group and the like are not described.

In the following Reference Examples and Examples, mass spectrum (MS), nuclear magnetic resonance spectrum (NMR) and melting point were measured by the following apparatus.

MS (mass spectrum) was measured by LC/MS (liquid chromatograph mass spectrometer). As the ionization method, API (Atmospheric Pressure Ionization, atmospheric pressure chemical ionization) method or ESI (Electron Spray Ionization) method was used. The data indicate measured value (found). Generally, a molecular ion peak is observed. In the case of a compound having an amino group (-NH₂), a peak after elimination of NH₃ may be observed as a fragment ion. In the case of a salt, a molecular ion peak or fragment ion peak of free form is generally observed.

### Example 1

### N-(2-methoxy-1-(4-(trifluoromethoxy)phenyl)ethyl)-2-oxo-1,2-dihydro-1,5-naphthyridine-4-carboxamide

### A) 4-ethenylphenyl trifluoromethyl ether

To a suspension of potassium 2-methylpropan-2-oate (6.10 g) in tetrahydrofuran (80 mL) was added methyl(triphenyl)phosphonium iodide (22.1 g) over 5 min under ice-cooling. The reaction mixture was stirred for 30 min, and a solution of 4-(trifluoromethoxy)benzaldehyde (8.00 g) in tetrahydrofuran (20 mL) was gradually added. The reaction mixture was stirred at room temperature for 1.5 hr, water was added, and the mixture was extracted with ethyl acetate. The extract was washed with water, and then with saturated brine, and dried over anhydrous sodium sulfate. The solvent was evaporated under reduced pressure. The residue was purified by silica gel column chromatography (petroleum ether) to give the title compound (5.30 g).
1H NMR (300 MHz, CDC13) δ 5.30 (1H, d, J = 10.8 Hz), 5.75 (1H, d, J = 17.7 Hz), 6.72 (1H, dd, J = 17.7, 10.8 Hz), 7.19 (2H, d, J = 7.8 Hz), 7.44 (2H, d, J = 6.9 Hz).

### B) 2-(4-(trifluoromethoxy)phenyl)oxirane

To a solution of 4-ethenylphenyl trifluoromethyl ether (5.30 g) in chloroform (50 mL) was added 3-chloroperbenzoic acid (11.4 g) at room temperature. The reaction mixture was stirred at room temperature for 2 hr, and washed with aqueous sodium thiosulfate solution, and then with saturated brine. The organic layer was dried over anhydrous sodium sulfate, and the solvent was evaporated under reduced pressure. The residue was purified by silica gel column chromatography (petroleum ether/dichloromethane) to give the title compound (4.20 g).
1H NMR (400 MHz, CDC13) δ 2.76 (1H, dd, J = 5.6, 2.4 Hz), 3.16 (1H, dd, J = 5.2, 4.0 Hz), 3.87 (1H, dd, J = 4.0, 2.8 Hz), 7.20 (2H, d, J = 8.0 Hz), 7.31 (2H, d, J = 8.0 Hz).

### C) 2-methoxy-1-(4-(trifluoromethoxy)phenyl)ethanol

To a solution of 2-(4-(trifluoromethoxy)phenyl)oxirane (4.20 g) in N,N-dimethylacetamide (20 mL) was added sodium methoxide (5.56 g) at room temperature. The reaction mixture was stirred at 60°C for 3 hr. The reaction mixture was cooled to room temperature, ethyl acetate was added, and the mixture was washed with water, and then with saturated brine, and dried over anhydrous sodium sulfate. The solvent was evaporated under reduced pressure. The residue was purified by silica gel column chromatography (dichloromethane/ethyl acetate) to give the title compound (2.75 g).
1H NMR (300 MHz, CDC13) δ 2.87 (1H, d, J = 2.7 Hz), 3.33-3.45 (4H, m), 3.55 (1H, dd, J = 9.6, 3.0 Hz), 4.92 (1H, d, J = 8.7 Hz), 7.22 (2H, d, J = 8.1 Hz), 7.43 (2H, d, J = 8.7 Hz).

### D) 2-methoxy-1-(4-(trifluoromethoxy)phenyl)ethanone

To a solution of 2-methoxy-1-(4-(trifluoromethoxy)phenyl)ethanol (2.70 g) in dichloromethane (50 mL) was added 1,1,1-triacetoxy-1,1-dihydro-1,2-benzoiodoxol-3(1H)-one (6.28 g) at room temperature. The reaction mixture was stirred at room temperature for 2 hr, and the insoluble material was filtered off. The filtrate was washed with sodium thiosulfate, and then with saturated brine, and dried over anhydrous sodium sulfate. The solvent was evaporated under reduced pressure. The residue was purified by silica gel column chromatography (dichloromethane) to give the title compound (2.00 g).
1H NMR (400 MHz, CDC13) δ 3.51 (3H, s), 4.68 (2H, s), 7.30 (2H, d, J = 8.0 Hz), 8.02 (2H, d, J = 9.2 Hz).

### E) 2-methoxy-1-(4-(trifluoromethoxy)phenyl)ethanamine

To a solution of 2-methoxy-1-(4-(trifluoromethoxy)phenyl)ethanone (2.00 g) in ethanol (80 mL) were added potassium carbonate (4.70 g) and hydroxylamine hydrochloride (1.18 g) at room temperature. The reaction mixture was heated under reflux overnight, and the solvent was evaporated under reduced pressure. To the residue was added ethyl acetate, and the mixture was washed with water, and then with saturated brine, and dried over anhydrous sodium sulfate. The solvent was evaporated under reduced pressure to give a crude product (1.50 g) of N-hydroxy-2-methoxy-1-(4-(trifluoromethoxy)phenyl)ethanimine. To a solution of the obtained crude product (1.50 g) of N-hydroxy-2-methoxy-1-(4-(trifluoromethoxy)phenyl)ethanimine in methanol (50 mL) was added 10% palladium-carbon (containing water (50%), 0.20 g) at room temperature. The reaction mixture was stirred under a hydrogen atmosphere, and stirred at room temperature overnight. The insoluble material was filtered off, and the filtrate was concentrated under reduced pressure to give the title compound (1.3 g).
1H NMR (300 MHz, CDC13) δ 3.72-3.40 (4H, m), 3.49 (1H, dd, J = 9.0, 3.0 Hz), 4.22 (1H, dd, J = 8.1, 3.6 Hz), 7.20 (2H, d, J = 8.1 Hz), 7.42 (2H, d, J = 8.1 Hz).

### F) ethyl tert-butyl (3-nitropyridin-2-yl)propanedioate

To a suspension of potassium t-butoxide (26.6 g) in tetrahydrofuran (500 mL) was gradually added ethyl tert-butyl propanedioate (45 mL) at 60°C, a solution of 2-chloro-3-nitropyridine (25.0 g) in tetrahydrofuran (50 mL) was added at 60°C. The reaction mixture was heated under reflux for 3 hr, and the solvent was evaporated under reduced pressure. To the residue was added 1M hydrochloric acid (100 mL), and the mixture was extracted with ethyl acetate. The extract was washed with saturated brine, and dried over anhydrous sodium sulfate. The solvent was evaporated under reduced pressure to give the title compound (66.7 g).
1H NMR (400 MHz, CDC13) δ 1.30 (3H, t, J = 7.2 Hz), 1.49 (9H, s), 4.29-4.33 (2H, m), 5.43 (1H, s), 7.51 (1H, dd, J = 8.4, 4.2 Hz), 8.46 (1H, d, J = 8.4 Hz), 8.82 (1H, d, J = 5.2 Hz).

### G) ethyl (3-nitropyridin-2-yl)acetate

To a solution of ethyl tert-butyl (3-nitropyridin-2-yl)propanedioate (66.7 g) in dichloromethane (300 mL) was added trifluoroacetic acid (100 mL). The reaction mixture was stirred at room temperature overnight, and the solvent was evaporated under reduced pressure. To the residue was added aqueous sodium hydrogen carbonate solution (100 mL), and the mixture was extracted with ethyl acetate. The extract was washed with saturated brine, and dried over anhydrous sodium sulfate. The solvent was evaporated under reduced pressure to give the title compound (29.0 g).
1H NMR (400 MHz, CDC13) δ 1.26 (3H, t, J = 7.2 Hz), 4.20 (2H, q, J = 7.2 Hz), 4.33 (2H, s), 7.48 (1H, dd, J = 8.0, 1.2 Hz), 8.43 (1H, dd, J = 8.4, 1.2 Hz), 8.80 (1H, dd, J = 8.4, 1.2 Hz).

### H) ethyl (3-aminopyridin-2-yl)acetate

A mixture of ethyl (3-nitropyridin-2-yl)acetate (29.0 g) and 10% palladium-carbon (containing water (50%), 2.90 g) in ethanol (400 mL) was stirred under a hydrogen atmosphere (50 psi) at room temperature for 3 hr. The insoluble material was filtered off, and the filtrate was concentrated under reduced pressure to give the title compound (25.5 g).
1H NMR (400 MHz, CDC13) δ 1.26 (3H, t, J = 7.2 Hz), 3.85 (2H, s), 4.17 (2H, q, J = 7.2 Hz), 4.32 (2H, br s), 6.99-7.06 (2H, m), 7.99-8.01 (1H, m).

### I) diethoxyacetic acid

To a solution of ethyl diethoxyacetate (5.00 g) in ethanol (20 mL) was added 1M aqueous sodium hydroxide solution (60 mL). The reaction mixture was stirred at room temperature for 2 hr, and ethanol was evaporated under reduced pressure. An aqueous solution of the residue was washed with diethyl ether, and adjusted to pH 3 - 4 with 2M hydrochloric acid, and extracted with ethyl acetate. The extract was washed with saturated brine, and dried over anhydrous sodium sulfate. The solvent was evaporated under reduced pressure to give the title compound (3.00 g).
1H NMR (400 MHz, CDCl3) δ 1.27 (6H, t, J = 7.2 Hz), 3.64-3.77 (4H, m), 4.96 (1H, s), 8.91 (1H, br s).

### J) ethyl (3-((diethoxyacetyl)amino)pyridin-2-yl)acetate

To a solution of ethyl (3-aminopyridin-2-yl)acetate (25.5 g) and diethoxyacetic acid (23.0 g) in N,N-dimethylformamide (300 mL) was added diisopropylethylamine (70.3 mL), and O-(7-azabenzotriazol-1-yl)-N,N,N',N'-tetramethyluronium hexafluorophosphate (80.7 g) was added. The reaction mixture was stirred at room temperature overnight, water was added, and the mixture was extracted with ethyl acetate. The extract was washed with saturated aqueous sodium hydrogen carbonate solution, and dried over anhydrous sodium sulfate. The solvent was evaporated under reduced pressure. The residue was purified by silica gel column chromatography (petroleum ether/ethyl acetate) to give the title compound (26.0 g).
1H NMR (400 MHz, CDC13) δ 1.27-1.33 (9H, m), 3.69-3.83 (4H, m), 3.90 (2H, s), 4.20 (2H, q, J = 7.2 Hz), 4.97 (1H, s), 7.25-7.28 (1H, m), 8.29-8.35 (2H, m), 9.53 (1H, br s) .

### K) ethyl 2-oxo-1,2-dihydro-1,5-naphthyridine-4-carboxylate

To a solution of ethyl (3-((diethoxyacetyl)amino)pyridin-2-yl)acetate (3.00 g) in trifluoroacetic acid (50 mL) were added water (2 mL), and then 2 drops of a solution of trifluoroacetic acid solution (iodine (30 mg) of iodine in trifluoroacetic acid (10 mL). The reaction mixture was stirred at 50°C overnight, and the solvent was evaporated under reduced pressure. To the residue were added toluene (50 mL) and piperidine (3 mL), the reaction mixture was heated under reflux for 6 hr, and the solvent was evaporated under reduced pressure. The residue was purified by silica gel column chromatography (petroleum ether/ethyl acetate) to give the title compound (550 mg).
1H NMR (400 MHz, CDC13) δ 1.44 (3H, t, J = 7.2 Hz), 4.53 (2H, q, J = 7.2 Hz), 7.07 (1H, s), 7.48-7.51 (1H, m), 7.74-7.77 (1H, m), 8.66-8.68 (1H, m), 12.38 (1H, br s).

### L) 2-oxo-1,2-dihydro-1,5-naphthyridine-4-carboxylic acid

To a solution of ethyl 2-oxo-1,2-dihydro-1,5-naphthyridine-4-carboxylate (70 mg) in ethanol (5 mL) was added 2M aqueous sodium hydroxide solution (4 mL). The reaction mixture was stirred at room temperature for 2 hr, and ethanol was evaporated under reduced pressure. The residue was adjusted to pH 5 with 1M hydrochloric acid, and the obtained solid was collected by filtration to give the title compound (53 mg).
1H NMR (400 MHz, DMSO-d6) δ 7.02 (1H, s), 7.64-7.67 (1H, m), 7.80 (1H, d, J = 8.8 Hz), 8.55 (1H, d, J = 4.0 Hz), 12.26 (1H, d, J = 10.4 Hz), 14.86 (1H, br s).

### M) N-(2-methoxy-1-(4-(trifluoromethoxy)phenyl)ethyl)-2-oxo-1,2-dihydro-1,5-naphthyridine-4-carboxamide

To a solution of 2-oxo-1,2-dihydro-1,5-naphthyridine-4-carboxylic acid (300 mg) and triethylamine (800 mg) in tetrahydrofuran (10 mL) was added isobutyl chloroformate (301 mg). The reaction mixture was stirred at room temperature for 1.5 hr, and a solution of 2-methoxy-1-(4-(trifluoromethoxy)phenyl)ethanamine (557 mg) in tetrahydrofuran (2 mL) was gradually added. The reaction mixture was further stirred at room temperature for 2 hr, and the solvent was evaporated under reduced pressure. To the residue was added dichloromethane, and the mixture was washed with water and saturated brine, and dried over anhydrous sodium sulfate. The solvent was evaporated under reduced pressure. The residue was purified by silica gel column chromatography (dichloromethane/methanol), and the obtained solid was recrystallized from ethyl acetate to give the title compound (127 mg).
1H NMR (400 MHz, CDC13) δ 3.45 (3H, s), 3.80 (2H, s), 5.45 (1H, d, J = 6.8 Hz), 7.19 (2H, d, J = 8.0 Hz), 7.48 (2H, d, J = 8.4 Hz), 7.56 (1H, dd, J = 8.4, 4.0 Hz), 7.92-7.93 (2H, m), 8.62 (1H, d, J=4.0Hz), 11.74 (1H, d, J=7.6Hz), 13.04 (1H, br s) .

### Example 2

### N-((1S)-2-methoxy-1-(4-(trifluoromethoxy)phenyl)ethyl)-6-methylpyrazolo[1,5-a]pyrimidine-3-carboxamide

### A) ethyl 6-methylpyrazolo[1,5-a]pyrimidine-3-carboxylate

A solution of ethyl 3-amino-1H-pyrazole-4-carboxylate (10.0 g) and (E)-3-ethoxy-2-methylacrylic aldehyde (7.66 mL) in acetic acid (70 mL) was stirred at 100°C for 4 hr, and the solvent was evaporated under reduced pressure. To the residue was added aqueous sodium hydrogen carbonate solution, and the mixture was extracted with ethyl acetate. The extract was washed with saturated brine, and dried over anhydrous sodium sulfate. The solvent was evaporated under reduced pressure to give the title compound (13.7 g).
MS (API+): [M+H]+206.2.

### B) 6-methylpyrazolo[1,5-a]pyrimidine-3-carboxylic acid

To a mixed solution of ethyl 6-methylpyrazolo[1,5-a]pyrimidine-3-carboxylate (6.44 g) in tetrahydrofuran (20 mL) and methanol (20 mL) was added 2M aqueous sodium hydroxide solution (23.5 mL). The reaction mixture was stirred at 60°C overnight, and acidified with 10% aqueous citric acid solution. The precipitated solid was collected by filtration, and washed with water to give the title compound (3.91 g).
MS (API+): [M+H]+178.0.

### C) N-(2-methoxy-1-(4-(trifluoromethoxy)phenyl)ethyl)-6-methylpyrazolo[1,5-a]pyrimidine-3-carboxamide

To a solution of 6-methylpyrazolo[1,5-a]pyrimidine-3-carboxylic acid (299 mg) and 2-methoxy-1-(4-(trifluoromethoxy)phenyl)ethanamine (436 mg) in N,N-dimethylformamide (6 mL) were added 1-hydroxybenzotriazole monohydrate (310 mg) and 1-(3-dimethylaminopropyl)-3-ethylcarbodiimide hydrochloride (388 mg). The reaction mixture was stirred at room temperature for 2 hr, poured into water, and the mixture was extracted with ethyl acetate. The extract was washed with water, and dried over anhydrous sodium sulfate. The solvent was evaporated under reduced pressure. The residue was purified by silica gel column chromatography (hexane/ethyl acetate), and then silica gel column chromatography (NH, hexane/ethyl acetate), and the obtained solid was washed with hexane/ethyl acetate to give the title compound (473 mg).
MS (API+): [M+H]+395.1.

### D) N-((1S)-2-methoxy-1-(4- (trifluoromethoxy)phenyl)ethyl)-6-methylpyrazolo[1,5-a]pyrimidine-3-carboxamide

The racemate (398 mg) of N-(2-methoxy-1-(4-(trifluoromethoxy)phenyl)ethyl)-6-methylpyrazolo[1,5-a]pyrimidine-3-carboxamide was resolved by HPLC (column: CHIRALPAK AD, 50 mmIDx500 mmL, manufactured by Daicel Corporation, mobile phase:hexane/ethanol = 700/300) to give the title compound (193 mg) having a shorter retention time.
MS (API+): [M+H]+395.3.

### Example 3

### N-(2-methoxy-1-(4-(trifluoromethoxy)phenyl)ethyl)pyrazolo[1,5-a]pyrimidine-7-carboxamide

### A) 2-methoxy-1-(4-(trifluoromethoxy)phenyl)ethanamine hydrochloride

To a solution of 2-methoxy-1-(4-(trifluoromethoxy)phenyl)ethanone (4.78 g) in ethanol (120 mL) were added hydroxylamine hydrochloride (2.84 g) and triethylamine (5.69 mL) at room temperature. The reaction mixture was stirred at room temperature for 5.5 hr, and the solvent was evaporated under reduced pressure. To the residue was added water, and the mixture was extracted with ethyl acetate. The extract was washed with water and saturated brine, and dried over anhydrous magnesium sulfate. The solvent was evaporated under reduced pressure to give a crude product of N-hydroxy-2-methoxy-1-(4-(trifluoromethoxy)phenyl)ethanimine. To a solution of the obtained crude product in ethanol (160 mL) was added 10% palladium-carbon (containing water (50%), 350 mg). The reaction mixture was stirred under a hydrogen atmosphere at room temperature for 16 hr, the catalyst was filtered off, and the filtrate was concentrated under reduced pressure. To a solution of the residue in ethyl acetate (10 mL) was added 4M hydrogen chloride/ethyl acetate solution (100 mL), the mixture was stirred at room temperature for 2 hr, and the solvent was evaporated under reduced pressure. The residue was washed with diisopropyl ether to give the title compound (4.2 g).
1H NMR (300 MHz, DMSO-d6) δ 3.34 (3H, s), 3.58-3.74 (2H, m), 4.58 (1H, dd, J = 6.6, 5.5 Hz), 7.46 (2H, d, J = 8.7 Hz), 7.65 (2H, d, J = 8.3 Hz), 8.57 (3H, brs).

### B) ethyl pyrazolo[1,5-a]pyrimidine-7-carboxylate

To ethoxyethene (5.28 g) was added ethyl chloro(oxo)acetate (5 g) at 0°C, the reaction mixture was stirred at room temperature overnight, and concentrated under reduced pressure to give a crude product of ethyl 4-ethoxy-2-oxobut-3-enoate (8.21 g). A mixture of the obtained ethyl 4-ethoxy-2-oxobut-3-enoate (3 g) and 1H-pyrazole-3-amine and acetic acid (40 mL) was stirred at 100°C for 2 hr. The reaction mixture was concentrated under reduced pressure, and the residue was purified by silica gel column chromatography (hexane/ethyl acetate) to give the title compound (750 mg) containing isomer.
MS (API+): [M+H]+192.1.

### C) pyrazolo[1,5-a]pyrimidine-7-carboxylic acid

A mixture of ethyl pyrazolo[1,5-a]pyrimidine-7-carboxylate (750 mg) containing isomer, 1M aqueous sodium hydroxide solution (10 mL) and tetrahydrofuran (15 mL) was stirred at room temperature for 3 hr. The reaction mixture was added to 1M hydrochloric acid, and the mixture was extracted with ethyl acetate. The organic layer was washed with saturated brine, dried over anhydrous magnesium sulfate, and concentrated under reduced pressure to give the title compound (650 mg) containing isomer.
1H NMR (300 MHz, DMSO-d6) δ 6.90 (1H, d, J = 2.6 Hz), 7.39 (1H, d, J = 4.1 Hz), 8.33 (1H, d, J = 2.3 Hz), 8.67 (1H, d, J = 4.1 Hz), 12.81-15.98 (1H, m).

### D) N-(2-methoxy-1-(4-(trifluoromethoxy)phenyl)ethyl)pyrazolo[1,5-a]pyrimidine-7-carboxamide

A mixture of pyrazolo[1,5-a]pyrimidine-7-carboxylic acid (430 mg) containing isomer, 2-methoxy-1-(4-(trifluoromethoxy)phenyl)ethanamine hydrochloride (716 mg), 1-hydroxybenzotriazole monohydrate (484 mg), 1-(3-dimethylaminopropyl)-3-ethylcarbodiimide hydrochloride (606 mg), triethylamine (0.735 mL) and N,N-dimethylformamide (15 mL) was stirred at room temperature overnight, and stirred at 100°C for 5 hr. The reaction mixture was added to saturated aqueous sodium hydrogen carbonate solution, and the mixture was extracted with ethyl acetate. The organic layer was washed with saturated aqueous sodium hydrogen carbonate solution and saturated brine, dried over anhydrous magnesium sulfate, and concentrated under reduced pressure. The residue was purified by silica gel column chromatography (hexane/ethyl acetate) to give the title compound (496 mg).
MS (API+): [M+H]+381.1.

### Example 4

### N-(2-methoxy-1-(4-(trifluoromethoxy)phenyl)ethyl)-2-methylpyrazolo[1,5-a]pyrimidine-7-carboxamide

By a method similar to that in Example 3, the title compound was obtained.
MS (API+): [M+H]+395.1.

### Example 5

### 3-chloro-N-(2-methoxy-1-(4-(trifluoromethoxy)phenyl)ethyl)pyrazolo[1,5-a]pyrimidine-7-carboxamide

A mixture of N-(2-methoxy-1-(4-(trifluoromethoxy)phenyl)ethyl)pyrazolo[1,5-a]pyrimidine-7-carboxamide (90 mg), N-chlorosuccinimide (34.8 mg) and acetonitrile (3 mL) was stirred at 100°C overnight. To the reaction mixture was added silica gel, the mixture was concentrated under reduced pressure, and the residue was purified by silica gel column chromatography (hexane/ethyl acetate) to give the title compound (97 mg).
MS (API+): [M+H]+415.1.

### Example 6

### N-(2-methoxy-1-(4-(trifluoromethoxy)phenyl)ethyl)-5-methylpyrazolo[1,5-a]pyrimidine-7-carboxamide

By a method similar to that in Example 3, the title compound was obtained.
MS (API+): [M+H]+395.1.

### Example 7

### N-(2-methoxy-1-(4-(trifluoromethoxy)phenyl)ethyl)[1,2,4]triazolo[1,5-a]pyrimidine-7-carboxamide

By a method similar to that in Example 3, the title compound was obtained.
MS (API+): [M+H]+382.1.

### Example 8

### N-(2-methoxy-1-(4-(trifluoromethoxy)phenyl)ethyl)-1,6-naphthyridine-8-carboxamide

To a solution of 1,6-naphthyridine-8-carboxylic acid (132 mg), 2-methoxy-1-(4-(trifluoromethoxy)phenyl)ethanamine hydrochloride (216 mg) and triethylamine (0.158 mL) in N,N-dimethylformamide (6 mL) were added 1-hydroxybenzotriazole monohydrate (139 mg) and 1-(3-dimethylaminopropyl)-3-ethylcarbodiimide hydrochloride (174 mg). The reaction mixture was stirred at room temperature for 1.5 hr, aqueous sodium hydrogen carbonate solution was added, and the mixture was extracted with ethyl acetate. The extract was washed with water, and dried over anhydrous sodium sulfate. The solvent was evaporated under reduced pressure. The residue was purified by silica gel column chromatography (hexane/ethyl acetate), and the obtained solid was washed with hexane/ethyl acetate to give the title compound (189 mg).
MS (API+): [M+H]+392.1.

### Example 9

### N-(2-methoxy-1-(4-(trifluoromethoxy)phenyl)ethyl)-3H-imidazo[4,5-b]pyridine-7-carboxamide

By a method similar to that in Example 8, the title compound was obtained.
MS (ESI+): [M+H]+380.1.

### Example 10

### N-(2-methoxy-1-(4-(trifluoromethoxy)phenyl)ethyl)-7H-purine-6-carboxamide

### A) butyl 7H-purine-6-carboxylate

To a mixture of 6-chloro-7H-purine (200 mg), acetonitrile (15 mL) and n-butanol (15 mL) were added N,N-diisopropylethylamine (0.23 mL), palladium(II) acetate (9 mg) and 1,1'-bis(diphenylphosphino)ferrocene (29 mg) at room temperature. The reaction mixture was stirred under carbon monoxide atmosphere (120 psi) in an autoclave (manufactured by Parr) at 80°C for 16 hr. The reaction mixture was cooled to room temperature, and volatile product was removed under reduced pressure. The residue was diluted with water and the mixture was extracted with ethyl acetate. The organic layer was washed with water and saturated brine, dried over anhydrous sodium sulfate, and concentrated under reduced pressure. The residue was purified by silica gel column chromatography (dichloromethane/methanol) to give the title compound (200 mg).
MS (API+): [M+H]+221.5.

### B) 7H-purine-6-carboxylic acid

To a mixture of butyl 7H-purine-6-carboxylate (280 mg) and tetrahydrofuran (12 mL) was added 1M aqueous lithium hydroxide solution (2.5 mL), and the mixture was stirred at room temperature for 1 hr. The reaction mixture was concentrated under reduced pressure, and the residue was neutralized with 4M hydrogen chloride/dioxane solution, and concentrated under reduced pressure to give the title compound (200 mg).
1H NMR (400 MHz, DMSO-d6) δ 8.75 (1H, s), 9.05 (1H, s), 13.35 (1H, br s).

### C) N-(2-methoxy-1-(4-(trifluoromethoxy)phenyl)ethyl)-7H-purine-6-carboxamide

To a solution of 7H-purine-6-carboxylic acid (100 mg) in N,N-dimethylformamide (10 mL) were added triethylamine (0.2 mL), 1-hydroxybenzotriazole anhydride (115.2 mg), 1-(3-dimethylaminopropyl)-3-ethylcarbodiimide hydrochloride (115.2 mg), 2-methoxy-1-(4-(trifluoromethoxy)phenyl)ethanamine hydrochloride (115.7 mg) and N,N-dimethyl-4-aminopyridine (1.5 mg), and the mixture was stirred at 60°C for 3 hr. The reaction mixture was allowed to cool to room temperature, saturated aqueous ammonium chloride solution was added, and the organic product was extracted with dichloromethane. The organic layer was washed with water and saturated brine, dried over anhydrous sodium sulfate, and concentrated under reduced pressure. The residue was purified by HPLC (C18, mobile phase: water/acetonitrile (0.05% TFA-containing system)) to give the title compound (15 mg).
MS (API+): [M+H]+382.0.

### Example 11

### N-(2-methoxy-1-(4-(trifluoromethoxy)phenyl)ethyl)-4-oxo-4,5-dihydro-1H-pyrazolo[4,3-c]pyridine-3-carboxamide

### A) 1-(tert-butyl)-4-methoxy-1H-pyrazolo[4,3-c]pyridine-3-carbonitrile

A mixture of 1-(tert-butyl)-4-methoxy-1H-pyrazolo[4,3-c]pyridin-3-yl trifluoromethanesulfonate (353 mg), zinc cyanide (70.4 mg) and tetrakis(triphenylphosphine)palladium(0) (115 mg) in N,N-dimethylformamide (10 mL) was stirred under a nitrogen stream at 100°C overnight. To the reaction mixture was added water at room temperature, and the mixture was extracted with ethyl acetate. The organic layer was dried over anhydrous sodium sulfate, and the solvent was evaporated under reduced pressure. The residue was purified by silica gel column chromatography (hexane/ethyl acetate) to give the title compound (110 mg).
MS (API+): [M+H]+231.1.

### B) 1-(tert-butyl)-4-methoxy-1H-pyrazolo[4,3-c]pyridine-3-carboxylic acid

To a mixture of 1-(tert-butyl)-4-methoxy-1H-pyrazolo[4,3-c]pyridine-3-carbonitrile (100 mg) in methanol (50 mL) was added 2M aqueous sodium hydroxide solution (10 mL) at room temperature, and the mixture was stirred at 100°C for 120 hr. The reaction mixture was neutralized with 1M hydrochloric acid (10 ml) under ice-cooling, and extracted with ethyl acetate. The organic layer was dried over anhydrous sodium sulfate, and the solvent was evaporated under reduced pressure to give the title compound (100 mg).
MS (API+): [M+H]+250.1.

### C) 1-(tert-butyl)-4-methoxy-N-(2-methoxy-1-(4-(trifluoromethoxy)phenyl)ethyl)-1H-pyrazolo[4,3-c]pyridine-3-carboxamide

By a method similar to that in Example 8, the title compound was obtained.
MS (API+): [M+H]+467.1.

### D) 1-(tert-butyl)-N-(2-methoxy-1-(4-(trifluoromethoxy)phenyl)ethyl)-4-oxo-4,5-dihydro-1H-pyrazolo[4,3-c]pyridine-3-carboxamide

A mixture of 1-(tert-butyl)-4-methoxy-N-(2-methoxy-1-(4-(trifluoromethoxy)phenyl)ethyl)-1H-pyrazolo[4,3-c]pyridine-3-carboxamide (100 mg), chlorotrimethylsilane (0.219 mL) and sodium iodide (64 mg) in acetonitrile (10 mL) was stirred at 60°C for 2 hr, and the solvent was evaporated under reduced pressure. The residue was purified by silica gel column chromatography (ethyl acetate/methanol) to give the title compound (12 mg).
MS (API+): [M+H]+453.2.

### E) N-(2-methoxy-1-(4-(trifluoromethoxy)phenyl)ethyl)-4-oxo-4,5-dihydro-1H-pyrazolo[4,3-c]pyridine-3-carboxamide

A mixture of 1-(tert-butyl)-N-(2-methoxy-1-(4-(trifluoromethoxy)phenyl)ethyl)-4-oxo-4,5-dihydro-1H-pyrazolo[4,3-c]pyridine-3-carboxamide (30 mg) in trifluoroacetic acid (10 mL) and water (1 mL) was stirred at 120°C for 120 hr, and the solvent was evaporated under reduced pressure. The residue was purified by silica gel column chromatography (ethyl acetate/methanol) to give the title compound (3 mg).
MS (API+): [M+H]+397.1.

### Example 12

### N-((1S)-1-(3-fluoro-4-(trifluoromethoxy)phenyl)-2-methoxyethyl)-6-methylpyrazolo[1,5-a]pyrimidine-3-carboxamide

### A) N,2-dimethoxy-N-methylacetamide

To a mixture of N,O-dimethylhydroxylamine hydrochloride (24.7 g) and potassium carbonate (63.7 g) in acetonitrile (330 mL) was added 2-methoxyacetyl chloride (25.0 g) at not more than 10°C. The reaction mixture was stirred at room temperature overnight, the insoluble material was filtered off, and the filtrate was concentrated under reduced pressure. The residue was distilled (boiling point: 60°C/0.8 KPa) to give the title compound (25.8 g).
1H NMR (300 MHz, CDC13) δ 3.20 (3H, s), 3.47 (3H, d, J = 0.8 Hz), 3.69 (3H, s), 4.22 (2H, s).

### B) 1-(3-fluoro-4-(trifluoromethoxy)phenyl)-2-methoxyethanone

To a mixture of 4-bromo-2-fluoro-1-(trifluoromethoxy)benzene (25.0 g) and N,2-dimethoxy-N-methylacetamide (15.4 g) and tetrahydrofuran (400 mL) was gradually added 1.6M n-butyllithium/hexane solution (72.4 mL) under a nitrogen atmosphere at -78°C. The reaction mixture was stirred under a nitrogen atmosphere at -78°C for 20 min. After stirring, the mixture was neutralized with 0.1M hydrochloric acid, and extracted with ethyl acetate. The extract was washed with water and saturated brine, and dried over anhydrous sodium sulfate. The solvent was evaporated under reduced pressure. The residue was purified by silica gel chromatography (hexane/ethyl acetate) to give the title compound (15.7 g).
1H NMR (300 MHz, CDC13) δ 3.50 (3H, s), 4.63 (2H, s), 7.42 (1H, ddd, J = 8.6, 7.3, 1.5 Hz), 7.75-7.81 (1H, m), 7.83 (1H, dd, J = 10.2, 1.9 Hz).

### C) 1-(3-fluoro-4-(trifluoromethoxy)phenyl)-N-hydroxy-2-methoxyethanimine

To a solution of 1-(3-fluoro-4-(trifluoromethoxy)phenyl)-2-methoxyethanone (7.56 g) in ethanol (250 mL) were added hydroxylamine hydrochloride (4.17 g) and triethylamine (8.36 mL) at room temperature. The reaction mixture was stirred at room temperature for 16 hr, and the solvent was evaporated under reduced pressure. To the residue was added water, and the mixture was extracted with ethyl acetate. The extract was washed with water and saturated brine, and dried over anhydrous magnesium sulfate. The solvent was evaporated under reduced pressure. The residue was purified by silica gel chromatography (hexane/ethyl acetate) to give the title compound (7.88 g).
MS (API+): [M+H]+268.0.

### D) tert-butyl (1-(3-fluoro-4-(trifluoromethoxy)phenyl)-2-methoxyethyl)carbamate

To a solution of 1-(3-fluoro-4-(trifluoromethoxy)phenyl)-N-hydroxy-2-methoxyethanimine (7.88 g) in ethanol (200 mL) was added 10% palladium-carbon (containing water (50%), 1.00 g). The reaction mixture was stirred under a hydrogen atmosphere, and stirred at room temperature overnight. The insoluble material was filtered off, and the filtrate was concentrated under reduced pressure. To a solution of the residue in tetrahydrofuran (200 mL) were added di-tert-butyl dicarbonate (7.53 mL) and triethylamine (6.17 mL), and the mixture was stirred at room temperature for 16 hr. Water was added to the reaction mixture, and the mixture was extracted with ethyl acetate. The extract was washed with saturated brine, and dried over anhydrous magnesium sulfate. The solvent was evaporated under reduced pressure. The residue was purified by silica gel column chromatography (hexane/ethyl acetate) to give the title compound (8.25 g).
1H NMR (300 MHz, CDC13) δ 1.42 (9H, br s), 3.35 (3H, s), 3.49-3.67 (2H, m), 4.78 (1H, br s), 5.34 (1H, br s), 7.08-7.29 (3H, m) .

### E) tert-butyl ((1S)-1-(3-fluoro-4-(trifluoromethoxy)phenyl)-2-methoxyethyl)carbamate

The racemate (12.48 g) of tert-butyl (1-(3-fluoro-4-(trifluoromethoxy)phenyl)-2-methoxyethyl)carbamate was resolved by HPLC (column: CHIRALPAK AD, 50 mmIDx500 mmL, manufactured by Daicel Corporation, mobile phase:hexane/ethanol = 950/50) to give the title compound (5.73 g) having a shorter retention time.
MS (API+) , found: 254.0.

### F) (1S)-1-(3-fluoro-4-(trifluoromethoxy)phenyl)-2-methoxyethanamine hydrochloride

A mixture of tert-butyl ((1S)-1-(3-fluoro-4-(trifluoromethoxy)phenyl)-2-methoxyethyl)carbamate (5.73 g) and 4M hydrogen chloride/ethyl acetate solution (100 mL) was stirred at room temperature for 3 hr. The solvent was evaporated under reduced pressure, and the residue was washed with diisopropyl ether to give the title compound (3.22 g).
MS (API+), found: 254.0.

### G) N-((1S)-1-(3-fluoro-4-(trifluoromethoxy)phenyl)-2-methoxyethyl)-6-methylpyrazolo[1,5-a]pyrimidine-3-carboxamide

By a method similar to that in Example 8, the title compound was obtained from 6-methylpyrazolo[1,5-a]pyrimidine-3-carboxylic acid and (1S)-1-(3-fluoro-4-(trifluoromethoxy)phenyl)-2-methoxyethanamine hydrochloride.
MS (API+): [M+H]+413.1.

### Example 13

### N-((1S)-1-(3-fluoro-4-(trifluoromethoxy)phenyl)-2-methoxyethyl)-2-oxo-1,2-dihydro-1,5-naphthyridine-4-carboxamide

To a solution of 2-oxo-1,2-dihydro-1,5-naphthyridine-4-carboxylic acid (106 mg), (1S)-1-(3-fluoro-4-(trifluoromethoxy)phenyl)-2-methoxyethanamine hydrochloride (177 mg) and diisopropylethylamine (0.292 mL) in N,N-dimethylformamide (5 mL) was added 0-(7-azabenzotriazol-1-yl)-N,N,N',N'-tetramethyluronium hexafluorophosphate (317 mg). The reaction mixture was stirred at 60°C for 2.5 hr, and poured into water, and the mixture was extracted with ethyl acetate. The extract was washed with saturated brine, and dried over anhydrous sodium sulfate. The solvent was evaporated under reduced pressure. The residue was purified by silica gel column chromatography (hexane/ethyl acetate, ethyl acetate, later ethyl acetate/methanol), and the obtained solid was washed with hexane/ethyl acetate to give the title compound (216 mg).
MS (API+): [M+H]+426.1.

### Example 14

### N-(1-(3-fluoro-4-(trifluoromethoxy)phenyl)-2-hydroxy-2-methylpropyl)-6-methylpyrazolo[1,5-a]pyrimidine-3-carboxamide

### A) ethyl 6-methylpyrazolo[1,5-a]pyrimidine-3-carboxylate

A solution of ethyl 3-amino-1H-pyrazole-4-carboxylate (10.0 g) and (E)-3-ethoxy-2-methylacrylic aldehyde (7.66 mL) in acetic acid (70 mL) was stirred at 100°C for 4 hr, and the solvent was evaporated under reduced pressure. To the residue was added aqueous sodium hydrogen carbonate solution, and the mixture was extracted with ethyl acetate. The extract was washed with saturated brine, and dried over anhydrous sodium sulfate. The solvent was evaporated under reduced pressure to give the title compound (13.7 g).
MS (API+): [M+H]+206.2.

### B) 6-methylpyrazolo[1,5-a]pyrimidine-3-carboxylic acid

To a mixed solution of ethyl 6-methylpyrazolo[1,5-a]pyrimidine-3-carboxylate (6.44 g) in tetrahydrofuran (20 mL) and methanol (20 mL) was added 2M aqueous sodium hydroxide solution (23.5 mL). The reaction mixture was stirred at 60°C overnight, and acidified with 10% aqueous citric acid solution. The precipitated solid was collected by filtration, and washed with water to give the title compound (3.91 g).
MS (API+): [M+H]+178.0.

### C) 2-amino-2-(3-fluoro-4-(trifluoromethoxy)phenyl)acetonitrile

3-Fluoro-4-(trifluoromethoxy)benzaldehyde (10 g) was dissolved in 2M ammonia/methanol solution (96 mL), and titanium (IV) tetraisopropoxide (15.5 mL) was added under ice-cooling. The reaction mixture was stirred at the same temperature for 15 min, trimethylsilane carbonitrile (9.61 mL) was added, and the mixture was stirred at room temperature for 20 hr. The solvent was evaporated under reduced pressure, and to the residue were added ethyl acetate and saturated aqueous sodium hydrogen carbonate solution. The insoluble material was filtered off through celite, and the filtrate was extracted with ethyl acetate. The extract was washed with water and saturated brine, and dried over anhydrous magnesium sulfate. The solvent was evaporated under reduced pressure. The residue was purified by silica gel column chromatography (ethyl acetate/hexane) to give the title compound (9.4 g).
1H NMR (300 MHz, CDC13) δ 2.01 (2H, br s), 4.93 (1H, s), 7.29-7.53 (3H, m).

### D) tert-butyl (cyano(3-fluoro-4-(trifluoromethoxy)phenyl)methyl)carbamate

To a solution of 2-amino-2-(3-fluoro-4-(trifluoromethoxy)phenyl)acetonitrile (9.4 g) in tetrahydrofuran (200 mL) were added di-tert-butyl dicarbonate (10.3 mL) and triethylamine (7.27 mL) at room temperature. The reaction mixture was stirred at 40°C for 20 hr, water was added, and the mixture was extracted with ethyl acetate. The extract was washed with saturated brine, and dried over anhydrous magnesium sulfate. The solvent was evaporated under reduced pressure. The residue was purified by silica gel column chromatography (ethyl acetate/hexane) to give the title compound (8.47 g).
MS (API+): [M+H]+335.1.

### E) tert-butyl (2-amino-1-(3-fluoro-4-(trifluoromethoxy)phenyl)-2-oxoethyl)carbamate

To a mixture of tert-butyl (cyano(3-fluoro-4-(trifluoromethoxy)phenyl)methyl)carbamate (9.4 g), potassium carbonate (3.89 g) and dimethyl sulfoxide (200 mL) was added 35% aqueous hydrogen peroxide (4.66 mL) at room temperature. The reaction mixture was stirred at room temperature for 4 hr, water was added, and the mixture was extracted with ethyl acetate. The extract was washed with water, and then with saturated brine, and dried over anhydrous magnesium sulfate. The solvent was evaporated under reduced pressure. The residue was purified by silica gel column chromatography (ethyl acetate/hexane) to give the title compound (3.84 g).
MS (API+) , found: 253.0.

### F) methyl 2-((tert-butoxycarbonyl)amino)-2-(3-fluoro-4-(trifluoromethoxy)phenyl)acetate

To a solution of tert-butyl (2-amino-1-(3-fluoro-4-(trifluoromethoxy)phenyl)-2-oxoethyl)carbamate (3.84 g) in methanol (100 mL) was added 8M aqueous sodium hydroxide solution (2.8 mL) at room temperature. The reaction mixture was heated under reflux overnight, and ethanol was evaporated under reduced pressure. The residue was neutralized with 1M hydrochloric acid, and extracted with ethyl acetate. The extract was washed with saturated brine, and dried over anhydrous magnesium sulfate. The solvent was evaporated under reduced pressure. To a mixture of the residue (3.85 g) and potassium carbonate (1.81 g) in N,N-dimethylformamide (50 mL) was added methyl iodide (0.750 mL) at room temperature. The reaction mixture was stirred at room temperature for 2 hr, water was added, and the mixture was extracted with ethyl acetate. The extract was washed with water and saturated brine, and dried over anhydrous magnesium sulfate. The solvent was evaporated under reduced pressure. The residue was purified by silica gel column chromatography (hexane/ethyl acetate) to give the title compound (1.90 g).
MS (API+) , found: 268.0.

### G) tert-butyl (1-(3-fluoro-4-(trifluoromethoxy)phenyl)-2-hydroxy-2-methylpropyl)carbamate

To a solution of methyl 2-((tert-butoxycarbonyl)amino)-2-(3-fluoro-4-(trifluoromethoxy)phenyl)acetate (1.90 g) in tetrahydrofuran (60 mL) was gradually added 3M methylmagnesium bromide/diethyl ether solution (5.17 mL) at 0°C. The reaction mixture was stirred at room temperature for 3 hr, 1M hydrochloric acid was added at 0°C, and the mixture was extracted with ethyl acetate. The extract was washed with water and saturated brine, and dried over anhydrous magnesium sulfate. The solvent was evaporated under reduced pressure. The residue was purified by silica gel column chromatography (hexane/ethyl acetate) to give the title compound (1.53 g).
1H NMR (300 MHz, CDC13) δ 1.06 (3H, s), 1.29-1.50 (13H, m), 4.38-4.52 (1H, m), 5.47-5.60 (1H, m), 7.07-7.30 (3H, m).

### H) 1-amino-1-(3-fluoro-4-(trifluoromethoxy)phenyl)-2-methylpropan-2-ol hydrochloride

To tert-butyl (1-(3-fluoro-4-(trifluoromethoxy)phenyl)-2-hydroxy-2-methylpropyl)carbamate (3.01 g) was added 4M hydrogen chloride/ethyl acetate solution (30 mL). The reaction mixture was stirred at room temperature for 1 hr, and the solvent was evaporated under reduced pressure. The residue was washed with diisopropyl ether to give the title compound (2.22 g).
MS (API+) , found: 268.1.

### I) -N-(1-(3-fluoro-4-(trifluoromethoxy)phenyl)-2-hydroxy-2 methylpropyl)-6-methylpyrazolo[1,5-a]pyrimidine-3-carboxamide

To a solution of 6-methylpyrazolo[1,5-a]pyrimidine-3-carboxylic acid (122 mg) and 1-amino-1-(3-fluoro-4-(trifluoromethoxy)phenyl)-2-methylpropan-2-ol hydrochloride (220 mg) in N,N-dimethylformamide (6 mL) were added 1-hydroxybenzotriazole monohydrate (127 mg), 1-(3-dimethylaminopropyl)-3-ethylcarbodiimide hydrochloride (158 mg) and triethylamine (0.288 mL). The reaction mixture was stirred at room temperature for 16 hr, poured into water, and the mixture was extracted with ethyl acetate. The extract was washed with water, and dried over anhydrous sodium sulfate. The solvent was evaporated under reduced pressure. The obtained solid was washed with hexane/ethyl acetate to give the title compound (271 mg).
MS (API+): [M+H]+427.2.
1H NMR (300 MHz, DMSO-d6) δ 1.03 (3H, s), 1.26 (3H, s), 2.40 (3H, s), 4.95 (1H, d, J = 8.7 Hz), 4.99 (1H, s), 7.30-7.38 (1H, m), 7.42-7.55 (2H, m), 8.44 (1H, s), 8.78-8.86 (2H, m), 9.16-9.22 (1H, m).

### Example 15

### N-((1S)-1-(3-fluoro-4-(trifluoromethoxy)phenyl)-2-hydroxy-2-methylpropyl)-6-methylpyrazolo[1,5-a]pyrimidine-3-carboxamide

The racemate (250 mg) of N-(1-(3-fluoro-4-(trifluoromethoxy)phenyl)-2-hydroxy-2-methylpropyl)-6-methylpyrazolo[1,5-a]pyrimidine-3-carboxamide was resolved by HPLC (column: CHIRALPAK AD, 50 mmIDx500 mmL, manufactured by Daicel Corporation, mobile phase:hexane/ethanol = 700/300) and crystallized from hexane/ethyl acetate to give the title compound (88.9 mg) having a shorter retention time.
MS (API+): [M+H]+427.2.
1H NMR (300 MHz, DMSO-d6) δ 1.03 (3H, s), 1.26 (3H, s), 2.40 (3H, s), 4.91-5.03 (2H, m), 7.30-7.38 (1H, m), 7.43-7.55 (2H, m), 8.44 (1H, s), 8.78-8.88 (2H, m), 9.17-9.22 (1H, m) .

### Example 16

### N-((1R)-1-(3-fluoro-4-(trifluoromethoxy)phenyl)-2-hydroxy-2-methylpropyl)-6-methylpyrazolo[1,5-a]pyrimidine-3-carboxamide

The racemate (250 mg) of N-(1-(3-fluoro-4-(trifluoromethoxy)phenyl)-2-hydroxy-2-methylpropyl)-6-methylpyrazolo[1,5-a]pyrimidine-3-carboxamide was resolved by HPLC (column: CHIRALPAK AD, 50 mmIDx500 mmL, manufactured by Daicel Corporation, mobile phase:hexane/ethanol = 700/300), and crystallized from hexane/ethyl acetate to give the title compound (87.7 mg) having a longer retention time.
MS (API+): [M+H]+427.2.
1H NMR (300 MHz, DMSO-d6) δ 1.03 (3H, s), 1.26 (3H, s), 2.40 (3H, s), 4.90-5.03 (2H, m), 7.30-7.38 (1H, m), 7.41-7.56 (2H, m), 8.44 (1H, s), 8.77-8.88 (2H, m), 9.15-9.24 (1H, m).

### Example 17

### N-(2-methoxy-1-(4-(1H-pyrazol-1-yl)phenyl)ethyl)-6-methylpyrazolo[1,5-a]pyrimidine-3-carboxamide

### A) 1-(4-bromophenyl)-2-methoxyethanone

To a solution of 2-bromo-1-(4-bromophenyl)ethanone (15.6 g) in methanol (100 mL) were added silver(I) carbonate (20.0 g) and boron trifluoride diethyl ether complex (8.7 mL) at room temperature. The reaction mixture was stirred under a nitrogen atmosphere at 60°C for 16 hr, the insoluble material was filtered off, and the filtrate was concentrated under reduced pressure. To the residue were added ethyl acetate and saturated brine, and the insoluble material was filtered off. The organic layer and the aqueous layer of the filtrate were separated, and the aqueous layer was extracted with ethyl acetate. The extract was dried over anhydrous sodium sulfate, and the solvent was evaporated under reduced pressure to give the title compound (11.6 g).
MS (API+): [M+H]+229.0.

### B) 2-(4-bromophenyl)-2-(methoxymethyl)-1,3-dioxolane

A mixture of 1-(4-bromophenyl)-2-methoxyethanone (3.00 g), p-toluenesulfonic acid monohydrate (0.225 g), ethylene glycol (4.06 g) and toluene (100 mL) was heated under reflux for 16 hr using Dean-Stark, and the solvent was evaporated under reduced pressure. The residue was purified by silica gel column chromatography (petroleum ether/ethyl acetate) to give the title compound (3.00 g).
1H NMR (400 MHz, CDC13) δ 3.40 (3H, s), 3.57 (2H, s), 3.83-3.86 (2H, m), 4.10-4.13 (2H, m), 7.38-7.41 (2H, m), 7.47-7.49 (2H, m) .

### C) 1-(4-(2-(methoxymethyl)-1,3-dioxolan-2-yl)phenyl)-1H-pyrazole

A mixture of 2-(4-bromophenyl)-2-(methoxymethyl)-1,3-dioxolane (3.0 g), 1H-pyrazole (0.748 g), quinolin-8-ol (0.319 g), copper(I) iodide (0.209 g), potassium carbonate (3.00 g) and dimethyl sulfoxide (50 mL) was stirred under a nitrogen atmosphere at 140°C overnight, and the insoluble material was filtered off through celite. The filtrate was poured into saturated aqueous ammonium chloride solution, and the mixture was extracted with ethyl acetate. The extract was dried over anhydrous magnesium sulfate, and the solvent was evaporated under reduced pressure. The residue was purified by silica gel column chromatography (hexane/ethyl acetate) to give the title compound (2.80 g).
MS (API+): [M+H]+261.2.

### D) 2-methoxy-1-(4-(1H-pyrazol-1-yl)phenyl)ethanone

A mixture of 1-(4-(2-(methoxymethyl)-1,3-dioxolan-2-yl)phenyl)-1H-pyrazole (2.4 g), 4M hydrogen chloride/ethanol solution (15 mL) and ethanol (20 mL) was heated under reflux overnight, and the insoluble material was filtered off through celite. The filtrate was poured into saturated brine, and the mixture was extracted with ethyl acetate. The extract was washed with saturated brine, and dried over anhydrous magnesium sulfate. The solvent was evaporated under reduced pressure. The residue was purified by silica gel column chromatography (hexane/ethyl acetate) to give the title compound (1.40 g).
MS (API+): [M+H]+217.2.

### E) N-hydroxy-2-methoxy-1-(4-(1H-pyrazol-1-yl)phenyl)ethanimine

A mixture of 2-methoxy-1-(4-(1H-pyrazol-1-yl)phenyl)ethanone (1.30 g), hydroxylamine hydrochloride (0.538 g), triethylamine (1.20 g) and ethanol (20 mL) was heated under reflux overnight, water was added, and the mixture was extracted with ethyl acetate. The extract was washed with saturated brine, and dried over anhydrous magnesium sulfate. The solvent was evaporated under reduced pressure to give the title compound (1.20 g).
MS (API+): [M+H]+232.2.

### F) 2-methoxy-1-(4-(1H-pyrazol-1-yl)phenyl)ethanamine

A mixture of N-hydroxy-2-methoxy-1-(4-(1H-pyrazol-1-yl)phenyl)ethanimine (1.20 g), 10% palladium-carbon (containing water (50%), 0.520 g) and methanol (30 mL) was stirred under a hydrogen atmosphere at room temperature overnight. The insoluble material was filtered off, and the filtrate was concentrated under reduced pressure. The residue was purified by silica gel column chromatography (dichloromethane/methanol) to give the title compound (0.583 g).
MS (API+): [M+H]+218.2.

### G) N-(2-methoxy-1-(4-(1H-pyrazol-1-yl)phenyl)ethyl)-6-methylpyrazolo[1,5-a]pyrimidine-3-carboxamide

To a solution of 6-methylpyrazolo[1,5-a]pyrimidine-3-carboxylic acid (115 mg) and 1-(4-(1H-pyrazol-1-yl)phenyl)-2-methoxyethanamine (148 mg) in N,N-dimethylformamide (5 mL) were added 1-hydroxybenzotriazole monohydrate (119 mg) and 1-(3-dimethylaminopropyl)-3-ethylcarbodiimide hydrochloride (149 mg). The reaction mixture was stirred at room temperature for 16 hr, and water was added. The precipitated solid was collected by filtration, washed with water and hexane/ethyl acetate, and dried under reduced pressure. The obtained solid was suspended in ethyl acetate/methanol, and the solid was collected by filtration, and dried under reduced pressure to give the title compound (158 mg).
MS (API+): [M+H]+377.2.

### Example 18

### 5-chloro-N-(1-(3-fluoro-4-(trifluoromethoxy)phenyl)-2-methoxyethyl)pyrazolo[1,5-a]pyrimidine-3-carboxamide

### A) 5-chloropyrazolo[1,5-a]pyrimidine-3-carbonyl chloride

To 5-hydroxypyrazolo[1,5-a]pyrimidine-3-carboxylic acid (4.24 g) was added phosphorus oxychloride (50 mL), and diisopropylethylamine (13.6 mL) was gradually added. The reaction mixture was heated under reflux for 3 hr, and phosphorus oxychloride was evaporated under reduced pressure. To the residue was added ethyl acetate, and the mixture was cooled to 0°C. Water was added, and the mixture was extracted with ethyl acetate. The extract was washed with water and saturated brine, filtered through a silica gel pad, and the solvent was evaporated under reduced pressure to give the title compound (3.07 g).
1H NMR (300 MHz, DMSO-d6) δ 7.37 (1H, d, J = 7.2 Hz), 8.61 (1H, s), 9.30 (1H, d, J = 7.2 Hz).

### B) 5-chloro-N-(1-(3-fluoro-4-(trifluoromethoxy)phenyl)-2-methoxyethyl)pyrazolo[1,5-a]pyrimidine-3-carboxamide

To a solution of 5-chloropyrazolo[1,5-a]pyrimidine-3-carbonyl chloride (1.51 g) and 1-(3-fluoro-4-(trifluoromethoxy)phenyl)-2-methoxyethanamine hydrochloride (2.13 g) in N,N-dimethylformamide (30 mL) was added triethylamine (2.92 mL) at room temperature. The reaction mixture was stirred at room temperature for 1 hr, poured into water, and extracted with ethyl acetate. The extract was washed with water, and dried over anhydrous sodium sulfate. The solvent was evaporated under reduced pressure. The residue was purified by silica gel column chromatography (hexane/ethyl acetate), and the obtained solid was washed with hexane/ethyl acetate to give the title compound (0.958 g).
MS (API+): [M+H]+433.1.

### Example 19

### 6-bromo-N-(1-(3-fluoro-4-(trifluoromethoxy)phenyl)-2-hydroxy-2-methylpropyl)pyrazolo[1,5-a]pyrimidine-3-carboxamide

### A) 6-bromopyrazolo[1,5-a]pyrimidine-3-carboxylic acid

To a mixed solution of 3-amino-1H-pyrazole-4-carboxylic acid (2.63 g) in ethanol (30 mL) and acetic acid (30 mL) was added 2-bromomalonaldehyde (3.11 g). The reaction mixture was stirred at 80°C for 1.5 hr, and cooled to room temperature. The precipitated solid was collected by filtration, and washed with water and dried under reduced pressure to give the title compound (3.58 g).
1H NMR (300 MHz, DMSO-d6) δ 8.58 (1H, s), 8.88 (1H, d, J = 2.3 Hz), 9.76 (1H, d, J = 2.3 Hz), 12.54 (1H, br s).

### B) 6-bromo-N-(1-(3-fluoro-4-(trifluoromethoxy)phenyl)-2-hydroxy-2-methylpropyl)pyrazolo[1,5-a]pyrimidine-3-carboxamide

By a method similar to that in Example 8, the title compound was obtained.
MS (API+): [M+H]+491.0.

### Example 20

### 5-chloro-N-(1-(3-fluoro-4-(trifluoromethoxy)phenyl)-2-hydroxy-2-methylpropyl)pyrazolo[1,5-a]pyrimidine-3-carboxamide

By a method similar to that in Example 18, step B, the title compound was obtained.
MS (API+): [M+H]+491.1.

### Example 21

### N-(1-(3-fluoro-4-(trifluoromethoxy)phenyl)-2-hydroxy-2-methylpropyl)-6-vinylpyrazolo[1,5-a]pyrimidine-3-carboxamide

To a mixture of 6-bromo-N-(1-(3-fluoro-4-(trifluoromethoxy)phenyl)-2-hydroxy-2-methylpropyl)pyrazolo[1,5-a]pyrimidine-3-carboxamide (142 mg), 4,4,5,5-tetramethyl-2-vinyl-1,3,2-dioxaborolane (53.5 mg), potassium carbonate (160 mg), 1,2-dimethoxyethane (3 mL) and water (1.5 mL) was added tetrakistriphenylphosphinepalladium (0) (16.7 mg) at room temperature. The reaction mixture was stirred under a nitrogen atmosphere by using microwave at 150°C for 25 min, poured into water, and the mixture was extracted with ethyl acetate. The extract was dried over anhydrous sodium sulfate, and the solvent was evaporated under reduced pressure. The residue was purified by silica gel column chromatography (hexane/ethyl acetate), and the obtained solid was washed with hexane/ethyl acetate to give the title compound (57.9 mg).
MS (API+): [M+H]+439.1.

### Example 22

### 6-ethyl-N-(1-(3-fluoro-4-(trifluoromethoxy)phenyl)-2-hydroxy-2-methylpropyl)pyrazolo[1,5-a]pyrimidine-3-carboxamide

To a solution of N-(1-(3-fluoro-4-(trifluoromethoxy)phenyl)-2-hydroxy-2-methylpropyl)-6-vinylpyrazolo[1,5-a]pyrimidine-3-carboxamide (51.7 mg) in methanol (4 mL) was added 10% palladium-carbon (containing water (50%), 5.0 mg). The reaction mixture was stirred under a hydrogen atmosphere, and at room temperature for 2 hr. The insoluble material was filtered off, and the filtrate was concentrated under reduced pressure. The residue was purified by silica gel column chromatography (hexane/ethyl acetate), and then by preparative TLC (hexane/ethyl acetate) to give the title compound (43.1 mg).
MS (API+): [M+H]+441.2.

### Example 23

### 6-cyclopropyl-N-(1-(3-fluoro-4-(trifluoromethoxy)phenyl)-2-hydroxy-2-methylpropyl)pyrazolo[1,5-a]pyrimidine-3-carboxamide

To a mixture of 6-bromo-N-(1-(3-fluoro-4-(trifluoromethoxy)phenyl)-2-hydroxy-2-methylpropyl)pyrazolo[1,5-a]pyrimidine-3-carboxamide (157 mg), cyclopropylboronic acid (68.6 mg) and potassium t-butoxide (118 mg) and toluene (30 mL) were added palladium(II) acetate (3.6 mg) and tricyclohexylphosphine (9.0 mg). The reaction mixture was stirred under an argon atmosphere at 105°C for 16 hr, poured into water, and the mixture was extracted with ethyl acetate. The extract was dried over anhydrous sodium sulfate, and the solvent was evaporated under reduced pressure. The residue was purified by silica gel column chromatography (hexane/ethyl acetate), and the obtained solid was washed with hexane/ethyl acetate to give the title compound (12.5 mg).
MS (API+): [M+H]+453.2.

### Example 24

### N-(1-(3-fluoro-4-(trifluoromethoxy)phenyl)-2-hydroxy-2-methylpropyl)-6-(prop-1-en-2-yl)pyrazolo[l,5-a]pyrimidine-3-carboxamide

By a method similar to that in Example 21, the title compound was obtained.
MS (API+): [M+H]+453.2.

### Example 25

### N-(1-(3-fluoro-4-(trifluoromethyl)phenyl)-2-hydroxy-2-methylpropyl)-6-methylpyrazolo[1,5-a]pyrimidine-3-carboxamide

### A) ethyl 2-((diphenylmethylene)amino)-2-(3-fluoro-4-(trifluoromethyl)phenyl)acetate

A mixture of ethyl 2-((diphenylmethylene)amino)acetate (2.94 g), 4-bromo-2-fluoro-1-(trifluoromethyl)benzene (2.43 g) and tripotassium phosphate (6.37 g) in toluene (30 mL) was argon-substituted, and bis(tri-tert-butylphosphine)palladium (0) (0.256 g) was added at room temperature. The reaction mixture was stirred at 80°C for 21 hr, bis(tri-tert-butylphosphine)palladium (0) (0.256 g) was added, and the mixture was stirred at 100°C for 16 hr. To the reaction mixture were added water and ethyl acetate, and the insoluble material was filtered off. The organic layer of the filtrate was washed with saturated brine, dried over anhydrous sodium sulfate, and the solvent was evaporated under reduced pressure. The residue was purified by silica gel column chromatography (hexane/ethyl acetate) to give the title compound (3.02 g).
MS (API+): [M+H]+430.1.

### B) 1-amino-1-(3-fluoro-4-(trifluoromethyl)phenyl)-2-methylpropan-2-ol hydrochloride

A solution of ethyl 2-((diphenylmethylene)amino)-2-(3-fluoro-4-(trifluoromethyl)phenyl)acetate (200 mg) in tetrahydrofuran (3 mL) was stirred while ice-cooling under an argon atmosphere, and 1M methylmagnesium bromide/tetrahydrofuran solution (1.4 mL) was added dropwise. The reaction mixture was stirred for 40 min, 2M hydrochloric acid (3 mL) was added, and the mixture was stirred at room temperature for 2 hr. The reaction mixture was washed with diethyl ether, the aqueous layer was separated and neutralized with 2M aqueous sodium hydroxide solution, and extracted with ethyl acetate. The extract was washed with saturated brine, dried over anhydrous sodium sulfate, and the solvent was evaporated under reduced pressure. To the residue was added 4M hydrogen chloride/ethyl acetate solution (1 mL), and the resulting solid was washed with diisopropyl ether to give the title compound (103 mg).
MS (API+): found: 252.1.

### C) N-(1-(3-fluoro-4-(trifluoromethyl)phenyl)-2-hydroxy-2-methylpropyl)-6-methylpyrazolo[1,5-a]pyrimidine-3-carboxamide

A mixture of 1-amino-1-(3-fluoro-4-(trifluoromethyl)phenyl)-2-methylpropan-2-ol hydrochloride (100 mg), 6-methylpyrazolo[1,5-a]pyrimidine-3-carboxylic acid (63 mg), 1-(3-dimethylaminopropyl)-3-ethylcarbodiimide hydrochloride (92 mg), 1-hydroxybenzotriazole (56.4 mg) and triethylamine (0.15 mL) in N,N-dimethylformamide (1 mL) was stirred at room temperature for 20 hr. Water was added to the reaction mixture, and the mixture was extracted with ethyl acetate. The extract was washed with saturated brine, dried over anhydrous sodium sulfate, and the solvent was evaporated under reduced pressure. The residue was purified by silica gel column chromatography (hexane/ethyl acetate), and the resulting solid was washed with heptane/ethyl acetate to give the title compound (104 mg).
MS (API+): [M+H]+411.2.
1H NMR (300 MHz, DMSO-d6) δ 1.05 (3H, s), 1.28 (3H, s), 2.40 (3H, s), 5.00 (1H, d, J = 8.7 Hz), 5.04 (1H, s), 7.41-7.52 (2H, m), 7.67-7.77 (1H, m), 8.44 (1H, s), 8.82 (1H, d, J = 2.3 Hz), 8.86 (1H, d, J = 8.7 Hz), 9.18-9.22 (1H, m).

### Example 26

### N-(1-(3-fluoro-4-(trifluoromethoxy)phenyl)-2-hydroxy-2-methylpropyl)-6-isopropylpyrazolo[1,5-a]pyrimidine-3-carboxamide

A mixture of N-(1-(3-fluoro-4-(trifluoromethoxy)phenyl)-2-hydroxy-2-methylpropyl)-6-(prop-1-en-2-yl)pyrazolo[1,5-a]pyrimidine-3-carboxamide (97.0 mg), 5% palladium-carbon-ethylenediamine complex (15 mg), methanol (10 mL) and tetrahydrofuran (2 mL) was stirred under a hydrogen atmosphere at room temperature for 3 hr. The insoluble material was filtered off, and the filtrate was concentrated under reduced pressure. The residue was purified by silica gel column chromatography (hexane/ethyl acetate), and the obtained solid was washed with hexane/ethyl acetate to give the title compound (72.3 mg).
MS (API+): [M+H]+455.2.

### Example 27

### 5-(dimethylamino)-N-(1-(3-fluoro-4-(trifluoromethoxy)phenyl)-2-hydroxy-2-methylpropyl)pyrazolo[1,5-a]pyrimidine-3-carboxamide

To a solution of 5-chloro-N-(1-(3-fluoro-4-(trifluoromethoxy)phenyl)-2-hydroxy-2-methylpropyl)pyrazolo[1,5-a]pyrimidine-3-carboxamide (35.7 mg) in 2-methyl-2-butanol (1.5 mL) were added dimethylamine hydrochloride (13.0 mg) and N,N-diisopropylethylamine (0.042 mL), and the mixture was heated by a microwave synthesizer at 130°C for 30 min. To a reaction solution were added water and ethyl acetate, and the organic layer was extracted and the solvent was evaporated by an air blowing apparatus. The residue was purified by HPLC (column: YMC Triart C18, mobile phase: acetonitrile/10 mM aqueous ammonium hydrogen carbonate solution), and the solvent was evaporated by an air blowing apparatus to give the title compound (22.0 mg).
MS (API+): [M+H]+456.0.

### Example 28

### 5-(dimethylamino)-N-(1-(3-fluoro-4-(trifluoromethoxy)phenyl)-2-methoxyethyl)pyrazolo[1,5-a]pyrimidine-3-carboxamide

By a method similar to that in Example 27, the title compound was obtained from 5-chloro-N-(1-(3-fluoro-4-(trifluoromethoxy)phenyl)-2-methoxyethyl)pyrazolo[1,5-a]pyrimidine-3-carboxamide and dimethylamine hydrochloride.
MS (API+): [M+H]+442.0.

### Example 29

### 5-(ethyl(methyl)amino)-N-(1-(3-fluoro-4-(trifluoromethoxy)phenyl)-2-hydroxy-2-methylpropyl)pyrazolo[1,5-a]pyrimidine-3-carboxamide

By a method similar to that in Example 27, the title compound was obtained.
MS (API+): [M+H]+470.1.

### Example 30

### 5-(ethyl(methyl)amino)-N-(1-(3-fluoro-4-(trifluoromethoxy)phenyl)-2-methoxyethyl)pyrazolo[1,5-a]pyrimidine-3-carboxamide

By a method similar to that in Example 28, the title compound was obtained.
MS (API+): [M+H]+456.1.

### Example 31

### 5-(azetidin-1-yl)-N-(1-(3-fluoro-4-(trifluoromethoxy)phenyl)-2-hydroxy-2-methylpropyl)pyrazolo[1,5-a]pyrimidine-3-carboxamide

By a method similar to that in Example 27, the title compound was obtained.
MS (API+): [M+H]+468.1.

### Example 32

### 5-(azetidin-1-yl)-N-(1-(3-fluoro-4-(trifluoromethoxy)phenyl)-2-methoxyethyl)pyrazolo[1,5-a]pyrimidine-3-carboxamide

By a method similar to that in Example 28, the title compound was obtained.
MS (API+): [M+H]+454.1.

### Example 33

### N-(1-(3-fluoro-4-(trifluoromethoxy)phenyl)-2-hydroxy-2-methylpropyl)-5-(pyrrolidin-1-yl)pyrazolo[1,5-a]pyrimidine-3-carboxamide

By a method similar to that in Example 27, the title compound was obtained.
MS (API+): [M+H]+482.1.

### Example 34

### N-(1-(3-fluoro-4-(trifluoromethoxy)phenyl)-2-methoxyethyl)-5-(pyrrolidin-1-yl)pyrazolo[1,5-a]pyrimidine-3-carboxamide

By a method similar to that in Example 28, the title compound was obtained.
MS (API+): [M+H]+468.1.

### Example 35

### N-(1-(3-fluoro-4-(trifluoromethoxy)phenyl)-2-hydroxy-2-methylpropyl)-5-(piperidin-1-yl)pyrazolo[1,5-a]pyrimidine-3-carboxamide

By a method similar to that in Example 27, the title compound was obtained.
MS (API+): [M+H]+496.1.

### Example 36

### N-(1-(3-fluoro-4-(trifluoromethoxy)phenyl)-2-methoxyethyl)-5-(piperidin-1-yl)pyrazolo[1,5-a]pyrimidine-3-carboxamide

By a method similar to that in Example 28, the title compound was obtained.
MS (API+): [M+H]+482.1.

### Example 37

### N-(1-(3-fluoro-4-(trifluoromethoxy)phenyl)-2-hydroxy-2-methylpropyl)-5-(morpholin-4-yl)pyrazolo[l,5-a]pyrimidine-3-carboxamide

By a method similar to that in Example 27, the title compound was obtained.
MS (API+): [M+H]+498.1.

### Example 38

### N-(1-(3-fluoro-4-(trifluoromethoxy)phenyl)-2-methoxyethyl)-5-(morpholin-4-yl)pyrazolo[1,5-a]pyrimidine-3-carboxamide

By a method similar to that in Example 28, the title compound was obtained.
MS (API+): [M+H]+484.1.

### Example 39

### N-(1-(3-fluoro-4-(trifluoromethoxy)phenyl)-2-hydroxy-2-methylpropyl)-5-(3-methoxyazetidin-1-yl)pyrazolo[1,5-a]pyrimidine-3-carboxamide

By a method similar to that in Example 27, the title compound was obtained.
MS (API+): [M+H]+498.1.

### Example 40

### N-(1-(3-fluoro-4-(trifluoromethoxy)phenyl)-2-methoxyethyl)-5-(3-methoxyazetidin-1-yl)pyrazolo[1,5-a]pyrimidine-3-carboxamide

By a method similar to that in Example 28, the title compound was obtained.
MS (API+): [M+H]+484.1.

### Example 41

### 5-(3,3-difluoropyrrolidin-1-yl)-N-(1-(3-fluoro-4-(trifluoromethoxy)phenyl)-2-hydroxy-2-methylpropyl)pyrazolo[1,5-a]pyrimidine-3-carboxamide

By a method similar to that in Example 27, the title compound was obtained.
MS (API+): [M+H]+518.1.

### Example 42

### 5-(3,3-difluoropyrrolidin-1-yl)-N-(1-(3-fluoro-4-(trifluoromethoxy)phenyl)-2-methoxyethyl)pyrazolo[1,5-a]pyrimidine-3-carboxamide

By a method similar to that in Example 28, the title compound was obtained.
MS (API+): [M+H]+504.1.

### Example 43

### N-(1-(3-fluoro-4-(trifluoromethoxy)phenyl)-2-hydroxy-2-methylpropyl)-5-(3-oxa-8-azabicyclo[3.2.1]oct-8-yl)pyrazolo[1,5-a]pyrimidine-3-carboxamide

By a method similar to that in Example 27, the title compound was obtained.
MS (API+): [M+H]+524.1.

### Example 44

### N-(1-(3-fluoro-4-(trifluoromethoxy)phenyl)-2-methoxyethyl)-5-(3-oxa-8-azabicyclo[3.2.1]oct-8-yl)pyrazolo[1,5-a]pyrimidine-3-carboxamide

By a method similar to that in Example 28, the title compound was obtained.
MS (API+): [M+H]+510.1.

### Example 45

### N-(1-(3-fluoro-4-(trifluoromethoxy)phenyl)-2-hydroxy-2-methylpropyl)-5-(3-oxa-6-azabicyclo[3.1.1]hept-6-yl)pyrazolo[1,5-a]pyrimidine-3-carboxamide

By a method similar to that in Example 27, the title compound was obtained.
MS (API+): [M+H]+510.1.

### Example 46

### N-(1-(3-fluoro-4-(trifluoromethoxy)phenyl)-2-methoxyethyl)-5-(3-oxa-6-azabicyclo[3.1.1]hept-6-yl)pyrazolo[1,5-a]pyrimidine-3-carboxamide

By a method similar to that in Example 28, the title compound was obtained.
MS (API+): [M+H]+496.1.

### Example 47

### N-(1-(3-fluoro-4-(trifluoromethoxy)phenyl)-2-hydroxy-2-methylpropyl)-5-((1R, 5S)-6-oxa-3-azabicyclo[3.1.1]hept-3-yl)pyrazolo[1,5-a]pyrimidine-3-carboxamide

By a method similar to that in Example 27, the title compound was obtained.
MS (API+): [M+H]+510.1.

### Example 48

### N-(1-(3-fluoro-4-(trifluoromethoxy)phenyl)-2-methoxyethyl)-5-((1R, 5S)-6-oxa-3-azabicyclo[3.1.1]hept-3-yl)pyrazolo[1,5-a]pyrimidine-3-carboxamide

By a method similar to that in Example 28, the title compound was obtained.
MS (API+): [M+H]+496.1.

### Example 49

### N-(1-(3-fluoro-4-(trifluoromethoxy)phenyl)-2-hydroxy-2-methylpropyl)-5-methylpyrazolo[1,5-a]pyrimidine-3-carboxamide

To a solution of 5-chloro-N-(1-(3-fluoro-4-(trifluoromethoxy)phenyl)-2-hydroxy-2-methylpropyl)pyrazolo[1,5-a]pyrimidine-3-carboxamide (35.7 mg) in 2-methyl-2-butanol (1.5 mL) were added 2M aqueous cesium carbonate solution (0.1 mL), bis(di-tert-butyl(4-dimethylaminophenylphosphine)dichloropalladium(II)) (5.6 mg), and trimethylboroxine (20 mg), and the mixture was heated by a microwave synthesizer at 130°C for 30 min. The reaction solution was filtered through celite, and water and ethyl acetate were added. The organic layer was extracted, and the solvent was evaporated by an air blowing apparatus. The residue was purified by HPLC (column: YMC Triart C18, mobile phase: acetonitrile/10 mM aqueous ammonium hydrogen carbonate solution), and the solvent was evaporated by an air blowing apparatus to give the title compound (19.9 mg).
MS (API+): [M+H]+427.0.

### Example 50

### N-(1-(3-fluoro-4-(trifluoromethoxy)phenyl)-2-methoxyethyl)-5-methylpyrazolo[1,5-a]pyrimidine-3-carboxamide

By a method similar to that in Example 49, the title compound was obtained.
MS (API+): [M+H]+412.9.

### Example 51

### 5-cyclopropyl-N-(1-(3-fluoro-4-(trifluoromethoxy)phenyl)-2-hydroxy-2-methylpropyl)pyrazolo[1,5-a]pyrimidine-3-carboxamide

By a method similar to that in Example 49, the title compound was obtained.
MS (API+): [M+H]+453.1.

### Example 52

### 5-cyclopropyl-N-(1-(3-fluoro-4-(trifluoromethoxy)phenyl)-2-methoxyethyl)pyrazolo[1,5-a]pyrimidine-3-carboxamide

By a method similar to that in Example 49, the title compound was obtained.
MS (API+): [M+H]+439.0.

### Example 53

### N-(1-(3-fluoro-4-(trifluoromethoxy)phenyl)-2-hydroxy-2-methylpropyl)-5-(1-methyl-1H-pyrazol-3-yl)pyrazolo[1,5-a]pyrimidine-3-carboxamide

By a method similar to that in Example 49, the title compound was obtained.
MS (API+): [M+H]+493.1.

### Example 54

### N-(1-(3-fluoro-4-(trifluoromethoxy)phenyl)-2-methoxyethyl)-5-(1-methyl-1H-pyrazol-3-yl)pyrazolo[1,5-a]pyrimidine-3-carboxamide

By a method similar to that in Example 49, the title compound was obtained.
MS (API+): [M+H]+479.1.

### Example 55

### N-(1-(3-fluoro-4-(trifluoromethoxy)phenyl)-2-hydroxy-2-methylpropyl)-5-(1-methyl-1H-pyrazol-4-yl)pyrazolo[1,5-a]pyrimidine-3-carboxamide

By a method similar to that in Example 49, the title compound was obtained.
MS (API+): [M+H]+493.1.

### Example 56

### N-(1-(3-fluoro-4-(trifluoromethoxy)phenyl)-2-methoxyethyl)-5-(1-methyl-1H-pyrazol-4-yl)pyrazolo[1,5-a]pyrimidine-3-carboxamide

By a method similar to that in Example 49, the title compound was obtained.
MS (API+): [M+H]+479.1.

### Example 57

### N-(1-(3-fluoro-4-(trifluoromethoxy)phenyl)-2-methoxyethyl)-5-(1-methyl-1H-pyrazol-5-yl)pyrazolo[1,5-a]pyrimidine-3-carboxamide

By a method similar to that in Example 49, the title compound was obtained.
MS (API+): [M+H]+479.1.

### Example 58

### N-(2-hydroxy-2-methyl-1-(4-(trifluoromethoxy)phenyl)propyl)-6-methylpyrazolo[1,5-a]pyrimidine-3-carboxamide

By a method similar to that in Example 8, the title compound was obtained.
MS (API+): [M+H]+409.2.

### Example 59

### 5-(azetidin-1-yl)-N-(1-(3-fluoro-4-(trifluoromethoxy)phenyl)-2-hydroxy-2-methylpropyl)-6-methylpyrazolo[1,5-a]pyrimidine-3-carboxamide

### A) ethyl 5,7-dihydroxy-6-methylpyrazolo[1,5-a]pyrimidine-3-carboxylate

A solution of ethyl 5-amino-1H-pyrazole-4-carboxylate (22.4 g), diethyl 2-methylmalonate (24.6 mL) and tributylamine (51.6 mL) was stirred at 160°C overnight. After cooling to room temperature, the mixture was poured into a solution of 1M aqueous sodium hydroxide, and extracted twice with ethyl acetate. The aqueous layer was separated, and adjusted to pH 1 with 1M hydrochloric acid. The resulting solid was collected by filtration, washed with water, and dried under reduced pressure to give the title compound (21.0 g).
MS (API+): [M+H]+238.1.

### B) ethyl 5,7-dichloro-6-methylpyrazolo[1,5-a]pyrimidine-3-carboxylate

A mixture of ethyl 5,7-dihydroxy-6-methylpyrazolo[1,5-a]pyrimidine-3-carboxylate (21.0 g) and phosphorus oxychloride (206 mL) was stirred at 90°C overnight, and concentrated under reduced pressure. The residue was poured into ice water, and the mixture was extracted with ethyl acetate. The extract was washed with saturated brine, dried over anhydrous sodium sulfate, and the solvent was evaporated under reduced pressure to give a crude product (8.12 g). A mixture of the crude product (2.0 g) and phosphorus oxychloride (25.5 mL) was stirred at 110°C for 3 days. The reaction mixture was concentrated under reduced pressure, the residue was diluted with ethyl acetate and poured into ice water, and the organic layer was separated. The organic layer was washed with saturated brine, dried over anhydrous magnesium sulfate, and the solvent was evaporated under reduced pressure. The residue was purified by silica gel column chromatography (hexane/ethyl acetate) to give the title compound (0.66 g).
1H NMR (300 MHz, DMSO-d6) δ 1.32 (3H, t, J = 7.1 Hz), 2.49 (3H, s), 4.31 (2H, q, J = 7.2 Hz), 8.69 (1H, s).

### C) ethyl 5-chloro-6-methylpyrazolo[1,5-a]pyrimidine-3-carboxylate

A mixture of ethyl 5,7-dichloro-6-methylpyrazolo[1,5-a]pyrimidine-3-carboxylate (660 mg) and zinc powder (338 mg), concentrated aqueous ammonia (2.0 mL), saturated brine (6.0 mL), and tetrahydrofuran (6.0 mL) was stirred at room temperature for 2 hr. The reaction mixture was filtered, washed with ethyl acetate, and the organic layer was separated. The organic layer was washed with saturated brine, dried over anhydrous magnesium sulfate, and the solvent was evaporated under reduced pressure. The residue was purified by silica gel column chromatography (hexane/ethyl acetate) to give the title compound (198 mg).
1H NMR (300 MHz, DMSO-d6) δ 1.31 (3H, t, J = 7.0 Hz), 2.36 (3H, d, J = 1.1 Hz), 4.30 (2H, q, J = 7.2 Hz), 8.59 (1H, s), 9.34 (1H, d, J = 0.8 Hz).

### D) 5-(azetidin-1-yl)-N-(1-(3-fluoro-4-(trifluoromethoxy)phenyl)-2-hydroxy-2-methylpropyl)-6-methylpyrazolo[1,5-a]pyrimidine-3-carboxamide

To a solution of ethyl 5-chloro-6-methylpyrazolo[1,5-a]pyrimidine-3-carboxylate (120 mg) in acetonitrile (2.5 mL) were added azetidine (40 µL) and triethylamine (105 µL) at room temperature. The reaction mixture was stirred at room temperature overnight, poured into water, and the mixture was extracted with ethyl acetate. The extract was washed with saturated brine, and dried over anhydrous magnesium sulfate. The solvent was evaporated under reduced pressure. The obtained crude product was used for the next reaction without purification. By a method similar to that in Example 8, the title compound was obtained from the obtained crude product.
MS (API+): [M+H]+482.2.

### Example 60

### N-(1-(3-fluoro-4-(trifluoromethoxy)phenyl)-2-hydroxy-2-methylpropyl)-5,6-dimethylpyrazolo[1,5-a]pyrimidine-3-carboxamide

### A) ethyl 5,6-dimethylpyrazolo[1,5-a]pyrimidine-3-carboxylate

A mixture of ethyl 5-chloro-6-methylpyrazolo[1,5-a]pyrimidine-3-carboxylate (72.0 mg), bis(di-tert-butyl(4-dimethylaminophenyl)phosphine)dichloropalladium(II) (21.3 mg), 2M aqueous cesium carbonate solution (0.300 mL), trimethylboroxine (84.0 µL) and 2-methyl-2-butanol (2.20 mL) was heated under microwave irradiation at 120°C for 1 hr. After cooling to room temperature, the reaction mixture was diluted with ethyl acetate and aqueous ammonium chloride solution, and extracted with ethyl acetate. The organic layer was separated, washed with water and saturated brine, and dried over anhydrous sodium sulfate. The solvent was evaporated under reduced pressure. The residue was purified by silica gel chromatography (NH, ethyl acetate/hexane) to give the title compound (25.0 mg).
1H NMR (300 MHz, CDC13) δ 1.41 (3H, t, J = 7.1 Hz), 2.35 (3H, d, J = 0.8 Hz), 2.68 (3H, s), 4.41 (2H, q, J = 7.1 Hz), 8.42 (1H, s), 8.44 (1H, s).

### B) N-(1-(3-fluoro-4-(trifluoromethoxy)phenyl)-2-hydroxy-2-methylpropyl)-5,6-dimethylpyrazolo[1,5-a]pyrimidine-3-carboxamide

A mixture of ethyl 5,6-dimethylpyrazolo[1,5-a]pyrimidine-3-carboxylate (23.0 mg), 2M aqueous sodium hydroxide solution (0.420 mL), tetrahydrofuran (3.00 mL) and ethanol (1.50 mL) was stirred at 60°C for 18 hr. After cooling to room temperature, the reaction mixture was neutralized with 2M hydrochloric acid (0.420 mL), and the solvent was evaporated under reduced pressure. The obtained crude product was used for the next reaction without purification. A mixture of the crude product, 1-amino-1-(3-fluoro-4-(trifluoromethoxy)phenyl)-2-methylpropan-2-ol hydrochloride (31.8 mg), 0-(7-azabenzotriazol-1-yl)-N,N,N',N'-tetramethyluronium hexafluorophosphate (51.7 mg), N,N-diisopropylethylamine (73.0 µL) and dimethylformamide (2.00 mL) was stirred at room temperature for 3 hr. The reaction mixture was diluted with ethyl acetate and water, and the mixture was extracted with ethyl acetate. The organic layer was separated, washed with saturated brine, and dried over anhydrous sodium sulfate. The solvent was evaporated under reduced pressure. The residue was purified by silica gel chromatography (ethyl acetate/hexane and methanol/ethyl acetate) to give the title compound (35.0 mg).
MS (API+) : [M+H]+441.1.

### Example 61

### N-(1-(3-fluoro-4-(trifluoromethoxy)phenyl)-3-hydroxy-3-methylbutyl)-6-methylpyrazolo[1,5-a]pyrimidine-3-carboxamide

### A) 3-amino-3-(3-fluoro-4-(trifluoromethoxy)phenyl)propanoic acid

A mixed solution of 3-fluoro-4-(trifluoromethoxy)benzaldehyde (4.16 g), ammonium acetate (3.08 g) and malonic acid (2.08 g) in ethanol (50 mL) was stirred under a nitrogen atmosphere at 80°C for 15 hr. After cooling the reaction mixture, the resulting solid was collected by filtration, and washed with ethanol and then diisopropyl ether to give the title compound (2.84 g).
MS (API+): [M+H]+268.1.

### B) methyl 3-amino-3-(3-fluoro-4-(trifluoromethoxy)phenyl)propanoate

To a mixture of concentrated sulfuric acid (1.2 mL) and methanol (10 mL) was added 3-amino-3-(3-fluoro-4-(trifluoromethoxy)phenyl)propanoic acid (2.84 g) by small portions, and the mixture was stirred at room temperature for 2 hr. The solvent was evaporated under reduced pressure, the residue was neutralized with 2M aqueous sodium hydroxide solution, and the mixture was extracted with ethyl acetate. The extract was washed with saturated brine, dried over anhydrous sodium sulfate, and the solvent was evaporated under reduced pressure to give a crude product of the title compound. The present compound was used for the next reaction without further purification.
MS (API+): [M+H]+282.1.

### C) methyl 3-((tert-butoxycarbonyl)amino)-3-(3-fluoro-4-(trifluoromethoxy)phenyl)propanoate

To a solution of methyl 3-amino-3-(3-fluoro-4-(trifluoromethoxy)phenyl)propanoate (2.99 g) in tetrahydrofuran (30 mL) was added dropwise a solution of di-tert-butyl dicarbonate (2.32 g) in tetrahydrofuran (10 mL) under ice-cooling. The reaction mixture was stirred at room temperature for 3 days, water was added, and the mixture was extracted with ethyl acetate. The extract was washed with saturated brine, dried over anhydrous sodium sulfate, and the solvent was evaporated under reduced pressure to give a crude product of the title compound. The present compound was used for the next reaction without further purification.
MS (API-): [M-H]-380.1.

### D) tert-butyl (1-(3-fluoro-4-(trifluoromethoxy)phenyl)-3-hydroxy-3-methylbutyl)carbamate

A solution of methyl 3-((tert-butoxycarbonyl)amino)-3-(3-fluoro-4-(trifluoromethoxy)phenyl)propanoate (4.05 g) in tetrahydrofuran (50 mL) was stirred while ice-cooling under an argon atmosphere, and 1M methylmagnesium bromide/tetrahydrofuran solution (42.5 mL) was added dropwise. The reaction mixture was stirred while warming to room temperature for 14 hr, 1M methylmagnesium bromide/tetrahydrofuran solution (4.4 mL) was added, and the mixture was further stirred for 1 hr. The reaction mixture was treated with aqueous ammonium chloride solution, and extracted with ethyl acetate. The extract was washed with saturated brine, dried over anhydrous magnesium sulfate, and the solvent was evaporated under reduced pressure. The residue was purified by silica gel column chromatography (hexane/ethyl acetate) to give a crude product (1.07 g) of the title compound. The present compound was used for the next reaction without further purification.

### E) 4-amino-4-(3-fluoro-4-(trifluoromethoxy)phenyl)-2-methylbutan-2-ol hydrochloride

A mixture of tert-butyl (1-(3-fluoro-4-(trifluoromethoxy)phenyl)-3-hydroxy-3-methylbutyl)carbamate (1.07 g) and 4M hydrogen chloride/ethyl acetate solution (6.5 mL) was stirred at room temperature for 1.5 hr. The solvent was evaporated under reduced pressure, the residue was neutralized with saturated aqueous sodium hydrogen carbonate, and the mixture was extracted with ethyl acetate. The extract was washed with saturated brine, and dried over anhydrous magnesium sulfate. The solvent was evaporated under reduced pressure. The residue was purified by silica gel column chromatography (hexane/ethyl acetate), the obtained oil was treated with 4M hydrogen chloride/ethyl acetate solution (4 mL), and the solvent was evaporated under reduced pressure. The resulting solid was washed with diisopropyl ether to give the title compound (0.459 g).
MS (API+): found: 282.1.

### F) N-(1-(3-fluoro-4-(trifluoromethoxy)phenyl)-3-hydroxy-3-methylbutyl)-6-methylpyrazolo[1,5-a]pyrimidine-3-carboxamide

By a method similar to that in Example 8, the title compound was obtained.
MS (API+) : [M+H]+441.1.

### Example 62

### N-(1-(3-fluoro-4-(trifluoromethoxy)phenyl)-2-hydroxy-2-methylpropyl)pyrazolo[1,5-a]pyrimidine-3-carboxamide

By a method similar to that in Example 8, the title compound was obtained.
MS (API+) : [M+H]+ 413.1.

### Example 63

### N-(1-(3-fluoro-4-(trifluoromethoxy)phenyl)-2-hydroxy-2-methylpropyl)-5-(1H-pyrazol-1-yl)pyrazolo[1,5-a]pyrimidine-3-carboxamide

A mixture of 5-chloro-N-(1-(3-fluoro-4-(trifluoromethoxy)phenyl)-2-hydroxy-2-methylpropyl)pyrazolo[1,5-a]pyrimidine-3-carboxamide (143 mg), 1H-pyrazole (32.7 mg), potassium carbonate (66.4 mg) and dimethylformamide (2.00 mL) was stirred at 70°C for 12 hr. The reaction mixture was diluted with ethyl acetate and water, and extracted with ethyl acetate. The organic layer was separated, washed with saturated brine, and dried over anhydrous sodium sulfate. The solvent was evaporated under reduced pressure. The residue was purified by silica gel chromatography (ethyl acetate/hexane), and crystallized from ethyl acetate/heptane to give the title compound (124 mg).
MS (API+) : [M+H]+479.1.

### Example 64

### N-(1-(3-fluoro-4-(trifluoromethoxy)phenyl)-2-hydroxy-2-methylpropyl)-5-(4-methyl-1H-pyrazol-1-yl)pyrazolo[1,5-a]pyrimidine-3-carboxamide

By a method similar to that in Example 63, the title compound was obtained.
MS (API+) : [M+H]+493.2.

### Example 65

### N-(2-ethyl-1-(3-fluoro-4-(trifluoromethoxy)phenyl)-2-hydroxybutyl)-6-methylpyrazolo[1,5-a]pyrimidine-3-carboxamide

### A) ethyl 2-((diphenylmethylene)amino)-2-(3-fluoro-4-(trifluoromethoxy)phenyl)acetate

By a method similar to that in Example 25, step A, the title compound (15.1 g) was obtained.
MS (API+) : [M+H]+ 446.1.

### B) 3-(amino(3-fluoro-4-(trifluoromethoxy)phenyl)methyl)pentan-3-ol hydrochloride

By a method similar to that in Example 25, step B, the title compound was obtained from ethyl 2-((diphenylmethylene)amino)-2-(3-fluoro-4-(trifluoromethoxy)phenyl)acetate and 3M ethylmagnesium bromide/diethyl ether solution.
MS (API+) : found: 296.1.

### C) N-(2-ethyl-1-(3-fluoro-4-(trifluoromethoxy)phenyl)-2-hydroxybutyl)-6-methylpyrazolo[1,5-a]pyrimidine-3-carboxamide

By a method similar to that in Example 8, the title compound was obtained.
MS (API+) : [M+H]+455.2.

### Example 66

### 5-cyclopropyl-N-((1S)-1-(3-fluoro-4-(trifluoromethoxy)phenyl)-2-hydroxy-2-methylpropyl)-1-methyl-7-oxo-1,7-dihydropyrazolo[1,5-a]pyrimidine-3-carboxamide

### A) ethyl 2-((diphenylmethylene)amino)-2-(3-fluoro-4-(trifluoromethoxy)phenyl)acetate

By a method similar to that in Example 25, step A, the title compound was obtained.
MS (API+): [M+H]+446.1.

### B) tert-butyl (1-(3-fluoro-4-(trifluoromethoxy)phenyl)-2-hydroxy-2-methylpropyl)carbamate

To a solution of ethyl 2-((diphenylmethylene)amino)-2-(3-fluoro-4-(trifluoromethoxy)phenyl)acetate (27.2 g) in tetrahydrofuran (305 mL) was slowly added a solution (183 mL) of 1M methylmagnesium bromide in tetrahydrofuran at 0°C. The reaction mixture was stirred under an argon atmosphere at 0°C for 1 hr. To the reaction mixture was added 2M hydrochloric acid (245 mL) at 0°C, and the mixture was stirred at room temperature for 2 hr. Tetrahydrofuran in the reaction mixture was evaporated under reduced pressure, and the reaction mixture was extracted with diethyl ether. The aqueous layer was neutralized with 2M aqueous sodium hydroxide solution, and extracted with ethyl acetate. The organic layer was dried over anhydrous magnesium sulfate, and the solvent was evaporated under reduced pressure to give a crude product (7.60 g). The obtained crude product was used for the next reaction without purification. A solution of the crude product (7.60 g), di-tert-butyl dicarbonate (7.26 mL) and triethylamine (5.95 mL) in tetrahydrofuran (142 mL) was stirred at room temperature overnight. The reaction mixture was concentrated under reduced pressure, and the residue was purified by silica gel column chromatography (hexane/ethyl acetate) to give the title compound (9.70 g).
MS (API-): [M-H]-366.0.

### C) tert-butyl ((1S)-1-(3-fluoro-4-(trifluoromethoxy)phenyl)-2-hydroxy-2-methylpropyl)carbamate

The racemate (9.70 g) of tert-butyl (1-(3-fluoro-4-(trifluoromethoxy)phenyl)-2-hydroxy-2-methylpropyl)carbamate was resolved by HPLC (column: CHIRALPAK AD, 50 mmIDx500 mmL, manufactured by Daicel Corporation, mobile phase:hexane/ethanol = 950/50) to give the title compound (4.65 g) having a shorter retention time.
MS (API-): [M-H]-366.0.

### D) (S)-1-amino-1-(3-fluoro-4-(trifluoromethoxy)phenyl)-2-methylpropan-2-ol hydrochloride

To a mixture of tert-butyl ((1S)-1-(3-fluoro-4-(trifluoromethoxy)phenyl)-2-hydroxy-2-methylpropyl)carbamate (2.86 g) and ethyl acetate (40 mL) was added 4M hydrogen chloride/ethyl acetate (40 mL). The reaction mixture was stirred at room temperature for 4 hr, and the solvent was evaporated under reduced pressure. The residue was washed with diisopropyl ether to give the title compound (2.39 g).
MS (API+): found: 268.1.

### E) ethyl 5-cyclopropyl-7-hydroxypyrazolo[1,5-a]pyrimidine-3-carboxylate

To a mixed solution of ethyl 3-amino-1H-pyrazole-4-carboxylate (2.01 g) in ethanol (30 mL) and acetic acid (30 mL) was added ethyl 3-cyclopropyl-3-oxopropanate (2.14 g). The reaction mixture was stirred at 100°C for 4 hr, and the solvent was evaporated under reduced pressure. To the residue was added ethyl acetate, and the obtained solid was collected by filtration and washed with ethyl acetate to give the title compound (1.83 g).
MS (API+) : [M+H]+248.1.

### F) 5-cyclopropyl-N-((1S)-1-(3-fluoro-4-(trifluoromethoxy)phenyl)-2-hydroxy-2-methylpropyl)-1-methyl-7-oxo-1,7-dihydropyrazolo[1,5-a]pyrimidine-3-carboxamide

To a solution of ethyl 5-cyclopropyl-7-hydroxypyrazolo[1,5-a]pyrimidine-3-carboxylate (503 mg) and methyl iodide (0.509 mL) in N,N-dimethylformamide (15 mL) was added potassium carbonate (563 mg). The reaction mixture was stirred at 80°C for 5 hr, poured into water, and the mixture was extracted with ethyl acetate. The extract was washed with water, and dried over anhydrous sodium sulfate. The solvent was evaporated under reduced pressure. The obtained solid was washed with hexane/ethyl acetate to give a crude product (305 mg) of ethyl 5-cyclopropyl-l-methyl-7-oxo-1,7-dihydropyrazolo[1,5-a]pyrimidine-3-carboxylate. To a mixed solution of the obtained crude product (302 mg) of ethyl 5-cyclopropyl-1-methyl-7-oxo-1,7-dihydropyrazolo[1,5-a]pyrimidine-3-carboxylate in tetrahydrofuran (8 mL) and methanol (4 mL) was added 1M aqueous sodium hydroxide solution (3.27 mL). The reaction mixture was stirred at room temperature for 16 hr, and the solvent was evaporated under reduced pressure. To the residue was added water, and 1M hydrochloric acid (3.27 mL) was added. The precipitated solid was collected by filtration, and washed with water to give a crude product (125 mg) of 5-cyclopropyl-1-methyl-7-oxo-1,7-dihydropyrazolo[1,5-a]pyrimidine-3-carboxylic acid. To a solution of the obtained crude product (124 mg) of 5-cyclopropyl-1-methyl-7-oxo-1,7-dihydropyrazolo[1,5-a]pyrimidine-3-carboxylic acid, (S)-1-amino-1-(3-fluoro-4-(trifluoromethoxy)phenyl)-2-methylpropan-2-ol hydrochloride (169 mg) in N,N-dimethylformamide (6 mL) were added 1-hydroxybenzotriazole monohydrate (97 mg), 1-(3-dimethylaminopropyl)-3-ethylcarbodiimide hydrochloride (122 mg) and triethylamine (0.221 mL). The reaction mixture was stirred at room temperature for 16 hr, poured into aqueous sodium hydrogen carbonate solution, and the mixture was extracted with ethyl acetate. The extract was washed with water, and dried over anhydrous sodium sulfate. The solvent was evaporated under reduced pressure. The residue was purified by silica gel column chromatography (hexane/ethyl acetate), and then preparative HPLC (column: YMC Triart C18, mobile phase: acetonitrile/10 mM aqueous ammonium hydrogen carbonate solution) to give the title compound (95.7 mg).
MS (API+) : [M+H]+483.2.

### Example 67

### N-((1S)-1-(3-fluoro-4-(trifluoromethoxy)phenyl)-2-hydroxy-2-methylpropyl)-5-(methoxymethyl)-1-methyl-7-oxo-1,7-dihydropyrazolo[1,5-a]pyrimidine-3-carboxamide

### A) ethyl 7-hydroxy-5-(methoxymethyl)pyrazolo[1,5-a]pyrimidine-3-carboxylate

By a method similar to that in Example 66, step A to step E, the title compound was obtained.
MS (API+): [M+H]+252.1.

### B) N-((1S)-1-(3-fluoro-4-(trifluoromethoxy)phenyl)-2-hydroxy-2-methylpropyl)-5-(methoxymethyl)-1-methyl-7-oxo-1,7-dihydropyrazolo[1,5-a]pyrimidine-3-carboxamide

To a solution of ethyl 7-hydroxy-5-(methoxymethyl)pyrazolo[1,5-a]pyrimidine-3-carboxylate (425 mg) in N,N-dimethylformamide (8 mL) was gradually added sodium hydride (60% containing, 81 mg) at 0°C. The reaction mixture was stirred at room temperature for 30 min, and methyl iodide (0.423 mL) was added. The reaction mixture was stirred at room temperature for 30 min and then at 80°C for 2 hr, poured into water, and the mixture was extracted with ethyl acetate. The extract was washed with water, and dried over anhydrous sodium sulfate. The solvent was evaporated under reduced pressure. The obtained solid was washed with hexane/ethyl acetate to give a crude product (164 mg) of ethyl 5-(methoxymethyl)-l-methyl-7-oxo-1,7-dihydropyrazolo[1,5-a]pyrimidine-3-carboxylate. To a mixed solution of the obtained crude product (165 mg) of ethyl 5-(methoxymethyl)-1-methyl-7-oxo-1,7-dihydropyrazolo[1,5-a]pyrimidine-3-carboxylate in tetrahydrofuran (5 mL) and methanol (2.5 mL) was added 1M aqueous sodium hydroxide solution (1.87 mL). The reaction mixture was stirred at room temperature for 16 hr, 1M hydrochloric acid (1.87 mL) was added, and the solvent was evaporated under reduced pressure. To a solution of the residue and (S)-1-amino-1-(3-fluoro-4-(trifluoromethoxy)phenyl)-2-methylpropan-2-ol hydrochloride (198 mg) in N,N-dimethylformamide (4 mL) were added 1-hydroxybenzotriazole monohydrate (114 mg), 1-(3-dimethylaminopropyl)-3-ethylcarbodiimide hydrochloride (143 mg) and triethylamine (0.259 mL). The reaction mixture was stirred at room temperature for 16 hr, poured into aqueous sodium hydrogen carbonate solution, and the mixture was extracted with ethyl acetate. The extract was washed with water, and dried over anhydrous sodium sulfate. The solvent was evaporated under reduced pressure. The residue was purified by silica gel column chromatography (hexane/ethyl acetate), and then preparative HPLC (column: YMC Triart C18, mobile phase: acetonitrile/10 mM aqueous ammonium hydrogen carbonate solution) to give the title compound (36.5 mg).
MS (API+): [M+H]+487.2.

### Example 68

### N-((1S)-2-hydroxy-2-methyl-1-(4-(trifluoromethoxy)phenyl)propyl)-5-(1H-1,2,3-triazol-1-yl)pyrazolo[1,5-a]pyrimidine-3-carboxamide

A mixture of (S)-5-chloro-N-(2-hydroxy-2-methyl-1-(4-(trifluoromethoxy)phenyl)propyl)pyrazolo[1,5-a]pyrimidine-3-carboxamide (200 mg), potassium carbonate (97.0 mg), 1H-1,2,3-triazole (48.3 mg) and N,N-dimethylformamide (2.00 mL) was stirred at 95°C for 30 min. The reaction mixture was diluted with ethyl acetate and water, and the mixture was extracted with ethyl acetate. The organic layer was separated, washed with saturated brine, and dried over anhydrous sodium sulfate. The solvent was evaporated under reduced pressure. The residue was purified by silica gel chromatography (ethyl acetate/hexane) to give the title compound (98 mg).
1H NMR (300 MHz, CDC13) δ 1.18 (3H, s), 1.52 (3H, s), 1.72 (1H, s), 5.03 (1H, d, J = 8.3 Hz), 7.14-7.22 (2H, m), 7.42-7.48 (2H, m), 7.94 (1H, d, J = 1.3 Hz), 8.04 (1H, d, J = 7.6 Hz), 8.65 (1H, s), 8.90-8.98 (2H, m), 9.06 (1H, d, J = 1.3 Hz).

### Example 69-I

### N-((1S)-2-hydroxy-2-methyl-1-(4-(trifluoromethoxy)phenyl)propyl)-5-(6-methylpyridin-3-yl)pyrazolo[1,5-a]pyrimidine-3-carboxamide

### A) ethyl 5-hydroxypyrazolo[1,5-a]pyrimidine-3-carboxylate

To a solution of ethyl 3-amino-1H-pyrazole-4-carboxylate (15.0 g) and ethyl 3-ethoxy-2-propenoate (21.0 mL) in N,N-dimethylformamide (322 mL) was added cesium carbonate (56.7 g). The reaction mixture was stirred overnight at 100°C, water was added, and acetic acid was sequentially added to adjust the pH to about 4. The obtained solid was collected by filtration, and washed with water to give the title compound (17.1 g).
¹H NMR (300 MHz, DMSO-d₆) δ 1.28 (3H, t, J = 7.0 Hz), 4.27 (2H, q, J = 6.9 Hz), 6.14 (1H, d, J = 7.9 Hz), 8.13 (1H, s), 8.56 (1H, d, J = 7.9 Hz), 11.75 (1H, brs).

### B) 5-hydroxypyrazolo[1,5-a]pyrimidine-3-carboxylic acid

To a mixture of ethyl 5-hydroxypyrazolo[1,5-a]pyrimidine-3-carboxylate (20.8 g), tetrahydrofuran (223 mL) and ethanol (111 mL) was added 2M aqueous sodium hydroxide solution (201 mL). The reaction mixture was stirred overnight at 50°C, and the organic solvent was evaporated under reduced pressure. To the residue was added 2M hydrochloric acid (201 mL), and the precipitated solid was collected by filtration and washed with water/ethanol to give the title compound (17.7 g).
MS (API-): [M-H]⁻177. 9.

### C) 5-chloropyrazolo[1,5-a]pyrimidine-3-carbonyl chloride

To 5-hydroxypyrazolo[1,5-a]pyrimidine-3-carboxylic acid (4.24 g) was added phosphorus oxychloride (50 mL), and N,N-diisopropylethylamine (13.6 mL) was slowly added. The reaction mixture was heated under reflux for 3 hr, and phosphorus oxychloride was evaporated under reduced pressure. To the residue was added ethyl acetate, and the mixture was cooled to 0°C, water was added, and the mixture was extracted with ethyl acetate. The extract was washed with water and saturated brine and filtered through a silica gel pad, and the solvent was evaporated under reduced pressure to give the title compound (3.07 g).
¹H NMR (300 MHz, DMSO-d₆) δ 7.37 (1H, d, J = 7.2 Hz), 8.61 (1H, s), 9.30 (1H, d, J = 7.2 Hz).

### D) (S)-5-chloro-N-(2-hydroxy-2-methyl-1-(4-(trifluoromethoxy)phenyl)propyl)pyrazolo[1,5-a]pyrimidine-3-carboxamide

To a mixture of 5-chloropyrazolo[1,5-a]pyrimidine-3-carbonyl chloride (1.07 g), (S)-1-amino-2-methyl-1-(4-(trifluoromethoxy)phenyl)propan-2-ol hydrochloride (1.42 g) and acetonitrile (20 mL) was added triethylamine (1.60 mL). The reaction mixture was stirred at room temperature for 3 hr, water was added, and the mixture was extracted with ethyl acetate. The extract was washed with saturated brine and dried over anhydrous sodium sulfate, and the solvent was evaporated under reduced pressure. The residue was purified by silica gel column chromatography (hexane/ethyl acetate) and the obtained solid was washed with diisopropyl ether to give the title compound (1.76 g).
MS (API+) : [M+H]⁺429.1.

### E) N-((1S)-2-hydroxy-2-methyl-1-(4-(trifluoromethoxy)phenyl)propyl)-5-(6-methylpyridin-3-yl)pyrazolo[1,5-a]pyrimidine-3-carboxamide

To a mixture of (S)-5-chloro-N-(2-hydroxy-2-methyl-1-(4-(trifluoromethoxy)phenyl)propyl)pyrazolo[1,5-a]pyrimidine-3-carboxamide (55.0 mg), 2-methyl-5-(4,4,5,5-tetramethyl-1,3,2-dioxaborolan-2-yl)pyridine (43.9 mg), potassium carbonate (26.6 mg), toluene (4 mL) and water (0.4 mL) was added bis(di-tert-butyl(4-dimethylaminophenyl)phosphine)dichloropalladium (II) (9.1 mg) at room temperature. The reaction mixture was stirred under a nitrogen atmosphere and using microwave at 150°C for 25 min and purified by silica gel column chromatography (hexane/ethyl acetate). The obtained solid was washed with hexane/ethyl acetate to give the title compound (39.3 mg).
MS (API+) : [M+H]⁺486.2.
¹H NMR (300 MHz, DMSO-d₆) δ 1.03 (3H, s), 1.33 (3H, s), 2.60 (3H, s), 4.93 (1H, d, J = 8.3 Hz), 5.19 (1H, s), 7.23-7.33 (2H, m), 7.48-7.59 (3H, m), 7.98 (1H, d, J = 7.5 Hz), 8.50 (1H, s), 8.81 (1H, dd, J = 8.3, 2.3 Hz), 9.24 (1H, d, J = 8.7 Hz), 9.41 (1H, d, J = 7.5 Hz), 9.54 (1H, d, J = 2.3 Hz).

### Example 69-II

### N-((1S)-2-hydroxy-2-methyl-1-(4-(trifluoromethoxy)phenyl)propyl)-5-(6-methylpyridin-3-yl)pyrazolo[1,5-a]pyrimidine-3-carboxamide

### A) ethyl 5-hydroxypyrazolo[1,5-a]pyrimidine-3-carboxylate

A mixture of ethyl 3-amino-1H-pyrazole-4-carboxylate (10.7 g), ethyl 3-ethoxyacrylate (15 mL) and cesium carbonate (33.8 g) in N,N-dimethylformamide (200 mL) was stirred at 110°C for 18 hr. The solvent was evaporated under reduced pressure, and the residue was diluted with water and acidified with acetic acid. The precipitated solid was collected by filtration, washed with water, and dried to give the title compound (13.7 g).
¹H NMR (300 MHz, DMSO-d₆) δ 1.28 (3H, t, J = 7.2 Hz), 4.27 (2H, q, J = 7.2 Hz), 6.15 (1H, d, J = 7.9 Hz), 8.13 (1H, s), 8.56 (1H, d, J = 7.9 Hz), 11.77 (1H, brs).

### B) ethyl 5-chloropyrazolo[1,5-a]pyrimidine-3-carboxylate

Ethyl 5-hydroxypyrazolo[1,5-a]pyrimidine-3-carboxylate (18.0 g) was suspended in acetonitrile (40 mL), phosphorus oxychloride (40 mL) was added at room temperature, and the mixture was stirred under a nitrogen atmosphere at 110°C for 4 hr. The reaction mixture was cooled, and concentrated under reduced pressure. The residue was diluted with ethyl acetate and neutralized with aqueous sodium bicarbonate solution, and insoluble material was filtered off with Celite. The organic layer of the filtrate was purified by a silica gel pad, and the solvent was evaporated under reduced pressure. The precipitated solid was washed with ethyl acetate/diisopropyl ether to give the title compound (14.1 g). The mother liquor was further concentrated under reduced pressure and the resulting solid was washed with ethyl acetate/diisopropyl ether to give the title compound (2.40 g).
¹H NMR (300 MHz, CDC13) δ 1.42 (3H, t, J = 7.2 Hz), 4.43 (2H, q, J = 7.2 Hz), 6.99 (1H, d, J = 7.2 Hz), 8.56 (1H, s), 8.63 (1H, d, J = 7.2 Hz).

### C) ethyl 5-(6-methylpyridin-3-yl)pyrazolo[1,5-a]pyrimidine-3-carboxylate

A mixture of ethyl 5-chloropyrazolo[1,5-a]pyrimidine-3-carboxylate (1.7 g), 2-methyl-5-(4,4,5,5-tetramethyl-1,3,2-dioxaborolan-2-yl)pyridine (2 g), potassium phosphate (3.4 g), toluene (32 mL) and water (8 mL) was purged with argon, bis(di-tert-butyl(4-dimethylaminophenyl)phosphine)dichloropalladium (II) (267 mg) was added at room temperature, and the reaction mixture was heated to 110°C and stirred for 15 hr. The reaction mixture was cooled, water and ethyl acetate and tetrahydrofuran were added, and insoluble material was filtered off. The organic layer of the filtrate was washed with saturated brine and dried over anhydrous sodium sulfate, and the solvent was evaporated under reduced pressure. The resulting solid was washed with ethyl acetate/diisopropyl ether to give the title compound (1.69 g). The mother liquor was further purified by silica gel column chromatography (hexane/ethyl acetate) and the obtained solid was washed with ethyl acetate/diisopropyl ether to give the title compound (0.29 g).
MS (API+): [M+H]⁺283.1.

### D) 5-(6-methylpyridin-3-yl)pyrazolo[1,5-a]pyrimidine-3-carboxylic acid

Ethyl 5-(6-methylpyridin-3-yl)pyrazolo[1,5-a]pyrimidine-3-carboxylate (1.98 g) was suspended in a mixture of ethanol (15 mL) and tetrahydrofuran (30 mL), 2M aqueous sodium hydroxide solution (7 mL) was added, and the reaction mixture was stirred at 60°C for 18 hr. The organic solvent of the reaction mixture was evaporated under reduced pressure, and the residue was acidified with 2M hydrochloric acid. The resulting solid was collected by filtration, washed with water, and dried to give the title compound (1.8 g).
MS (API+) : [M+H]⁺255.1.

### E) N-((1S)-2-hydroxy-2-methyl-1-(4-(trifluoromethoxy)phenyl)propyl)-5-(6-methylpyridin-3-yl)pyrazolo[1,5-a]pyrimidine-3-carboxamide

5-(6-Methylpyridin-3-yl)pyrazolo[1,5-a]pyrimidine-3-carboxylic acid (2.87 g), (S)-1-amino-2-methyl-1-(4-(trifluoromethoxy)phenyl)propan-2-ol hydrochloride (3.22 g) and triethylamine (4.72 mL) were suspended in acetonitrile (120 mL), 1-hydroxybenzotriazole (1.83 g) and 1-(3-dimethylaminopropyl)-3-ethylcarbodiimide hydrochloride (2.60 g) were added thereto, and the mixture was stirred under a nitrogen atmosphere at room temperature for 4 hr. To the reaction mixture was added water and the mixture was extracted with ethyl acetate. The extract was washed with saturated brine and dried over anhydrous sodium sulfate, and the solvent was evaporated under reduced pressure. The residue was purified by silica gel column chromatography (NH, hexane/ethyl acetate), and the obtained solid was recrystallized from ethyl acetate/heptane to give the title compound (3.24 g). The mother liquor was further concentrated and the obtained solid was recrystallized from ethyl acetate/heptane to give the title compound (0.80 g).
MS (API+) : [M+H]⁺486.1.
¹H NMR (300 MHz, DMSO-d₆) δ 1.04 (3H, s), 1.33 (3H, s), 2.60 (3H, s), 4.93 (1H, d, J = 8.5 Hz), 5.19 (1H, s), 7.24-7.32 (2H, m), 7.49-7.58 (3H, m), 7.98 (1H, d, J = 7.5 Hz), 8.50 (1H, s), 8.81 (1H, dd, J = 8.3, 2.3 Hz), 9.24 (1H, d, J = 8.5 Hz), 9.41 (1H, d, J = 7.5 Hz), 9.54 (1H, d, J = 2.3 Hz).

### Example 70-I

### 5-(1-(difluoromethyl)-1H-pyrazol-4-yl)-N-((1S)-2-hydroxy-2-methyl-1-(4-(trifluoromethoxy)phenyl)propyl)pyrazolo[1,5-a]pyrimidine-3-carboxamide

### A) ethyl 5-hydroxypyrazolo[1,5-a]pyrimidine-3-carboxylate

To a solution of ethyl 3-amino-1H-pyrazole-4-carboxylate (15.0 g) and ethyl 3-ethoxy-2-propenate (21.0 mL) in N,N-dimethylformamide (322 mL) was added cesium carbonate (56.7 g). The reaction mixture was stirred at 100°C overnight, water was added, and then adjusted to about pH 4 with acetic acid. The obtained solid were collected by filtration, and washed with water to give the title compound (17.1 g).
1H NMR (300 MHz, DMSO-d6) δ 1.28 (3H, t, J = 7.0 Hz), 4.27 (2H, q, J = 6.9 Hz), 6.14 (1H, d, J = 7.9 Hz), 8.13 (1H, s), 8.56 (1H, d, J = 7.9 Hz), 11.75 (1H, brs).

### B) 5-hydroxypyrazolo[1,5-a]pyrimidine-3-carboxylic acid

To a mixture of ethyl 5-hydroxypyrazolo[1,5-a]pyrimidine-3-carboxylate (20.8 g), tetrahydrofuran (223 mL) and ethanol (111 mL) was added 2M aqueous sodium hydroxide solution (201 mL). The reaction mixture was stirred at 50°C overnight, and the organic solvent was evaporated under reduced pressure. To the residue was added 2M hydrochloric acid (201 mL), and the precipitated solid was collected by filtration, and washed with water/ethanol to give the title compound (17.7 g).
MS (API-): [M-H]-177.9.

### C) 5-chloropyrazolo[1,5-a]pyrimidine-3-carbonyl chloride

To 5-hydroxypyrazolo[1,5-a]pyrimidine-3-carboxylic acid (4.24 g) was added phosphorus oxychloride (50 mL), and N,N-diisopropylethylamine (13.6 mL) was gradually added. The reaction mixture was heated under reflux for 3 hr, and phosphorus oxychloride was evaporated under reduced pressure. To the residue was added ethyl acetate, the mixture was cooled to 0°C, water was added, and the mixture was extracted with ethyl acetate. The extract was washed with water and saturated brine, filtered through a silica gel pad, and the solvent was evaporated under reduced pressure to give the title compound (3.07 g).
1H NMR (300 MHz, DMSO-d6) δ 7.37 (1H, d, J = 7.2 Hz), 8.61 (1H, s), 9.30 (1H, d, J = 7.2 Hz).

### D) (S)-5-chloro-N-(2-hydroxy-2-methyl-1-(4-(trifluoromethoxy)phenyl)propyl)pyrazolo[1,5-a]pyrimidine-3-carboxamide

To a mixture of 5-chloropyrazolo[1,5-a]pyrimidine-3-carbonyl chloride (1.07 g), (S)-1-amino-2-methyl-1-(4-(trifluoromethoxy)phenyl)propan-2-ol hydrochloride (1.42 g), and acetonitrile (20 mL) was added triethylamine (1.60 mL). The reaction mixture was stirred at room temperature for 3 hr, water was added, and the mixture was extracted with ethyl acetate. The extract was washed with saturated brine, and dried over anhydrous sodium sulfate. The solvent was evaporated under reduced pressure. The residue was purified by silica gel column chromatography (hexane/ethyl acetate), and the obtained solid was washed with diisopropyl ether to give the title compound (1.76 g).
MS (API+): [M+H]+429.1.

### E) 1-(difluoromethyl)-4-(4,4,5,5-tetramethyl-1,3,2-dioxaborolan-2-yl)-1H-pyrazole

To a mixture of 4-(4,4,5,5-tetramethyl-1,3,2-dioxaborolan-2-yl)-1H-pyrazole (1.50 g), sodium chlorodifluoroacetate (1.41 g) and acetonitrile (35 mL) was added 18-crown-6 (0.409 g). The reaction mixture was stirred at 90°C for 16 hr, poured into water, and the mixture was extracted with ethyl acetate. The extract was washed with saturated brine, and dried over anhydrous sodium sulfate. The solvent was evaporated under reduced pressure to give the title compound (1.54 g).
MS (API+) : [M+H]+245.1.

### F) 5-(1-(difluoromethyl)-1H-pyrazol-4-yl)-N-((1S)-2-hydroxy-2-methyl-1-(4-(trifluoromethoxy)phenyl)propyl)pyrazolo[1,5-a]pyrimidine-3-carboxamide

To a mixture of (S)-5-chloro-N-(2-hydroxy-2-methyl-1-(4-(trifluoromethoxy)phenyl)propyl)pyrazolo[1,5-a]pyrimidine-3-carboxamide (158 mg), 1-(difluoromethyl)-4-(4,4,5,5-tetramethyl-1,3,2-dioxaborolan-2-yl)-1H-pyrazole (135 mg), potassium carbonate (76.0 mg), toluene (10 mL) and water (1 mL) was added bis(di-tert-butyl(4-dimethylaminophenyl)phosphine)dichloropalladium(II) (9.1 mg) at room temperature. The reaction mixture was stirred under a nitrogen atmosphere by using microwave at 150°C for 25 min. After stirring, the mixture was purified by silica gel column chromatography (NH, hexane/ethyl acetate), and then silica gel column chromatography (hexane/ethyl acetate) to give the title compound (109 mg).
MS (API+) : [M+H]+511.1.
1H NMR (300 MHz, DMSO-d6) δ 1.04 (3H, s), 1.35 (3H, s), 4.88 (1H, d, J = 8.3 Hz), 5.27 (1H, s), 7.23-7.34 (2H, m), 7.48-7.58 (2H, m), 7.72 (1H, d, J = 7.2 Hz), 7.96 (1H, t, J = 59.0 Hz), 8.44 (1H, s), 8.80 (1H, s), 9.29 (1H, s), 9.31-9.42 (2H, m).

### Example 70-II 5-(1-(difluoromethyl)-1H-pyrazol-4-yl)-N-((1S)-2-hydroxy-2-methyl-1-(4-(trifluoromethoxy)phenyl)propyl)pyrazolo[1,5-a]pyrimidine-3-carboxamide

### A) ethyl 5-hydroxypyrazolo[1,5-a]pyrimidine-3-carboxylate

A mixture of ethyl 3-amino-1H-pyrazole-4-carboxylate (10.74 g), ethyl 3-ethoxyacrylate (15 mL) and cesium carbonate (33.8 g) in N,N-dimethylformamide (200 mL) was stirred at 110°C for 18 hr. The solvent was evaporated under reduced pressure, and the residue was diluted with water, and acidified with acetic acid. The precipitated solid was collected by filtration, washed with water, and dried to give the title compound (13.70 g).
1H NMR (300 MHz, DMSO-d6) δ 1.28 (3H, t, J = 7.2 Hz), 4.27 (2H, q, J = 7.2 Hz), 6.15 (1H, d, J = 7.9 Hz), 8.13 (1H, s), 8.56 (1H, d, J = 7.9 Hz), 11.77 (1H, brs).

### B) ethyl 5-chloropyrazolo[1,5-a]pyrimidine-3-carboxylate

Ethyl 5-hydroxypyrazolo[1,5-a]pyrimidine-3-carboxylate (18.0 g) was suspended in acetonitrile (40 mL), phosphorus oxychloride (40 mL) was added at room temperature, and the mixture was stirred under a nitrogen atmosphere at 110°C for 4 hr. The reaction mixture was cooled, and concentrated under reduced pressure. The residue was diluted with ethyl acetate, and neutralized with aqueous sodium hydrogen carbonate solution, and the insoluble material was filtered off through celite. The organic layer of the filtrate was purified with a silica gel pad, and the solvent was evaporated under reduced pressure. The precipitated solid was washed with ethyl acetate/diisopropyl ether to give the title compound (14.05 g). Furthermore, the mother liquor was concentrated under reduced pressure, and the resulting solid was washed with ethyl acetate/diisopropyl ether to give the title compound (2.40 g).
1H NMR (300 MHz, CDC13) δ 1.42 (3H, t, J = 7.2 Hz), 4.43 (2H, q, J = 7.2 Hz), 6.99 (1H, d, J = 7.2 Hz), 8.56 (1H, s), 8.63 (1H, d, J = 7.2 Hz).

### C) ethyl 5-(1-(difluoromethyl)-1H-pyrazol-4-yl)pyrazolo[1,5-a]pyrimidine-3-carboxylate

A mixed solution of ethyl 5-chloropyrazolo[1,5-a]pyrimidine-3-carboxylate (2.0 g), 1-(difluoromethyl)-4-(4,4,5,5-tetramethyl-1,3,2-dioxaborolan-2-yl)-1H-pyrazole (3.24 g), bis(di-tert-butyl(4-dimethylaminophenyl)phosphine)dichloropalladium (II) (0.628 g) and potassium phosphate (2.82 g) in toluene (81 mL) and water (8.1 mL) was stirred under an argon stream at 100°C for 16 hr. The reaction mixture was cooled to room temperature, and extracted with ethyl acetate. The extract was washed with water and saturated brine, and dried over anhydrous magnesium sulfate. The solvent was evaporated under reduced pressure. The residue was purified by silica gel column chromatography (hexane/ethyl acetate) to give the title compound (1.8 g).
MS (API+) : [M+H]+308.1.

### D) 5-(1-(difluoromethyl)-1H-pyrazol-4-yl)pyrazolo[1,5-a]pyrimidine-3-carboxylic acid

To a mixture of ethyl 5-(1-(difluoromethyl)-1H-pyrazol-4-yl)pyrazolo[1,5-a]pyrimidine-3-carboxylate (1.75 g), tetrahydrofuran (50 mL) and ethanol (25 mL) was added 2M aqueous sodium hydroxide solution (11.4 mL). The reaction mixture was stirred at 80°C for 4 hr, and the organic solvent was evaporated under reduced pressure. The solution was acidified by adding 2 M hydrochloric acid to the residue, and the precipitated solid was collected by filtration, and washed with water to give the title compound (1.54 g).
MS (API+) : [M+H]+280.1.

### E) 5-(1-(difluoromethyl)-1H-pyrazol-4-yl)-N-((1S)-2-hydroxy-2-methyl-1-(4-(trifluoromethoxy)phenyl)propyl)pyrazolo[1,5-a]pyrimidine-3-carboxamide

To a solution of 5-(1-(difluoromethyl)-1H-pyrazol-4-yl)pyrazolo[1,5-a]pyrimidine-3-carboxylic acid (1.49 g) and (S)-1-amino-2-methyl-1-(4-(trifluoromethoxy)phenyl)propan-2-ol hydrochloride (1.61 g) in N,N-dimethylformamide (50 mL) were added 1-hydroxybenzotriazole monohydrate (0.984 g) and 1-(3-dimethylaminopropyl)-3-ethylcarbodiimide hydrochloride (1.23 g). The reaction mixture was stirred at 60°C for 1 hr, poured into aqueous sodium hydrogen carbonate solution, and the mixture was extracted with ethyl acetate. The extract was washed with water and saturated brine, and dried over anhydrous sodium sulfate. The solvent was evaporated under reduced pressure. The residue was purified by silica gel column chromatography (NH, hexane/ethyl acetate), and crystallized from hexane/isopropylalcohol to give the title compound (2.00 g).
MS (API+) : [M+H]+511.1.
1H NMR (300 MHz, DMSO-d6) δ 1.04 (3H, s), 1.35 (3H, s), 4.88 (1H, d, J = 8.3 Hz), 5.27 (1H, s), 7.22-7.33 (2H, m), 7.48-7.59 (2H, m), 7.72 (1H, d, J = 7.5 Hz), 7.96 (1H, t, J = 59.0 Hz), 8.44 (1H, s), 8.80 (1H, s), 9.29 (1H, s), 9.31-9.42 (2H, m).

### Example 71

### N-((1S)-2-hydroxy-2-methyl-1-(4-(trifluoromethoxy)phenyl)propyl)-5-(1H-pyrazol-1-yl)pyrazolo[1,5-a]pyrimidine-3-carboxamide

To a mixture of (S)-5-chloro-N-(2-hydroxy-2-methyl-1-(4-(trifluoromethoxy)phenyl)propyl)pyrazolo[1,5-a]pyrimidine-3-carboxamide (126 mg), 1H-pyrazole (24.1 mg) and N,N-dimethylformamide (4 mL) was added potassium carbonate (48.8 mg). The reaction mixture was stirred at 90°C for 3 hr, poured into water, and the mixture was extracted with ethyl acetate. The extract was washed with saturated brine, and dried over anhydrous sodium sulfate. The solvent was evaporated under reduced pressure. The residue was purified by silica gel column chromatography (hexane/ethyl acetate), and the obtained solid was washed with hexane/ethyl acetate to give the title compound (102 mg).
MS (API+) : [M+H]+461.2.

### Example 72

### N-((1S)-2-hydroxy-2-methyl-1-(4-(trifluoromethoxy)phenyl)propyl)-5-(pyridin-2-yl)pyrazolo[1,5-a]pyrimidine-3-carboxamide

### A) ethyl 5-hydroxypyrazolo[1,5-a]pyrimidine-3-carboxylate

A mixture of ethyl 3-amino-1H-pyrazole-4-carboxylate (10.74 g), ethyl 3-ethoxyacrylate (15 mL) and cesium carbonate (33.8 g) in N,N-dimethylformamide (200 mL) was stirred at 110°C for 18 hr. The solvent was evaporated under reduced pressure, and the residue was diluted with water, and acidified with acetic acid. The precipitated solid was collected by filtration, washed with water, and dried to give the title compound (13.70 g).
1H NMR (300 MHz, DMSO-d6) δ 1.28 (3H, t, J = 7.2 Hz), 4.27 (2H, q, J = 7.2 Hz), 6.15 (1H, d, J = 7.9 Hz), 8.13 (1H, s), 8.56 (1H, d, J = 7.9 Hz), 11.77 (1H, brs).

### B) ethyl 5-chloropyrazolo[1,5-a]pyrimidine-3-carboxylate

Ethyl 5-hydroxypyrazolo[1,5-a]pyrimidine-3-carboxylate (18.0 g) was suspended in acetonitrile (40 mL), phosphorus oxychloride (40 mL) was added at room temperature, and the mixture was stirred under a nitrogen atmosphere at 110°C for 4 hr. The reaction mixture was cooled, and concentrated under reduced pressure. The residue was diluted with ethyl acetate, neutralized with aqueous sodium bicarbonate, and the insoluble material was filtered off through celite. The organic layer of the filtrate was purified by a silica gel pad, and the solvent was evaporated under reduced pressure. The precipitated solid was washed with ethyl acetate/diisopropyl ether to give the title compound (14.05 g). Furthermore, the mother liquor was concentrated under reduced pressure, and the resulting solid was washed with ethyl acetate/diisopropyl ether to give the title compound (2.40 g).
1H NMR (300 MHz, CDC13) δ 1.42 (3H, t, J = 7.2 Hz), 4.43 (2H, q, J = 7.2 Hz), 6.99 (1H, d, J = 7.2 Hz), 8.56 (1H, s), 8.63 (1H, d, J = 7.2 Hz).

### C) ethyl 5-(pyridin-2-yl)pyrazolo[1,5-a]pyrimidine-3-carboxylate

A mixture of ethyl 5-chloropyrazolo[1,5-a]pyrimidine-3-carboxylate (300 mg), lithium 4-methyl-1-(pyridin-2-yl)-2,6,7-trioxa-1-borabicyclo[2.2.2]octan-1-uide (566 mg), triphenylphosphine (139 mg), copper triiodide (127 mg), palladium acetate (29.9 mg) and N,N-dimethylformamide (5.00 mL) was heated at 80°C for 2 hr. The reaction mixture was filtered through a silica gel pad (NH and Si02), and concentrated under reduced pressure. To the residue was added ethyl acetate, and the resulting insoluble solid was collected by filtration, purified by silica gel chromatography (NH, ethyl acetate/hexane), and crystallized from diisopropyl ether to give the title compound (309 mg).
1H NMR (300 MHz, CDC13) δ 1.47 (3H, t, J = 7.1 Hz), 4.46 (2H, q, J = 7.1 Hz), 7.40-7.47 (1H, m), 7.86-7.95 (1H, m), 8.25 (1H, d, J = 7.4 Hz), 8.59 (1H, s), 8.71-8.76 (2H, m), 8.80 (1H, d, J = 7.4 Hz).

### D) N-((1S)-2-hydroxy-2-methyl-1-(4-(trifluoromethoxy)phenyl)propyl)-5-(pyridin-2-yl)pyrazolo[1,5-a]pyrimidine-3-carboxamide

A mixture of ethyl 5-(pyridin-2-yl)pyrazolo[1,5-a]pyrimidine-3-carboxylate (117 mg), 4M sodium hydroxide (0.545 mL), tetrahydrofuran (3.50 mL) and ethanol(3.50 mL) was stirred at 60°C for 2 hr. The reaction mixture was cooled to 0°C, neutralized with 6M hydrochloric acid (0.363 mL), and the solvent was evaporated under reduced pressure.

A mixture of the obtained residue (94.0 mg), (S)-1-amino-2-methyl-1-(4-(trifluoromethoxy)phenyl)propan-2-ol hydrochloride (94 mg), 0-(7-azabenzotriazol-1-yl)-N,N,N',N'-tetramethyluronium hexafluorophosphate (163 mg), N,N-diisopropylethylamine (0.172 mL) and N,N-dimethylformamide (5.00 mL) was stirred at room temperature for 12 hr. The reaction mixture was diluted with ethyl acetate and water, and the mixture was extracted with ethyl acetate. The organic layer was separated, washed with saturated brine, and dried over anhydrous sodium sulfate. The solvent was evaporated under reduced pressure. The residue was purified by silica gel chromatography (NH, ethyl acetate/hexane) to give the title compound (130 mg).
1H NMR (300 MHz, CDC13) δ 1.21 (3H, s), 1.49 (3H, s), 1.96 (1H, s), 5.14 (1H, d, J = 8.3 Hz), 7.14-7.22 (2H, m), 7.44-7.56 (3H, m), 7.88-7.97 (1H, m), 8.26 (1H, d, J = 7.4 Hz), 8.64 (1H, s), 8.75-8.88 (3H, m), 9.25 (1H, d, J = 8.3 Hz).

### Example 73

### 5-(3-fluoropyridin-4-yl)-N-((1S)-2-hydroxy-2-methyl-1-(4-(trifluoromethoxy)phenyl)propyl)pyrazolo[1,5-a]pyrimidine-3-carboxamide

A mixture of (S)-5-chloro-N-(2-hydroxy-2-methyl-1-(4-(trifluoromethoxy)phenyl)propyl)pyrazolo[1,5-a]pyrimidine-3-carboxamide (100 mg), 3-fluoro-4-(4,4,5,5-tetramethyl-1,3,2-dioxaborolan-2-yl)pyridine (156 mg), bis(di-tert-butyl(4-dimethylaminophenyl)phosphine)dichloropalladium (II) (16.5 mg), potassium carbonate (97.0 mg), toluene (2.00 mL) and water (0.20 mL) was heated under microwave irradiation at 120°C for 9 hr. The reaction mixture was purified by silica gel chromatography (NH, ethyl acetate/hexane), and crystallized from ethyl acetate/heptane to give the title compound (77.0 mg).
1H NMR (300 MHz, CDC13) δ 1.18 (3H, s), 1.46 (3H, s), 1.90 (1H, brs), 5.12 (1H, d, J = 8.5 Hz), 7.13-7.20 (2H, m), 7.44-7.52 (2H, m), 7.65-7.70 (1H, m), 8.33 (1H, dd, J = 6.8, 5.1 Hz), 8.66 (1H, d, J = 5.1 Hz), 8.69-8.74 (2H, m), 8.90 (1H, d, J = 7.4 Hz), 9.07 (1H, d, J = 8.3 Hz).

### Example 74

### N-((1S)-2-hydroxy-2-methyl-1-(4-(trifluoromethoxy)phenyl)propyl)-5-(4-methyl-1H-1,2,3-triazol-1-yl)pyrazolo[1,5-a]pyrimidine-3-carboxamide

A solution of (S)-5-chloro-N-(2-hydroxy-2-methyl-1-(4-(trifluoromethoxy)phenyl)propyl)pyrazolo[1,5-a]pyrimidine-3-carboxamide (200 mg), 4-methyl-1H-1,2,3-triazole (46.5 mg) and potassium carbonate (97 mg) in N,N-dimethylformamide (2 mL) was stirred at 80°C for 2 hr. Water was added to the reaction mixture at room temperature, and the mixture was extracted with ethyl acetate. The extract was washed with water and saturated brine, and dried over anhydrous magnesium sulfate. The solvent was evaporated under reduced pressure, and the residue was fractionated by HPLC (C18, mobile phase: water/acetonitrile (0.1% TFA-containing system)). The obtained fraction with a shorter retention time was added saturated aqueous sodium hydrogen carbonate solution, and the mixture was extracted with ethyl acetate. The extract was washed with water and saturated brine, and dried over anhydrous magnesium sulfate. The solvent was evaporated under reduced pressure. The residue was crystallized from hexane/ethyl acetate to give the title compound (37.5 mg).
MS (API+): [M+H]+476.2.

### Example 75

### N-((1S)-1-(3-fluoro-4-(trifluoromethoxy)phenyl)-2-hydroxy-2-methylpropyl)-1-methyl-7-oxo-5-(pyridin-2-yl)-1,7-dihydropyrazolo[1,5-a]pyrimidine-3-carboxamide A) methyl 3-oxo-3-(pyridin-2-yl)propanoate

To a solution of picolinic acid (3.08 g) in tetrahydrofuran (62.5 mL) was added 1,1'-carbonyldiimidazole (4.86 g) at room temperature. The reaction mixture was stirred under an argon atmosphere at room temperature for 2 hr. To the reaction mixture were added monomethylmonopotassium malonate (3.90 g) and magnesium chloride (2.38 g) at room temperature, and the mixture was stirred under an argon atmosphere at room temperature overnight. The reaction mixture was neutralized with 2M hydrochloric acid, and extracted with ethyl acetate. The organic layer was washed with saturated brine, and dried over anhydrous magnesium sulfate. The solvent was evaporated under reduced pressure. The residue was purified by silica gel column chromatography (ethyl acetate/hexane) to give the title compound (2.48 g).
MS (API+) : [M+H]+180.1.

### B) ethyl 7-hydroxy-5-(pyridin-2-yl)pyrazolo[1,5-a]pyrimidine-3-carboxylate

A mixture of ethyl 5-amino-1H-pyrazole-4-carboxylate (1.09 g), methyl 3-oxo-3-(pyridin-2-yl)propanoate (1.38 g), and acetic acid (23.3 mL) was stirred at 120°C for 3 days. The reaction mixture was cooled to room temperature, diisopropyl ether was added, and the mixture was stirred at room temperature for 30 min. The solid was collected by filtration, and washed with diisopropyl ether to give the title compound (1.60 g).
MS (API+) : [M+H]+285.1.

### C) 7-hydroxy-5-(pyridin-2-yl)pyrazolo[1,5-a]pyrimidine-3-carboxylic acid

A mixture of ethyl 7-hydroxy-5-(pyridin-2-yl)pyrazolo[1,5-a]pyrimidine-3-carboxylate (853 mg), 2M aqueous sodium hydroxide solution (7.50 mL), ethanol (7.50 mL), and tetrahydrofuran (7.50 mL) was stirred at 60°C overnight. To the reaction mixture was added 2M hydrochloric acid (7.50 mL) at 0°C, and ethanol and tetrahydrofuran were evaporated under reduced pressure. The resulting solid was collected by filtration, washed with water, and dried under reduced pressure to give the title compound (645 mg).
MS (API+) : [M+H]+257.1.

### D) (S)-N-(1-(3-fluoro-4-(trifluoromethoxy)phenyl)-2-hydroxy-2-methylpropyl)-7-hydroxy-5-(pyridin-2-yl)pyrazolo[1,5-a]pyrimidine-3-carboxamide

A mixture of 7-hydroxy-5-(pyridin-2-yl)pyrazolo[1,5-a]pyrimidine-3-carboxylic acid (256 mg), (S)-1-amino-1-(3-fluoro-4-(trifluoromethoxy)phenyl)-2-methylpropan-2-ol hydrochloride (334 mg), 1-hydroxybenzotriazole monohydrate (184 mg), 1-(3-dimethylaminopropyl)-3-ethylcarbodiimide hydrochloride (230 mg), triethylamine (0.335 mL), and N,N-dimethylformamide (10.0 mL) was stirred at 60°C overnight. The reaction mixture was stirred at room temperature, poured into water, and the mixture was extracted with ethyl acetate. The organic layer was washed with saturated brine, and dried over anhydrous magnesium sulfate. The solvent was evaporated under reduced pressure. The residue was purified by silica gel column chromatography (ethyl acetate/hexane) to give the title compound (165 mg) containing impurity. The obtained resultant product was used for the next reaction without further purification.
MS (API+) : [M+H]+506.2.

### E) N-((1S)-1-(3-fluoro-4-(trifluoromethoxy)phenyl)-2-hydroxy-2-methylpropyl)-1-methyl-7-oxo-5-(pyridin-2-yl)-1,7-dihydropyrazolo[1,5-a]pyrimidine-3-carboxamide

To a solution of (S)-N-(1-(3-fluoro-4-(trifluoromethoxy)phenyl-2-hydroxy-2-methylpropyl)-7-hydroxy-5-(pyridin-2-yl)pyrazolo[1,5-a]pyrimidine-3-carboxamide (165 mg) in N,N-dimethylformamide (3.27 mL) were added methyl iodide (40.8 µL) and potassium carbonate (90 mg) at room temperature. The reaction mixture was stirred at 60°C overnight, poured into water, and the mixture was extracted with ethyl acetate. The organic layer was washed with saturated brine, and dried over anhydrous magnesium sulfate. The solvent was evaporated under reduced pressure. The residue was purified by silica gel column chromatography (NH, ethyl acetate/hexane), pulverized with diisopropyl ether, and washed to give the title compound (79 mg).
MS (API+) : [M+H]+520.2.

### Example 76

### N-((1S)-2-hydroxy-2-methyl-1-(4-(trifluoromethoxy)phenyl)propyl)-5-(3-methyl-2-oxopyridine-1(2H)-yl)pyrazolo[1,5-a]pyrimidine-3-carboxamide

A mixture of (S)-5-chloro-N-(2-hydroxy-2-methyl-1-(4-(trifluoromethoxy)phenyl)propyl)pyrazolo[1,5-a]pyrimidine-3-carboxamide (100 mg), 2-hydroxy-3-methylpyridine (31 mg), cesium carbonate (152 mg) and toluene (2 mL) was argon-substituted, and tris(dibenzylideneacetone)dipalladium(0) (21 mg) and 4,5-bis(diphenylphosphino)-9,9-dimethylxanthene (27 mg) were added at room temperature. The reaction mixture was heated to 100°C under an argon atmosphere and stirred for 15 hr. To the reaction mixture were added water and ethyl acetate, and the organic layer was washed with saturated brine, dried over anhydrous magnesium sulfate, and the solvent was evaporated under reduced pressure. The residue was purified by silica gel column chromatography (NH, hexane/ethyl acetate), and the obtained oil was solidified and pulverized with diisopropyl ether to give the title compound (36 mg).
MS (API+) : [M+H]+502.2.

### Example 77

### N-((1S)-2-hydroxy-2-methyl-1-(4-(trifluoromethoxy)phenyl)propyl)-1-methyl-7-oxo-5-(pyridin-2-yl)-1,7-dihydropyrazolo[1,5-a]pyrimidine-3-carboxamide

By a method similar to that in Example 75, steps A to E, the title compound (81 mg) was obtained.
MS (API+) : [M+H]+502.2.

### Example 78

### N-((1S)-2-hydroxy-2-methyl-1-(4-(trifluoromethoxy)phenyl)propyl)-6-methyl-5-(1H-1,2,3-triazol-1-yl)pyrazolo[1,5-a]pyrimidine-3-carboxamide

A mixture of ethyl 5-chloro-6-methylpyrazolo[1,5-a]pyrimidine-3-carboxylate (200 mg), 1H-1,2,3-triazole (63.4 mg), potassium carbonate (138 mg) and N,N-dimethylformamide (2 mL) was stirred at room temperature overnight. The reaction mixture was poured into water, and the mixture was extracted with ethyl acetate. The organic layer was washed with saturated brine, dried over magnesium sulfate, and concentrated under reduced pressure to give a solid (232 mg). To the obtained solid (200 mg) were added (S)-1-amino-2-methyl-1-(4-(trifluoromethoxy)phenyl)propan-2-ol hydrochloride (250 mg), triethylamine (0.256 mL), 1.8M trimethylaluminum/toluene solution (1.02 mL) and toluene (5 mL), and the mixture was stirred at 150°C for 10 min under microwave irradiation. The reaction mixture was added to saturated aqueous sodium hydrogen carbonate solution at room temperature, and the mixture was extracted with ethyl acetate. The organic layer was washed with saturated brine, dried over magnesium sulfate, and the solvent was evaporated under reduced pressure. The residue was fractionated by HPLC (C18, mobile phase: water/acetonitrile (0.1% TFA-containing system)), to the obtained fraction was added saturated aqueous sodium hydrogen carbonate solution, and the mixture was extracted with ethyl acetate, dried over anhydrous magnesium sulfate, and concentrated under reduced pressure to give the title compound (73.2 mg).
MS (API+): [M+H]+476.2.

### Example 79

### N-((1S)-2-hydroxy-2-methyl-1-(4-(trifluoromethoxy)phenyl)propyl)-6-methyl-5-(4-(trifluoromethyl)-1H-imidazol-1-yl)pyrazolo[1,5-a]pyrimidine-3-carboxamide

### A) ethyl 5,7-dihydroxy-6-methylpyrazolo[1,5-a]pyrimidine-3-carboxylate

A mixture of 5-amino-1H-pyrazole-4-carboxylic acid (22.4 g), diethyl 2-methylmalonate (24.6 mL), and tributylamine (51.6 mL) was stirred at 160°C overnight. After cooling to room temperature, the mixture was poured into a 1M aqueous sodium hydroxide solution, and the mixture was extracted twice with ethyl acetate. The aqueous layer was separated, and adjusted to pH 1 with 1M hydrochloric acid. The resulting solid was collected by filtration, washed with water, and dried under reduced pressure to give the title compound (21.0 g).
MS (API+) : [M+H]+238.1.

### B) ethyl 5,7-dichloro-6-methylpyrazolo[1,5-a]pyrimidine-3-carboxylate

A mixture of ethyl 5,7-dihydroxy-6-methylpyrazolo[1,5-a]pyrimidine-3-carboxylate (21.0 g) and phosphorus oxychloride (206 mL) was stirred at 90°C overnight, and concentrated under reduced pressure. To the residue was added ice water, and the mixture was extracted with ethyl acetate. The extract was washed with saturated brine, dried over anhydrous sodium sulfate, and the solvent was evaporated under reduced pressure to give a crude product (8.12 g). A mixture of the crude product (2.0 g) and phosphorus oxychloride (25.5 mL) was stirred at 110°C for 3 days. The reaction mixture was concentrated under reduced pressure, the residue was diluted with ethyl acetate and poured into ice water, and the organic layer was separated. The organic layer was washed with saturated brine, dried over anhydrous magnesium sulfate, and the solvent was evaporated under reduced pressure. The residue was purified by silica gel column chromatography (hexane/ethyl acetate) to give the title compound (0.66 g).
1H NMR (300 MHz, DMSO-d6) δ 1.32 (3H, t, J = 7.1 Hz), 2.49 (3H, s), 4.31 (2H, q, J = 7.2 Hz), 8.69 (1H, s).

### C) ethyl 5-chloro-6-methylpyrazolo[1,5-a]pyrimidine-3-carboxylate

A mixture of ethyl 5,7-dichloro-6-methylpyrazolo[1,5-a]pyrimidine-3-carboxylate (660 mg) and zinc powder (338 mg), concentrated aqueous ammonia (2.0 mL), saturated brine (6.0 mL), and tetrahydrofuran (6.0 mL) was stirred at room temperature for 2 hr. The reaction mixture was filtered and washed with ethyl acetate, and the organic layer was separated. The organic layer was washed with saturated brine, dried over anhydrous magnesium sulfate, and the solvent was evaporated under reduced pressure. The residue was purified by silica gel column chromatography (hexane/ethyl acetate) to give the title compound (198 mg).
1H NMR (300 MHz, DMSO-d6) δ 1.31 (3H, t, J = 7.0 Hz), 2.36 (3H, d, J = 1.1 Hz), 4.30 (2H, q, J = 7.2 Hz), 8.59 (1H, s), 9.34 (1H, d, J = 0.8 Hz).

### D) ethyl 6-methyl-5-(4-(trifluoromethyl)-1H-imidazol-1-yl)pyrazolo[1,5-a]pyrimidine-3-carboxylate

A mixture of ethyl 5-chloro-6-methylpyrazolo[1,5-a]pyrimidine-3-carboxylate (250 mg), 4-(trifluoromethyl)-1H-imidazole (156 mg), potassium carbonate (173 mg) and N,N-dimethylacetamide (2 mL) was stirred at room temperature overnight. The reaction mixture was added to saturated brine at room temperature, and the mixture was extracted with ethyl acetate. The organic layer was washed with saturated brine, dried over magnesium sulfate, and concentrated under reduced pressure. The residue was pulverized and washed with diisopropyl ether and hexane to give the title compound (336 mg).
MS (API+) : [M+H]+340.1.

### E) N-((1S)-2-hydroxy-2-methyl-1-(4-(trifluoromethoxy)phenyl)propyl)-6-methyl-5-(4-(trifluoromethyl)-1H-imidazol-1-yl)pyrazolo[1,5-a]pyrimidine-3-carboxamide

Ethyl 6-methyl-5-(4-(trifluoromethyl)-1H-imidazol-1-yl)pyrazolo[1,5-a]pyrimidine-3-carboxylate (300 mg), (S)-1-amino-2-methyl-1-(4-(trifluoromethoxy)phenyl)propan-2-ol hydrochloride (303 mg), 1.8M trimethylaluminum/toluene solution (1.47 mL) and toluene (2.5 mL) were added, and the mixture was stirred at 170°C for 30 min under microwave irradiation. The reaction mixture was added to saturated aqueous sodium hydrogen carbonate solution at room temperature, and the mixture was filtered through celite, and the filtrate was extracted with ethyl acetate. The organic layer was washed with saturated brine, dried over magnesium sulfate, and the solvent was evaporated under reduced pressure. The residue was purified by silica gel column chromatography (ethyl acetate/hexane), and the residue was pulverized and washed with ethyl acetate/diisopropyl ether to give the title compound (241 mg). MS (API+) : [M+H]+543.2.
1H NMR (300 MHz, CDC13) δ 1.07-1.20 (3H, m), 1.44 (3H, s), 1.70 (1H, s), 2.64 (3H, d, J = 0.8 Hz), 5.02 (1H, d, J = 8.3 Hz), 7.09-7.21 (2H, m), 7.37-7.52 (2H, m), 8.14-8.23 (1H, m), 8.46 (1H, s), 8.64 (1H, s), 8.68 (1H, d, J = 8.7 Hz), 8.82 (1H, d, J = 1.1 Hz).

### Example 80-I

### N-((1S)-2-hydroxy-2-methyl-1-(4-(trifluoromethoxy)phenyl)propyl)-6-methyl-5-(4-methyl-1H-1,2,3-triazol-1-yl)pyrazolo[1,5-a]pyrimidine-3-carboxamide A) ethyl 5,7-dihydroxy-6-methylpyrazolo[1,5-a]pyrimidine-3-carboxylate

A mixture of ethyl 5-amino-1H-pyrazole-4-carboxylate (22.4 g) and diethyl 2-methylmalonate (24.6 mL), tributylamine (51.6 mL) was stirred overnight at 160°C. After cooling to room temperature, the mixture was poured into 1M aqueous sodium hydroxide solution, and washed twice with ethyl acetate. The pH of the aqueous layer was adjusted to 1 with 1M hydrochloric acid. The resulting solid was collected by filtration, washed with water, and dried under reduced pressure to give the title compound (21.0 g).
MS (API+): [M+H]⁺238.1.

### B) ethyl 5,7-dichloro-6-methylpyrazolo[1,5-a]pyrimidine-3-carboxylate

A mixture of ethyl 5,7-dihydroxy-6-methylpyrazolo[1,5-a]pyrimidine-3-carboxylate (21.0 g) and phosphorus oxychloride (206 mL) was stirred overnight at 90°C, and concentrated under reduced pressure. To the residue was added ice water, and the mixture was extracted with ethyl acetate. The extract was washed with saturated brine, and dried over anhydrous magnesium sulfate. The solvent was evaporated under reduced pressure and the residue was washed with ethyl acetate to give a crude product (8.12 g). A mixture of the crude product (2.0 g) and phosphorus oxychloride (25.5 mL) was stirred at 110°C for 3 days. The reaction mixture was concentrated under reduced pressure, and the residue was diluted with ethyl acetate and poured into ice water, and the organic layer was separated. The organic layer was washed with saturated brine and dried over anhydrous magnesium sulfate, and the solvent was evaporated under reduced pressure. The residue was purified by silica gel column chromatography (hexane/ethyl acetate) to give the title compound (0.660 g).
¹H NMR (300 MHz, DMSO-d₆) δ 1.32 (3H, t, J = 7.1 Hz), 2.49 (3H, s), 4.31 (2H, q, J = 7.2 Hz), 8.69 (1H, s).

### C) ethyl 5-chloro-6-methylpyrazolo[1,5-a]pyrimidine-3-carboxylate

A mixture of ethyl 5,7-dichloro-6-methylpyrazolo[1,5-a]pyrimidine-3-carboxylate (660 mg) and zinc powder (338 mg), conc. aqueous ammonia (2.0 mL), saturated brine (6.0 mL), tetrahydrofuran (6.0 mL) was stirred at room temperature for 2 hr. The reaction mixture was filtered, washed with ethyl acetate, and the organic layer was separated. The organic layer was washed with saturated brine and dried over anhydrous magnesium sulfate, and the solvent was evaporated under reduced pressure. The residue was purified by silica gel column chromatography (hexane/ethyl acetate) to give the title compound (198 mg).
¹H NMR (300 MHz, DMSO-d₆) δ 1.31 (3H, t, J = 7.0 Hz), 2.36 (3H, d, J = 1.1 Hz), 4.30 (2H, q, J = 7.2 Hz), 8.59 (1H, s), 9.34 (1H, d, J = 0.8 Hz).

### D) ethyl 6-methyl-5-(4-methyl-1H-1,2,3-triazol-1-yl)pyrazolo[1,5-a]pyrimidine-3-carboxylate

A mixture of ethyl 5-chloro-6-methylpyrazolo[1,5-a]pyrimidine-3-carboxylate (400 mg), 4-methyl-1H-1,2,3-triazole (153 mg), potassium carbonate (277 mg) and N,N-dimethylformamide (5 mL) was stirred at room temperature overnight. The reaction mixture was poured into water at room temperature, and the mixture was extracted with ethyl acetate. The organic layer was washed with saturated brine, dried over anhydrous magnesium sulfate, and concentrated under reduced pressure. The residue was purified by silica gel column chromatography (ethyl acetate/hexane) to give the title compound (106 mg).
MS (API+): [M+H]⁺287.1.

### E) N-((1S)-2-hydroxy-2-methyl-1-(4-(trifluoromethoxy)phenyl)propyl)-6-methyl-5-(4-methyl-1H-1,2,3-triazol-1-yl)pyrazolo[1,5-a]pyrimidine-3-carboxamide

A mixture of ethyl 6-methyl-5-(4-methyl-1H-1,2,3-triazol-1-yl)pyrazolo[1,5-a]pyrimidine-3-carboxylate (105 mg), (S)-1-amino-2-methyl-1-(4-(trifluoromethoxy)phenyl)propan-2-ol hydrochloride (110 mg), 1.8M trimethylaluminum-toluene solution (0.611 mL) and toluene (2 mL) was stirred under microwave irradiation at 165°C for 25 min. The reaction mixture was cooled to room temperature, (S)-1-amino-2-methyl-1-(4-(trifluoromethoxy)phenyl)propan-2-ol hydrochloride (20 mg) was added, and the mixture was stirred under microwave irradiation at 165°C for 20 min. The reaction mixture was added to saturated aqueous sodium hydrogen carbonate solution at room temperature, and the mixture was filtered through Celite. The filtrate was extracted with ethyl acetate. The organic layer was washed with saturated brine and dried over magnesium sulfate, and concentrated under reduced pressure. The residue was purified by silica gel column chromatography (ethyl acetate/hexane) to give the title compound (29.4 mg).
MS (API+) : [M+H]⁺490.2.
¹H NMR (300 MHz, CDC13) δ 1.17 (3H, s), 1.48 (3H, s), 1.70 (1H, s), 2.50 (3H, d, J = 0.8 Hz), 2.84 (3H, d, J = 0.8 Hz), 5.05 (1H, d, J = 8.3 Hz), 7.12-7.22 (2H, m), 7.38-7.50 (2H, m), 8.60 (1H, s), 8.68-8.83 (3H, m).

### Example 80-II

### N-((1S)-2-hydroxy-2-methyl-1-(4-(trifluoromethoxy)phenyl)propyl)-6-methyl-5-(4-methyl-1H-1,2,3-triazol-1-yl)pyrazolo[1,5-a]pyrimidine-3-carboxamide A) tert-butyl 3-amino-1H-pyrazole-4-carboxylate

A mixture of tert-butyl 2-cyanoacetate (24.3 g) and 1-tert-butoxy-N,N,N',N'-tetramethylmethanediamine (35.5 mL) was stirred at room temperature for 30 min. The reaction mixture was concentrated under reduced pressure to give a crude product of tert-butyl 2-cyano-3-(dimethylamino)acrylate. A solution of the obtained crude product and hydrazine monohydrate (8.39 mL) in methanol (344 mL) was stirred overnight at 70°C. The reaction mixture was cooled to room temperature and poured into water, and the mixture was extracted with ethyl acetate. The organic layer was washed with saturated brine, and dried over anhydrous magnesium sulfate. The solvent was evaporated under reduced pressure and the residue was crystallized from diisopropyl ether to give the title compound (18.6 g).
MS (API+): [M+H]⁺ 184.1.

### B) methyl 3,3-dimethoxy-2-methylpropanoate

To a solution of methyl methacrylate (51.2 g) in ethyl acetate (301 mL) was added a solution of bromine (26.2 mL) in ethyl acetate (20 mL) at 0°C. The reaction mixture was stirred at room temperature for 16 hr, and saturated aqueous sodium thiosulfate solution was added. The reaction mixture was diluted with water, and extracted with ethyl acetate. The organic layer was washed with saturated brine, dried over anhydrous sodium sulfate, and the solvent was evaporated under reduced pressure to give methyl 2,3-dibromo-2-methylpropanoate (143 g). A 28% solution (213 g) of sodium methoxide in methanol was diluted with methanol (221 mL), heated to 70°C, and a solution of the obtained crude product (143 g) in methanol (20 mL) was added. The reaction mixture was stirred at 70°C for 3 hr and diluted with diethyl ether (550 mL) and water. The organic layer was washed with water and saturated brine and dried over anhydrous sodium sulfate, and the solvent was evaporated under reduced pressure to give the title compound (39.4 g).
¹H NMR (300 MHz, CDC13) δ 1.17 (3H, d, J = 7.2 Hz), 2.71-2.86 (1H, m), 3.35 (3H, s), 3.38 (3H, s), 3.70 (3H, s), 4.50 (1H, d, J = 7.7 Hz).

### C) tert-butyl 5-hydroxy-6-methylpyrazolo[1,5-a]pyrimidine-3-carboxylate

To a solution of tert-butyl 3-amino-1H-pyrazole-4-carboxylate (6.80 g) and methyl 3,3-dimethoxy-2-methylpropanoate (9.03 g) in N,N-dimethylformamide (74.2 mL) was added cesium carbonate (21.8 g), and the reaction mixture was stirred at 100°C for 16 hr. The reaction mixture was diluted with water, and adjusted to about pH 4 with acetic acid. The resulting solid was collected by filtration, washed with water, and dried under reduced pressure to give the title compound (9.24 g).
MS (API+): [M+H]⁺250.1.

### D) tert-butyl 5-chloro-6-methylpyrazolo[1,5-a]pyrimidine-3-carboxylate

To a solution of triphenylphosphine (8.14 g) in 1,2-dichloroethane (80.0 mL) was added carbon tetrachloride (2.92 mL) at room temperature. The reaction mixture was stirred under a nitrogen atmosphere at room temperature for 30 min, and a suspension of tert-butyl 5-hydroxy-6-methylpyrazolo[1,5-a]pyrimidine-3-carboxylate (1.5 g) in 1,2-dichloroethane (70 mL) was added at room temperature. The reaction mixture was stirred under a nitrogen atmosphere at 75°C to 85°C for 4.5 hr, and concentrated under reduced pressure. The residue was diluted with ethyl acetate and water, and the mixture was extracted with ethyl acetate. The extract was washed with saturated brine and dried over anhydrous sodium sulfate, and the solvent was evaporated under reduced pressure. The residue was purified by silica gel column chromatography (hexane/ethyl acetate) to give the title compound (1.54 g).
¹H NMR (300 MHz, CDC13) δ 1.62 (9H, s), 2.42 (3H, d, J = 1.1 Hz), 8.40 (1H, s), 8.52 (1H, d, J = 1.1 Hz).

### E) tert-butyl 6-methyl-5-(4-methyl-1H-1,2,3-triazol-1-yl)pyrazolo[1,5-a]pyrimidine-3-carboxylate

To a solution of tert-butyl 5-chloro-6-methylpyrazolo[1,5-a]pyrimidine-3-carboxylate (607 mg) in N,N-dimethylformamide (39.8 mL) were added 4-methyl-1H-1,2,3-triazole (208 mg) and potassium carbonate (378 mg). The reaction mixture was stirred at room temperature for 2 hr, water was added, and the mixture was extracted with ethyl acetate. The extract was washed with water and saturated brine, dried over anhydrous sodium sulfate, and the solvent was evaporated under reduced pressure. The residue was purified by silica gel column chromatography (hexane/ethyl acetate) to give the title compound (167 mg).
MS (API+): [M+H]⁺315.1.

### F) tert-butyl 5-azido-6-methylpyrazolo[1,5-a]pyrimidine-3-carboxylate

A mixture of tert-butyl 5-chloro-6-methylpyrazolo[1,5-a]pyrimidine-3-carboxylate (268 mg), sodium azide (98 mg), and ethanol (5.00 mL) was stirred overnight at 60°C. The reaction mixture was cooled to room temperature, poured into water, and the mixture was extracted with ethyl acetate. The organic layer was washed with saturated brine and dried over anhydrous magnesium sulfate, and the solvent was evaporated under reduced pressure to give the title compound (279 mg). The obtained crude product was used without purification for the next reaction.
MS (API+) : [M+H]⁺275.1.

### G) tert-butyl 6-methyl-5-(4-methyl-1H-1,2,3-triazol-1-yl)pyrazolo[1,5-a]pyrimidine-3-carboxylate

A mixture of tert-butyl 5-azido-6-methylpyrazolo[1,5-a]pyrimidine-3-carboxylate (239 mg) and 2-(triphenylphosphoranylidene)propionaldehyde (291 mg), tetrahydrofuran (4.36 mL) was stirred at 60°C for 5 hr. The reaction mixture was cooled to room temperature and the solvent was evaporated under reduced pressure. The residue was purified by silica gel column chromatography (ethyl acetate/hexane) to give the title compound (256 mg).
MS (API+) : [M+H]⁺315.2.

### H) N-((1S)-2-hydroxy-2-methyl-1-(4-(trifluoromethoxy)phenyl)propyl)-6-methyl-5-(4-methyl-1H-1,2,3-triazol-1-yl)pyrazolo[1,5-a]pyrimidine-3-carboxamide

To a solution of tert-butyl 6-methyl-5-(4-methyl-1H-1,2,3-triazol-1-yl)pyrazolo[1,5-a]pyrimidine-3-carboxylate (1.12 g) in toluene (50.8 mL) was added trifluoroacetic acid (29.0 mL) at 0°C. The reaction mixture was stirred at room temperature overnight, and concentrated under reduced pressure to give a crude product (990 mg) of 6-methyl-5-(4-methyl-1H-1,2,3-triazol-1-yl)pyrazolo[1,5-alpyrimidine-3-carboxylic acid. To a solution of the obtained crude product (987 mg) and (1S)-1-amino-2-methyl-1-(4-(trifluoromethoxy)phenyl)propan-2-ol hydrochloride (1.15 g) in N,N-dimethylformamide (127 mL) were added 1-hydroxybenzotriazole monohydrate (724 mg), 1-(3-dimethylaminopropyl)-3-ethylcarbodiimide hydrochloride (897 mg) and triethylamine (2.69 mL). The reaction mixture was stirred at room temperature overnight, water was added, and the mixture was extracted with ethyl acetate. The extract was washed with saturated aqueous sodium hydrogen carbonate solution, water and saturated brine and dried over anhydrous sodium sulfate, and the solvent was evaporated under reduced pressure. The residue was purified by silica gel column chromatography (hexane/ethyl acetate) to give the title compound (1.54 g).
MS (API+) : [M+H]⁺490.2.
¹H NMR (300 MHz, CDC13) δ 1.17 (3H, s), 1.48 (3H, s), 1.72 (1H, s), 2.50 (3H, d, J = 0.8 Hz), 2.84 (3H, d, J = 0.8 Hz), 5.05 (1H, d, J = 8.3 Hz), 7.17 (2H, d, J = 7.9 Hz), 7.38-7.48 (2H, m), 8.60 (1H, s), 8.67-8.82 (3H, m).

### I) N-((1S)-2-hydroxy-2-methyl-1-(4-(trifluoromethoxy)phenyl)propyl)-6-methyl-5-(4-methyl-1H-1,2,3-triazol-1-yl)pyrazolo[1,5-a]pyrimidine-3-carboxamide

N-((1S)-2-hydroxy-2-methyl-1-(4-(trifluoromethoxy)phenyl)propyl)-6-methyl-5-(4-methyl-1H-1,2,3-triazol-1-yl)pyrazolo[1,5-a]pyrimidine-3-carboxamide (3.07 g) was dissolved in ethyl acetate (220 mL) at 50°C to 60°C, and hexane (150 mL) was added at 50°C to 60°C. The reaction mixture was stirred at room temperature for 2 hr, hexane (70 mL) was added, and the mixture was stirred at room temperature for 10 min. The resulting solid was collected by filtration and dried under reduced pressure to give the title compound (2.48 g).
MS (API+) : [M+H]⁺490.2.
¹H NMR (300 MHz, CDC13) δ 1.17 (3H, s), 1.48 (3H, s), 1.74 (1H, s), 2.50 (3H, s), 2.84 (3H, d, J = 0.8 Hz), 5.05 (1H, d, J = 8.3 Hz), 7.17 (2H, d, J = 7.9 Hz), 7.40-7.49 (2H, m), 8.60 (1H, s), 8.69-8.83 (3H, m).

### Example 81

### N-((1S)-1-(3-fluoro-4-(trifluoromethoxy)phenyl)-2-hydroxy-2-methylpropyl)-5,6-dimethylpyrazolo[1,5-a]pyrimidine-3-carboxamide

By a method similar to that in Example 60, the title compound was obtained.
MS (API+) : [M+H]+441.1.

### Example 82

### 5-((2R,6S)-2,6-dimethylmorpholin-4-yl)-N-((1S)-1-(3-fluoro-4-(trifluoromethoxy)phenyl)-2-hydroxy-2-methylpropyl)pyrazolo[1,5-a]pyrimidine-3-carboxamide

By a method similar to that in Example 59, the title compound was obtained.
MS (API+) : [M+H]+526.1.

### Example 83

### 5-(1-(difluoromethyl)-1H-pyrazol-4-yl)-N-((1S)-2-hydroxy-2-methyl-1-(4-(trifluoromethoxy)phenyl)propyl)-6-methylpyrazolo[1,5-a]pyrimidine-3-carboxamide

### A) tert-butyl 5-chloro-6-methylpyrazolo[1,5-a]pyrimidine-3-carboxylate

By a method similar to that in Example 80-II, step A to step D, the title compound was obtained.
MS (API+): found: 212.1.

### B) tert-butyl 5-(1-(difluoromethyl)-1H-pyrazol-4-yl)-6-methylpyrazolo[1,5-a]pyrimidine-3-carboxylate

By a method similar to that in Example 70-II, step C, the title compound was obtained.
MS (API+): found: 284.1.

### C) 5-(1-(difluoromethyl)-1H-pyrazol-4-yl)-6-methylpyrazolo[1,5-a]pyrimidine-3-carboxylic acid

To a suspension of tert-butyl 5-(1-(difluoromethyl)-1H-pyrazol-4-yl)-6-methylpyrazolo[1,5-a]pyrimidine-3-carboxylate (367 mg) in acetonitrile (5.25 mL) was added methanesulfonic acid (682 µL) at room temperature. The reaction mixture was stirred at room temperature for 1 hr, and 1M aqueous sodium hydroxide solution (10.5 mL) was added at 0°C. Acetonitrile was evaporated under reduced pressure. The resulting solid was filtered off, washed with water, and dried under reduced pressure to give the title compound (103 mg).
MS (API+): [M+H]+294.1.

### D) 5-(1-(difluoromethyl)-1H-pyrazol-4-yl)-N-((1S)-2-hydroxy-2-methyl-1-(4-(trifluoromethoxy)phenyl)propyl)-6-methylpyrazolo[1,5-a]pyrimidine-3-carboxamide

By a method similar to that in Example 70, step E, the title compound was obtained.
MS (API+) : [M+H]+525.2.

### Example 84

### N-((1S)-2-hydroxy-2-methyl-1-(4-(trifluoromethoxy)phenyl)propyl)-6-methyl-5-(5-methyl-1H-1,2,3-triazol-1-yl)pyrazolo[1,5-a]pyrimidine-3-carboxamide

By a method similar to that in Example 80-II, step A to step D, step F and step G, Example 84, step C, and Example 70, step E, the title compound was obtained.
MS (API+) : [M+H]+490.3.

### Example 85

### 5-(4-fluoro-1H-pyrazol-1-yl)-N-((1S)-2-hydroxy-2-methyl-1-(4-(trifluoromethoxy)phenyl)propyl)-6-methylpyrazolo[1,5-a]pyrimidine-3-carboxamide

By a method similar to that in Example 80-II, step A to step D, Example 80-I, step D, Example 84, step C, and Example 70, step E, the title compound was obtained.
MS (API+) : [M+H]+ 493.1.

### Example 86

### 5-(3-methoxypyridin-4-yl)-N-((1S)-2-methoxy-1-(4-(trifluoromethoxy)phenyl)ethyl)pyrazolo[1,5-a]pyrimidine-3-carboxamide

By a method similar to that in Example 73, the title compound was obtained.
MS (API+) : [M+H]+488.1.

### Example 87

### N-(1-(4-(difluoromethoxy)-3-fluorophenyl)-2-hydroxy-2-methylpropyl)-5-((2R,6S)-2,6-dimethylmorpholin-4-yl)pyrazolo[1,5-a]pyrimidine-3-carboxamide

### A) 1-amino-1-(4-(difluoromethoxy)-3-fluorophenyl)-2-methylpropan-2-ol hydrochloride

By a method similar to that in Example 25, step A and step B, the title compound was obtained.
MS (API+) : found: 250.1.

### B) N-(1-(4-(difluoromethoxy)-3-fluorophenyl)-2-hydroxy-2-methylpropyl)-5-((2R,6S)-2,6-dimethylmorpholin-4-yl)pyrazolo[1,5-a]pyrimidine-3-carboxamide

A mixture of 5-((2S,6R)-2,6-dimethylmorpholino)pyrazolo[1,5-a]pyrimidine-3-carboxylic acid (138 mg), 1-amino-1-(4-(difluoromethoxy)-3-fluorophenyl)-2-methylpropan-2-ol hydrochloride (143 mg), (1-cyano-2-ethoxy-2-oxoethylideneaminocy)dimethylamino-morpholinocarbenium hexafluorophosphate (257 mg), and triethylamine (0.167 mL) in acetonitrile (4 mL) was stirred at room temperature for 19 hr. Water was added to the reaction mixture, and the mixture was extracted with ethyl acetate. The extract was washed with saturated brine, dried over anhydrous sodium sulfate, and the solvent was evaporated under reduced pressure. The residue was purified by silica gel column chromatography (hexane/ethyl acetate), and solidified with diisopropyl ether/ethyl acetate to give the title compound (223 mg).
MS (API+) : [M+H]+508.2.

### Example 88

### 5-(1-(difluoromethyl)-1H-pyrazol-4-yl)-N-(1-(3-fluoro-4-(trifluoromethyl)phenyl)-2-hydroxy-2-methylpropyl)pyrazolo[1,5-a]pyrimidine-3-carboxamide

By a method similar to that in Example 70-II, step E, the title compound was obtained.
MS (API+) : [M+H]+513.1.

### Example 89

### N-((1S)-1-(3-fluoro-4-(trifluoromethoxy)phenyl)-2-hydroxy-2-methylpropyl)-5-(6-methylpyridazin-4-yl)pyrazolo[1,5-a]pyrimidine-3-carboxamide

By a method similar to that in Example 73, the title compound was obtained.
MS (API+) : [M+H]+505.1.

### Example 90

### N-(1-(4-(difluoromethoxy)-3-fluorophenyl)-2-hydroxy-2-methylpropyl)-6-methylpyrazolo[1,5-a]pyrimidine-3-carboxamide

By a method similar to that in Example 87, step B, the title compound was obtained.
MS (API+): [M+H]+409.1.

### Example 91

### N-((1S)-2-hydroxy-2-methyl-1-(4-(trifluoromethoxy)phenyl)propyl)-5-(2-methoxypyridin-3-yl)pyrazolo[1,5-a]pyrimidine-3-carboxamide

By a method similar to that in Example 73, the title compound was obtained.
MS (API+) : [M+H]+502.1.

### Example 92

### N-((1S)-2-methoxy-1-(4-(trifluoromethoxy)phenyl)ethyl)-7-methyl-2-oxo-1,2-dihydro-1,5-naphthyridine-4-carboxamide

By a method similar to that in Example 1, steps F to M, the title compound was obtained.
MS (API+) : [M+H]+422.1.

### Example 93

### 6-chloro-N-((1S)-1-(3-fluoro-4-(trifluoromethoxy)phenyl)-2-hydroxy-2-methylpropyl)pyrazolo[1,5-a]pyrimidine-3-carboxamide

By a method similar to that in Example 8, the title compound was obtained.
MS (API+) : [M+H]+447.1.

### Example 94

### N-((1S)-1-(3-fluoro-4-(trifluoromethoxy)phenyl)-2-hydroxy-2-methylpropyl)pyrazolo[1,5-a]pyrimidine-3-carboxamide

By a method similar to that in Example 8, the title compound was obtained.
MS (API+) : [M+H]+413.1.

### Example 95

### N-(1-(3-fluoro-4-(trifluoromethoxy)phenyl)-2-hydroxy-2-methylpropyl)-5-(3-methyl-1H-pyrazol-1-yl)pyrazolo[1,5-a]pyrimidine-3-carboxamide

By a method similar to that in Example 63, the title compound was obtained.
MS (API+) : [M+H]+493.1.

### Example 96

### N-(2-hydroxy-2-methyl-1-(3-methyl-4-(trifluoromethoxy)phenyl)propyl)-6-methylpyrazolo[1,5-a]pyrimidine-3-carboxamide

By a method similar to that in Example 25, the title compound was obtained.
MS (API+) : [M+H]+423.1.

### Example 97

### N-((1S)-1-(3-fluoro-4-(trifluoromethoxy)phenyl)-2-hydroxy-2-methylpropyl)-5-(3-methoxypyridin-4-yl)pyrazolo[1,5-a]pyrimidine-3-carboxamide

By a method similar to that in Example 73, the title compound was obtained.
MS (API+) : [M+H]+520.2.

### Example 98

### 5-(3-chloro-2-oxopyridine-1(2H)-yl)-N-((1S)-2-hydroxy-2-methyl-1-(4-(trifluoromethoxy)phenyl)propyl)pyrazolo[1,5-a]pyrimidine-3-carboxamide

By a method similar to that in Example 76, the title compound was obtained.
MS (API+) : [M+H]+522.1.

### Example 99

### 5-(azetidin-1-yl)-N-((1S)-1-(3-fluoro-4-(trifluoromethoxy)phenyl)-2-hydroxy-2-methylpropyl)-6-methylpyrazolo[1,5-a]pyrimidine-3-carboxamide

By a method similar to that in Example 59, the title compound was obtained.
MS (API+): [M+H]+482.2.

### Example 100

### 5-(dimethylamino)-N-((1S)-1-(3-fluoro-4-(trifluoromethoxy)phenyl)-2-hydroxy-2-methylpropyl)-6-methylpyrazolo[1,5-a]pyrimidine-3-carboxamide

By a method similar to that in Example 59, the title compound was obtained.
MS (API+) : [M+H]+470.2.

### Example 101

### 5-(2-cyanophenyl)-N-((1S)-1-(3-fluoro-4-(trifluoromethoxy)phenyl)-2-hydroxy-2-methylpropyl)pyrazolo[1,5-a]pyrimidine-3-carboxamide

By a method similar to that in Example 73, the title compound was obtained.
MS (API+) : [M+H]+514.1.

### Example 102

### N-(1-(4-(difluoromethoxy)phenyl)-2-hydroxy-2-methylpropyl)-6-methylpyrazolo[1,5-a]pyrimidine-3-carboxamide

By a method similar to that in Example 25, the title compound was obtained.
MS (API+) : [M+H]+391.1.

### Example 103

### 5-cyclopropyl-N-((1S)-1-(3-fluoro-4-(trifluoromethoxy)phenyl)-2-hydroxy-2-methylpropyl)-7-oxo-1,7-dihydropyrazolo[1,5-a]pyrimidine-3-carboxamide

By a method similar to that in Example 75, steps B to D, the title compound was obtained.
MS (API+) : [M+H]+469.2.

### Example 104

### N-((1S)-1-(3-fluoro-4-(trifluoromethoxy)phenyl)-2-hydroxy-2-methylpropyl)-5-(1H-pyrazol-1-yl)pyrazolo[1,5-a]pyrimidine-3-carboxamide

By a method similar to that in Example 63, the title compound was obtained.
MS (API+) : [M+H]+479.1.

### Example 105

### 5-ethyl-N-((1S)-1-(3-fluoro-4-(trifluoromethoxy)phenyl)-2-hydroxy-2-methylpropyl)-1-methyl-7-oxo-1,7-dihydropyrazolo[1,5-a]pyrimidine-3-carboxamide

By a method similar to that in Example 75, step B to step D, the title compound was obtained.
MS (API+): [M+H]+ 471.2.

### Example 106

### N-((1S)-1-(3-fluoro-4-(trifluoromethoxy)phenyl)-2-hydroxy-2-methylpropyl)-5-isopropyl-1-methyl-7-oxo-1,7-dihydropyrazolo[1,5-a]pyrimidine-3-carboxamide

By a method similar to that in Example 75, step B to step D, the title compound was obtained.
MS (API+) : [M+H]+485.2.

### Example 107

### N-((1S)-2-hydroxy-2-methyl-1-(4-(trifluoromethoxy)phenyl)propyl)-5-(pyrimidin-5-yl)pyrazolo[1,5-a]pyrimidine-3-carboxamide

By a method similar to that in Example 73, the title compound was obtained.
MS (API+) : [M+H]+473.1.

### Example 108

### N-((1S)-2-hydroxy-2-methyl-1-(4-(trifluoromethoxy)phenyl)propyl)-5-(1-methyl-1H-pyrazol-3-yl)pyrazolo[1,5-a]pyrimidine-3-carboxamide

By a method similar to that in Example 73, the title compound was obtained.
MS (API+) : [M+H]+475.1.

### Example 109

### N-((1S)-2-hydroxy-2-methyl-1-(4-(trifluoromethoxy)phenyl)propyl)-5-(4-methyl-2H-1,2,3-triazol-2-yl)pyrazolo[1,5-a]pyrimidine-3-carboxamide

By a method similar to that in Example 74, the title compound was obtained.
MS (API+) : [M+H]+ 476.2.

### Example 110

### N-((1S)-2-hydroxy-2-methyl-1-(4-(trifluoromethoxy)phenyl)propyl)-6-methylpyrazolo[1,5-a]pyrimidine-3-carboxamide

By a method similar to that in Example 2, step A to step C, the title compound was obtained.
MS (API+): [M+H]+ 409.2.

### Example 111

### 2-amino-N-((1S)-2-hydroxy-2-methyl-1-(4-(trifluoromethoxy)phenyl)propyl)-6-methylpyrazolo[1,5-a]pyrimidine-3-carboxamide

By a method similar to that in Example 14, step A, step B and step I, the title compound was obtained.
MS (API+) : [M+H]+424.1.

### Example 112

### 2-amino-N-((1S)-1-(3-fluoro-4-(trifluoromethoxy)phenyl)-2-hydroxy-2-methylpropyl)-6-methylpyrazolo[1,5-a]pyrimidine-3-carboxamide

By a method similar to that in Example 111, the title compound was obtained.
MS (API+) : [M+H]+442.1.

### Example 113

### N-((1S)-2-hydroxy-2-methyl-1-(4-(trifluoromethoxy)phenyl)propyl)-5-((2R)-2-methylmorpholin-4-yl)pyrazolo[1,5-a]pyrimidine-3-carboxamide

By a method similar to that in Example 37, the title compound was obtained.
MS (API+): [M+H]+494.2.

### Example 114

### N-((1S)-2-hydroxy-2-methyl-1-(4-(trifluoromethoxy)phenyl)propyl)-5-(1,3-thiazol-2-yl)pyrazolo[1,5-a]pyrimidine-3-carboxamide

A mixture of (S)-5-chloro-N-(2-hydroxy-2-methyl-1-(4-(trifluoromethoxy)phenyl)propyl)pyrazolo[1,5-a]pyrimidine-3-carboxamide (57.6 mg), 2-tributylstannylthiazole (60.3 mg), tetrakistriphenylphosphinepalladium (0) (7.8 mg) and toluene (2 mL) was stirred under a nitrogen atmosphere by using microwave at 150°C for 30 min. The reaction mixture was purified by silica gel column chromatography (NH, hexane/ethyl acetate), and the obtained solid was washed with hexane/ethyl acetate to give the title compound (56.9 mg).
MS (API+) : [M+H]+478.1.

### Example 115

### N-((1S)-2-hydroxy-2-methyl-1-(4-(trifluoromethoxy)phenyl)propyl)-5-(4-methyl-1H-imidazol-1-yl)pyrazolo[1,5-a]pyrimidine-3-carboxamide

By a method similar to that in Example 74, the title compound was obtained.
MS (API+) : [M+H]+475.2.

### Example 116

### N-(2-methoxy-1-(4-(trifluoromethoxy)phenyl)ethyl)-6-methylpyrazolo[1,5-a]pyrimidine-3-carboxamide

By a method similar to that in Example 2, step C, the title compound was obtained.
MS (API+) : [M+H]+395.1.

### Example 117

### 5-((1S or 1R)-1-(difluoromethyl)-1H-pyrazol-4-yl)-N-(1-(3-fluoro-4-(trifluoromethyl)phenyl)-2-hydroxy-2-methylpropyl)pyrazolo[1,5-a]pyrimidine-3-carboxamide

By a method similar to that in Example 15, the title compound was obtained.
MS (API+) : [M+H]+513.0.

### Example 118

### 5-((1R or 1S)-1-(difluoromethyl)-1H-pyrazol-4-yl)-N-(1-(3-fluoro-4-(trifluoromethyl)phenyl)-2-hydroxy-2-methylpropyl)pyrazolo[1,5-a]pyrimidine-3-carboxamide

By a method similar to that in Example 16, the title compound was obtained.
MS (API+) : [M+H]+513.0.

### Example 119

### N-((1S)-2-hydroxy-2-methyl-1-(4-(trifluoromethoxy)phenyl)propyl)-5-(tetrahydro-2H-pyran-4-yl)pyrazolo[1,5-a]pyrimidine-3-carboxamide

### A) (S)-5-(3,6-dihydro-2H-pyran-4-yl)-N-(2-hydroxy-2-methyl-1-(4-(trifluoromethoxy)phenyl)propyl)pyrazolo[1,5-a]pyrimidine-3-carboxamide

To a mixture of (S)-5-chloro-N-(2-hydroxy-2-methyl-1-(4-(trifluoromethoxy)phenyl)propyl)pyrazolo[1,5-a]pyrimidine-3-carboxamide (142 mg), 2-(3,6-dihydro-2H-pyran-4-yl)-4,4,5,5-tetramethyl-1,3,2-dioxaborolane (104 mg), potassium carbonate (68.5 mg), toluene (4 mL) and water (0.4 mL) was added bis(di-tert-butyl(4-dimethylaminophenyl)phosphine)dichloropalladium(II) (23.4 mg) at room temperature. The reaction mixture was stirred under a nitrogen atmosphere by using microwave at 150°C for 25 min, and purified by silica gel column chromatography (hexane/ethyl acetate) to give the title compound (110 mg).
MS (API+) : [M+H]+477.2.

### B) N-((1S)-2-hydroxy-2-methyl-1-(4-(trifluoromethoxy)phenyl)propyl)-5-(tetrahydro-2H-pyran-4-yl)pyrazolo[1,5-a]pyrimidine-3-carboxamide

A mixture of (S)-5-(3,6-dihydro-2H-pyran-4-yl)-N-(2-hydroxy-2-methyl-1-(4-(trifluoromethoxy)phenyl)propyl)pyrazolo[1,5-a]pyrimidine-3-carboxamide, 5% palladium-carbon-ethylenediamine complex (15 mg), methanol (10 mL) and tetrahydrofuran (2 mL) was stirred under a hydrogen atmosphere at room temperature for 16 hr. The insoluble material was filtered off, and the filtrate was concentrated under reduced pressure. The residue was purified by silica gel column chromatography (hexane/ethyl acetate) to give the title compound (83.0 mg).
MS (API+): [M+H]+479.2.

### Example 120

### N-((1S)-2-hydroxy-2-methyl-1-(4-(trifluoromethoxy)phenyl)propyl)-5-(prop-1-en-2-yl)pyrazolo[1,5-a]pyrimidine-3-carboxamide

By a method similar to that in Example 70-I, step F, the title compound was obtained.
MS (API+) : [M+H]+435.2.

### Example 121

### 5-(4,5-dimethyl-1H-1,2,3-triazol-1-yl)-N-((1S)-2-hydroxy-2-methyl-1-(4-(trifluoromethoxy)phenyl)propyl)pyrazolo[1,5-a]pyrimidine-3-carboxamide

### A) (S)-5-chloro-N-(2-hydroxy-2-methyl-1-(4-(trifluoromethoxy)phenyl)propyl)pyrazolo[1,5-a]pyrimidine-3-carboxamide

By a method similar to that in Example 69-I, step A to step D, the title compound was obtained.
MS (API+): [M+H]+429.1.

### B) 5-(4,5-dimethyl-1H-1,2,3-triazol-1-yl)-N-((1S)-2-hydroxy-2-methyl-1-(4-(trifluoromethoxy)phenyl)propyl)pyrazolo[1, 5-a]pyrimidine-3-carboxamide

A mixture of (S)-5-chloro-N-(2-hydroxy-2-methyl-1-(4-(trifluoromethoxy)phenyl)propyl)pyrazolo[1,5-a]pyrimidine-3-carboxamide (500 mg), sodium azide (114 mg), and ethanol (10 mL) was stirred at 80°C for 2 hr. The reaction mixture was stirred at room temperature, poured into saturated brine, and the mixture was extracted with ethyl acetate. The organic layer was separated, washed with saturated brine, dried over anhydrous magnesium sulfate, and the solvent was evaporated under reduced pressure. A mixture of the obtained crude product (104 mg), 2-butyne (450 mg) and toluene (2 mL) was stirred under microwave irradiation at 150°C for 1 hr. The residue was purified by silica gel column chromatography (ethyl acetate/hexane) to give a mixture of a starting material and a resultant product. To the obtained mixture were added copper(I) iodide (3 mg) and N,N-dimethylformamide (1 mL), and the mixture was stirred at 100°C for 1 hr. The residue was purified by silica gel column chromatography (ethyl acetate/hexane) to give a mixture of the starting material and the resultant product. Furthermore, to the obtained mixture were added copper(I) iodide (3 mg) and N,N-dimethylformamide (1 mL), and the mixture was stirred at 100°C for 1 hr. The residue was purified by silica gel column chromatography (ethyl acetate/hexane) to give the title compound (46 mg).
MS (API+) : [M+H]+490.2.

### Example 122

### N-((1S)-2-hydroxy-2-methyl-1-(4-(trifluoromethoxy)phenyl)propyl)-5-(1H-1,2,4-triazol-1-yl)pyrazolo[1,5-a]pyrimidine-3-carboxamide

By a method similar to that in Example 63, the title compound was obtained.
MS (API+): [M+H]+462.1.

### Example 123

### 5-(2,4-dimethyl-1H-imidazol-1-yl)-N-((1S)-2-hydroxy-2-methyl-1-(4-(trifluoromethoxy)phenyl)propyl)pyrazolo[1,5-a]pyrimidine-3-carboxamide

By a method similar to that in Example 71, the title compound was obtained.
MS (API+): [M+H]+489.2.

### Example 124

### 5-(2,2-dimethylmorpholin-4-yl)-N-((1S)-2-hydroxy-2-methyl-1-(4-(trifluoromethoxy)phenyl)propyl)pyrazolo[1,5-a]pyrimidine-3-carboxamide

By a method similar to that in Example 37, the title compound was obtained.
MS (API+) : [M+H]+508.2.

### Example 125

### N-(1-(4-(difluoromethoxy)-3-fluorophenyl)-2-hydroxy-2-methylpropyl)-5-(6-methylpyridin-3-yl)pyrazolo[1,5-a]pyrimidine-3-carboxamide

By a method similar to that in Example 69-I, step D and step E, the title compound was obtained.
MS (API+): [M+H]+486.1.

### Example 126

### 2-chloro-N-((1S)-2-hydroxy-2-methyl-1-(4-(trifluoromethoxy)phenyl)propyl)-6-methylpyrazolo[1,5-a]pyrimidine-3-carboxamide

### A) ethyl 3-amino-5-bromo-1H-pyrazole-4-carboxylate

To a solution of ethyl 3-amino-1H-pyrazole-4-carboxylate (4.65 g) in acetonitrile (100 mL) was added N-bromosuccinimide (5.87 g) at room temperature by small portions, and the reaction mixture was stirred for 2 hr. The reaction mixture was concentrated under reduced pressure, and the residue was diluted with ethyl acetate, washed with aqueous sodium thiosulfate solution, and then saturated brine, dried over anhydrous sodium sulfate, and the solvent was evaporated under reduced pressure. The residue was purified by silica gel column chromatography (hexane/ethyl acetate), and the resulting solid was washed with diisopropyl ether/ethyl acetate to give the title compound (3.65 g).
MS (API+): [M+H]+234.0, 236.0.

### B) ethyl 2-bromo-6-methylpyrazolo[1,5-a]pyrimidine-3-carboxylate

By a method similar to that in Example 14, step A, the title compound was obtained.
MS (API+): [M+H]+284.0, 286.0.

### C) ethyl 2-chloro-6-methylpyrazolo[1,5-a]pyrimidine-3-carboxylate

A mixture of ethyl 2-bromo-6-methylpyrazolo[1,5-a]pyrimidine-3-carboxylate (1.96 g), and copper(I) chloride in 1-methyl-2-pyrrolidinone (20 mL) was stirred under an argon atmosphere at 150°C for 5 hr. To the reaction mixture were added water and ethyl acetate, and the insoluble material was filtered off. The organic layer of the filtrate was separated, washed with saturated brine, and dried over anhydrous sodium sulfate. The residue was purified by silica gel column chromatography (hexane/ethyl acetate), and the resulting solid was washed with diisopropyl ether to give the title compound (0.375 g).
1H NMR (300 MHz, CDCl3) δ 1.44 (3H, t, J = 7.2 Hz), 2.44 (3H, d, J = 0.9 Hz), 4.47 (2H, q, J = 7.0 Hz), 8.43 (1H, dd, J = 2.1, 0.9 Hz), 8.66 (1H, d, J = 2.1 Hz).

### D) 2-chloro-N-((1S)-2-hydroxy-2-methyl-1-(4-(trifluoromethoxy)phenyl)propyl)-6-methylpyrazolo[1,5-a]pyrimidine-3-carboxamide

By a method similar to that in Example 14, step B and step I, the title compound was obtained.
MS (API+) : [M+H]+443.1.

### Example 127

### N-((1S)-1-(3-fluoro-4-(trifluoromethoxy)phenyl)-2-hydroxy-2-methylpropyl)-5-(pyridin-2-yl)pyrazolo[1,5-a]pyrimidine-3-carboxamide

By a method similar to that in Example 72, the title compound was obtained.
MS (API+) : [M+H]+490.1.

### Example 128

### N-(1-(4-(difluoromethoxy)-3-fluorophenyl)-2-hydroxy-2-methylpropyl)-5-(2-methoxypyridin-3-yl)pyrazolo[1,5-a]pyrimidine-3-carboxamide

By a method similar to that in Example 73, the title compound was obtained.
MS (API+) : [M+H]+502.1.

### Example 129

### N-((1S)-2-hydroxy-2-methyl-1-(4-(trifluoromethoxy)phenyl)propyl)-5-(3-methoxypyridin-4-yl)pyrazolo[1,5-a]pyrimidine-3-carboxamide

By a method similar to that in Example 73, the title compound was obtained.
MS (API+) : [M+H]+502.1.

### Example 130

### N-((1S)-1-(3-fluoro-4-(trifluoromethoxy)phenyl)-2-hydroxy-2-methylpropyl)-1,5,6-trimethyl-7-oxo-1,7-dihydropyrazolo[1,5-a]pyrimidine-3-carboxamide

### A) ethyl 1,5,6-trimethyl-7-oxo-1,7-dihydropyrazolo[1,5-a]pyrimidine-3-carboxylate

To a mixture of ethyl 3-amino-1H-pyrazole-4-carboxylate (1.0 g) in acetonitrile (30 mL) was added sodium hydride (335 mg) at 0°C. After stirring at 0°C for 30 min, methyl iodide (443 µL) was added to the mixture. The reaction mixture was stirred at room temperature overnight, poured into saturated aqueous ammonium chloride solution at room temperature, and the mixture was extracted with ethyl acetate. The organic layer was separated, washed with saturated brine, and dried over anhydrous magnesium sulfate. The solvent was evaporated under reduced pressure. A mixture of the obtained crude product (1.08 g), ethyl 2-methyl-3-oxobutanoate (923 mg), and polyphosphoric acid (3.0 g) was stirred at 120°C for 4 hr. The reaction mixture was poured into saturated aqueous sodium hydrogen carbonate solution at room temperature, and the mixture was extracted with ethyl acetate. The organic layer was separated, washed with saturated brine, and dried over anhydrous magnesium sulfate. The solvent was evaporated under reduced pressure. The residue was pulverized and washed with diisopropyl ether to give the title compound (557 mg).
MS (API+) : [M+H]+250.1.

### B) 1,5,6-trimethyl-7-oxo-1,7-dihydropyrazolo[1,5-a]pyrimidine-3-carboxylic acid

A mixture of ethyl 1,5,6-trimethyl-7-oxo-1,7-dihydropyrazolo[1,5-a]pyrimidine-3-carboxylate (540 mg) and 2M aqueous sodium hydroxide solution (5 mL) in methanol (15 mL) was stirred at room temperature for 2 hr. Methanol was evaporated under reduced pressure, 1M hydrochloric acid was added to the residue, and the resulting solid was collected by filtration to give the title compound (548 mg).
MS (API+): [M+H]+222.1.

### C) N-((1S)-1-(3-fluoro-4-(trifluoromethoxy)phenyl)-2-hydroxy-2-methylpropyl)-1,5,6-trimethyl-7-oxo-1,7-dihydropyrazolo[1,5-a]pyrimidine-3-carboxamide

A mixture of 1,5,6-trimethyl-7-oxo-1,7-dihydropyrazolo[1,5-a]pyrimidine-3-carboxylic acid (70 mg) and benzotriazol-1-yloxy-trisdimethylaminophosphonium hexafluorophosphate (280 mg), (S)-1-amino-1-(3-fluoro-4-(trifluoromethoxy)phenyl)-2-methylpropan-2-ol hydrochloride (96 mg), and triethylamine (64 mg) in N,N-dimethylaminoacetamide (2 mL) was stirred at room temperature for 2 days. The reaction mixture was poured into saturated aqueous sodium hydrogen carbonate solution at room temperature, and the mixture was extracted with ethyl acetate and tetrahydrofuran. The organic layer was separated, dried over anhydrous magnesium sulfate, and the solvent was evaporated under reduced pressure. The residue was pulverized and washed with water and diisopropyl ether to give the title compound (112 mg).
MS (API+) : [M+H]+471.2.

### Example 131

### N-((1S)-1-(3-fluoro-4-(trifluoromethoxy)phenyl)-2-hydroxy-2-methylpropyl)-1,5-dimethyl-7-oxo-1,7-dihydropyrazolo[1,5-a]pyrimidine-3-carboxamide

By a method similar to that in Example 130, the title compound was obtained.
MS (API+): [M+H]+457.2.

### Example 132

### 5-(2-chloro-1H-imidazol-1-yl)-N-((1S)-2-hydroxy-2-methyl-1-(4-(trifluoromethoxy)phenyl)propyl)-6-methylpyrazolo[1,5-a]pyrimidine-3-carboxamide

By a method similar to that in Example 80-I, the title compound was obtained.
MS (API+) : [M+H]+509.2.

### Example 133

### N-((1S)-1-(3-fluoro-4-(trifluoromethoxy)phenyl)-2-hydroxy-2-methylpropyl)-5-(3-methyl-1H-1,2,4-triazol-1-yl)pyrazolo[1,5-a]pyrimidine-3-carboxamide

By a method similar to that in Example 63, the title compound was obtained.
MS (API+): [M+H]+494.1.

### Example 134

### N-((1S)-1-(3-fluoro-4-(trifluoromethoxy)phenyl)-2-hydroxy-2-methylpropyl)-5-(1H-1,2,4-triazol-1-yl)pyrazolo[1,5-a]pyrimidine-3-carboxamide

By a method similar to that in Example 63, the title compound was obtained.
MS (API+) : [M+H]+480.1.

### Example 135

### 5-(3,3-dimethyl-2-oxopyrrolidin-1-yl)-N-((1S)-2-hydroxy-2-methyl-1-(4-(trifluoromethoxy)phenyl)propyl)pyrazolo[1,5-a]pyrimidine-3-carboxamide

By a method similar to that in Example 76, the title compound was obtained.
MS (API+) : [M+H]+506.1.

### Example 136

### N-(1-(2-fluoro-4-(trifluoromethoxy)phenyl)-2-hydroxy-2-methylpropyl)-5-(4-methyl-1H-imidazol-1-yl)pyrazolo[1,5-a]pyrimidine-3-carboxamide

By a method similar to that in Example 25, step A and step B, Example 69-I, step D and Example 63, the title compound was obtained.
MS (API+) : [M+H]+493.1.

### Example 137

### N-(2-hydroxy-2-methyl-1-(3-methyl-4-(trifluoromethoxy)phenyl)propyl)-5-(6-methylpyridin-3-yl)pyrazolo[1,5-a]pyrimidine-3-carboxamide

By a method similar to that in Example 69-I, step D and step E, the title compound was obtained.
MS (API+) : [M+H]+500.2.

### Example 138

### N-((1S)-2-hydroxy-2-methyl-1-(4-(trifluoromethoxy)phenyl)propyl)-6-methyl-5-(2H-1,2,3-triazol-2-yl)pyrazolo[1,5-a]pyrimidine-3-carboxamide

By a method similar to that in Example 78, the title compound was obtained.
MS (API+): [M+H]+476.2.

### Example 139

### N-((1S)-2-hydroxy-2-methyl-1-(4-(trifluoromethoxy)phenyl)propyl)-5-(1H-tetrazol-1-yl)pyrazolo[1,5-a]pyrimidine-3-carboxamide

By a method similar to that in Example 63, the title compound was obtained.
MS (API+) : [M+H]+463.0.

### Example 140

### 6-fluoro-N-((1S)-1-(3-fluoro-4-(trifluoromethoxy)phenyl)-2-hydroxy-2-methylpropyl)-5-methylpyrazolo[1,5-a]pyrimidine-3-carboxamide

### A) ethyl 6-fluoro-7-hydroxy-5-methylpyrazolo[1,5-a]pyrimidine-3-carboxylate

A mixture of ethyl 5-amino-1H-pyrazole-4-carboxylate (23.6 g) and ethyl 2-fluoro-3-oxobutanoate (22.9 mL) in acetic acid (304 mL) was stirred at 100°C overnight. After cooling to room temperature, the reaction mixture was evaporated under reduced pressure. The solid was pulverized and washed with water and diisopropyl ether to give the title compound (33.1 g). MS (API+) : [M+H]+240.1.

### B) 6-fluoro-7-hydroxy-5-methylpyrazolo[1,5-a]pyrimidine-3-carboxylic acid

By a method similar to that in Example 69-I, step B, the title compound was obtained.
MS (API-): [M-H]-209.9.

### C) 6-fluoro-N-((1S)-1-(3-fluoro-4-(trifluoromethoxy)phenyl)-2-hydroxy-2-methylpropyl)-5-methylpyrazolo[1,5-a]pyrimidine-3-carboxamide

By a method similar to that in Example 59, step B, Example 18, step B, and Example 59, step C, the title compound was obtained.
MS (API+) : [M+H]+445.1.

### Example 141

### N-((1S)-2-hydroxy-2-methyl-1-(4-(trifluoromethoxy)phenyl)propyl)-5-(2-methyl-1H-imidazol-1-yl)pyrazolo[1,5-a]pyrimidine-3-carboxamide

By a method similar to that in Example 71, the title compound was obtained.
MS (API+) : [M+H]+475.2.

### Example 142

### N-((1S or 1R)-1-(4-(difluoromethoxy)-3-fluorophenyl)-2-hydroxy-2-methylpropyl)-6-methylpyrazolo[1,5-a]pyrimidine-3-carboxamide

By a method similar to that in Example 66, step B to step D and Example 14, step I, the title compound was obtained.
MS (API+): [M+H]+409.1.

### Example 143

### N-((1R or 1S)-1-(3-fluoro-4-(trifluoromethyl)phenyl)-2-hydroxy-2-methylpropyl)-6-methylpyrazolo[1,5-a]pyrimidine-3-carboxamide

By a method similar to that in Example 15, the title compound was obtained.
MS (API+) : [M+H]+411.1.

### Example 144

### 6-chloro-N-((1S)-1-(3-fluoro-4-(trifluoromethoxy)phenyl)-2-hydroxy-2-methylpropyl)-1,5-dimethyl-7-oxo-1,7-dihydropyrazolo[1,5-a]pyrimidine-3-carboxamide

A mixture of N-((1S)-1-(3-fluoro-4-(trifluoromethoxy)phenyl)-2-hydroxy-2-methylpropyl)-1,5-dimethyl-7-oxo-1,7-dihydropyrazolo[1,5-a]pyrimidine-3-carboxamide (45 mg) and 1-chloropyrrolidine-2,5-dione (16 mg) in tetrahydrofuran (3 mL) was stirred at 80°C overnight. The reaction mixture was evaporated under reduced pressure. The residue was purified by silica gel column chromatography (hexane/ethyl acetate), and the obtained residue was pulverized and washed with diisopropyl ether to give the title compound (22 mg).
MS (API+) : [M+H]+491.1.

### Example 145

### N-((1S)-1-(3-fluoro-4-(trifluoromethoxy)phenyl)-2-hydroxy-2-methylpropyl)-1,6-dimethyl-7-oxo-1,7-dihydropyrazolo[1,5-a]pyrimidine-3-carboxamide

By a method similar to that in Example 129, the title compound was obtained.
MS (API+): [M+H]+457.1.

### Example 146

### N-(1-(4-(difluoromethoxy)-3-fluorophenyl)-2-hydroxy-2-methylpropyl)-1,5,6-trimethyl-7-oxo-1,7-dihydropyrazolo[1,5-a]pyrimidine-3-carboxamide

By a method similar to that in Example 129, the title compound was obtained.
MS (API+) : [M+H]+453.2.

### Example 147

### 5-(2-chloro-1H-imidazol-1-yl)-N-((1S)-2-hydroxy-2-methyl-1-(4-(trifluoromethoxy)phenyl)propyl)pyrazolo[1,5-a]pyrimidine-3-carboxamide

By a method similar to that in Example 71, the title compound was obtained.
MS (API+) : [M+H]+495.2.

### Example 148

### N-(1-(4-(difluoromethoxy)-3-fluorophenyl)-2-hydroxy-2-methylpropyl)-5-(1-(difluoromethyl)-1H-pyrazol-4-yl)pyrazolo[1,5-a]pyrimidine-3-carboxamide

By a method similar to that in Example 87, step B, the title compound was obtained.
MS (API+) : [M+H]+511.1.

### Example 149

### N-((1S)-2-hydroxy-2-methyl-1-(4-(trifluoromethoxy)phenyl)propyl)-5-(2-methylpyridin-4-yl)pyrazolo[1,5-a]pyrimidine-3-carboxamide

By a method similar to that in Example 73, the title compound was obtained.
MS (API+): [M+H]+486.1.

### Example 150

### N-((1S)-1-(3-fluoro-4-(trifluoromethoxy)phenyl)-2-hydroxy-2-methylpropyl)-5-(6-methylpyridin-3-yl)pyrazolo[1,5-a]pyrimidine-3-carboxamide

By a method similar to that in Example 73, the title compound was obtained.
MS (API+) : [M+H]+504.1.

### Example 151

### 2-chloro-N-((1S)-1-(3-fluoro-4-(trifluoromethoxy)phenyl)-2-hydroxy-2-methylpropyl)-6-methylpyrazolo[1,5-a]pyrimidine-3-carboxamide

By a method similar to that in Example 14, step I, the title compound was obtained.
MS (API+) : [M+H]+461.1.

### Example 152

### N-((1S)-1-(3-fluoro-4-(trifluoromethoxy)phenyl)-2-methoxyethyl)-7-methoxy-2-oxo-1,2-dihydro-1,5-naphthyridine-4-carboxamide

### A) 1-tert-butyl 3-ethyl 2-(5-bromo-3-nitropyridin-2-yl)malonate

To a suspension of tert-butyl ethyl malonate (1.19 g) and potassium t-butoxide (0.834 g) in tetrahydrofuran (12 mL) was added a solution of 5-bromo-2-chloro-3-nitropyridine (1.00 g) in tetrahydrofuran (3 mL) at 60°C. The reaction mixture was stirred at 60°C for 3 hr, and the solvent was evaporated under reduced pressure. To the residue was added water, and the mixture was extracted with ethyl acetate. The extract was dried over anhydrous sodium sulfate, and the solvent was evaporated under reduced pressure. The residue was purified by silica gel chromatography (hexane/ethyl acetate) to give the title compound (945 mg).
1H NMR (300 MHz, DMSO-d6) δ 1.19 (3H, t, J = 7.2 Hz), 1.41 (9H, s), 4.13-4.28 (2H, m), 5.42 (1H, s), 8.87 (1H, d, J = 1.9 Hz), 9.08 (1H, d, J = 2.3 Hz).

### B) ethyl 2-(5-bromo-3-nitropyridin-2-yl)acetate

To a solution of 1-tert-butyl 3-ethyl 2-(5-bromo-3-nitropyridin-2-yl)malonate (945 mg) in toluene (10 mL) was added trifluoroacetic acid (5 mL). The reaction mixture was stirred at room temperature for 4 hr, and the solvent was evaporated under reduced pressure. The residue was purified by silica gel chromatography (hexane/ethyl acetate) to give the title compound (648 mg).
1H NMR (300 MHz, DMSO-d6) δ 1.17 (3H, t, J = 7.2 Hz), 4.10 (2H, q, J = 7.2 Hz), 4.21 (2H, s), 8.82 (1H, d, J = 2.3 Hz), 9.04 (1H, d, J = 2.3 Hz).

### C) ethyl 2-(5-hydroxy-3-nitropyridin-2-yl)acetate

A mixture of ethyl 2-(5-bromo-3-nitropyridin-2-yl)acetate (9.26 g), 1,1'-bis(diphenylphosphino)ferrocene (2.34 g), 4,4,4',4',5,5,5',5'-octamethyl-2,2'-bi-1,3,2-dioxaborolane (16.3 g), potassium acetate (12.6 g) and N,N-dimethylformamide (160 mL) was stirred under an argon atmosphere at 80°C overnight. Water was added to the reaction mixture, the insoluble material was filtered off, and the filtrate was extracted with ethyl acetate. The extract was washed with water, and dried over anhydrous sodium sulfate. The solvent was evaporated under reduced pressure. The residue was dissolved in tetrahydrofuran (160 mL) and water (3 mL), and sodium perborate tetrahydrate (7.39 g) was added. The reaction mixture was stirred at room temperature for 2 hr, poured into water, and the mixture was extracted with ethyl acetate. The extract was dried over anhydrous sodium sulfate, and the solvent was evaporated under reduced pressure. The residue was purified by silica gel chromatography (hexane/ethyl acetate) to give the title compound (4.18 g).
1H NMR (300 MHz, DMSO-d6) δ 1.12-1.21 (3H, m), 3.96-4.16 (4H, m), 7.83 (1H, d, J = 2.6 Hz), 8.41 (1H, d, J = 2.6 Hz), 10.97 (1H, brs).

### D) ethyl 2-(5-methoxy-3-nitropyridin-2-yl)acetate

To a mixed solution of ethyl 2-(5-hydroxy-3-nitropyridin-2-yl)acetate (4.18 g), potassium carbonate (3.83 g) and N,N-dimethylformamide (90 mL) was added methyl iodide (1.38 mL) at 0°C. The reaction mixture was stirred at room temperature for 2 hr, poured into water, and the mixture was extracted with ethyl acetate. The extract was washed with water, and dried over anhydrous sodium sulfate. The solvent was evaporated under reduced pressure. The residue was purified by silica gel column chromatography (hexane/ethyl acetate) to give the title compound (2.17 g).
MS (API+) : [M+H]+241.1.

### E) N-((1S)-1-(3-fluoro-4-(trifluoromethoxy)phenyl)-2-methoxyethyl)-7-methoxy-2-oxo-1,2-dihydro-1,5-naphthyridine-4-carboxamide

By a method similar to that in Example 1, steps H to M, the title compound was obtained.
MS (API+): [M+H]+456.2.

### Example 153

### N-((1S)-1-(3-fluoro-4-(trifluoromethoxy)phenyl)-2-methoxyethyl)-5-(3-methoxypyridin-4-yl)pyrazolo[1,5-a]pyrimidine-3-carboxamide

By a method similar to that in Example 73, the title compound was obtained.
MS (API+) : [M+H]+506.1.

### Example 154

### 5-(3,5-dimethyl-1,2-oxazol-4-yl)-N-((1S)-2-hydroxy-2-methyl-1-(4-(trifluoromethoxy)phenyl)propyl)pyrazolo[1,5-a]pyrimidine-3-carboxamide

By a method similar to that in Example 69-I, the title compound was obtained.
MS (API+) : [M+H]+490.2.

### Example 155

### (1S)-2-methyl-1-(((6-methyl-5-(4-methyl-1H-1,2,3-triazol-1-yl)pyrazolo[1,5-a]pyrimidin-3-yl)carbonyl)amino)-1-(4-(trifluoromethoxy)phenyl)propan-2-yl acetate

To a solution of N-((1S)-2-hydroxy-2-methyl-1-(4-(trifluoromethoxy)phenyl)propyl)-6-methyl-5-(4-methyl-1H-1,2,3-triazol-1-yl)pyrazolo[1,5-a]pyrimidine-3-carboxamide (301 mg) in tetrahydrofuran (10 mL) were added acetic anhydride (0.464 mL) and N,N-dimethyl-4-aminopyridine (376 mg). The reaction mixture was stirred at 70°C for 16 hr, and the solvent was evaporated under reduced pressure. To the residue was added water, and the mixture was extracted with ethyl acetate. The extract was dried over anhydrous sodium sulfate, and the solvent was evaporated under reduced pressure. The residue was purified by silica gel column chromatography (hexane/ethyl acetate), and recrystallized from isopropyl alcohol/hexane to give the title compound (129 mg).
MS (API+) : [M+H]+532.2.

### Example 156

### N-((1S)-1-(3-fluoro-4-(trifluoromethoxy)phenyl)-2-hydroxy-2-methylpropyl)-5-(1H-1,2,3-triazol-1-yl)pyrazolo[1,5-a]pyrimidine-3-carboxamide

By a method similar to that in Example 63, the title compound was obtained.
MS (API+) : [M+H]+480.0.

### Example 157

### N-((1S)-2-hydroxy-2-methyl-1-(4-(trifluoromethoxy)phenyl)propyl)-5-(6-methoxypyridin-2-yl)pyrazolo[1,5-a]pyrimidine-3-carboxamide

By a method similar to that in Example 72, the title compound was obtained.
MS (API+) : [M+H]+502.1.

### Example 158

### N-((1S)-1-(3-fluoro-4-(trifluoromethoxy)phenyl)-2-hydroxy-2-methylpropyl)-5-(2-methoxypyridin-3-yl)pyrazolo[1,5-a]pyrimidine-3-carboxamide

By a method similar to that in Example 73, the title compound was obtained.
MS (API+) : [M+H]+520.1.

### Example 159

### 5-(3-fluoropyridin-4-yl)-N-((1S)-1-(3-fluoro-4-(trifluoromethoxy)phenyl)-2-methoxyethyl)pyrazolo[1,5-a]pyrimidine-3-carboxamide

By a method similar to that in Example 73, the title compound was obtained.
MS (API+): [M+H]+494.0.

### Example 160

### N-((1S)-2-hydroxy-2-methyl-1-(4-(trifluoromethoxy)phenyl)propyl)-5-(pyrazin-2-yl)pyrazolo[1,5-a]pyrimidine-3-carboxamide

A mixture of (S)-5-chloro-N-(2-hydroxy-2-methyl-1-(4-(trifluoromethoxy)phenyl)propyl)pyrazolo[1,5-a]pyrimidine-3-carboxamide (100 mg), tetrakis(triphenylphosphine)palladium(0) (26.9 mg), (tributylstannyl)pyrazine (0.110 mL), and toluene (2.00 mL) was heated under microwave irradiation at 120°C for 5 hr. The reaction mixture was purified by silica gel chromatography (NH, ethyl acetate/hexane), and crystallized from ethyl acetate/heptane to give the title compound (83.0 mg).
MS (API+) : [M+H]+473.1.

### Example 161

### N-((1S)-2-hydroxy-2-methyl-1-(4-(trifluoromethoxy)phenyl)propyl)-6-methyl-5-(3-methyl-1H-1,2,4-triazol-1-yl)pyrazolo[1,5-a]pyrimidine-3-carboxamide A) tert-butyl 3-amino-1H-pyrazole-4-carboxylate

A mixture of tert-butyl 2-cyanoacetate (24.3 g) and 1-tert-butoxy-N,N,N',N'-tetramethylmethanediamine (35.5 mL) was stirred at room temperature for 30 min. The reaction mixture was concentrated under reduced pressure to give a crude product of tert-butyl 2-cyano-3-(dimethylamino)acrylate. A solution of the obtained crude product and hydrazine monohydrate (8.39 mL) in methanol (344 mL) was stirred overnight at 70°C. The reaction mixture was cooled to room temperature, poured into water, and the mixture was extracted with ethyl acetate. The organic layer was separated, washed with saturated brine, and dried over anhydrous magnesium sulfate. The solvent was evaporated under reduced pressure and the residue was crystallized from isopropyl ether to give the title compound (18.6 g).
MS (API+) : [M+H]⁺184.1.

### B) methyl 3,3-dimethoxy-2-methylpropanoate

To a solution of methyl methacrylate (51.2 g) in ethyl acetate (301 mL) was added a solution of bromine (26.2 mL) in ethyl acetate (20 mL) at 0°C. The reaction mixture was stirred at room temperature for 16 hr, and saturated aqueous sodium thiosulfate solution was added. The reaction mixture was diluted with water, and the mixture was extracted with ethyl acetate. The organic layer was washed with saturated brine and dried over anhydrous sodium sulfate, and the solvent was evaporated under reduced pressure to give a crude product of methyl 2,3-dibromo-2-methylpropanoate (143 g). 28% Methanol solution (213 g) of sodium methoxide was diluted with methanol (221 mL), heated to 70°C, and a solution of the obtained crude product (143 g) in methanol (20 mL) was added. The reaction mixture was stirred at 70°C for 3 hr, and diluted with diethyl ether (550 mL) and water. The organic layer was washed with water and saturated brine and dried over anhydrous sodium sulfate, and the solvent was evaporated under reduced pressure to give the title compound (39.4 g).
¹H NMR (300 MHz, CDCl₃) δ 1.17 (3H, d, J = 7.2 Hz), 2.71-2.86 (1H, m), 3.35 (3H, s), 3.38 (3H, s), 3.70 (3H, s), 4.50 (1H, d, J = 7.7 Hz).

### C) tert-butyl 5-hydroxy-6-methylpyrazolo[1,5-a]pyrimidine-3-carboxylate

To a solution of tert-butyl 3-amino-1H-pyrazole-4-carboxylate (6.80 g) and methyl 3,3-dimethoxy-2-methylpropanoate (9.03 g) in N,N-dimethylformamide (74.2 mL) was added cesium carbonate (21.8 g), and the reaction mixture was stirred at 100°C for 16 hr. The reaction mixture was diluted with water, and adjusted to about pH 4 with acetic acid. The resulting solid was collected by filtration, washed with water, and dried under reduced pressure to give the title compound (9.24 g).
MS (API+) : [M+H]⁺250.1

### D) tert-butyl 5-chloro-6-methylpyrazolo[1,5-a]pyrimidine-3-carboxylate

To a solution of triphenylphosphine (8.14 g) in 1,2-dichloroethane (80.0 mL) was added carbon tetrachloride (2.92 mL) at room temperature. The reaction mixture was stirred under a nitrogen atmosphere at room temperature for 30 min, and a suspension of tert-butyl 5-hydroxy-6-methylpyrazolo[1,5-a]pyrimidine-3-carboxylate (1.5 g) in 1,2-dichloroethane (70 mL) was added at room temperature. The reaction mixture was stirred under a nitrogen atmosphere at 75°C to 85°C for 4.5 hr, and concentrated under reduced pressure. The residue was diluted with water, and extracted with ethyl acetate. The extract was washed with saturated brine and dried over anhydrous sodium sulfate, and the solvent was evaporated under reduced pressure. The residue was purified by silica gel column chromatography (hexane/ethyl acetate) to give the title compound (1.54 g).
¹H NMR (300 MHz, CDCl₃) δ 1.62 (9H, s), 2.42 (3H, d, J = 1.1 Hz), 8.40 (1H, s), 8.52 (1H, d, J = 1.1 Hz).

### E) tert-butyl 6-methyl-5-(3-methyl-1H-1,2,4-triazol-1-yl)pyrazolo[1,5-a]pyrimidine-3-carboxylate

To a solution of tert-butyl 5-chloro-6-methylpyrazolo[1,5-a]pyrimidine-3-carboxylate (1.08 g) in N,N-dimethylformamide (20.2 mL) were added 3-methyl-1H-1,2,4-triazole (469 mg) and potassium carbonate (781 mg) at room temperature. The reaction mixture was stirred at room temperature under an atmosphere dried with calcium chloride, and stirred overnight and then at 60°C for 2 hr. The reaction mixture was cooled to room temperature, and water was added. The resulting solid was collected by filtration, washed with water, and dried under reduced pressure to give the title compound (870 mg) containing a regioisomer.
MS (API+): [M+H]⁺315.2.

### F) 6-methyl-5-(3-methyl-1H-1,2,4-triazol-1-yl)pyrazolo[1,5-a]pyrimidine-3-carboxylic acid

To a suspension of tert-butyl 6-methyl-5-(3-methyl-1H-1,2,4-triazol-1-yl)pyrazolo[1,5-a]pyrimidine-3-carboxylate (870 mg) in acetonitrile (13.8 mL) was added methanesulfonic acid (1.26 mL) at 0°C. The reaction mixture was stirred at room temperature for 5 hr, and at 60°C for 30 min. 1M sodium hydroxide aqueous solution (19.4 mL) was added at 0°C, and acetonitrile was evaporated under reduced pressure. The resulting solid was collected by filtration, washed with water, and dried under reduced pressure to give the title compound (606 mg) .
MS (API+) : [M+H]⁺259.1.

### G) methyl 2-((tert-butoxycarbonyl)amino)-2-(4-(trifluoromethoxy)phenyl)acetate

To a mixture of 4-(trifluoromethoxy)benzaldehyde (19.0 g) and ammonium carbonate (25.9 g) in a mixed solvent of ethanol (114 mL) and water (45.6 mL) was slowly added an aqueous solution (71.1 mL) of potassium cyanide (8.14 g) at 50°C. The reaction mixture was stirred at 60°C for 3 hr, and cooled to room temperature, and the ethanol was evaporated under reduced pressure. The pH of the residue was adjusted to 1 with conc. hydrochloric acid at 0°C, and the resulting solid was filtered off, and washed with water. To an aqueous solution (100 mL) of potassium hydroxide (23.6 g) was added the solid obtained by the above-mentioned operation at room temperature, and the reaction mixture was stirred at 90°C for 3 days. The reaction mixture was cooled to room temperature, and neutralized with conc. hydrochloric acid. The resulting solid was filtered off, and washed with water to give a crude product (13.3 g) of 2-amino-2-(4-(trifluoromethoxy)phenyl)acetic acid. To a solution of the obtained crude product (13.3 g) in tetrahydrofuran (113 mL) were added di-tert-butyl dicarbonate (19.7 mL) and 2 M aqueous sodium hydroxide solution (85 mL) at room temperature. The reaction mixture was stirred overnight at room temperature, and poured into water, and the mixture was washed with diethyl ether. The pH of the aqueous layer was adjusted to 3 with 1 M hydrochloric acid at 0°C, and the mixture was extracted with ethyl acetate. The extract was washed with saturated brine, and dried over anhydrous magnesium sulfate, and the solvent was evaporated under reduced pressure to give a crude product (11.3 g) of 2-((tert-butoxycarbonyl)amino)-2-(4-(trifluoromethoxy)phenyl)acetic acid. To a solution of the obtained crude product (11.3 g) in N,N-dimethylformamide (84 mL) were added methyl iodide (2.53 mL) and potassium carbonate (5.59 g) at room temperature. The reaction mixture was stirred at room temperature for 2 hr, and poured into water, and the mixture was extracted with ethyl acetate. The extract was washed with saturated brine, and dried over anhydrous magnesium sulfate, and the solvent was evaporated under reduced pressure. The residue was purified by silica gel column chromatography (hexane/ethyl acetate) to give the title compound (8.20 g).
MS (API-): [M-H]⁻ 348.1.

### H) tert-butyl (2-hydroxy-2-methyl-1-(4-(trifluoromethoxy)phenyl)propyl)carbamate

To a solution of methyl 2-((tert-butoxycarbonyl)amino)-2-(4-(trifluoromethoxy)phenyl)acetate (5.00 g) in tetrahydrofuran (71.6 mL) was slowly added 1 M methylmagnesium bromide/tetrahydrofuran solution (57.3 mL) at 0°C. The reaction mixture was stirred at 0°C for 1 hr under argon atmosphere, saturated aqueous ammonium chloride solution was added thereto at 0°C, and the mixture was extracted with ethyl acetate. The extract was washed with saturated brine, and dried over anhydrous magnesium sulfate, and the solvent was evaporated under reduced pressure. The residue was purified by silica gel column chromatography (hexane/ethyl acetate) to give the title compound (3.99 g).
MS (API-): [M-H]⁻ 348.2.

### I) (S)-1-amino-2-methyl-1-(4-(trifluoromethoxy)phenyl)propan-2-ol hydrochloride

The racemate (17.9 g) of tert-butyl (2-hydroxy-2-methyl-1-(4-(trifluoromethoxy)phenyl)propyl)carbamate was resolved by HPLC (column: CHIRALPAK AD, 50 mmID×500 mmL, manufactured by Daicel Corporation, mobile phase: hexane/ethanol = 950/50) to give tert-butyl {(1S)-2-hydroxy-2-methyl-1-[4-(trifluoromethoxy)phenyl]propyl}carbamate (8.06 g) having a shorter retention time. To tert-butyl {(1S)-2-hydroxy-2-methyl-1-[4-(trifluoromethoxy)phenyl]propyl}carbamate (8.06 g) was added 4M hydrogen chloride/ethyl acetate solution (115 mL). The reaction mixture was stirred at room temperature for 15 min, and the solvent was evaporated under reduced pressure. To the residue was added diisopropyl ether, and the resulting crystals were collected by filtration to give the title compound (5.39 g).
MS (API+), found: 250.1.

### J) N-((1S)-2-hydroxy-2-methyl-1-(4-(trifluoromethoxy)phenyl)propyl)-6-methyl-5-(3-methyl-1H-1,2,4-triazol-1-yl)pyrazolo[1,5-a]pyrimidine-3-carboxamide

To a suspension of 6-methyl-5-(3-methyl-1H-1,2,4-triazol-1-yl)pyrazolo[1,5-a]pyrimidine-3-carboxylic acid (606 mg) in N,N-dimethylformamide (11.7 mL) were added (S)-1-amino-2-methyl-1-(4-(trifluoromethoxy)phenyl)propan-2-ol hydrochloride (805 mg), 1-hydroxybenzotriazole monohydrate (431 mg), 1-(3-dimethylaminopropyl)-3-ethylcarbodiimide hydrochloride (540 mg), and triethylamine (392 µL). The reaction mixture was stirred at room temperature for 2 hr, poured into water, and the mixture was extracted with ethyl acetate. The extract was washed with saturated brine and dried over anhydrous magnesium sulfate, and the solvent was evaporated under reduced pressure. The residue was purified by silica gel column chromatography (NH, hexane/ethyl acetate) and recrystallized from ethyl acetate and diisopropyl ether to give the title compound (858 mg).
MS (API+) : [M+H]⁺490.3.
¹H NMR (300 MHz, DMSO-d₆) δ 1.00 (3H, s), 1.31 (3H, s), 2.46 (3H, s), 2.64 (3H, d, J = 0.8 Hz), 4.87 (1H, d, J = 8.0 Hz), 5.32 (1H, s), 7.27 (2H, d, J = 8.0 Hz), 7.51 (2H, d, J = 8.7 Hz), 8.49 (1H, s), 8.85 (1H, d, J = 8.3 Hz), 9.46-9.53 (2H, m).

### Example 162

### N-((1S)-2-hydroxy-2-methyl-1-(4-(trifluoromethoxy)phenyl)propyl)-6-methyl-5-(1H-pyrazol-1-yl)pyrazolo[1,5-a]pyrimidine-3-carboxamide

By a method similar to that in Example 161, the title compound was obtained.
MS (API+) : [M+H]+475.3.

### Example 163

### 8-bromo-N-((1S)-2-hydroxy-2-methyl-1-(4-(trifluoromethoxy)phenyl)propyl)-1,6-naphthyridine-2-carboxamide

By a method similar to that in Example 13, the title compound was obtained.
MS (API+): [M+H]+484.1, 486.1.

### Example 164

### N-((1S)-2-hydroxy-2-methyl-1-(4-(trifluoromethoxy)phenyl)propyl)-8-phenyl-1,6-naphthyridine-2-carboxamide

By a method similar to that in Example 73, the title compound was obtained.
MS (API+): [M+H]+482.3.

### Example 165

### N-(1-(4-(difluoromethoxy)phenyl)-2-hydroxy-2-methylpropyl)-5-(1-(difluoromethyl)-1H-pyrazol-4-yl)pyrazolo[1,5-a]pyrimidine-3-carboxamide

By a method similar to that in Example 14, step I, the title compound was obtained.
MS (API+) : [M+H]+493.2.

### Example 166

### N-((1S or 1R)-1-(4-(difluoromethoxy)phenyl)-2-hydroxy-2-methylpropyl)-5-(1-(difluoromethyl)-1H-pyrazol-4-yl)pyrazolo[1,5-a]pyrimidine-3-carboxamide

By a method similar to that in Example 15, the title compound was obtained.
MS (API+) : [M+H]+493.2.

### Example 167

### N-((1R or 1S)-1-(4-(difluoromethoxy)phenyl)-2-hydroxy-2-methylpropyl)-5-(1-(difluoromethyl)-1H-pyrazol-4-yl)pyrazolo[1,5-a]pyrimidine-3-carboxamide

By a method similar to that in Example 16, the title compound was obtained.
MS (API+): [M+H]+493.2.

The compounds of Examples prepared according to the above-mentioned method or a method analogous thereto are shown in the following tables. Mass in the tables means measured value.

**[Table 1-1]**

| **EX.** | **IUPAC NAME** | **STRUCTURE** | **Mass (M+1)** | **Mass (M-1**) |
|---|---|---|---|---|
| 1 | N-(2-methoxy-1-(4-(trifluoromethoxy)phenyl)ethyl)-2-oxo-1,2-dihydro-1,5-naphthyridine-4-carboxamide | 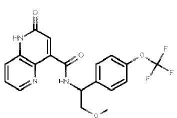 | | |
| | 1H NMR (400 MHz, CDCl3) δ 1.44 (3H, t, J=7.2 Hz), 4.53 (2H, q, J=7.2 Hz), 7.07 (1H, s), 7.48-7.51 (1H, m), 7.74-7.77 (1H, m), 8.66-8.68 (1H, m), 12.38 (1H, brs). | | | |
| 2 | N-((1S)-2-methoxy-1-(4-(trifluoromethoxy)phenyl)ethyl)-6-methylpyrazolo[1,5-a]pyrimidine-3-carboxamide | 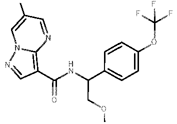 | 395.3 | |
| 3 | N-(2-methoxy-1-(4-(trifluoromethoxy)phenyl)ethyl)pyrazolo[1,5-a]pyrimidine-7-carboxamide | 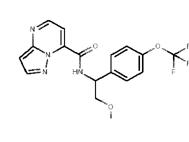 | 381.1 | |
| 4 | N-(2-methoxy-1-(4-(trifluoromethoxy)phenyl)ethyl)-2-methylpyrazolo[1,5-a]pyrimidine-7-carboxamide | 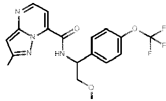 | 395.1 | |
| 5 | 3-chloro-N-(2-methoxy-1-(4-(trifluoromethoxy)phenyl)ethyl)-pyrazolo[1,5-a]pyrimidine-7-carboxamide | 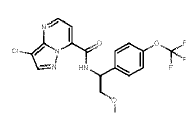 | 415.1 | |
| 6 | N-(2-methoxy-1-(4-(trifluoromethoxy)phenyl)ethyl)-5-methylpyrazolo[1,5-a]pyrimidine-7-carboxamide | 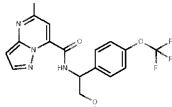 | 395.1 | |
| 7 | N-(2-methoxy-1-(4-(trifluoromethoxy)phenyl)ethyl)-[1,2,4]triazolo[1,5-a]pyrimidine-7-carboxamide | 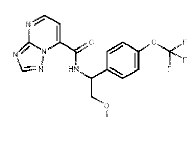 | 382.1 | |
| 8 | N-(2-methoxy-1-(4-(trifluoromethoxy)phenyl)ethyl)-1,6-naphthyridine-8-carboxamide | 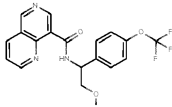 | 392.1 | |
| 9 | N-(2-methoxy-1-(4-(trifluoromethoxy)phenyl)ethyl)-3H-imidazo[4,5-b]pyridine-7-carboxamide | 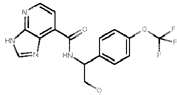 | 380.1 | |

**[Table 1-2]**

| | | | | |
|---|---|---|---|---|
| 10 | N-(2-methoxy-1-(4-(trifluoromethoxy)phenyl)ethyl)-7H-purine-6-carboxamide | 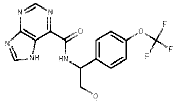 | 382 | |
| 11 | N-(2-methoxy-1-(4-(trifluoromethoxy)phenyl)ethyl)-4-oxo-4,5-dihydro-1H-pyrazolo[4,3-c]pyridine-3-carboxamide | 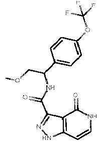 | 397.1 | |
| 12 | N-((1S)-1-(3-fluoro-4-(trifluoromethoxy)phenyl)-2-methoxyethyl)-6-methylpyrazolo-[1,5-a]pyrimidine-3-carboxamide | 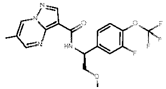 | 413.1 | |
| 13 | N-((1S)-1-(3-fluoro-4-(trifluoromethoxy)phenyl)-2-methoxyethyl)-2-oxo-1,2-dihydro-1,5-naphthyridine-4-carboxamide | 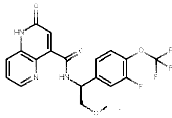 | 426.1 | |
| 14 | N-(1-(3-fluoro-4-(trifluoromethoxy)phenyl)-2-hydroxy-2-methylpropyl)-6-methylpyrazolo-[1,5-a]pyrimidine-3-carboxamide | 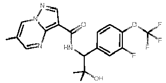 | 427.2 | |
| 15 | N-((1S)-1-(3-fluoro-4-(trifluoromethoxy)phenyl)-2-hydroxy-2-methylpropyl)-6-methylpyrazolo-[1,5-a]pyrimidine-3-carboxamide | 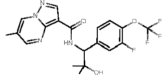 | 427.2 | |
| 16 | N-((1R)-1-(3-fluoro-4-(trifluoromethoxy)phenyl)-2-hydroxy-2-methylpropyl)-6-methylpyrazolo[1,5-a]pyrimidine-3-carboxamide | 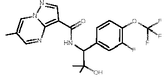 | 427.2 | |
| 17 | N-(2-methoxy-1-(4-(1H-pyrazol-1-yl)phenyl)ethyl)-6-methylpyrazolo[1,5-a]pyrimidine-3-carboxamide | 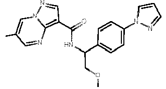 | 377.2 | |
| 18 | 5-chloro-N-(1-(3-fluoro-4-(trifluoromethoxy)phenyl)-2-methoxyethyl)pyrazolo[1,5-a]pyrimidine-3-carboxamide | 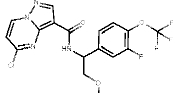 | 433.1 | |
| 19 | 6-bromo-N-(1-(3-fluoro-4-(trifluoromethoxy)phenyl)-2-hydroxy-2-methylpropyl)-pyrazolo[1,5-a]pyrimidine-3-carboxamide | 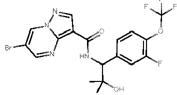 | 491 | |

**[Table 1-3]**

| | | | | |
|---|---|---|---|---|
| 20 | 5-chloro-N-(1-(3-fluoro-4-(trifluoromethoxy)phenyl)-2-hydroxy-2-methylpropyl)pyrazolo[1,5-a]pyrimidine-3-carboxamide | 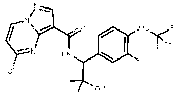 | 491.1 | |
| 21 | N-(1-(3-fluoro-4-(trifluoromethoxy)phenyl)-2-hydroxy-2-methylpropyl)-6-vinylpyrazolo[1,5-a]pyrimidine-3-carboxamide | 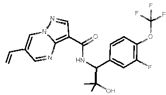 | 439.1 | |
| 22 | 6-ethyl-N-(1-(3-fluoro-4-(trifluoromethoxy)phenyl)-2-hydroxy-2-methylpropyl)pyrazolo[1,5-a]pyrimidine-3-carboxamide | 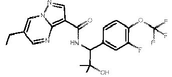 | 441.2 | |
| 23 | 6-cyclopropyl-N-(1-(3-fluoro-4-(trifluoromethoxy)phenyl)-2-hydroxy-2-methylpropyl)pyrazolo[1,5-a]pyrimidine-3-carboxamide | 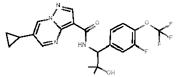 | 453.2 | |
| 24 | N-(1-(3-fluoro-4-(trifluoromethoxy)phenyl)-2-hydroxy-2-methylpropyl)-6-(prop-1-en-2-yl)pyrazolo[1,5-a]pyrimidine-3-carboxamide | 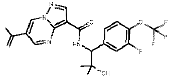 | 453.2 | |
| 25 | N-(1-(3-fluoro-4-(trifluoromethyl)phenyl)-2-hydroxy-2-methylpropyl)-6-methylpyrazolo[1,5-a]pyrimidine-3-carboxamide | 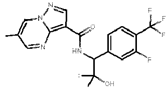 | 411.2 | |
| 26 | N-(1-(3-fluoro-4-(trifluoromethoxy)phenyl)-2-hydroxy-2-methylpropyl)-6-isopropylpyrazolo[1,5-a]pyrimidine-3-carboxamide | 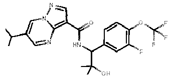 | 455.2 | |
| 27 | 5-(dimethylamino)-N-(1-(3-fluoro-4-(trifluoromethoxy)phenyl)-2-hydroxy-2-methylpropyl)pyrazolo[1,5-a]pyrimidine-3-carboxamide | 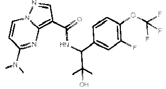 | 456.0 | |
| 28 | 5-(dimethylamino)-N-(1-(3-fluoro-4-(trifluoromethoxy)phenyl)-2-methoxyethyl)pyrazolo[1,5-a]pyrimidine-3-carboxamide | 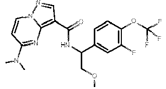 | 442.0 | |
| 29 | 5-(ethyl(methyl)amino)-N-(1-(3-fluoro-4-(trifluoromethoxy)phenyl)-2-hydroxy-2-methylpropyl)pyrazolo[1,5-a]pyrimidine-3-carboxamide | 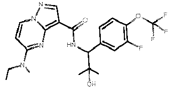 | 470.1 | |

**[Table 1-4]**

| | | | | |
|---|---|---|---|---|
| 30 | 5-(ethyl(methyl)amino)-N-(1-(3-fluoro-4-(trifluoromethoxy)phenyl)-2-methoxyethyl)pyrazolo[1,5-a]pyrimidine-3-carboxamide | 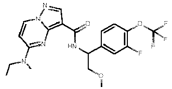 | 456.1 | |
| 31 | 5-(azetidin-1-yl)-N-(1-(3-fluoro-4-(trifluoromethoxy)phenyl)-2-hydroxy-2-methylpropyl)pyrazolo[1,5-a]pyrimidine-3-carboxamide | 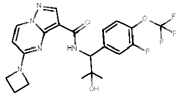 | 468.1 | |
| 32 | 5-(azetidin-1-yl)-N-(1-(3-fluoro-4-(trifluoromethoxy)phenyl)-2-methoxyethyl)pyrazolo[1,5-a]pyrimidine-3-carboxamide | 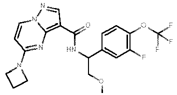 | 454.1 | |
| 33 | N-(1-(3-fluoro-4-(trifluoromethoxy)phenyl)-2-hydroxy-2-methylpropyl)-5-(pyrrolidin-1-yl)pyrazolo[1,5-a]pyrimidine-3-carboxamide | 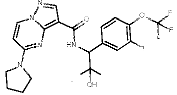 | 482.1 | |
| 34 | N-(1-(3-fluoro-4-(trifluoromethoxy)phenyl)-2-methoxyethyl)-5-(pyrrolidin-1-yl)pyrazolo[1,5-a]pyrimidine-3-carboxamide | 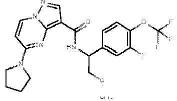 | 468.1 | |
| 35 | N-(1-(3-fluoro-4-(trifluoromethoxy)phenyl)-2-hydroxy-2-methylpropyl)-5-(piperidin-1-yl)pyrazolo[1,5-a]pyrimidine-3-carboxamide | 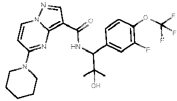 | 496.1 | |
| 36 | N-(1-(3-fluoro-4-(trifluoromethoxy)phenyl)-2-methoxyethyl)-5-(piperidin-1-yl)pyrazolo[1,5-a]pyrimidine-3-carboxamide | 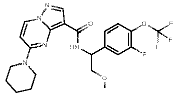 | 482.1 | |
| 37 | N-(1-(3-fluoro-4-(trifluoromethoxy)phenyl)-2-hydroxy-2-methylpropyl)-5-(morpholin-4-yl)pyrazolo[1,5-a]pyrimidine-3-carboxamide | 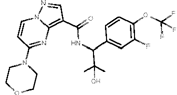 | 498.1 | |
| 38 | N-(1-(3-fluoro-4-(trifluoromethoxy)phenyl)-2-methoxyethyl)-5-(morpholin-4-yl)pyrazolo[1,5-a]pyrimidine-3-carboxamide | 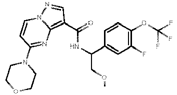 | 484.1 | |
| 39 | N-(1-(3-fluoro-4-(trifluoromethoxy)phenyl)-2-hydroxy-2-methylpropyl)-5-(3-methoxyazetidin-1-yl)pyrazolo[1,5-a]pyrimidine-3-carboxamide | 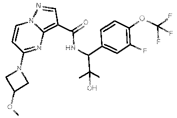 | 498.1 | |

**[Table 1-5]**

| | | | | |
|---|---|---|---|---|
| 40 | N-(1-(3-fluoro-4-(trifluoromethoxy)phenyl)-2-methoxyethyl)-5-(3-methoxyazetidin-1-yl)pyrazolo[1,5-a]pyrimidine-3-carboxamide | 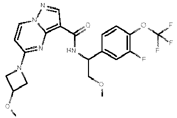 | 484.1 | |
| 41 | 5-(3,3-difluoropyrrolidin-1-yl)-N-(1-(3-fluoro-4-(trifluoromethoxy)phenyl)-2-hydroxy-2-methylpropyl)pyrazolo[1,5-a]pyrimidine-3-carboxamide | 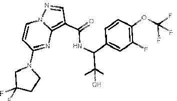 | 518.1 | |
| 42 | 5-(3,3-difluoropyrrolidin-1-yl)-N-(1-(3-fluoro-4-(trifluoromethoxy)phenyl)-2-methoxyethyl)pyrazolo[1,5-a]pyrimidine-3-carboxamide | 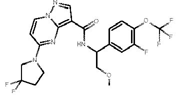 | 504.1 | |
| 43 | N-(1-(3-fluoro-4-(trifluoromethoxy)phenyl)-2-hydroxy-2-methylpropyl)-5-(3-oxa-8-azabicyclo[3.2.1]oct-8-yl)pyrazolo[1,5-a]pyrimidine-3-carboxamide | 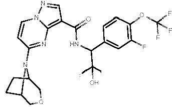 | 524.1 | |
| 44 | N-(1-(3-fluoro-4-(trifluoromethoxy)phenyl)-2-methoxyethyl)-5-(3-oxa-8-azabicyclo[3.2.1]oct-8-yl)pyrazolo[1,5-a]pyrimidine-3-carboxamide | 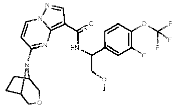 | 510.1 | |
| 45 | N-(1-(3-fluoro-4-(trifluoromethoxy)phenyl)-2-hydroxy-2-methylpropyl)-5-(3-oxa-6-azabicyclo[3.1.1]hept-6-yl)pyrazolo[1,5-a]pyrimidine-3-carboxamide | 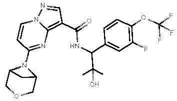 | 510.1 | |
| 46 | N-(1-(3-fluoro-4-(trifluoromethoxy)phenyl)-2-methoxyethyl)-5-(3-oxa-6-azabicyclo[3.1.1]hept-6-yl)pyrazolo[1,5-a]pyrimidine-3-carboxamide | 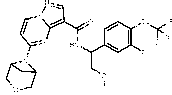 | 496.1 | |
| 47 | N-(1-(3-fluoro-4-(trifluoromethoxy)phenyl)-2-hydroxy-2-methylpropyl)-5-((1R,5S)-6-oxa-3-azabicyclo[3.1.1]hept-3-yl)pyrazolo[1,5-a]pyrimidine-3-carboxamide | 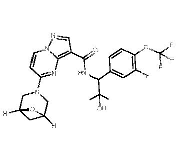 | 510.1 | |
| 48 | N-(1-(3-fluoro-4-(trifluoromethoxy)phenyl)-2-methoxyethyl)-5-((1R,5S)-6-oxa-3-azabicyclo[3.1.1]hept-3-yl)pyrazolo[1,5-a]pyrimidine-3-carboxamide | 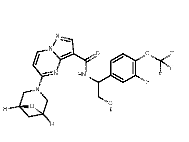 | 496.1 | |
| 49 | N-(1-(3-fluoro-4-(trifluoromethoxy)phenyl)-2-hydroxy-2-methylpropyl)-5-methylpyrazolo[1,5-a]pyrimidine-3-carboxamide | 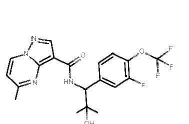 | 427.0 | |

**[Table 1-6]**

| | | | | |
|---|---|---|---|---|
| 50 | N-(1-(3-fluoro-4-(trifluoromethoxy)phenyl)-2-methoxyethyl)-5-methylpyrazolo[1,5-a]pyrimidine-3-carboxamide | 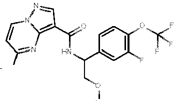 | 412.9 | |
| 51 | 5-cyclopropyl-N-(1-(3-fluoro-4-(trifluoromethoxy)phenyl)-2-hydroxy-2-methylpropyl)pyrazolo[1,5-a]pyrimidine-3-carboxamide | 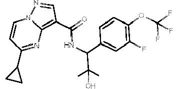 | 453.1 | |
| 52 | 5-cyclopropyl-N-(1-(3-fluoro-4-(trifluoromethoxy)phenyl)-2-methoxyethyl)pyrazolo[1,5-a]pyrimidine-3-carboxamide | 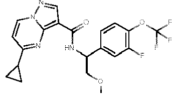 | 439.0 | |
| 53 | N-(1-(3-fluoro-4-(trifluoromethoxy)phenyl)-2-hydroxy-2-methylpropyl)-5-(1-methyl-1H-pyrazol-3-yl)pyrazolo[1,5-a]pyrimidine-3-carboxamide | 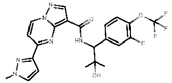 | 493.1 | |
| 54 | N-(1-(3-fluoro-4-(trifluoromethoxy)phenyl)-2-methoxyethyl)-5-(1-methyl-1H-pyrazol-3-yl)pyrazolo[1,5-a]pyrimidine-3-carboxamide | 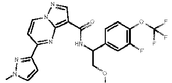 | 479.1 | |
| 55 | N-(1-(3-fluoro-4-(trifluoromethoxy)phenyl)-2-hydroxy-2-methylpropyl)-5-(1-methyl-1H-pyrazol-4-yl)pyrazolo[1,5-a]pyrimidine-3-carboxamide | 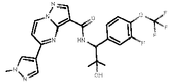 | 493.1 | |
| 56 | N-(1-(3-fluoro-4-(trifluoromethoxy)phenyl)-2-methoxyethyl)-5-(1-methyl-1H-pyrazol-4-yl)pyrazolo[1,5-a]pyrimidine-3-carboxamide | 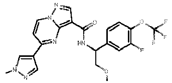 | 479.1 | |
| 57 | N-(1-(3-fluoro-4-(trifluoromethoxy)phenyl)-2-methoxyethyl)-5-(1-methyl-1H-pyrazol-5-yl)pyrazolo[1,5-a]pyrimidine-3-carboxamide | 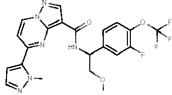 | 479.1 | |
| 58 | N-(2-hydroxy-2-methyl-1-(4-(trifluoromethoxy)phenyl)-propyl)-6-methylpyrazolo[1,5-a]pyrimidine-3-carboxamide | 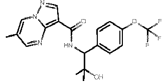 | 409.2 | |
| 59 | 5-(azetidin-1-yl)-N-(1-(3-fluoro-4-(trifluoromethoxy)phenyl)-2-hydroxy-2-methylpropyl)-6-methylpyrazolo[1,5-a]pyrimidine-3-carboxamide | 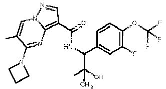 | 482.2 | |

**[Table 1-7]**

| | | | | |
|---|---|---|---|---|
| 60 | N-(1-(3-fluoro-4-(trifluoromethoxy)phenyl)-2-hydroxy-2-methylpropyl)-5,6-dimethylpyrazolo[1,5-a]pyrimidine-3-carboxamide | 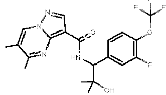 | 441.1 | |
| 61 | N-(1-(3-fluoro-4-(trifluoromethoxy)phenyl)-3-hydroxy-3-methylbutyl)-6-methylpyrazolo[1,5-a]pyrimidine-3-carboxamide | 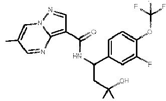 | 441.1 | |
| 62 | N-(1-(3-fluoro-4-(trifluoromethoxy)phenyl)-2-hydroxy-2-methylpropyl)pyrazolo[1,5-a]pyrimidine-3-carboxamide | 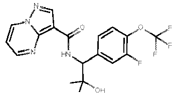 | 413.1 | |
| 63 | N-(1-(3-fluoro-4-(trifluoromethoxy)phenyl)-2-hydroxy-2-methylpropyl)-5-(1H-pyrazol-1-yl)pyrazolo[1,5-a]pyrimidine-3-carboxamide | 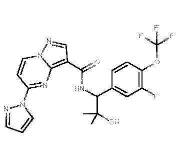 | 479.1 | |
| 64 | N-(1-(3-fluoro-4-(trifluoromethoxy)phenyl)-2-hydroxy-2-methylpropyl)-5-(4-methyl-1H-pyrazol-1-yl)pyrazolo[1,5-a]pyrimidine-3-carboxamide | 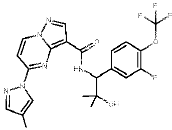 | 493.2 | |
| 65 | N-(2-ethyl-1-(3-fluoro-4-(trifluoromethoxy)phenyl)-2-hydroxybutyl)-6-methylpyrazolo[1,5-a]pyrimidine-3-carboxamide | 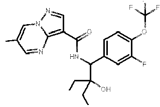 | 455.2 | |
| 66 | 5-cyclopropyl-N-((1S)-1-(3-fluoro-4-(trifluoromethoxy)phenyl)-2-hydroxy-2-methylpropyl)-1-methyl-7-oxo-1,7-dihydropyrazolo[1,5-a]pyrimidine-3-carboxamide | 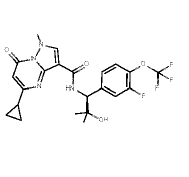 | 483.2 | |
| 67 | N-((1S)-1-(3-fluoro-4-(trifluoromethoxy)phenyl)-2-hydroxy-2-methylpropyl)-5-(methoxymethyl)-1-methyl-7-oxo-1,7-dihydropyrazolo[1,5-a]pyrimidine-3-carboxamide | 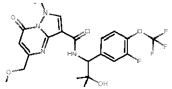 | 487.2 | |

**[Table 1-8]**

| | | | | |
|---|---|---|---|---|
| 68 | N-((1S)-2-hydroxy-2-methyl-1-(4-(trifluoromethoxy)phenyl)propyl)-5-(1H-1,2,3-triazol-1-yl)pyrazolo[1,5-a]pyrimidine-3-carboxamide | 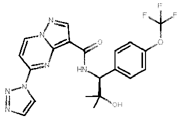 | | |
| | ¹H NMR (300 MHz, CDCl₃) δ 1.18 (3H, s), 1.52 (3H, s), 1.72 (1H, s), 5.03 (1H, d, J=8.3 Hz), 7.14-7.22 (2H, m), 7.42-7.48 (2H, m), 7.94 (1H, d, J=1.3 Hz), 8.04 (1H, d, J-7.6 Hz), 8.65 (1H, s), 8.90-8.98 (2H, m), 9.06 (1H, d, J=1.3 | | | |
| 69 | N-((1S)-2-hydroxy-2-methyl-1-(4-(trifluoromethoxy)phenyl)propyl)-5-(6-methylpyridin-3-yl)pyrazolo[1,5-a]pyrimidine-3-carboxamide | 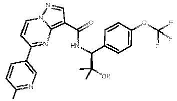 | 486.2 | |
| 70 | 5-(1-(difluoromethyl)-1H-pyrazol-4-yl)-N-((1S)-2-hydroxy-2-methyl-1-(4-(trifluoromethoxy)-phenyl)propyl)pyrazolo[1,5-a]pyrimidine-3-carboxamide | 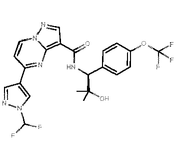 | 511.1 | |
| 71 | N-((1S)-2-hydroxy-2-methyl-1-(4-(trifluoromethoxy)phenyl)propyl)-5-(1H-pyrazol-1-yl)pyrazolo[1,5-a]pyrimidine-3-carboxamide | 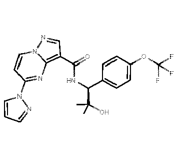 | 461.2 | |
| 72 | N-((1S)-2-hydroxy-2-methyl-1-(4-(trifluoromethoxy)phenyl)propyl)-5-(pyridin-2-yl)pyrazolo[1,5-a]pyrimidine-3-carboxamide | 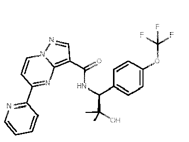 | | |
| | ¹H NMR (300 MHz, CDCl₃) δ 1.21 (3H, s), 1.49 (3H, s), 1.96 (1H, s), 5.14 (1H, d, J=8.3 Hz), 7.14-7.22 (2H, m), 7.44-7.56 (3H, m), 7.88-7.97 (1H, m), 8.26 (1H, d, J=7.4 Hz), 8.64 (1H, s), 8.75-8.88 (3H, m), 9.25 (1H, d, J=8.3 Hz). | | | |
| 73 | 5-(3-fluoropyridin-4-yl)-N-((1S)-2-hydroxy-2-methyl-1-(4-(trifluoromethoxy)phenyl)propyl)-pyrazolo[1,5-a]pyrimidine-3-carboxamide | 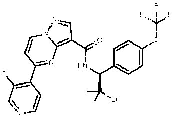 | | |
| | ¹H NMR (300 MHz, CDCl₃) δ 1.18 (3H, s), 1.46 (3H, s), 1.90 (1H, brs), 5.12 (1H, d, J=8.5 Hz), 7.13-7.20 (2H, m), 7.44-7.52 (2H, m), 7.65-7.70 (1H, m), 8.33 (1H, dd, J=6.8, 5.1 Hz), 8.66 (1H, d, J=5.1 Hz), 8.69-8.74 (2H, m), 8.90 (1H, d, J=7.4 Hz), 9.07 (1H, d, J=8.3 Hz). | | | |

**[Table 1-9]**

| | | | | |
|---|---|---|---|---|
| 74 | N-((1S)-2-hydroxy-2-methyl-1-(4-(trifluoromethoxy)phenyl)propyl)-5-(4-methyl-1H-1,2,3-triazol-1-yl)pyrazolo[1,5-a]pyrimidine-3-carboxamide | 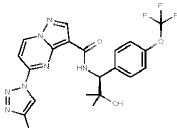 | 476.2 | |
| 75 | N-((1S)-1-(3-fluoro-4-(trifluoromethoxy)phenyl)-2-hydroxy-2-methylpropyl)-1-methyl-7-oxo-5-(pyridin-2-yl)-1,7-dihydropyrazolo[1,5-a]pyrimidine-3-carboxamide | 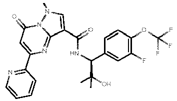 | 520.2 | |
| 76 | N-((1S)-2-hydroxy-2-methyl-1-(4-(trifluoromethoxy)phenyl)propyl)-5-(3-methyl-2-oxopyridin-1(2H)-yl)pyrazolo[1,5-a]pyrimidine-3-carboxamide | 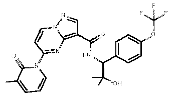 | 502.2 | |
| 77 | N-((1S)-2-hydroxy-2-methyl-1-(4-(trifluoromethoxy)phenyl)propyl)-1-methyl-7-oxo-5-(pyridin-2-yl)-1,7-dihydropyrazolo[1,5-a]pyrimidine-3-carboxamide | 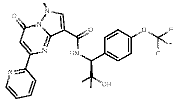 | 502.2 | |
| 78 | N-((1S)-2-hydroxy-2-methyl-1-(4-(trifluoromethoxy)phenyl)propyl)-6-methyl-5-(1H-1,2,3-triazol-1-yl)pyrazolo[1,5-a]pyrimidine-3-carboxamide | 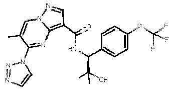 | 476.2 | |
| 79 | N-((1S)-2-hydroxy-2-methyl-1-(4-(trifluoromethoxy)phenyl)propyl)-6-methyl-5-(4-(trifluoromethyl)-1H-imidazol-1-yl)pyrazolo[1,5-a]pyrimidine-3-carboxamide | 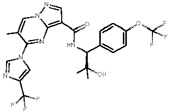 | 543.2 | |
| 80 | N-((1S)-2-hydroxy-2-methyl-1-(4-(trifluoromethoxy)phenyl)propyl)-6-methyl-5-(4-methyl-1H-1,2,3-triazol-1-yl)pyrazolo[1,5-a]pyrimidine-3-carboxamide | 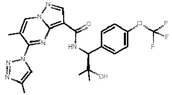 | 490.2 | |

**[Table 1-10]**

| | | | | |
|---|---|---|---|---|
| 81 | N-((1S)-1-(3-fluoro-4-(trifluoro-methoxy)phenyl)-2-hydroxy-2-methylpropyl)-5,6-dimethylpyrazolo[1,5-a]pyrimidine-3-carboxamide | 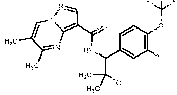 | 441.1 | |
| 82 | 5-((2R,6S)-2,6-dimethylmorpholin-4-yl)-N-((1S)-1-(3-fluoro-4-(trifluoromethoxy)phenyl)-2-hydroxy-2-methylpropyl)-pyrazolo[1,5-a]pyrimidine-3-carboxamide | 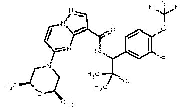 | 526.1 | |
| 83 | 5-(1-(difluoromethyl)-1H-pyrazol-4-yl)-N-((1S)-2-hydroxy-2-methyl-1-(4-(trifluoromethoxy)-phenyl)propyl)-6-methylpyrazolo-[1,5-a]pyrimidine-3-carboxamide | 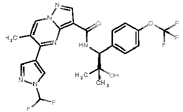 | 525.2 | |
| 84 | N-((1S)-2-hydroxy-2-methyl-1-(4-(trifluoromethoxy)phenyl)propyl)-6-methyl-5-(5-methyl-1H-1,2,3-triazol-1-yl)pyrazolo[1,5-a]pyrimidine-3-carboxamide | 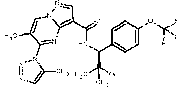 | 490.3 | |
| 85 | 5-(4-fluoro-1H-pyrazol-1-yl)-N-((1S)-2-hydroxy-2-methyl-1-(4-(trifluoromethoxy)phenyl)propyl)-6-methylpyrazolo[1,5-a]pyrimidine-3-carboxamide | 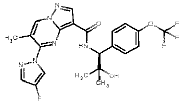 | 493.1 | |
| 86 | 5-(3-methoxypyridin-4-yl)-N-((1S)-2-methoxy-1-(4-(trifluoro-methoxy)phenyl)ethyl)pyrazolo[1,5 -a]pyrimidine-3-carboxamide | 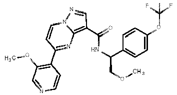 | 488.1 | |
| 87 | N-(1-(4-(difluoromethoxy)-3-fluorophenyl)-2-hydroxy-2-methylpropyl)-5-((2R,6S)-2,6-dimethylmorpholin-4-yl)pyrazolo-[1,5-a]pyrimidine-3-carboxamide | 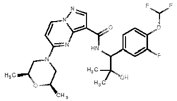 | 508.2 | |
| 88 | 5-(1-(difluoromethyl)-1H-pyrazol-4-yl)-N-(1-(3-fluoro-4-(trifluoromethyl)phenyl)-2-hydroxy-2-methylpropyl)-pyrazolo[1,5-a]pyrimidine-3-carboxamide | 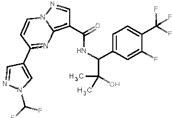 | 513.1 | |

**[Table 1-11]**

| | | | | |
|---|---|---|---|---|
| 89 | N-((1S)-1-(3-fluoro-4-(trifluoromethoxy)phenyl)-2-hydroxy-2-methylpropyl)-5-(6-methylpyridazin-4-yl)pyrazolo-[1,5-a]pyrimidine-3-carboxamide | 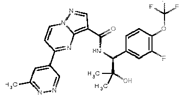 | 505.1 | |
| 90 | N-(1-(4-(difluoromethoxy)-3-fluorophenyl)-2-hydroxy-2-methylpropyl)-6-methylpyrazolo-[1,5-a]pyrimidine-3-carboxamide | 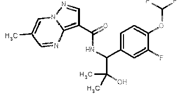 | 409.1 | |
| 91 | N-((1S)-2-hydroxy-2-methyl-1-(4-(trifluoromethoxy)phenyl)propyl)-5-(2-methoxypyridin-3-yl)pyrazolo[1,5-a]pyrimidine-3-carboxamide | 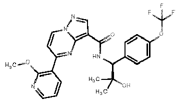 | 502.1 | |
| 92 | N-((1S)-2-methoxy-1-(4-(trifluoromethoxy)phenyl)ethyl)-7-methyl-2-oxo-1,2-dihydro-1,5-naphthyridine-4-carboxamide | 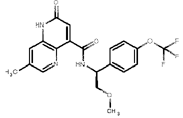 | 422.2 | |
| 93 | 6-chloro-N-((1S)-1-(3-fluoro-4-(trifluoromethoxy)phenyl)-2-hydroxy-2-methylpropyl)-pyrazolo[1,5-a]pyrimidine-3-carboxamide | 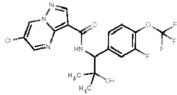 | 447.1 | |
| 94 | N-((1S)-1-(3-fluoro-4-(trifluoromethoxy)phenyl)-2-hydroxy-2-methylpropyl)pyrazolo[1,5-a]pyrimidine-3-carboxamide | 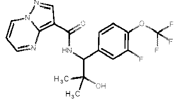 | 413.1 | |
| 95 | N-(1-(3-fluoro-4-(trifluoromethoxy)phenyl)-2-hydroxy-2-methylpropyl)-5-(3-methyl-1H-pyrazol-1-yl)pyrazolo[1,5-a]pyrimidine-3-carboxamide | 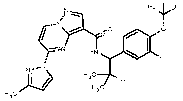 | 493.1 | |
| 96 | N-(2-hydroxy-2-methyl-1-(3-methyl-4-(trifluoromethoxy)-phenyl)propyl)-6-methylpyrazolo-[1,5-a]pyrimidine-3-carboxamide | 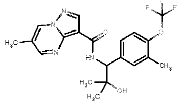 | 423.1 | |

**[Table 1-12]**

| | | | | |
|---|---|---|---|---|
| 97 | N-((1S)-1-(3-fluoro-4-(trifluoromethoxy)phenyl)-2-hydroxy-2-methylpropyl)-5-(3-methoxypyridin-4-yl)pyrazolo[1,5-a]pyrimidine-3-carboxamide | 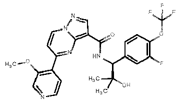 | 520.2 | |
| 98 | 5-(3-chloro-2-oxopyridin-1(2H)-yl)-N-((1S)-2-hydroxy-2-methyl-1-(4-(trifluoromethoxy)-phenyl)propyl)pyrazolo[1,5-a]pyrimidine-3-carboxamide | 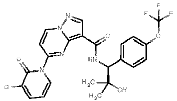 | 522.1 | |
| 99 | 5-(azetidin-1-yl)-N-((1S)-1-(3-fluoro-4-(trifluoromethoxy)phenyl)-2-hydroxy-2-methylpropyl)-6-methylpyrazolo[1,5-a]pyrimidine-3-carboxamide | 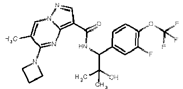 | 482.2 | |
| 100 | 5-(dimethylamino)-N-((1S)-1-(3-fluoro-4-(trifluoromethoxy)phenyl)-2-hydroxy-2-methylpropyl)-6-methylpyrazolo[1,5-a]pyrimidine-3-carboxamide | 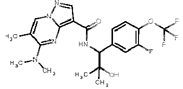 | 470.2 | |
| 101 | 5-(2-cyanophenyl)-N-((1S)-1-(3-fluoro-4-(trifluoromethoxy)-phenyl)-2-hydroxy-2-methylpropyl)pyrazolo[1,5-a]pyrimidine-3-carboxamide | 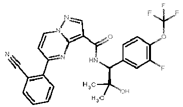 | 514.1 | |
| 102 | N-(1-(4-(difluoromethoxy)phenyl)-2-hydroxy-2-methylpropyl)-6-methylpyrazolo[1,5-a]pyrimidine-3-carboxamide | 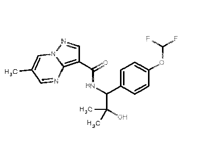 | 391.1 | |
| 103 | 5-cyclopropyl-N-((1S)-1-(3-fluoro-4-(trifluoromethoxy)-phenyl)-2-hydroxy-2-methylpropyl)-7-oxo-1,7-dihydropyrazolo[1,5-a]pyrimidine-3-carboxamide | 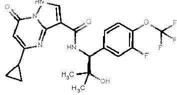 | 469.2 | |
| 104 | N-((1S)-1-(3-fluoro-4-(trifluoromethoxy)phenyl)-2-hydroxy-2-methylpropyl)-5-(1H-pyrazol-1-yl)pyrazolo[1,5-a]pyrimidine-3-carboxamide | 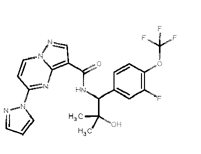 | 479.1 | |

**[Table 1-13]**

| | | | | |
|---|---|---|---|---|
| 105 | 5-ethyl-N-((1S)-1-(3-fluoro-4-(trifluoromethoxy)phenyl)-2-hydroxy-2-methylpropyl)-1-methyl-7-oxo-1,7-dihydropyrazolo[1,5-a]pyrimidine-3-carboxamide | 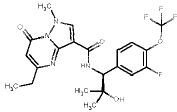 | 471.2 | |
| 106 | N-((1S)-1-(3-fluoro-4-(trifluoromethoxy)phenyl)-2-hydroxy-2-methylpropyl)-5-isopropyl-1-methyl-7-oxo-1,7-dihydropyrazolo[1,5-a]pyrimidine-3-carboxamide | 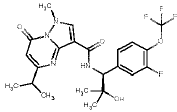 | 485.2 | |
| 107 | N-((1S)-2-hydroxy-2-methyl-1-(4-(trifluoromethoxy)phenyl)propyl)-5-(pyrimidin-5-yl)pyrazolo[1,5-a]pyrimidine-3-carboxamide | 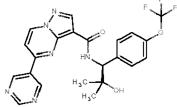 | 473.1 | |
| 108 | N-((1S)-2-hydroxy-2-methyl-1-(4-(trifluoromethoxy)phenyl)propyl)-5-(1-methyl-1H-pyrazol-3-yl)pyrazolo[1,5-a]pyrimidine-3-carboxamide | 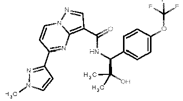 | 475.1 | |
| 109 | N-((1S)-2-hydroxy-2-methyl-1-(4-(trifluoromethoxy)phenyl)propyl)-5-(4-methyl-2H-1,2,3-triazol-2-yl)pyrazolo[1,5-a]pyrimidine-3-carboxamide | 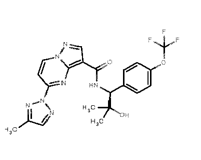 | 476.2 | |
| 110 | N-((1S)-2-hydroxy-2-methyl-1-(4-(trifluoromethoxy)phenyl)propyl)-6-methylpyrazolo[1,5-a]pyrimidine-3-carboxamide | 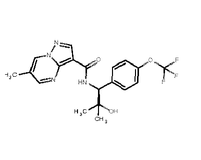 | 409.2 | |
| 111 | 2-amino-N-((1S)-2-hydroxy-2-methyl-1-(4-(trifluoromethoxy)-phenyl)propyl)-6-methylpyrazolo-[1,5-a]pyrimidine-3-carboxamide | 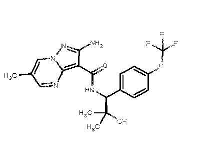 | 424.1 | |
| 112 | 2-amino-N-((1S)-1-(3-fluoro-4-(trifluoromethoxy)phenyl)-2-hydroxy-2-methylpropyl)-6-methylpyrazolo[1,5-a]pyrimidine-3-carboxamide | 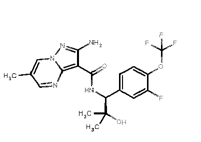 | 442.1 | |

**[Table 1-14]**

| | | | | |
|---|---|---|---|---|
| 113 | N-((1S)-2-hydroxy-2-methyl-1-(4-(trifluoromethoxy)phenyl)propyl)-5-((2R)-2-methylmorpholin-4-yl)pyrazolo[1,5-a]pyrimidine-3-carboxamide | 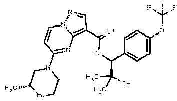 | 494.2 | |
| 114 | N-((1S)-2-hydroxy-2-methyl-1-(4-(trifluoromethoxy)phenyl)propyl)-5-(1,3-thiazol-2-yl)pyrazolo[1,5-a]pyrimidine-3-carboxamide | 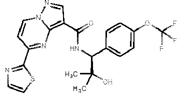 | 478.1 | |
| 115 | N-((1S)-2-hydroxy-2-methyl-1-(4-(trifluoromethoxy)phenyl)propyl)-5-(4-methyl-1H-imidazol-1-yl)pyrazolo[1,5-a]pyrimidine-3-carboxamide | 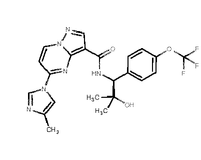 | 475.2 | |
| 116 | N-(2-methoxy-1-(4-(trifluoromethoxy)phenyl)ethyl)-6-methylpyrazolo[1,5-a]pyrimidine-3-carboxamide | 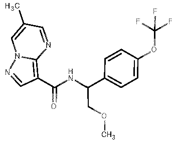 | 395.1 | |
| 117 | 5-((1S or 1R)-1-(difluoromethyl)-1H-pyrazol-4-yl)-N-(1-(3-fluoro-4-(trifluoromethyl)phenyl)-2-hydroxy-2-methylpropyl)-pyrazolo[1,5-a]pyrimidine-3-carboxamide | 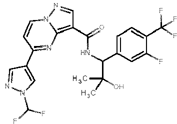 | 513.0 | |
| 118 | 5-((1R or 1S)-1-(difluoromethyl)-1H-pyrazol-4-yl)-N-(1-(3-fluoro-4-(trifluoromethyl)phenyl)-2-hydroxy-2-methylpropyl)-pyrazolo[1,5-a]pyrimidine-3-carboxamide | 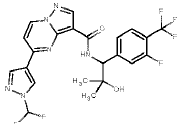 | 513.0 | |
| 119 | N-((1S)-2-hydroxy-2-methyl-1-(4-(trifluoromethoxy)phenyl)propyl)-5-(tetrahydro-2H-pyran-4-yl)pyrazolo[1,5-a]pyrimidine-3-carboxamide | 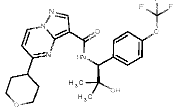 | 479.2 | |
| 120 | N-((1S)-2-hydroxy-2-methyl-1-(4-(trifluoromethoxy)phenyl)propyl)-5-(prop-1-en-2-yl)pyrazolo[1,5-a]pyrimidine-3-carboxamide | 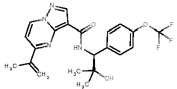 | 435.2 | |

**[Table 1-15]**

| | | | | |
|---|---|---|---|---|
| 121 | 5-(4,5-dimethyl-1H-1,2,3-triazol-1-yl)-N-((1S)-2-hydroxy-2-methyl-1-(4-(trifluoromethoxy)-phenyl)propyl)pyrazolo[1,5-a]pyrimidine-3-carboxamide | 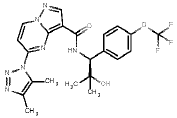 | 490.2 | |
| 122 | N-((1S)-2-hydroxy-2-methyl-1-(4-(trifluoromethoxy)phenyl)propyl)-5-(1H-1,2,4-triazol-1-yl)pyrazolo[1,5-a]pyrimidine-3-carboxamide | 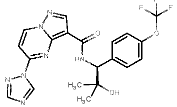 | 462.1 | |
| 123 | 5-(2,4-dimethyl-1H-imidazol-1-yl)-N-((1S)-2-hydroxy-2-methyl-1-(4-(trifluoromethoxy)-phenyl)propyl)pyrazolo[1,5-a]pyrimidine-3-carboxamide | 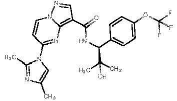 | 489.2 | |
| 124 | 5-(2,2-dimethylmorpholin-4-yl)-N-((1S)-2-hydroxy-2-methyl-1-(4-(trifluoromethoxy)phenyl)propyl)-pyrazolo[1,5-a]pyrimidine-3-carboxamide | 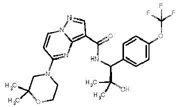 | 508.2 | |
| 125 | N-(1-(4-(difluoromethoxy)-3-fluorophenyl)-2-hydroxy-2-methylpropyl)-5-(6-methylpyridin-3-yl)pyrazolo[1,5-a]pyrimidine-3-carboxamide | 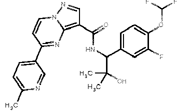 | 486.1 | |
| 126 | 2-chloro-N-((1S)-2-hydroxy-2-methyl-1-(4-(trifluoromethoxy)-phenyl)propyl)-6-methylpyrazolo-[1,5-a]pyrimidine-3-carboxamide | 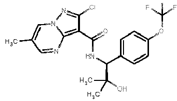 | 443.1 | |
| 127 | N-((1S)-1-(3-fluoro-4-(trifluoromethoxy)phenyl)-2-hydroxy-2-methylpropyl)-5-(pyridin-2-yl)pyrazolo[1,5-a]pyrimidine-3-carboxamide | 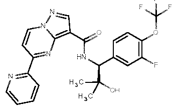 | 490.1 | |
| 128 | N-(1-(4-(difluoromethoxy)-3-fluorophenyl)-2-hydroxy-2-methylpropyl)-5-(2-methoxypyridin-3-yl)pyrazolo[1,5-a]pyrimidine-3-carboxamide | 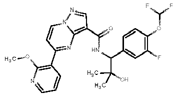 | 502.1 | |

**[Table 1-16]**

| | | | | |
|---|---|---|---|---|
| 129 | N-((1S)-2-hydroxy-2-methyl-1-(4-(trifluoromethoxy)phenyl)propyl)-5-(3-methoxypyridin-4-yl)pyrazolo[1,5-a]pyrimidine-3-carboxamide | 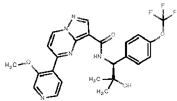 | 502.1 | |
| 130 | N-((1S)-1-(3-fluoro-4-(trifluoromethoxy)phenyl)-2-hydroxy-2-methylpropyl)-1,5,6-trimethyl-7-oxo-1,7-dihydropyrazolo[1,5-a]pyrimidine-3-carboxamide | 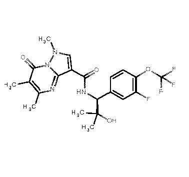 | 471.2 | |
| 131 | N-((1S)-1-(3-fluoro-4-(trifluoromethoxy)phenyl)-2-hydroxy-2-methylpropyl)-1,5-dimethyl-7-oxo-1,7-dihydropyrazolo[1,5-a]pyrimidine-3-carboxamide | 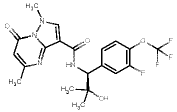 | 457.2 | |
| 132 | 5-(2-chloro-1H-imidazol-1-yl)-N-((1S)-2-hydroxy-2-methyl-1-(4-(trifluoromethoxy)phenyl)propyl)-6-methylpyrazolo[1,5-a]pyrimidine-3-carboxamide | 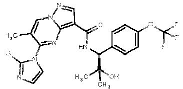 | 509.2 | |
| 133 | N-((1S)-1-(3-fluoro-4-(trifluoromethoxy)phenyl)-2-hydroxy-2-methylpropyl)-5-(3-methyl-1H-1,2,4-triazol-1-yl)pyrazolo[1,5-a]pyrimidine-3-carboxamide | 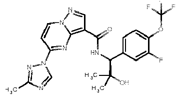 | 494.1 | |
| 134 | N-((1S)-1-(3-fluoro-4-(trifluoromethoxy)phenyl)-2-hydroxy-2-methylpropyl)-5-(1H-1,2,4-triazol-1-yl)pyrazolo[1,5-a]pyrimidine-3-carboxamide | 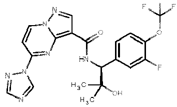 | 480.1 | |
| 135 | 5-(3,3-dimethyl-2-oxopyrrolidin-1-yl)-N-((1S)-2-hydroxy-2-methyl-1-(4-(trifluoromethoxy)-phenyl)propyl)pyrazolo[1,5-a]pyrimidine-3-carboxamide | 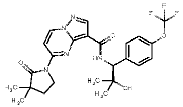 | 506.1 | |
| 136 | N-(1-(2-fluoro-4-(trifluoro-methoxy)phenyl)-2-hydroxy-2-methylpropyl)-5-(4-methyl-1H-imidazol-1-yl)pyrazolo[1,5-a]pyrimidine-3-carboxamide | 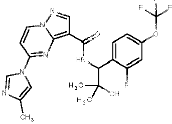 | 493.1 | |

**[Table 1-17]**

| | | | | |
|---|---|---|---|---|
| 137 | N-(2-hydroxy-2-methyl-1-(3-methyl-4-(trifluoromethoxy)-phenyl)propyl)-5-(6-methylpyridin-3-yl)pyrazolo[1,5-a]pyrimidine-3-carboxamide | 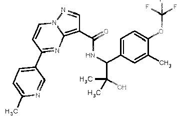 | 500.2 | |
| 138 | N-((1S)-2-hydroxy-2-methyl-1-(4-(trifluoromethoxy)phenyl)propyl)-6-methyl-5-(2H-1,2,3-triazol-2-yl)pyrazolo[1,5-a]pyrimidine-3-carboxamide | 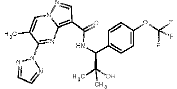 | 476.2 | |
| 139 | N-((1S)-2-hydroxy-2-methyl-1-(4-(trifluoromethoxy)phenyl)propyl)-5-(1H-tetrazol-1-yl)pyrazolo[1,5-a]pyrimidine-3-carboxamide | 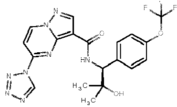 | 463.0 | |
| 140 | 6-fluoro-N-((1S)-1-(3-fluoro-4-(trifluoromethoxy)phenyl)-2-hydroxy-2-methylpropyl)-5-methylpyrazolo[1,5-a]pyrimidine-3-carboxamide | 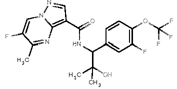 | 445.1 | |
| 141 | N-((1S)-2-hydroxy-2-methyl-1-(4-(trifluoromethoxy)phenyl)propyl)-5-(2-methyl-1H-imidazol-1-yl)pyrazolo[1,5-a]pyrimidine-3-carboxamide | 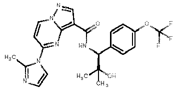 | 475.2 | |
| 142 | N-((1S or 1R)-1-(4-(difluoromethoxy)-3-fluorophenyl)-2-hydroxy-2-methylpropyl)-6-methylpyrazolo-[1,5-a]pyrimidine-3-carboxamide | 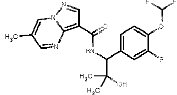 | 409.1 | |
| 143 | N-((1R or 1S)-1-(3-fluoro-4-(trifluoromethyl)phenyl)-2-hydroxy-2-methylpropyl)-6-methylpyrazolo[1,5-a]pyrimidine-3-carboxamide | 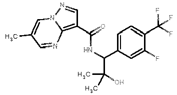 | 411.1 | |
| 144 | 6-chloro-N-((1S)-1-(3-fluoro-4-(trifluoromethoxy)phenyl)-2-hydroxy-2-methylpropyl)-1,5-dimethyl-7-oxo-1,7-dihydropyrazolo[1,5-a]pyrimidine-3-carboxamide | 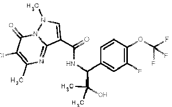 | 491.1 | |

**[Table 1-18]**

| | | | | |
|---|---|---|---|---|
| 145 | N-((1S)-1-(3-fluoro-4-(trifluoromethoxy)phenyl)-2-hydroxy-2-methylpropyl)-1,6-dimethyl-7-oxo-1,7-dihydropyrazolo[1,5-a]pyrimidine-3-carboxamide | 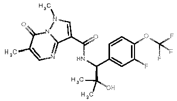 | 457.1 | |
| 146 | N-(1-(4-(difluoromethoxy)-3-fluorophenyl)-2-hydroxy-2-methylpropyl)-1,5,6-trimethyl-7-oxo-1,7-dihydropyrazolo[1,5-a]pyrimidine-3-carboxamide | 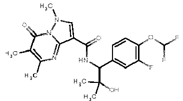 | 453.2 | |
| 147 | 5-(2-chloro-1H-imidazol-1-yl)-N-((1S)-2-hydroxy-2-methyl-1-(4-(trifluoromethoxy)phenyl)propyl)-pyrazolo[1,5-a]pyrimidine-3-carboxamide | 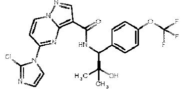 | 495.2 | |
| 148 | N-(1-(4-(difluoromethoxy)-3-fluorophenyl)-2-hydroxy-2-methylpropyl)-5-(1-(difluoromethyl)-1H-pyrazol-4-yl)pyrazolo[1,5-a]pyrimidine-3-carboxamide | 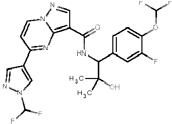 | 511.1 | |
| 149 | N-((1S)-2-hydroxy-2-methyl-1-(4-(trifluoromethoxy)phenyl)propyl)-5-(2-methylpyridin-4-yl)pyrazolo[1,5-a]pyrimidine-3-carboxamide | 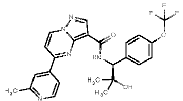 | 486.1 | |
| 150 | N-((1S)-1-(3-fluoro-4-(trifluoromethoxy)phenyl)-2-hydroxy-2-methylpropyl)-5-(6-methylpyridin-3-yl)pyrazolo[1,5-a]pyrimidine-3-carboxamide | 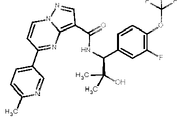 | 504.1 | |
| 151 | 2-chloro-N-((1S)-1-(3-fluoro-4-(trifluoromethoxy)phenyl)-2-hydroxy-2-methylpropyl)-6-methylpyrazolo[1,5-a]pyrimidine-3-carboxamide | 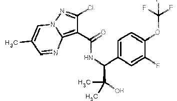 | 461.1 | |
| 152 | N-((1S)-1-(3-fluoro-4-(trifluoromethoxy)phenyl)-2-methoxyethyl)-7-methoxy-2-oxo-1,2-dihydro-1,5-naphthyridine-4-carboxamide | 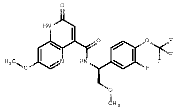 | 456.2 | |

**[Table 1-19]**

| | | | | |
|---|---|---|---|---|
| 153 | N-((1S)-1-(3-fluoro-4-(trifluoromethoxy)phenyl)-2-methoxyethyl)-5-(3-methoxypyridin-4-yl)pyrazolo[1,5-a]pyrimidine-3-carboxamide | 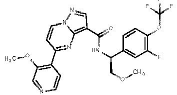 | 506.1 | |
| 154 | 5-(3,5-dimethyl-1,2-oxazol-4-yl)-N-((1S)-2-hydroxy-2-methyl-1-(4-(trifluoromethoxy)phenyl)propyl)-pyrazolo[1,5-a]pyrimidine-3-carboxamide | 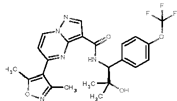 | 490.2 | |
| 155 | (1S)-2-methyl-1-(((6-methyl-5-(4-methyl-1H-1,2,3-triazol-1-yl)pyrazolo[1,5-a]pyrimidin-3-yl)carbonyl)amino)-1-(4-(trifluoromethoxy)phenyl)propan-2-yl acetate | 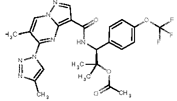 | 532.2 | |
| 156 | N-((1S)-1-(3-fluoro-4-(trifluoromethoxy)phenyl)-2-hydroxy-2-methylpropyl)-5-(1H-1,2,3-triazol-1-yl)pyrazolo[1,5-a]pyrimidine-3-carboxamide | 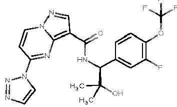 | 480.0 | |
| 157 | N-((1S)-2-hydroxy-2-methyl-1-(4-(trifluoromethoxy)phenyl)propyl)-5-(6-methoxypyridin-2-yl)pyrazolo[1,5-a]pyrimidine-3-carboxamide | 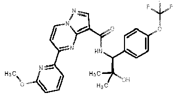 | 502.1 | |
| 158 | N-((1S)-1-(3-fluoro-4-(trifluoromethoxy)phenyl)-2-hydroxy-2-methylpropyl)-5-(2-methoxypyridin-3-yl)pyrazolo[1,5-a]pyrimidine-3-carboxamide | 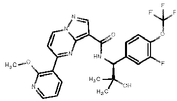 | 520.1 | |
| 159 | 5-(3-fluoropyridin-4-yl)-N-((1S)-1-(3-fluoro-4-(trifluoro-methoxy)phenyl)-2-methoxyethyl)-pyrazolo[1,5-a]pyrimidine-3-carboxamide | 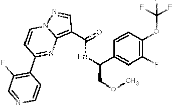 | 494.0 | |
| 160 | N-((1S)-2-hydroxy-2-methyl-1-(4-(trifluoromethoxy)phenyl)propyl)-5-(pyrazin-2-yl)pyrazolo[1,5-a]pyrimidine-3-carboxamide | 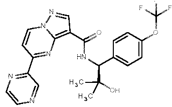 | 473.1 | |

**[Table 1-20]**

| | | | | |
|---|---|---|---|---|
| 161 | N-((1S)-2-hydroxy-2-methyl-1-(4-(trifluoromethoxy)phenyl)propyl)-6-methyl-5-(3-methyl-1H-1,2,4-triazol-1-yl)pyrazolo[1,5-a]pyrimidine-3-carboxamide | 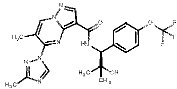 | 490.3 | |
| 162 | N-((1S)-2-hydroxy-2-methyl-1-(4-(trifluoromethoxy)phenyl)propyl)-6-methyl-5-(1H-pyrazol-1-yl)pyrazolo[1,5-a]pyrimidine-3-carboxamide | 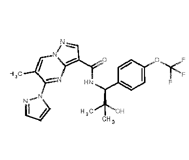 | 475.3 | |
| 163 | 8-bromo-N-((1S)-2-hydroxy-2-methyl-1-(4-(trifluoromethoxy)-phenyl)propyl)-1,6-naphthyridine-2-carboxamide | 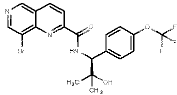 | 484.1 | |
| 164 | N-((1S)-2-hydroxy-2-methyl-1-(4-(trifluoromethoxy)phenyl)propyl)-8-phenyl-1,6-naphthyridine-2-carboxamide | 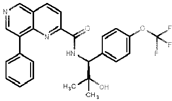 | 482.3 | |
| 165 | N-(1-(4-(difluoromethoxy)phenyl)-2-hydroxy-2-methylpropyl)-5-(1-(difluoromethyl)-1H-pyrazol-4-yl)pyrazolo[1,5-a]pyrimidine-3-carboxamide | 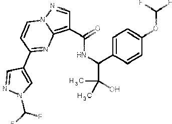 | 493.2 | |
| 166 | N-((1S or 1R)-1-(4-(difluoromethoxy)phenyl)-2-hydroxy-2-methylpropyl)-5-(1-(difluoromethyl)-1H-pyrazol-4-yl)pyrazolo[1,5-a]pyrimidine-3-carboxamide | 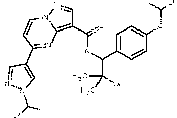 | 493.2 | |
| 167 | N-((1R or 1S)-1-(4-(difluoromethoxy)phenyl)-2-hydroxy-2-methylpropyl)-5-(1-(difluoromethyl)-1H-pyrazol-4-yl)pyrazolo[1,5-a]pyrimidine-3-carboxamide | 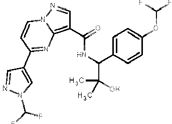 | 493.2 | |

Experimental Example 1

### PDE enzyme inhibitory assay

Human PDE2A3 full-length gene was transduced into Sf9, and human PDE2A3 enzyme was purified by His-tag affinity column and gel filtration. The enzyme was stored at -70°C until use. PDE activity was measured using a SPA (Scintillation Proximity Assay) (GE Healthcare). To evaluate the inhibitory activity of the compound, 10 µl of serial diluted compounds were reacted with 20 µl of PDE enzyme in assay buffer (50 mM HEPES-NaOH, 8.3 mM MgCl₂, 1.7 mM EGTA, 0.1 % BSA (pH 7.4)) for 30 min. at room temperature. Final concentration of DMSO in the reaction solution was 1 percent. Compounds were tested in duplicate in 96-well half-area plates (Corning) or 384-well OptiPlate (PerkinElmer). To start the reaction, 10 µl of substrate [³H] cGMP (final concentration 77 nM, PerkinElmer) was added to a total volume of 40 µl. After reaction for 60 min at room temperature, 20 µl of 20 mg/mL yttrium SPA beads containing zinc sulfate was added to terminate the PDE reaction. After being settled for further 1 hour, the assay plates were counted in a scintillation counter (PerkinElmer) to allow calculation of inhibition rate. Inhibition rate was calculated on the basis of 0% control wells with enzyme and DMSO, and 100% control wells without enzyme. The results are shown in Table 2.

**Table 2**

| Example No. | inhibitory rate (%) (1 µM) |
|---|---|
| 1 | 99 |
| 12 | 99 |
| 29 | 100 |
| 38 | 98 |
| 59 | 101 |
| 66 | 98 |
| 69 | 100 |
| 70 | 101 |
| 72 | 100 |
| 77 | 100 |
| 79 | 101 |
| 80 | 100 |
| 83 | 100 |
| 92 | 100 |
| 101 | 100 |
| 115 | 101 |
| 124 | 99 |
| 135 | 100 |
| 152 | 100 |
| 161 | 100 |
| 164 | 97 |

### Experimental Example 2

Improvement effect on MK-801 ((5S,10R)-5-methyl-10,11-dihydro-5H-5,10-epiminodibenzo[a,d][7]annulene)-induced disorder in contextual fear conditioning test.

### Experimental Animals

Male C57BL/6 mice were purchased from CLEA Japan, Inc. They were used for an experiment after a habituation period of at least one week after carrying them in the animal experimental facility. They were bred in the animal experimental facility with environment in which 12-hours light-dark cycle was performed, humidity and temperature were controlled, and free water drinking and feeding were allowed. Handling of experimental animals and experimental procedure of this study were approved by the Experimental Animal Ethics Committee of Takeda Pharmaceutical Company Limited.

### Drugs used

A test compound was suspended in 0.5% methylcellulose solution at a dose of 30 mg/kg, and orally administered. MK-801 (maleate) (Sigma-Aldrich, St Louis, MO) was dissolved in saline at a dose of 0.08 mg/kg, and subcutaneously administered. All drugs were administered to mice at a dose of 10 mL/kg.

Improvement effect on MK-801-induced deficits in contextual fear conditioning test.

Contextual fear conditioning test is widely used as memory and learning test system depending on the hippocampus and amygdala. The improvement effect of test compounds on MK-801-induced deficits was investigated by contextual fear conditioning test. The test compounds and MK-801 (maleate) were administered 60 min and 30 min, respectively, before the test. 0.5% methylcellulose and saline were administered to the control groups 60 min and 30 min, respectively, before the test. 0.5% methylcellulose and MK-801 (maleate) were administered to the vehicle groups 60 min and 30 min, respectively, before the test. In experiments, a chamber with a floor grid for electric shock and a freezing measuring device (O'Hara & Co., Ltd.) were used. The chamber was put in a soundproof box to block the noise from outside, and tests were performed. In the training session on day 1 of the experiment, after habituation for 3 minutes in the chamber, two electric footshocks were given to mice at 1-minute interval. In the test session on day 2 of the experiment, mice were placed in the same chamber for 7 minutes, and freezing was measured during that period. The freezing was calculated as a percentage of freezing rates during measuring period with use of an automatic analysis software manufactured by the O'Hara & Co., Ltd. All data were shown as the mean + standard error (n = 10). A comparison between two groups was performed by Student's t-test (^{*}p ≤ 0.05, comparison with control group. ^{#}p ≤ 0.05, comparison with group treated with MK-801 alone.). The results are shown in Fig. 1. compound A is the compound (30 mg/kg, p.o.) obtained in Example 69-II, compound B is the compound (30 mg/kg, p.o.) obtained in Example 80-II, and compound C is the compound (30 mg/kg, p.o.) obtained in Example 161.

The improvement effect on MK-801-induced deficits was shown by orally administering each of test compounds (A, B, C) to mice 60 minutes before the tests.

**Formulation Example 1**

| | |
|---|---|
| (1) compound of Example 1 | 10.0 g |
| (2) lactose | 70.0 g |
| (3) cornstarch | 50.0 g |
| (4) soluble starch | 7.0 g |
| (5) magnesium stearate | 3.0 g |

The compound of Example 1 (10.0 g) and magnesium stearate (3.0 g) are granulated in aqueous solution (70 mL) of soluble starch (7.0 g as soluble starch) and then dried, the resulting mixture is mixed with lactose (70.0 g) and cornstarch (50.0 g) (lactose, cornstarch, soluble starch and magnesium stearate are all products in compliance with Japanese Pharmacopoeia 14^{th} Edition). The mixture compressed to give tablets.

### INDUSTRIAL APPLICABILITY

According to the present invention, a compound having a PDE2A selective inhibitory action, and useful as a prophylactic or therapeutic drug for schizophrenia, Alzheimer's disease and the like can be provided.

This application is based on patent application No. 2013-153880 filed in Japan (filing date: July 24, 2013), No. 2013-220193 filed in Japan (filing date: October 23, 2013), and No. 2014-104701 filed in Japan (filing date: May 20, 2014), the contents of which are incorporated in full herein.

## Claims

1. A compound represented by the formula (I): 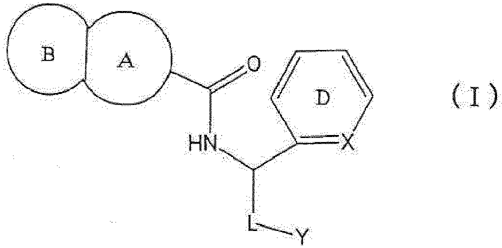 wherein
ring A is an optionally further substituted 5- or 6-membered nitrogen-containing heterocycle, ring B is an optionally substituted 5- or 6-membered nitrogen-containing heterocycle, and fused ring AB is an optionally further substituted heterocycle having two or more nitrogen atoms as ring-constituting atoms besides carbon atom, and optionally having 1 or 2 hetero atoms selected from an oxygen atom and a sulfur atom, said fused ring AB is aromatic,
ring D is a benzene ring or a pyridine ring, each of which further has a substituent,
X is a carbon atom or a nitrogen atom,
L is a bond or an optionally substituted C₁₋₂ alkylene group, and
Y is
(1) a group represented by the formula: 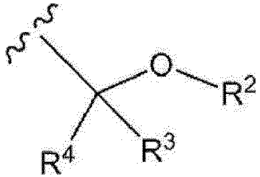 wherein R² and R³ are each independently a hydrogen atom or a substituent, R⁴ is a substituent, or R³ and R⁴ optionally form, together with the adjacent carbon atom, an optionally further substituted ring,
or
(2) a group represented by the formula -CH₂-O-R¹ wherein R¹ is a substituent,
(excluding N-[1-(4-fluorophenyl)-2-hydroxy-2,2-di(pyridin-3-yl)ethyl]-pyrazolo[1,5-a]pyridine-2-carboxamide) or a salt thereof.

2. The compound of claim 1, wherein Y is
(1) a group represented by the formula: 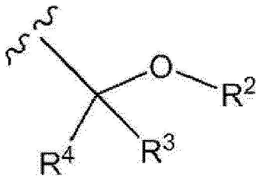
wherein R² is a hydrogen atom or a C₁₋₆ alkyl-carbonyl group;
R³ is a C₁₋₆ alkyl group; and
R⁴ is a C₁₋₆ alkyl group, or
(2) a group represented by the formula -CH₂-O-R¹ wherein R¹ is a C₁₋₆ alkyl group,
or a salt thereof.

3. The compound of claim 1, wherein ring A is a 5- or 6-membered nitrogen-containing heterocycle optionally substituted by 1 to 3 substituents selected from
(1) a hydroxy group,
(2) a C₁₋₆ alkyl group,
(3) a halogen atom, and
(4) an amino group
(excluding a pyrrole ring),
or a salt thereof.

4. The compound of claim 1, wherein ring B is a 5- or 6-membered nitrogen-containing heterocycle optionally substituted by 1 to 3 substituents selected from
(1) a C₁₋₆ alkyl group optionally substituted by 1 to 3 C₁₋₆ alkoxy groups,
(2) a halogen atom,
(3) a hydroxy group,
(4) a C₂₋₆ alkenyl group,
(5) a C₃₋₈ cycloalkyl group,
(6) a di-C₁₋₆ alkyl-amino group,
(7) a heterocyclic group optionally substituted by 1 to 3 substituents selected from
(i) a C₁₋₆ alkoxy group,
(ii) a halogen atom,
(iii) a C₁₋₆ alkyl group optionally substituted by 1 to 3 halogen atoms,
(iv) a C₁₋₆ alkoxy group,
(v) an oxo group, and
(vi) a cyano group,
(8) an oxo group,
(9) a phenyl group optionally substituted by a cyano group, and
(10) a C₁₋₆ alkoxy group
(excluding a pyrazine ring),
or a salt thereof.

5. The compound of claim 1, wherein fused ring AB is
(I)
a 1,5-naphthyridine ring,
a 1,6-naphthyridine ring,
a pyrazolo[1,5-a]pyrimidine ring,
a triazolo[1,5-a]pyrimidine ring,
an imidazo[4,5-b]pyridine ring,
a purine ring, or
a pyrazolo[4,3-c]pyridine ring, each of which is optionally substituted by 1 to 3 substituents selected from
(1) a hydroxy group,
(2) a halogen atom,
(3) a C₁₋₆ alkyl group,
(4) a C₂₋₆ alkenyl group,
(5) a C₃₋₈ cycloalkyl group,
(6) an amino group,
(7) a di-C₁₋₆ alkyl-amino group,
(8) a heterocyclic group optionally substituted by 1 to 3 substituents selected from
(i) a C₁₋₆ alkoxy group,
(ii) a halogen atom,
(iii) a C₁₋₆ alkyl group optionally substituted by 1 to 3 halogen atoms,
(iv) a C₁₋₆ alkoxy group,
(v) an oxo group, and
(iv) a cyano group,
(9) a C₆₋₁₄ aryl group optionally substituted by a cyano group, and
(10) a C₁₋₆ alkoxy group,
or
(II) a 7-oxo-1,7-dihydroa pyrazolo[1,5-a]pyrimidine ring optionally substituted by 1 to 3 substituents selected from
(1) a C₁₋₆ alkyl group optionally substituted by one C₁₋₆ alkoxy group,
(2) a C₃₋₈ cycloalkyl group,
(3) a heterocyclic group, and
(4) a halogen atom,
or a salt thereof.

6. The compound of claim 1, wherein ring D is a benzene ring substituted by 1 to 3 substituents selected from
(1) a C₁₋₆ alkoxy group optionally substituted by 1 to 5 halogen atoms,
(2) a halogen atom,
(3) a heterocyclic group, and
(4) a C₁₋₆ alkyl group optionally substituted by 1 to 3 halogen atoms,
or a salt thereof.

7. The compound of claim 1, wherein ring A is a 5- or 6-membered nitrogen-containing heterocycle optionally substituted by 1 to 3 substituents selected from
(1) a hydroxy group,
(2) a C₁₋₆ alkyl group,
(3) a halogen atom, and
(4) an amino group
(excluding a pyrrole ring);
ring B is a 5- or 6-membered nitrogen-containing heterocycle optionally substituted by 1 to 3 substituents selected from
(1) a C₁₋₆ alkyl group optionally substituted by 1 to 3 C₁₋₆ alkoxy groups,
(2) a halogen atom,
(3) a hydroxy group,
(4) a C₂₋₆ alkenyl group,
(5) a C₃₋₈ cycloalkyl group,
(6) a di-C₁₋₆ alkyl-amino group,
(7) a heterocyclic group optionally substituted by 1 to 3 substituents selected from
(i) a C₁₋₆ alkoxy group,
(ii) a halogen atom,
(iii) a C₁₋₆ alkyl group optionally substituted by 1 to 3 halogen atoms,
(iv) a C₁₋₆ alkoxy group,
(v) an oxo group, and
(iv) a cyano group,
(8) an oxo group,
(9) a phenyl group optionally substituted by a cyano group, and
(10) a C₁₋₆ alkoxy group
(excluding a pyrazine ring);
fused ring AB is
(I)
a 1,5-naphthyridine ring,
a 1,6-naphthyridine ring,
a pyrazolo[1,5-a]pyrimidine ring,
a triazolo[1,5-a]pyrimidine ring,
an imidazo[4,5-b]pyridine ring,
a purine ring, or
a pyrazolo[4,3-c]pyridine ring, each of which is optionally substituted by 1 to 3 substituents selected from
(1) a hydroxy group,
(2) a halogen atom,
(3) a C₁₋₆ alkyl group,
(4) a C₂₋₆ alkenyl group,
(5) a C₃₋₈ cycloalkyl group,
(6) an amino group,
(7) a di-C₁₋₆ alkyl-amino group,
(8) a heterocyclic group optionally substituted by 1 to 3 substituents selected from
(i) a C₁₋₆ alkoxy group,
(ii) a halogen atom,
(iii) a C₁₋₆ alkyl group optionally substituted by 1 to 3 halogen atoms,
(iv) a C₁₋₆ alkoxy group,
(v) an oxo group, and
(iv) a cyano group,
(9) a C₅₋₁₄ aryl group optionally substituted by a cyano group, and
(10) a C₁₋₆ alkoxy group,
or
(II) a 7-oxo-1,7-dihydroa pyrazolo[1,5-a]pyrimidine ring optionally substituted by 1 to 3 substituents selected from
(1) a C₁₋₆ alkyl group optionally substituted by one C₁₋₆ alkoxy group,
(2) a C₃₋₈ cycloalkyl group,
(3) a heterocyclic group, and
(4) a halogen atom;
ring D is a benzene ring substituted by 1 to 3 substituents selected from
(1) a C₁₋₆ alkoxy group optionally substituted by 1 to 5 halogen atoms,
(2) a halogen atom,
(3) a heterocyclic group, and
(4) a C₁₋₆ alkyl group optionally substituted by 1 to 3 halogen atoms;
X is a carbon atom;
L is a bond or methylene; and
Y is
(1) a group represented by the formula: 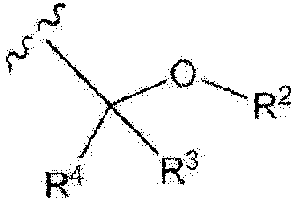
wherein R² is a hydrogen atom or a C₁₋₆ alkyl-carbonyl group;
R³ is a C₁₋₆ alkyl group; and
R⁴ is a C₁₋₆ alkyl group, or
(2) a group represented by the formula -CH₂-O-R¹ wherein R¹ is a C₁₋₆ alkyl group,
or a salt thereof.

8. The compound of claim 1, wherein ring A is a pyrazole ring, a pyridine ring, a pyrimidine ring, a dihydropyrazole ring, or a dihydropyridine ring, each of which is optionally substituted by 1 to 3 substituents selected from
(1) a hydroxy group,
(2) a C₁₋₆ alkyl group,
(3) a halogen atom, and
(4) an amino group;
ring B is
a pyridine ring,
a pyrimidine ring,
a pyrazole ring,
a triazole ring,
dihydropyrimidine ring, or
an imidazole ring, each of which is optionally substituted by 1 to 3 substituents selected from
(1) a C₁₋₆ alkyl group optionally substituted by 1 to 3 C₁₋₆ alkoxy groups,
(2) a halogen atom,
(3) a hydroxy group,
(4) a C₂₋₆ alkenyl group,
(5) a C₃₋₈ cycloalkyl group,
(6) a di-C₁₋₆ alkyl-amino group,
(7)
an azetidinyl group,
a pyrrolidinyl group,
a piperidyl group,
a morpholinyl group,
a 3-oxa-8-azabicyclo[3.2.1]octyl group,
a 3-oxa-6-azabicyclo[3.1.1]heptyl group,
a 6-oxa-3-azabicyclo[3.1.1]heptyl group,
a pyrazolyl group,
a triazolyl group,
a pyridyl group,
a pyridazinyl group,
a thiazolyl group,
an imidazolyl group,
a tetrahydropyranyl group,
a tetrazolyl group,
a pyrazinyl group,
an isoxazolyl group,
a dihydropyridyl group, or
a pyrimidinyl group, each of which is optionally substituted by 1 to 3 substituents selected from
(i) a C₁₋₆ alkoxy group,
(ii) a halogen atom,
(iii) a C₁₋₆ alkyl group optionally substituted by 1 to 3 halogen atoms,
(iv) a C₁₋₆ alkoxy group,
(v) an oxo group, and
(vi) a cyano group,
(8) an oxo group,
(9) a phenyl group optionally substituted by a cyano group, and
(10) a C₁₋₆ alkoxy group;
fused ring AB is
(I)
a 1,5-naphthyridine ring,
a 1,6-naphthyridine ring,
a pyrazolo[1,5-a]pyrimidine ring,
a triazolo[1,5-a]pyrimidine ring, an imidazo[4,5-b]pyridine ring,
a purine ring, or
a pyrazolo[4,3-c]pyridine ring, each of which is optionally substituted by 1 to 3 substituents selected from
(1) a hydroxy group,
(2) a halogen atom,
(3) a C₁₋₆ alkyl group,
(4) a C₂₋₆ alkenyl group,
(5) a C₃₋₈ cycloalkyl group,
(6) an amino group,
(7) a di-C₁₋₆ alkyl-amino group,
(8) an azetidinyl group,
a pyrrolidinyl group,
a piperidyl group,
a morpholinyl group,
a 3-oxa-8-azabicyclo[3.2.1]octyl group,
a 3-oxa-6-azabicyclo[3.1.1]heptyl group,
a 6-oxa-3-azabicyclo[3.1.1]heptyl group,
a pyrazolyl group,
an imidazolyl group,
a triazolyl group,
a pyridyl group,
a dihydropyridyl group,
a pyridazinyl group,
a pyrimidinyl group,
a thiazolyl group,
a tetrahydropyranyl group,
a tetrazolyl group,
a pyrazinyl group, or
an isoxazolyl group, each of which is optionally substituted by 1 to 3 substituents selected from
(i) a C₁₋₆ alkoxy group,
(ii) a halogen atom,
(iii) a C₁₋₆ alkyl group optionally substituted by 1 to 3 halogen atoms,
(iv) a C₁₋₆ alkoxy group, and
(v) an oxo group,
(9) a phenyl group optionally substituted by a cyano group, and
(10) a C₁₋₆ alkoxy group,
or
(II) a 7-oxo-1,7-dihydroa pyrazolo[1,5-a]pyrimidine ring optionally substituted by 1 to 3 substituents selected from
(1) a C₁₋₆ alkyl group optionally substituted by one C₁₋₆ alkoxy group,
(2) a C₃₋₈ cycloalkyl group,
(3) a pyridyl group, and
(4) a halogen atom;
ring D is a benzene ring substituted by 1 to 3 substituents selected from
(1) a C₁₋₆ alkoxy group optionally substituted by 1 to 3 halogen atoms,
(2) a halogen atom,
(3) a pyrazolyl group, and
(4) a C₁₋₆ alkyl group optionally substituted by 1 to 3 halogen atoms;
X is a carbon atom;
L is a bond or methylene; and
Y is
(1) a group represented by the formula: 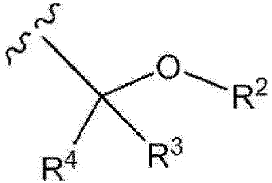
wherein R² is a hydrogen atom or a C₁₋₆ alkyl-carbonyl group;
R³ is a C₁₋₆ alkyl group; and
R⁴ is a C₁₋₆ alkyl group, or
(2) a group represented by the formula -CH₂-O-R¹ wherein R¹ is a C₁₋₆ alkyl group,
or a salt thereof.

9. The compound of claim 1, wherein ring A is a pyrazole ring, a pyridine ring, or a dihydropyridine ring each of which is optionally substituted by 1 to 3 substituents selected from
(1) a hydroxy group, and
(2) a C₁₋₆ alkyl group;
ring B is
a pyridine ring, or
a pyrimidine ring, each of which is optionally substituted by 1 to 3 substituents selected from
(1) a C₁₋₆ alkyl group,
(2) a C₃₋₈ cycloalkyl group,
(3) a di-C₁₋₆ alkyl-amino group,
(4) an azetidinyl group,
a pyrrolidinyl group,
a morpholinyl group,
a pyrazolyl group,
a triazolyl group,
a pyridyl group, or
an imidazolyl group, each of which is optionally substituted by 1 to 3 substituents selected from
(i) a C₁₋₆ alkyl group optionally substituted by 1 to 3 halogen atoms, and
(ii) an oxo group,
(5) an oxo group,
(6) a phenyl group optionally substituted by a cyano group, and
(7) a C₁₋₆ alkoxy group;
fused ring AB is
(I)
a 1,5-naphthyridine ring,
a 1,6-naphthyridine ring, or
a pyrazolo[1,5-a]pyrimidine ring, each of which is optionally substituted by 1 to 3 substituents selected from
(1) a hydroxy group,
(2) a C₁₋₆ alkyl group,
(3) a C₃₋₈ cycloalkyl group,
(4) a di-C₁₋₆ alkyl-amino group,
(5) an azetidinyl group,
a pyrrolidinyl group,
a morpholinyl group,
a pyrazolyl group,
an imidazolyl group,
a triazolyl group, or
a pyridyl group, each of which is optionally substituted by 1 to 3 substituents selected from
(i) a C₁₋₆ alkyl group optionally substituted by 1 to 3 halogen atoms, and
(ii) an oxo group,
(9) a phenyl group optionally substituted by a cyano group, and
(10) a C₁₋₆ alkoxy group,
or
(II) a 7-oxo-1,7-dihydroa pyrazolo[1,5-a]pyrimidine ring optionally substituted by 1 to 3 substituents selected from
(1) a C₁₋₆ alkyl group,
(2) a C₃₋₈ cycloalkyl group, and
(3) a pyridyl group;
ring D is a benzene ring substituted by 1 to 3 substituents selected from
(1) a C₁₋₆ alkoxy group optionally substituted by 1 to 3 halogen atoms, and
(2) a halogen atom;
X is a carbon atom;
L is a bond or methylene; and
Y is
(1) a group represented by the formula: 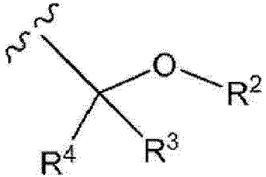
wherein R² is a hydrogen atom;
R³ is a C₁₋₆ alkyl group; and
R⁴ is a C₁₋₆ alkyl group, or
(2) a group represented by the formula -CH₂-O-R¹ wherein R¹ is a C₁₋₆ alkyl group,
or a salt thereof.

10. The compound of claim 1, wherein ring A is a pyrazole ring;
ring B is a pyrimidine ring substituted by 1 to 3 substituents selected from
(1) a C₁₋₆ alkyl group,
(2) a triazolyl group optionally substituted by 1 to 3 C₁₋₆ alkyl groups, and
(3) a pyridyl group optionally substituted by 1 to 3 C₁₋₆ alkyl groups;
fused ring AB is a pyrazolo[1,5-a]pyrimidine ring optionally substituted by 1 to 3 substituents selected from
(1) a C₁₋₆ alkyl group,
(2) a triazolyl group optionally substituted by 1 to 3 C₁₋₆ alkyl groups, and
(3) a pyridyl group optionally substituted by 1 to 3 C₁₋₆ alkyl groups;
ring D is a benzene ring substituted by a C₁₋₆ alkoxy group optionally substituted by 1 to 3 halogen atoms;
X is a carbon atom;
L is a bond; and
Y is a group represented by the formula: 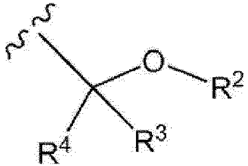
wherein R² is a hydrogen atom;
R³ is a C₁₋₆ alkyl group; and
R⁴ is a C₁₋₆ alkyl group, or a salt thereof,
or a salt thereof.

11. N-((1S)-2-Hydroxy-2-methyl-1-(4-(trifluoromethoxy)phenyl)propyl)-5-(6-methylpyridin-3-yl)pyrazolo[1,5-a]pyrimidine-3-carboxamide, or a salt thereof.

12. N-((1S)-2-Hydroxy-2-methyl-1-(4-(trifluoromethoxy)phenyl)propyl)-6-methyl-5-(4-methyl-1H-1,2,3-triazol-1-yl)pyrazolo[1,5-a]pyrimidine-3-carboxamide, or a salt thereof.

13. N-((1S)-2-Hydroxy-2-methyl-1-(4-(trifluoromethoxy)phenyl)propyl)-6-methyl-5-(3-methyl-1H-1,2,4-triazol-1-yl)pyrazolo[1,5-a]pyrimidine-3-carboxamide, or a salt thereof.

14. A medicament comprising the compound of any one of claims 1 to 13, or a salt thereof.

15. The medicament of claim 14, which is a phosphodiesterase 2A inhibitor.

16. The medicament of claim 14, which is a prophylactic or therapeutic drug for schizophrenia.

17. The compound of any one of claims 1 to 13 for use in the prophylaxis or treatment of schizophrenia, or a salt thereof.

18. A method of inhibiting of phosphodiesterase 2A in a mammal, comprising administering an effective amount of the compound of any one of the claims 1 to 13 or a salt thereof to the mammal.

19. A method for the prophylaxis or treatment of schizophrenia in a mammal, comprising administering an effective amount of the compound of any one of claims 1 to 13 or a salt thereof to the mammal.

20. Use of the compound of any one of claims 1 to 13 or a salt thereof in the production of a prophylactic or therapeutic drug for schizophrenia.
